(19) **Europäisches Patentamt / European Patent Office / Office européen des brevets**

(11) **EP 3 789 525 A1**

(12) **EUROPEAN PATENT APPLICATION**
published in accordance with Art. 153(4) EPC

(43) Date of publication:
**10.03.2021 Bulletin 2021/10**

(21) Application number: **19781749.7**

(22) Date of filing: **03.04.2019**

(51) Int Cl.:
**D02G 3/22** *(2006.01)*    **C07K 14/435** *(2006.01)*
**D01F 4/02** *(2006.01)*

(86) International application number:
**PCT/JP2019/014898**

(87) International publication number:
**WO 2019/194263 (10.10.2019 Gazette 2019/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.04.2018 JP 2018071894**

(71) Applicants:
• **Spiber Inc.**
 **Tsuruoka-shi, Yamagata 997-0052 (JP)**
• **Kojima Industries Corporation**
 **Toyota-shi, Aichi 471-8588 (JP)**

(72) Inventors:
• **IGARASHI, Takumi**
 **Tsuruoka-shi, Yamagata 997-0052 (JP)**
• **IKEDA, Atsushi**
 **Tsuruoka-shi, Yamagata 997-0052 (JP)**
• **ABE, Yunosuke**
 **Tsuruoka-shi, Yamagata 997-0052 (JP)**
• **HARADA, Seiji**
 **Toyota-shi, Aichi 471-8588 (JP)**

(74) Representative: **Handley, Matthew Edward**
 **Venner Shipley LLP**
 **200 Aldersgate**
 **London EC1A 4HD (GB)**

(54) **HIGHLY CONTRACTED SYNTHETIC FIBROIN TWISTED YARN AND PRODUCTION METHOD THEREFOR, AND SYNTHETIC FIBROIN TWISTED YARN AND METHOD FOR CONTRACTING SAME**

(57) An object of the present invention is to provide a high-shrinkage artificial fibroin twisted yarn having a sufficiently high shrinkage rate, and a safe method for producing the same. The high-shrinkage artificial fibroin twisted yarn according to the present invention is a shrunk artificial fibroin twisted yarn comprising a modified fibroin and having a shrinkage rate defined by the following Formula I of 2% or more:

$$\text{Shrinkage rate (\%)} = \{1\text{- (length of a shrunk artificial fibroin twisted yarn / length of the artificial fibroin twisted yarn after being twisted and before being brought into contact with water)}\} \times 100 \cdots$$

(Formula I).

## Description

### Technical Field

The present invention relates to a high-shrinkage artificial fibroin twisted yarn and a method for producing the same, and an artificial fibroin twisted yarn and method for shrinking the artificial fibroin twisted yarn.

### Background Art

Conventionally, twisted yarns formed of synthetic fibers or natural fibers or the like have been used as materials for various knitted or woven fabrics. As such twisted yarns, those that can satisfy the requirements for knitted or woven fabrics are selected in the production of the knitted or woven fabric.

In addition, in a case where more properties are required for the desired knitted or woven fabric, the material twisted yarn may be subjected to a predetermined processing. For example, when more flexibility and heat retention property, in addition to good touch texture, are desired for clothing, bedding, or the like, twisted yarns that had been subjected a shrinking processing to attain increased bulkiness may be used.

For example, artificial synthetic fibers such as a polyester fiber, a polyamide fiber, and an acrylic fiber that are generally used for clothing, bedding or the like have achieved a shrinkage rate of 40% or more by being brought into contact with boiling water (Patent Literature 1).

### Citation List

### Patent Literature

[Patent Literature 1] Japanese Unexamined Patent Publication No. 2009-121003

### Summary of Invention

### Technical Problem

However, in regard to shrinking processing of a twisted yarn containing an artificial protein, sufficient studies have not been performed. In addition, the shrinkage method disclosed in Patent Literature 1 handles boiling water of high temperature, and thus had a great danger.

The present invention aims to provide a high-shrinkage artificial fibroin twisted yarn having a sufficiently high shrinkage rate, and a safe method for producing the same. In addition, the present invention aims to provide an artificial fibroin twisted yarn with which the high-shrinkage artificial fibroin twisted yarn can be produced and a safe shrinkage method therefor.

### Solution to Problem

The present invention relates to, for example, each of the following inventions.

[1] A high-shrinkage artificial fibroin twisted yarn, which is a shrunk artificial fibroin twisted yarn comprising a modified fibroin and having a shrinkage rate defined by the following Formula I of 2% or more:

$$\text{Shrinkage rate (\%)} = \{1\text{-} \text{(length of a shrunk artificial fibroin twisted yarn / length of the artificial fibroin twisted yarn after being twisted and before being brought into contact with water)}\} \times 100 \cdots \text{(Formula I)}.$$

[2] The high-shrinkage artificial fibroin twisted yarn according to [1], wherein the modified fibroin is a modified spider silk fibroin.

[3] The high-shrinkage artificial fibroin twisted yarn according to [1] or [2], wherein the high-shrinkage artificial fibroin twisted yarn is an artificial fibroin twisted yarn shrunk by being brought into contact with water having a temperature lower than a boiling point.

[4] The high-shrinkage artificial fibroin twisted yarn according to [3], wherein the temperature of the water is 10°C to 90°C.

[5] The high-shrinkage artificial fibroin twisted yarn according to [3] or [4], wherein the high-shrinkage artificial fibroin twisted yarn is an artificial fibroin twisted yarn further shrunk by drying after being brought into contact with the water.

[6] A method for producing a high-shrinkage artificial fibroin twisted yarn, comprising a step of shrinking an artificial fibroin twisted yarn comprising a modified fibroin by bringing the artificial fibroin twisted yarn into contact with water having a temperature lower than a boiling point,

wherein a shrinkage rate defined by the following Formula I is 2% or more,

$$\text{Shrinkage rate (\%)} = \{1\text{-} \text{(length of a shrunk artificial fibroin twisted yarn / length of the artificial fibroin twisted yarn after being twisted and before being brought into contact with water)}\} \times 100 \cdots \text{(Formula I)}.$$

[7] The method for producing a high-shrinkage artificial fibroin twisted yarn according to [6], wherein the modified fibroin is a modified spider silk fibroin.

[8] The method for producing a high-shrinkage artificial fibroin twisted yarn according to [6] or [7], wherein the temperature of the water is 10°C to 90°C.

[9] The method for producing a high-shrinkage artificial fibroin twisted yarn according to any one of [6] to [8], wherein the shrinking step further comprises drying the artificial fibroin twisted yarn after being brought into contact with the water.

[10] A method for shrinking an artificial fibroin twisted yarn, comprising a step of shrinking an artificial fibroin twisted yarn comprising a modified fibroin by bringing the artificial fibroin twisted yarn into contact with water having a temperature lower than a boiling point,

wherein a shrinkage rate defined by the following Formula I is 2% or more,

$$\text{Shrinkage rate (\%)} = \{1\text{-} \text{(length of a shrunk artificial fibroin twisted yarn / length of the artificial fibroin twisted yarn after being twisted and before being brought into contact with water)}\} \times 100 \cdots \text{(Formula I)}.$$

[11] The method for shrinking an artificial fibroin twisted yarn according to [10], wherein the modified fibroin is a modified spider silk fibroin.

[12] The method for shrinking an artificial fibroin twisted yarn according to [10] or [11], wherein the temperature of the water is 10°C to 90°C.

[13] The method for shrinking an artificial fibroin twisted yarn according to any one of [10] to [12], wherein the shrinking step further comprises drying the artificial fibroin twisted yarn after being brought into contact with the water.

[14] An artificial fibroin twisted yarn comprising a modified fibroin and having a shrinkage rate defined by the following Formula II of 2% or more:

$$\text{Shrinkage (\%)} = \{1- \text{(length of artificial fibroin twisted yarn}$$

$$\text{subjected to shrinking processing including being brought into contact}$$

$$\text{with water having a temperature lower than a boiling point / length of}$$

$$\text{the artificial fibroin twisted yarn before being subjected to the shrinking}$$

$$\text{processing)}\} \times 100 \cdots \text{(Formula II)}.$$

[15] The high-shrinkage artificial fibroin twisted yarn according to any one of [1] to [5], the method for producing a high-shrinkage artificial fibroin twisted yarn according to any one of [6] to [9], the method for shrinking an artificial fibroin twisted yarn according to any one of [10] to [13], or the artificial fibroin twisted yarn according to [14], wherein the modified fibroin comprises a domain sequence represented by

$$\text{Formula 1: } [(A)_n \text{ motif-REP}]_m,$$

and
the domain sequence has an amino acid sequence with a reduced content of $(A)_n$ motifs, which corresponds to an amino acid sequence in which at least one or a plurality of $(A)_n$ motifs are deleted, as compared with a naturally occurring fibroin,
wherein in Formula 1, the $(A)_n$ motif represents an amino acid sequence composed of 2 to 27 amino acid residues and the number of alanine residues with respect to a total number of amino acid residues in the $(A)_n$ motif is 83% or more;
REP represents an amino acid sequence composed of 10 to 200 amino acid residues;
m represents an integer of 2 to 300;
a plurality of the $(A)_n$ motifs may have the same amino acid sequence or amino acid sequences different from each other; and
a plurality of REPs may have the same amino acid sequence or amino acid sequences different from each other.
[16] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to [15], wherein the domain sequence has an amino acid sequence that corresponds to an amino acid sequence in which at least one $(A)_n$ motif for every one to three $(A)_n$ motifs from the N-terminal side to the C-terminal side is deleted, as compared with a naturally occurring fibroin.
[17] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to [15], wherein the domain sequence has an amino acid sequence that corresponds to an amino acid sequence in which at least deletion of two consecutive $(A)_n$ motifs and deletion of one $(A)_n$ motif are repeated in this order from the N-terminal side to the C-terminal side, as compared with a naturally occurring fibroin.
[18] The high-shrinkage artificial fibroin twisted yarn according to any one of [1] to [5], the method for producing a high-shrinkage artificial fibroin twisted yarn according to any one of [6] to [9], the method for shrinking an artificial fibroin twisted yarn according to any one of [10] to [13], or the artificial fibroin twisted yarn according to [14], wherein the modified fibroin comprises a domain sequence represented by

$$\text{Formula 1: } [(A)_n \text{ motif-REP}]_m,$$

and
when the numbers of amino acid residues of REP of two adjacent $[(A)_n \text{ motif-REP}]$ units are sequentially compared from the N-terminal side to the C-terminal side, x/y is 50% or more, where x is a maximum value of a total value obtained by adding the numbers of amino acid residues of two adjacent $[(A)_n \text{ motif-REP}]$ units wherein when the number of amino acid residues of one REP having less amino acid residues is taken as 1, the ratio of the number of amino acids residues of the other REP satisfies 1.8 to 11.3; and where y is a total number of amino acid residues of the domain sequence,

wherein in Formula 1, the $(A)_n$ motif represents an amino acid sequence composed of 2 to 27 amino acid residues and the number of alanine residues with respect to a total number of amino acid residues in the $(A)_n$ motif is 83% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

[19] The high-shrinkage artificial fibroin twisted yarn according to any one of [1] to [5], the method for producing a high-shrinkage artificial fibroin twisted yarn according to any one of [6] to [9], the method for shrinking an artificial fibroin twisted yarn according to any one of [10] to [13], or the artificial fibroin twisted yarn according to [14], wherein the modified fibroin comprises a domain sequence represented by

$$\text{Formula 1: } [(A)_n \text{ motif-REP}]_m,$$

and

the domain sequence has an amino acid sequence with a reduced content of glycine residues, which corresponds to an amino acid sequence in which at least one or a plurality of glycine residues in REP are substituted with other amino acid residues, as compared with a naturally occurring fibroin,

wherein in Formula 1, the $(A)_n$ motif represents an amino acid sequence composed of 2 to 27 amino acid residues and the number of alanine residues with respect to a total number of amino acid residues in the $(A)_n$ motif is 83% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

[20] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to [19], wherein the domain sequence has an amino acid sequence that corresponds to an amino acid sequence in which, in at least one motif sequence selected from GGX and GPGXX (where X represents an amino acid residue other than glycine) in REP, at least one glycine residue in one or a plurality of the motif sequences is substituted with another amino acid residue, as compared with a naturally occurring fibroin.

[21] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to [20], wherein a proportion of the motif sequence in which a glycine residue is substituted with another amino acid residue is 10% or more with respect to all motif sequences.

[22] The high-shrinkage artificial fibroin twisted yarn according to any one of [1] to [5], the method for producing a high-shrinkage artificial fibroin twisted yarn according to any one of [6] to [9], the method for shrinking an artificial fibroin twisted yarn according to any one of [10] to [13], or the artificial fibroin twisted yarn according to [14], wherein the modified fibroin comprises a domain sequence represented by Formula 1: $[(A)_n \text{ motif-REP}]_m$, and

z/w is 50.9% or more, where z is the total number of amino acid residues of an amino acid sequence consisting of XGX (where X represents an amino acid residue other than glycine) contained in all REPs in the domain sequence excluding the sequence between the $(A)_n$ motif positioned closest to the C-terminal side and the C-terminal of the domain sequence; and where w is the total number of amino acid residues in the domain sequence excluding the sequence between the $(A)_n$ motif positioned closest to the C-terminal side and the C-terminal of the domain sequence,

wherein in Formula 1, the $(A)_n$ motif represents an amino acid sequence composed of 2 to 27 amino acid residues and the number of alanine residues with respect to a total number of amino acid residues in the $(A)_n$ motif is 83% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

[23] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to any one of [19] to [20], wherein the modified fibroin has an amino acid sequence that corresponds to an amino acid sequence in which one or a plurality of amino acid residues are further substituted, deleted, inserted, and/or added, in addition to substitution of one or a plurality of glycine residues in REP with other amino acid residues, as compared with a naturally occurring fibroin.

[24] The high-shrinkage artificial fibroin twisted yarn according to any one of [1] to [5], the method for producing a high-shrinkage artificial fibroin twisted yarn according to any one of [6] to [9], the method for shrinking an artificial fibroin twisted yarn according to any one of [10] to [13], or the artificial fibroin twisted yarn according to [14], wherein the modified fibroin comprises a domain sequence represented by

$$\text{Formula 1: } [(A)_n \text{ motif-REP}]_m,$$

and

the domain sequence has an amino acid sequence comprising a region having a locally high hydropathy index, which corresponds to an amino acid sequence in which one or a plurality of amino acid residues in REP are substituted with an amino acid residue having a high hydropathy index and/or one or a plurality of amino acid residues having a high hydropathy index is inserted into REP, as compared with a naturally occurring fibroin,

wherein in Formula 1, the $(A)_n$ motif represents an amino acid sequence composed of 2 to 27 amino acid residues and the number of alanine residues with respect to a total number of amino acid residues in the $(A)_n$ motif is 83% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

[25] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to [24], wherein the region having a locally high hydropathy index is composed of 2 to 4 consecutive amino acid residues.

[26] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to [24] or [25], wherein the amino acid residues having a high hydropathy index are selected from isoleucine (I), valine (V), leucine (L), phenylalanine (F), cysteine (C), methionine (M), and alanine (A).

[27] The high-shrinkage artificial fibroin twisted yarn according to any one of [1] to [5], the method for producing a high-shrinkage artificial fibroin twisted yarn according to any one of [6] to [9], the method for shrinking an artificial fibroin twisted yarn according to any one of [10] to [13], or the artificial fibroin twisted yarn according to [14], wherein the modified fibroin comprises a domain sequence represented by

$$\text{Formula 1: } [(A)_n \text{ motif-REP}]_m,$$

and

p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence,

wherein in Formula 1, the $(A)_n$ motif represents an amino acid sequence composed of 2 to 27 amino acid residues and the number of alanine residues with respect to a total number of amino acid residues in the $(A)_n$ motif is 83% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

[28] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to any one of [24] to [25], wherein the modified fibroin has an amino acid sequence that corresponds to an amino acid sequence in which one or a plurality of amino acid residues are substituted, deleted, inserted, and/or added, in addition to substitution of one or a plurality of amino acid residues in REP with amino acid residues having a high hydropathy index and/or insertion of one or a plurality of amino acid residues having a high hydropathy index into REP, as compared with a naturally occurring fibroin.

[29] The high-shrinkage artificial fibroin twisted yarn according to any one of [1] to [5], the method for producing a high-shrinkage artificial fibroin twisted yarn according to any one of [6] to [9], the method for shrinking an artificial fibroin twisted yarn according to any one of [10] to [13], or the artificial fibroin twisted yarn according to [14], wherein the modified fibroin comprises a domain sequence represented by Formula 1: $[(A)_n$ motif-REP$]_m$ or Formula 2:$[(A)_n$ motif-REP$]_m$-$(A)_n$ motif, and

the domain sequence has an amino acid sequence with a reduced content of glutamine residues, which corresponds to an amino acid sequence in which one or a plurality of glutamine residues in REP are deleted or substituted with other amino acid residues, as compared with a naturally occurring fibroin,

wherein in Formula 1 and Formula 2, the $(A)_n$ motif represents an amino acid sequence composed of 2 to 27 amino acid residues and the number of alanine residues with respect to a total number of amino acid residues in the $(A)_n$ motif is 80% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

[30] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to [29], wherein the modified fibroin contains a GPGXX (where X represents an amino acid residue other than a glycine residue) motif in REP, and a GPGXX motif content rate is 10% or more.

[31] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to [29] or [30], wherein a glutamine residue content rate of the modified fibroin is 9% or less.

[32] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to any one of [29] to [31], wherein the other amino acid residues are amino acid residues selected from the group consisting of isoleucine (I), valine (V), leucine (L), phenylalanine (F), cysteine (C), methionine (M), alanine (A), glycine (G), threonine (T), serine (S), tryptophan (W), tyrosine (Y), proline (P), and histidine (H).

[33] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to any one of [29] to [32], wherein the modified fibroin has a degree of hydrophobicity of REP of -0.8 or more.

[34] The high-shrinkage artificial fibroin twisted yarn, the method for producing a high-shrinkage artificial fibroin twisted yarn, the method for shrinking an artificial fibroin twisted yarn, or the artificial fibroin twisted yarn, according to any one of [29] to [33], wherein the modified fibroin has an amino acid sequence that corresponds to an amino acid sequence in which one or a plurality of amino acid residues are substituted, deleted, inserted, and/or added, in addition to deletion of one or a plurality of glutamine residues in REP or substitution with other amino acid residues, as compared with a naturally occurring fibroin.

[35] The high-shrinkage artificial fibroin twisted yarn according to any one of [1] to [5], the method for producing a high-shrinkage artificial fibroin twisted yarn according to any one of [6] to [9], the method for shrinking an artificial fibroin twisted yarn according to any one of [10] to [13], or the artificial fibroin twisted yarn according to [14], wherein the modified fibroin has a limiting oxygen index (LOI) value of 26.0 or more.

[36] The high-shrinkage artificial fibroin twisted yarn according to any one of [1] to [5], the method for producing a high-shrinkage artificial fibroin twisted yarn according to any one of [6] to [9], the method for shrinking an artificial fibroin twisted yarn according to any one of [10] to [13], or the artificial fibroin twisted yarn according to [14], wherein a maximum hygroscopic heat generation, as determined according to the following formula A, of the modified fibroin is more than 0.025°C/g:

Formula A: Maximum hygroscopic heat generation = {(Highest temperature of a sample when the sample has been transferred to a high humidity environment after being placed in a low humidity environment until a temperature of the sample reaches equilibrium) - (Temperature of the sample when the sample is being transferred to the high humidity environment after being placed in the low humidity environment until the temperature of the sample reaches equilibrium)} (°C) / Sample weight (g),

wherein in Formula A, the low humidity environment means an environment of a temperature of 20°C and a relative humidity of 40%, and the high humidity environment means an environment of a temperature of 20°C and a relative humidity of 90%.

**Advantageous Effects of Invention**

According to the present invention, a high-shrinkage artificial fibroin twisted yarn having a sufficiently high shrinkage rate and a safe method for producing the same are provided. Such a high-shrinkage artificial fibroin twisted yarn has an excellent touch texture and flexibility. In addition, according to the present invention, an artificial fibroin twisted yarn with which the high-shrinkage artificial fibroin twisted yarn can be produced and a safe shrinkage method therefor are provided.

**Brief Description of Drawings**

Fig. 1 is a schematic diagram illustrating a domain sequence of a modified fibroin according to one embodiment.
Fig. 2 is a diagram illustrating a distribution of z/w (%) values of a naturally occurring fibroin.
Fig. 3 is a diagram illustrating a distribution of x/y (%) values of a naturally occurring fibroin.
Fig. 4 is a schematic diagram illustrating a domain sequence of a modified fibroin according to one embodiment.
Fig. 5 is a schematic diagram illustrating a domain sequence of a modified fibroin according to one embodiment.
Fig. 6 is a schematic view illustrating an example of a spinning device for manufacturing a modified fibroin fiber.
Fig. 7 is a schematic view illustrating an example of a false twisting machine for producing an artificial fibroin false twisted yarn.
Fig. 8 is a graph illustrating an example of the results of a hygroscopic heat generation test.

**Description of Embodiments**

Hereinafter, embodiments for carrying out the present invention will be described in detail. However, the present invention is not limited to the following embodiments.

[High-shrinkage artificial fibroin twisted yarn]

A high-shrinkage artificial fibroin twisted yarn according to the present invention is a shrunk artificial fibroin twisted yarn containing a modified fibroin. The high-shrinkage artificial fibroin twisted yarn according to one embodiment has a shrinkage rate defined by the following Formula I of 2% or more.

$$\text{Shrinkage rate (\%)} = \{1- (\text{length of a shrunk artificial fibroin}$$

$$\text{twisted yarn} \,/\, \text{length of the artificial fibroin twisted yarn after being}$$

$$\text{twisted and before being brought into contact with water})\} \times 100 \cdots$$

$$\text{(Formula I)}.$$

<Modified Fibroin>

The modified fibroin according to the present embodiment is a protein containing a domain sequence represented by Formula 1: $[(A)_n\text{motif-REP}]_m$ or Formula 2 $[(A)_n\text{motif-REP}]_m\text{-}(A)_n$ motif. In the modified fibroin, an amino acid sequence (an N-terminal sequence and a C-terminal sequence) may be further added to either or both of the N-terminal side and the C-terminal side of the domain sequence. The N-terminal sequence and the C-terminal sequence are typically, but not limited to, regions that do not have a repetition of amino acid motifs characteristic of a fibroin, and that consist of amino acids of about 100 residues.

The "modified fibroin" in the present specification means an artificially produced fibroin (an artificial fibroin). The modified fibroin may be a fibroin in which the domain sequence is different from an amino acid sequence of a naturally occurring fibroin or the same as the amino acid sequence of a naturally occurring fibroin. The "naturally occurring fibroin" referred to in the present specification also is a protein containing a domain sequence represented by Formula 1: $[(A)_n\text{motif-REP}]_m$ or Formula 2 $[(A)_n\text{motif-REP}]_m\text{-}(A)_n$ motif.

The "modified fibroin" may be a modified fibroin having an amino acid sequence of a naturally occurring fibroin as it is, may be a modified fibroin whose amino acid sequence has been modified based on the amino acid sequence of a naturally occurring fibroin (for example, a modified fibroin whose amino acid sequence has been modified by altering a cloned gene sequence of a naturally occurring fibroin), or may be a modified fibroin that is artificially designed and synthesized independently of a naturally occurring fibroin (for example, a modified fibroin having a desired amino acid sequence by chemically synthesizing a nucleic acid encoding a designed amino acid sequence).

The "domain sequence" in the present specification is an amino acid sequence that produces a crystalline region (typically corresponding to the $(A)_n$ motif of an amino acid sequence) and an amorphous region (typically corresponding to REP of an amino acid sequence) unique to a fibroin, and refers to an amino acid sequence represented by Formula 1: $[(A)_n\text{motif-REP}]_m$ or Formula 2 $[(A)_n\text{motif-REP}]_m\text{-}(A)_n$ motif. Here the $(A)_n$ motif represents an amino acid sequence mainly containing alanine residues, and the number of amino acid residues in the $(A)_n$ motif is 2 to 27. The number of amino acid residues in the $(A)_n$ motif may be an integer of 2 to 20, 4 to 27, 4 to 20, 8 to 20, 10 to 20, 4 to 16, 8 to 16, or 10 to 16. Further, the proportion of the number of alanine residues with respect to the total number of amino acid residues in the $(A)_n$ motif may be 40% or more, and may be 60% or more, 70% or more, 80% or more, 83% or more, 85% or more, 86% or more, 90% or more, 95% or more, or 100% (meaning that the $(A)_n$ motif is composed of only alanine residues). Among a plurality of $(A)_n$ motifs present in the domain sequence, at least seven of the $(A)_n$ motifs may be composed only of alanine residues. REP represents an amino acid sequence composed of 2 to 200 amino acid residues. REP may be an amino acid sequence composed of 10 to 200 amino acid residues, and may be an amino acid sequence composed of 10 to 40, 10 to 60, 10 to 80, 10 to 100, 10 to 120, 10 to 140, 10 to 160, or 10 to 180 amino acid residues. m represents an integer of 2 to 300, and may be an integer of 8 to 300, 10 to 300, 20 to 300, 40 to 300, 60 to 300, 80 to 300, 100 to 300, 10 to 200, 20 to 200, 20 to 180, 20 to 160, 20 to 140, or 20 to 120. A plurality of $(A)_n$ motifs may have the same amino acid sequence or amino acid sequences different from each other. A plurality of REPs may have the same amino acid sequence or amino acid sequences different from each other.

The modified fibroin according to the present embodiment may be obtained, for example, by subjecting a cloned gene sequence of a naturally occurring fibroin to a modification of an amino acid sequence corresponding to, for example, substitution, deletion, insertion, and/or addition of one or a plurality of amino acid residues. The substitution, deletion, insertion, and/or addition of an amino acid residue may be performed by methods well known to those skilled in the art, such as site-directed mutagenesis. Specifically, the modification may be performed in accordance with the method described in literature such as Nucleic Acid Res. 10, 6487 (1982) and Methods in Enzymology, 100, 448 (1983).

A naturally occurring fibroin is a protein containing a domain sequence represented by Formula 1: $[(A)_n\text{motif-REP}]_m$ or Formula 2 $[(A)_n\text{motif-REP}]_m\text{-}(A)_n$ motif, and specific examples thereof include a fibroin produced by insects or spiders.

Examples of the fibroin produced by insects include silk proteins produced by silkworms such as Bombyx mori, Bombyx mandarina, Antheraea yamamai, Anteraea pernyi, Eriogyna pyretorum, Philosamia cynthia ricini, Samia cynthia, Caligula japonica, Antheraea mylitta, and Antheraea assama; and hornet silk proteins discharged by larvae of Vespa simillima xanthoptera.

More specific examples of the fibroin produced by insects include a silkworm fibroin L chain (GenBank Accession No. M76430 (nucleotide sequence), and AAA27840.1 (amino acid sequence)).

Examples of the fibroin produced by spiders include spider silk proteins produced by spiders belonging to the genus Araneus such as Araneus ventricosus, Araneus diadematus, Araneus pinguis, Araneus pentagrammicus and Araneus nojimai, spiders belonging to the genus Neoscona such as Neoscona scylla, Neoscona nautica, Neoscona adianta and Neoscona scylloides, spiders belonging to the genus Pronus such as Pronous minutes, spiders belonging to the genus Cyrtarachne such as Cyrtarachne bufo and Cyrtarachne inaequalis, spiders belonging to the genus Gasteracantha such as Gasteracantha kuhli and Gasteracantha mammosa, spiders belonging to the genus Ordgarius such as Ordgarius hobsoni and Ordgarius sexspinosus, spiders belonging to the genus Argiope such as Argiope amoena, Argiope minuta and Argiope bruennich, spiders belonging to the genus Arachnura such as Arachnura logio, spiders belonging to the genus Acusilas such as Acusilas coccineus, spiders belonging to the genus Cyrtophora such as Cyrtophora moluccensis, Cyrtophora exanthematica and Cyrtophora unicolor, spiders belonging to the genus Poltys such as Poltys illepidus, spiders belonging to the genus Cyclosa such as Cyclosa octotuberculata, Cyclosa sedeculata, Cyclosa vallata and Cyclosa atrata, and spiders belonging to the genus Chorizopes such as Chorizopes nipponicus; and spider silk proteins produced by spiders belonging to the genus Tetragnatha such as Tetragnatha praedonia, Tetragnatha maxillosa, Tetragnatha extensa and Tetragnatha squamata, spiders belonging to the genus Leucauge such as Leucauge magnifica, Leucauge blanda and Leucauge subblanda, spiders belonging to the genus Nephila such as Nephila clavata and Nephila pilipes, spiders belonging to the genus Menosira such as Menosira ornata, spiders belonging to the genus Dyschiriognatha such as Dyschiriognatha tenera, spiders belonging to the genus Latrodectus such as Latrodectus mactans, Latrodectus hasseltii, Latrodectus geometricus and Latrodectus tredecimguttatus, and spiders belonging to the family Tetragnathidae such as spiders belonging to the genus Euprosthenops. Examples of the spider silk proteins include traction yarn proteins such as MaSp (MaSp1 and MaSp2) and ADF (ADF3 and ADF4), and MiSp (MiSp1 and MiSp2).

More specific examples of the spider silk protein produced by spiders include fibroin-3 (adf-3) [derived from Araneus diadematus] (GenBank Accession No. AAC47010 (amino acid sequence), U47855 (nucleotide sequence)), fibroin-4 (adf-4) [derived from Araneus diadematus] (GenBank Accession No. AAC47011 (amino acid sequence), U47856 (nucleotide sequence)), dragline silk protein spidroin 1 [derived from Nephila clavipes] (GenBank Accession No. AAC04504 (amino acid sequence), U37520 (nucleotide sequence)), major ampullate spidroin 1 [derived from Latrodectus hesperus] (GenBank Accession No. ABR68856 (amino acid sequence), EF595246 (nucleotide sequence)), dragline silk protein spidroin 2 [derived from Nephila clavata] (GenBank Accession No. AAL32472 (amino acid sequence), AF441245 (nucleotide sequence)), major ampullate spidroin 1 [derived from Euprosthenops australis] (GenBank Accession No. CAJ00428 (amino acid sequence), AJ973155 (nucleotide sequence)) and major ampullate spidroin 2 [Euprosthenops australis] (GenBank Accession No. CAM32249.1 (amino acid sequence), AM490169 (nucleotide sequence)), minor ampullate silk protein 1 [Nephila clavipes] (GenBank Accession No. AAC14589.1 (amino acid sequence), minor ampullate silk protein 2 [Nephila clavipes] (GenBank Accession No. AAC14591.1 (amino acid sequence)), and minor ampullate spidroin-like protein [Nephilengys cruentata] (GenBank Accession No. ABR37278.1 (amino acid sequence)).

More specific examples of the naturally occurring fibroin further include a fibroin whose sequence information is registered in NCBI GenBank. For example, sequences thereof may be confirmed by extracting sequences that has a keyword of spidroin, ampullate, fibroin, "silk and polypeptide", or "silk and protein" in DEFINITION, among sequences containing INV as DIVISION in sequence information registered in NCBI GenBank; sequences that have a specific character string of a product from CDS; or sequences that have a specific character string described for TISSUE TYPE from SOURCE.

The modified fibroin according to the present embodiment may be a modified silk fibroin (a modified fibroin obtained by modifying an amino acid sequence of a silk protein produced by silkworm), or may be a modified spider silk fibroin (a modified fibroin obtained by modifying an amino acid sequence of a spider silk protein produced by spiders). As the modified fibroin, a modified spider silk fibroin is preferable. The modified spider silk fibroin is also excellent in heat retention, hygroscopic exothermicity, and/or flame retardancy.

Specific examples of the modified fibroin include a modified fibroin derived from a spigot dragline silk protein produced in the major ampullate gland of a spider (first modified fibroin), a modified fibroin having a domain sequence with a reduced content of glycine residues (second modified fibroin), a modified fibroin having a domain sequence with a reduced content of $(A)_n$ motifs (third modified fibroin), a modified fibroin with a reduced content of glycine residues and $(A)_n$ motifs (fourth modified fibroin), a modified fibroin having a domain sequence containing a region having a locally high hydropathy index (fifth modified fibroin), and a modified fibroin having a domain sequence with a reduced content of glutamine residues (sixth modified fibroin).

Examples of the first modified fibroin include a protein containing a domain sequence represented by

containing a domain sequence represented by Formula 1: [(A)ₙ motif-REP]ₘ. In the first modified fibroin, the number of amino acid

In the first modified fibroin, the number of amino acid residues of the $(A)_n$ motif is preferably an integer of 3 to 20, more preferably an integer of 4 to 20, still more preferably an integer of 8 to 20, even still more preferably an integer of 10 to 20, still further preferably an integer of 4 to 16, particularly preferably an integer of 8 to 16, and most preferably an integer of 10 to 16. In the first modified fibroin, the number of amino acid residues constituting REP in Formula 1 is preferably 10 to 200 residues, more preferably 10 to 150 residues, still more preferably 20 to 100 residues, and even still more preferably 20 to 75 residues. In the first modified fibroin, the total number of the glycine residues, the serine residues, and the alanine residues contained in the amino acid sequence represented by Formula 1:$[(A)_n$motif-REP$]_m$, is preferably 40% or more, more preferably 60% or more, is preferably 40% or more, more preferably 60% or more, and still more preferably 70% or more with respect to the total number of amino acid residues.

The first modified fibroin may be a polypeptide that contains a unit of an amino acid sequence represented by Formula 1:$[(A)_n$motif-REP$]_m$, and has the amino acid sequence set forth in any of SEQ ID NOs: 1 to 3 or an amino acid sequence having 90% or more homology with the amino acid sequence set forth in any of SEQ ID NOs: 1 to 3 as the C-terminal sequence.

The amino acid sequence set forth in SEQ ID NO: 1 is the same as the amino acid sequence consisting of 50 amino acid residues at the C-terminal of the amino acid sequence of ADF3 (GI: 1263287, NCBI), the amino acid sequence set forth in SEQ ID NO: 2 is the same as the amino acid sequence obtained by removing 20 residues from the C-terminal of the amino acid sequence set forth in SEQ ID NO: 1, and the amino acid sequence set forth in SEQ ID NO: 3 is the same as the amino acid sequence obtained by removing 29 residues from the C-terminal of the amino acid sequence set forth in SEQ ID NO: 1.

More specific examples of the first modified fibroin include a modified fibroin containing (1-i) the amino acid sequence set forth in SEQ ID NO: 4 (recombinant spider silk protein ADF3KaiLargeNRSH1), or (1-ii) an amino acid sequence having 90% or more sequence identity with the amino acid sequence set forth in SEQ ID NO: 4. The sequence identity is preferably 95% or more.

The amino acid sequence shown in SEQ ID NO 4 is obtained by performing mutation on the amino acid sequence of ADF3 in which an amino acid sequence (SEQ ID NO 5) consisting of a start codon, His10 tag, and HRV3C protease (Human rhinovirus 3C protease) recognition site is added to the N-terminal, such that the 1st to 13th repeat regions are approximately doubled, and the translation terminates at the 1154th amino acid residue. The amino acid sequence of the C-terminal of the amino acid sequence shown in SEQ ID NO 4 is the same as the amino acid sequence shown in SEQ ID NO 3.

The modified fibroin (1-i) may be a modified fibroin consisting of the amino acid sequence set forth in SEQ ID NO: 4.

The second modified fibroin has a domain sequence that has an amino acid sequence with a reduced content of glycine residues, as compared with a naturally occurring fibroin. It can be said that the second modified fibroin is a modified fibroin having an amino acid sequence that corresponds to an amino acid sequence in which, at least one or a plurality of glycine residues in REP are substituted with other amino acid residues, as compared with a naturally occurring fibroin.

The second modified fibroin may have a domain sequence that has an amino acid sequence corresponding to an amino acid sequence in which, in at least one motif sequence selected from GGX and GPGXX (where G represents a glycine residue, P represents a proline residue, and X represents an amino acid residue other than glycine) in REP, at least one glycine residue in one or a plurality of motif sequences is substituted with another amino acid residue, as compared with a naturally occurring fibroin.

In the second modified fibroin, the proportion of the above-described motif sequences in which a glycine residue is substituted with another amino acid residue may be 10% or more with respect to the all motif sequences.

The second modified fibroin may be a modified fibroin that contains a domain sequence represented by

contains a domain sequence represented by Formula 1: [(A)ₙ motif-REP]ₘ, that has an amino acid sequence in which z/w is 30% or

that has an amino acid sequence in which z/w is 30% or more, 40% or more, 50% or more, or 50.9% or more, where z is the total number of amino acid residues of an amino acid sequence consisting of XGX (where X represents an amino acid residue other than glycine) contained in all REPs in the domain sequence excluding the sequence between the

$(A)_n$ motif positioned closest to the C-terminal side and the C-terminal of the domain sequence; and where w is the total number of amino acid residues in the domain sequence excluding the sequence between the $(A)_n$ motif positioned closest to the C-terminal side and the C-terminal of the domain sequence. The number of alanine residues with respect to a total number of amino acid residues in the $(A)_n$ motif may be 83% or more, but is preferably 86% or more, more preferably 90% or more, still more preferably 95% or more, and even still more preferably 100% (which means that the $(A)_n$ motif consists only of alanine residues).

The second modified fibroin is preferably a modified fibroin in which one glycine residue of the GGX motif is substituted with another amino acid residue to increase the content ratio of the amino acid sequence consisting of XGX. In the second modified fibroin, the content ratio of the amino acid sequence consisting of GGX in the domain sequence is preferably 30% or less, more preferably 20% or less, still more preferably 10% or less, even still more preferably 6 % or less, further still more preferably 4% or less, and particularly preferably 2% or less. The content ratio of the amino acid sequence consisting of GGX in the domain sequence can be calculated by the same method as the following method for calculating a content ratio (z/w) of the amino acid sequence consisting of XGX.

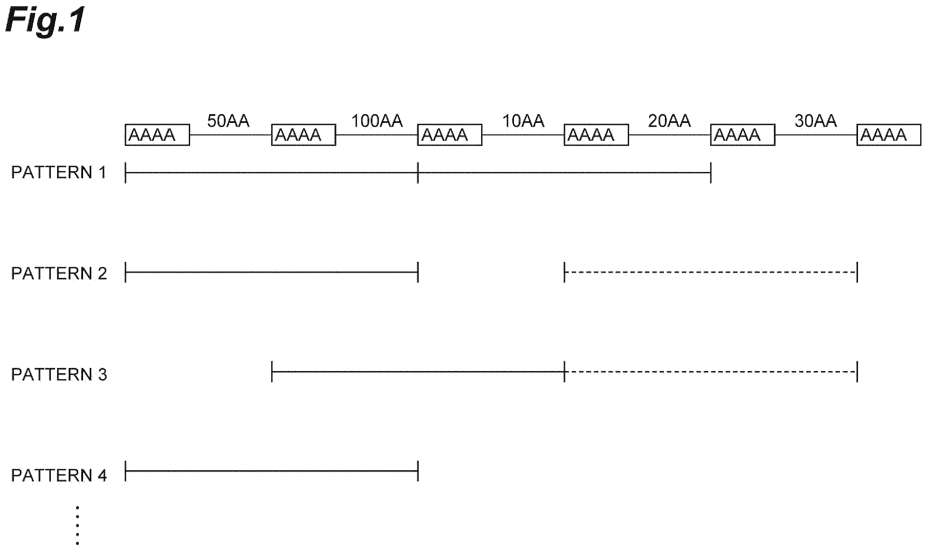

The method for calculating z/w will be described in more detail. First, in a fibroin (a modified fibroin or a naturally occurring fibroin) containing a domain sequence represented by

fibroin) containing a domain sequence represented by Formula 1: $[(A)_n$ motif-REP$]_m$, an amino acid sequence consisting of XGX is extracted

an amino acid sequence consisting of XGX is extracted from all REPs contained in the domain sequence excluding the sequence between the $(A)_n$ motif positioned closest to the C-terminal side and the C-terminal of the domain sequence. The total number of amino acid residues constituting XGX is z. For example, in a case where 50 amino acid sequences consisting of XGX (without an overlap) are extracted, z is $50 \times 3 = 150$. Further, for example, in a case where there is X contained in two XGXs (X at the center), as in the case of an amino acid sequence consisting of XGXGX, the calculation is performed by deducting the overlap (in the case of XGXGX, 5 amino acid residues). w is the total number of amino acid residues contained in the domain sequence excluding the sequence between the $(A)_n$ motif positioned closest to the C-terminal side and the C-terminal of the domain sequence. For example, in the case of the domain sequence illustrated in Fig. 1, w is $4 + 50 + 4 + 100 + 4 + 10 + 4 + 20 + 4 + 30 = 230$ (the $(A)_n$ motif positioned closest to the C-terminal side is excluded.). Next, z/w (%) can be calculated by dividing z by w.

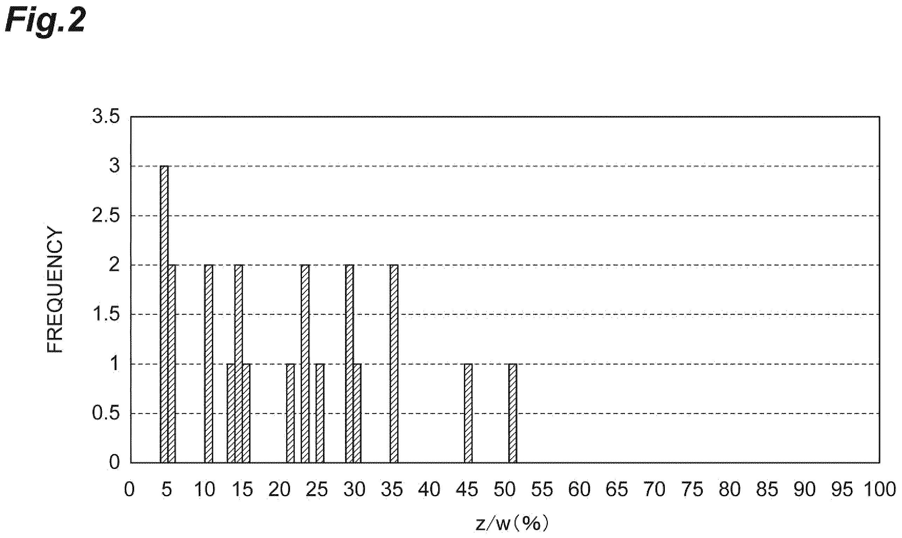

Here, z/w in a naturally occurring fibroin will be described. First, fibroins with amino acid sequence information registered in NCBI GenBank was checked by the exemplified method as described above, and 663 types of fibroins (of which 415 types were fibroins derived from spiders) were extracted. Among all the extracted fibroins, z/w was calculated from an amino acid sequence of a naturally occurring fibroin that contains a domain sequence represented by

fibroin that contains a domain sequence represented by Formula 1: $[(A)_n$ motif-REP$]_m$, and that has a content ratio of the amino acid sequence

and that has a content ratio of the amino acid sequence consisting of GGX in the fibroin of 6% or less, using the above-described calculation method. The results are shown in Fig. 2. In Fig. 2, the horizontal axis represents z/w (%), and the vertical axis represents frequency. As apparent from Fig. 2, z/w in all the naturally occurring fibroin is less than 50.9% (highest value: 50.86%).

In the second modified fibroin, z/w is preferably 50.9% or more, more preferably 56.1% or more, still more preferably 58.7% or more, even still more preferably 70% or more, and still further preferably 80% or more. The upper limit of z/w is not particularly limited, but may be, for example, 95% or less.

The second modified fibroin may be obtained, for example, by modifying a cloned gene sequence of a naturally occurring fibroin such that at least a part of the nucleotide sequence encoding a glycine residue is substituted to encode another amino acid residue. In this case, one glycine residue in the GGX motif and the GPGXX motif may be selected as the glycine residue to be modified, and also, substitution may be performed so that z/w becomes 50.9% or more. In addition, the second modified fibroin may also be obtained, for example, by designing an amino acid sequence that satisfies the above embodiment based on the amino acid sequence of a naturally occurring fibroin, and chemically synthesizing a nucleic acid encoding the designed amino acid sequence. In any case, in addition to the modification that corresponds to substitution of the glycine residue in REP in the amino acid sequence of a naturally occurring fibroin with another amino acid residue, a modification of the amino acid sequence that corresponds to substitution, deletion, insertion, and/or addition of one or a plurality of amino acid residues may further be performed.

The above another amino acid residue is not particularly limited as long as it is an amino acid residue other than the glycine residue, but is preferably a hydrophobic amino acid residue such as a valine (V) residue, a leucine (L) residue, a isoleucine (I) residue, a methionine (M) residue, a proline (P) residue, a phenylalanine (F) residue, and a tryptophan (W) residue, or a hydrophilic amino acid residues such as a glutamine (Q) residue, an asparagine (N) residue, a serine (S) residue, a lysine (K) residue, and a glutamic acid (E) residue, more preferably a valine (V) residue, a leucine (L) residue, an isoleucine (I) residue, a phenylalanine (F) residue, and a glutamine (Q) residue, and still more preferably a glutamine (Q) residue.

More specific examples of the second modified fibroin include a modified fibroin containing (2-i) the amino acid sequence set forth in SEQ ID NO: 6 (Met-PRT380), SEQ ID NO: 7 (Met-PRT410), SEQ ID NO: 8 (Met-PRT525), or SEQ ID NO: 9 (Met-PRT799), or (2-ii) an amino acid sequence having 90% or more sequence identity with the amino acid sequence set forth in SEQ ID NO: 6, SEQ ID NO: 7, SEQ ID NO: 8, or SEQ ID NO: 9.

The modified fibroin (2-i) will be described. The amino acid sequence set forth in SEQ ID NO: 6 is an amino acid sequence in which all GGXs in REP of the amino acid sequence set forth in SEQ ID NO: 10 (Met-PRT313) corresponding to a naturally occurring fibroin are substituted with GQXs. The amino acid sequence set forth in SEQ ID NO: 7 is an amino acid sequence in which every third $(A)_n$ motif from the N-terminal side to the C-terminal side is deleted from the amino acid sequence set forth in SEQ ID NO: 6, and further, one $[(A)_n$ motif-REP] is inserted before the C-terminal sequence. The amino acid sequence set forth in SEQ ID NO: 8 is an amino acid sequence in which two alanine residues are inserted at the C-terminal side of each $(A)_n$ motif of the amino acid sequence set forth in SEQ ID NO: 7, and further some of the glutamine (Q) residues are substituted with a serine (S) residue, and some of the amino acids on the C-terminal side are deleted so that the molecular weight is almost the same as that of SEQ ID NO 7. The amino acid sequence set forth in SEQ ID NO: 9 is an amino acid sequence in which a predetermined hinge sequence and a His tag sequence are added to the C-terminal of a sequence having four-time repetition of a region of 20 domain sequences present in the amino acid sequence set forth in SEQ ID NO: 7 (however, with several amino acid residues on the C-terminal side of the region are substituted).

The value of z/w in the amino acid sequence set forth SEQ ID NO: 10 (corresponding to a naturally occurring fibroin) is 46.8%. The values of z/w in the amino acid sequence set forth in SEQ ID NO: 6, the amino acid sequence set forth in SEQ ID NO: 7, the amino acid sequence set forth in SEQ ID NO: 8, and the amino acid sequence set forth in SEQ ID NO: 9 are 58.7%, 70.1%, 66.1%, and 70.0%, respectively. In addition, the values of x/y at the Giza ratio (which will be described later) of 1:1.8 to 11.3 in the amino acid sequences set forth in SEQ ID NO: 10, SEQ ID NO: 6, SEQ ID NO: 7, SEQ ID NO: 8, and SEQ ID NO: 9 are 15.0%, 15.0%, 93.4%, 92.7%, and 89.8%, respectively.

The modified fibroin (2-i) may be a modified fibroin consisting of the amino acid sequence set forth in SEQ ID NO: 6, SEQ ID NO: 7, SEQ ID NO: 8, or SEQ ID NO: 9.

The modified fibroin (2-ii) is a modified fibroin containing an amino acid sequence having 90% or more sequence identity with the amino acid sequence set forth in SEQ ID NO: 6, SEQ ID NO: 7, SEQ ID NO: 8, or SEQ ID NO: 9. The modified fibroin (2-ii) also is a protein containing a domain sequence represented by

protein containing a domain sequence represented by Formula 1: $[(A)_n$

motif-REP$]_m$.    The sequence identity is preferably 95% or more.

The sequence identity is preferably 95% or more.

The modified fibroin (2-ii) preferably has 90% or more sequence identity with the amino acid sequence set forth in SEQ ID NO: 6, SEQ ID NO: 7, SEQ ID NO: 8, or SEQ ID NO: 9, and that z/w is 50.9% or more, where z is the total number of amino acid residues in the amino acid sequence consisting of XGX (where X represents an amino acid residue other than glycine) contained in REP; and where w is the total number of amino acid residues in REP in the domain sequence.

The second modified fibroin may contain a tag sequence at either or both of the N-terminal and C-terminal. This makes it possible to isolate, immobilize, detect, and visualize the modified fibroin.

The tag sequence may be, for example, an affinity tag utilizing specific affinity (binding property, affinity) with other molecules. Specific examples of the affinity tag include a histidine tag (His tag). The His tag is a short peptide in which about 4 to 10 histidine residues are arranged and has a property of specifically binding to a metal ion such as nickel, and thus, it can be used for isolation of a modified fibroin by a chelating metal chromatography. Specific examples of the tag sequence include an amino acid sequence set forth in SEQ ID NO: 11 (amino acid sequence containing a His tag sequence and a hinge sequence).

In addition, a tag sequence such as a glutathione-S-transferase (GST) that specifically binds to glutathione and a maltose binding protein (MBP) that specifically binds to maltose may also be used.

Furthermore, an "epitope tag" utilizing an antigen-antibody reaction may also be used. By adding a peptide (an epitope) showing antigenicity as a tag sequence, an antibody against the epitope can be bound. Examples of the epitope tag include an HA (peptide sequence of hemagglutinin of an influenza virus) tag, a myc tag, and a FLAG tag. The modified fibroin can be easily purified with high specificity by utilizing the epitope tag.

It is also possible to further use a tag sequence which can be cleaved with a specific protease. By treating a protein adsorbed via the tag sequence with a protease, it is also possible to recover a modified fibroin cleaved from the tag sequence.

More specific examples of the modified fibroin containing the tag sequence include a modified fibroin containing (2-iii) the amino acid sequence set forth in SEQ ID NO: 12 (PRT380), SEQ ID NO: 13 (PRT410), SEQ ID NO: 14 (PRT525), or SEQ ID NO: 15 (PRT799), or (2-iv) an amino acid sequence having 90% or more sequence identity with the amino acid sequence set forth in SEQ ID NO: 12, SEQ ID NO: 13, SEQ ID NO: 14, or SEQ ID NO: 15.

The amino acid sequences set forth in SEQ ID NO: 16 (PRT313), SEQ ID NO: 12, SEQ ID NO: 13, SEQ ID NO: 14, and SEQ ID NO: 15 are amino acid sequences in which the amino acid sequence (containing a His tag sequence and a hinge sequence) set forth in SEQ ID NO: 11 is added to the N-terminal of the amino acid sequences set forth in SEQ ID NO: 10, SEQ ID NO: 6, SEQ ID NO: 7, SEQ ID NO: 8, and SEQ ID NO: 9, respectively.

The modified fibroin (2-iii) may be a modified fibroin consisting of the amino acid sequence set forth in SEQ ID NO: 12, SEQ ID NO: 13, SEQ ID NO: 14, or SEQ ID NO: 15.

The modified fibroin (2-iv) is a modified fibroin containing an amino acid sequence having 90% or more sequence identity with the amino acid sequence set forth in SEQ ID NO: 12, SEQ ID NO: 13, SEQ ID NO: 14, or SEQ ID NO: 15. The modified fibroin (2-iv) also is a protein containing a domain sequence represented by

protein containing a domain sequence represented by Formula 1: $[(A)_n$ motif-REP$]_m$.    The sequence identity is preferably 95% or more.

The sequence identity is preferably 95% or more.

It is preferable that the modified fibroin (2-iv) has 90% or more sequence identity with the amino acid sequence set forth in SEQ ID NO: 12, SEQ ID NO: 13, SEQ ID NO: 14, or SEQ ID NO: 15, and that z/w is 50.9% or more, where z is the total number of amino acid residues in the amino acid sequence consisting of XGX (where X represents an amino acid residue other than glycine) contained in REP; and where w is the total number of amino acid residues in REP in the domain sequence.

The second modified fibroin may contain a secretory signal for releasing the protein produced in a recombinant protein production system to the outside of the host. The sequence of the secretory signal may be set as appropriate, depending on the type of the host.

The third modified fibroin has a domain sequence that has an amino acid sequence with a reduced content of $(A)_n$ motifs, as compared with a naturally occurring fibroin. It can be said that the domain sequence of the third modified fibroin is a domain sequence having an amino acid sequence that corresponds to an amino acid sequence in which at least one or a plurality of the $(A)_n$ motifs are deleted, as compared with a naturally occurring fibroin.

The third modified fibroin may be a modified fibroin having an amino acid sequence that corresponds to an amino acid sequence in which 10% to 40% of the $(A)_n$ motifs are deleted from a naturally occurring fibroin.

The domain sequence of the third modified fibroin may be a domain sequence having an amino acid sequence that corresponds to an amino acid sequence in which at least one $(A)_n$ motif for every one to three $(A)_n$ motifs from the N-terminal side to the C-terminal side is deleted, as compared with a naturally occurring fibroin.

The domain sequence of the third modified fibroin may be a domain sequence having an amino acid sequence that corresponds to an amino acid sequence in which at least deletion of two consecutive $(A)_n$ motifs and deletion of one $(A)_n$ motif are repeated in this order from the N-terminal side to the C-terminal side, as compared with a naturally occurring fibroin.

The domain sequence of the third modified fibroin may be a domain sequence having an amino acid sequence that corresponds to an amino acid sequence in which, at least, every third $(A)_n$ motif from the N-terminal side to the C-terminal side is deleted.

The third modified fibroin may be a modified fibroin that contains a domain sequence represented by

contains a domain sequence represented by Formula 1: $[(A)_n$ motif-REP$]_m$, and that has an amino acid sequence in which, when the

and that has an amino acid sequence in which, when the numbers of amino acid residues of REP of two adjacent $[(A)_n$

motif-REP] units are sequentially compared from the N-terminal side to the C-terminal side, x/y is 20% or more, 30% or more, 40% or more, or 50% or more, where x is a maximum value of a total value obtained by adding the numbers of amino acid residues of two adjacent $[(A)_n$ motif-REP] units wherein when the number of amino acid residues of one REP having less amino acid residues is taken as 1, the ratio of the number of amino acids residues of the other REP satisfies 1.8 to 11.3; and where y is a total number of amino acid residues of the domain sequence. The number of alanine residues with respect to a total number of amino acid residues in the $(A)_n$ motif may be 83% or more, but is preferably 86% or more, more preferably 90% or more, still more preferably 95% or more, and even still more preferably 100% (which means that the $(A)_n$ motif consists only of alanine residues).

A method for calculating x/y will be described in more detail with reference to Fig. 1. Fig. 1 illustrates a domain sequence obtained by removing an N-terminal sequence and a C-terminal sequence from a modified fibroin. The domain sequence has a sequence of, from the N-terminal side (left side), $(A)_n$ motif-the first REP (50 amino acid residues)-$(A)_n$ motif-the second REP (100 amino acid residues)-$(A)_n$ motif-the third REP (10 amino acid residues)-$(A)_n$ motif-the fourth REP (20 amino acid residues)-$(A)_n$ motif-the fifth REP (30 amino acid residues)-$(A)_n$ motif.

The two adjacent $[(A)_n$ motif-REP] units are sequentially selected from the N-terminal side to the C-terminal side so that there is no overlap. There may be $[(A)_n$ motif-REP] unit that is not selected. Fig. 1 illustrates Pattern 1 (a comparison of the first REP and the second REP, and a comparison of the third REP and the fourth REP), Pattern 2 (a comparison of the first REP and the second REP, and a comparison of the fourth REP and the fifth REP), Pattern 3 (a comparison of the second REP and the third REP, and a comparison of the fourth REP and the fifth REP), and Pattern 4 (a comparison of the first REP and the second REP). There are selection methods other than these.

Next, for each pattern, the number of amino acid residues of each REP in the selected adjacent two $[(A)_n$ motif-REP] units is compared. The comparison is performed by determining the ratio, relative to one REP having less amino acid residues taken as 1, of the number of amino acid residues of the other REP. For example, in a case of comparing the first REP (50 amino acid residues) and the second REP (100 amino acid residues), the ratio of the number of amino acid residues of the second REP is 100/50 = 2, where the first REP having less amino acid residues is taken as 1. Similarly, in a case of comparing the fourth REP (20 amino acid residues) and the fifth REP (30 amino acid residues), the ratio of the number of amino acid residues of the fifth REP is 30/20 = 1.5, where the fourth REP having less amino acid residues is taken as 1.

In Fig. 1, a set of $[(A)_n$ motif-REP] units in which, where one REP having less amino acid residues is taken as 1, the ratio of the number of amino acid residues of the other REP is 1.8 to 11.3, is indicated by a solid line. In the present specification, this ratio is referred to as a Giza ratio. A set of $[(A)_n$ motif-REP] units in which, where a REP having less amino acid residues is taken as 1, the ratio of the number of amino acid residues of the other REP is less than 1.8 or more than 11.3, is indicated by a dotted line.

In each pattern, the numbers of all amino acid residues (not only REP but also the number of amino acid residues in the $(A)_n$ motif) of the two adjacent $[(A)_n$ motif-REP] units indicated by a solid line are added. Then, the total values obtained by addition are compared, and the total value of the pattern having the highest total value (a maximum value of the total value) is denoted as x. In the example illustrated in Fig. 1, the total value of Pattern 1 is the maximum.

Next, x/y (%) can be calculated by dividing x by the total number y of the amino acid residues of the domain sequence.

In the third modified fibroin, x/y is preferably 50% or more, more preferably 60% or more, still more preferably 65% or more, even still more preferably 70% or more, still further preferably 75% or more, and particularly preferably 80% or more. The upper limit of x/y is not particularly limited, but may be, for example, 100% or less. In a case where the Giza ratio is 1:1.9 to 11.3, x/y is preferably 89.6% or more, in a case where the Giza ratio is 1:1.8 to 3.4, x/y is more preferably 77.1% or more, in a case where the Giza ratio is 1:1.9 to 8.4, x/y is still more preferably 75.9% or more, and in a case where the Giza ratio is 1:1.9 to 4.1, x/y is even still more preferably 64.2% or more.

In a case where the third modified fibroin is a modified fibroin in which at least seven of a plurality of $(A)_n$ motifs present in the domain sequence are composed only of alanine residues, x/y is preferably 46.4% or more, more preferably 50% or more, still more preferably 55% or more, even still more preferably 60% or more, still further preferably 70% or more, and particularly preferably 80% or more. The upper limit of x/y is not particularly limited as long as it is 100% or less.

Here, x/y in a naturally occurring fibroin will be described. First, fibroins with amino acid sequence information registered in NCBI GenBank was checked by the exemplified method as described above, and 663 types of fibroins (of which 415 types were fibroins derived from spiders) were extracted. Among all the extracted fibroins, x/y was calculated from an amino acid sequence of a naturally occurring fibroin constituted with a domain sequence represented by

fibroin constituted with a domain sequence represented by Formula 1:

$[(A)_n$ motif-REP]$_m$, using the above-described calculation method.

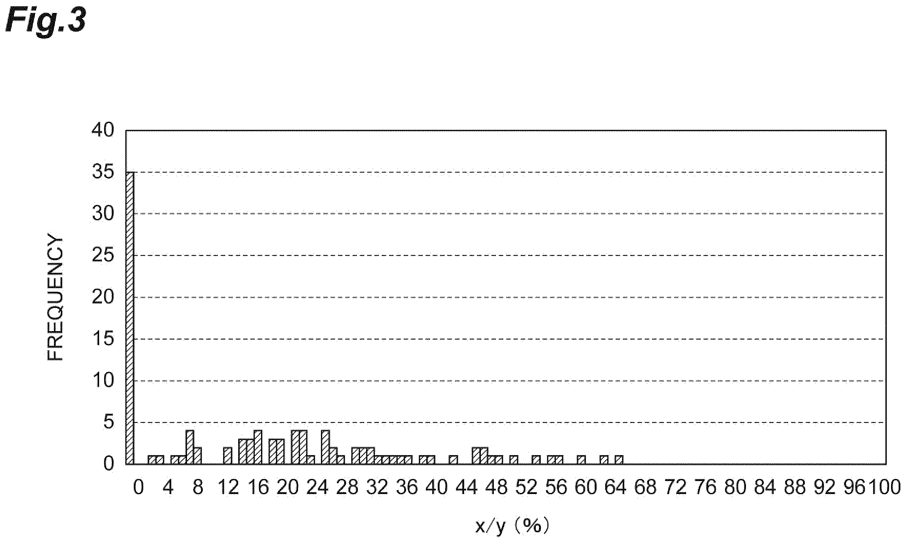

using the above-described calculation method. The results in a case where the Giza ratio is 1:1.9 to 4.1 are shown in Fig. 3.

In Fig. 3, the horizontal axis represents x/y (%) and the vertical axis represents a frequency. As apparent from Fig. 3, x/y in all the naturally occurring fibroin is less than 64.2% (highest value: 64.14%).

The third modified fibroin may be obtained, for example, by deleting one or a plurality of sequences encoding $(A)_n$ motif from a cloned gene sequence of a naturally occurring fibroin so that x/y is 64.2% or more. In addition, the third modified fibroin may also be obtained, for example, by designing an amino acid sequence that corresponds to an amino acid sequence in which one or a plurality of $(A)_n$ motifs are deleted from an amino acid sequence of a naturally occurring fibroin so that x/y becomes 64.2% or more, and chemically synthesizing a nucleic acid encoding the designed amino acid sequence. In any case, in addition to the modification that corresponds to deletion of the $(A)_n$ motif from an amino acid sequence of a naturally occurring fibroin, a modification of the amino acid sequence that corresponds to substitution, deletion, insertion, and/or addition of one or a plurality of amino acid residues may further be performed.

More specific examples of the third modified fibroin include a modified fibroin containing an amino acid sequence having (3-i) the amino acid sequence set forth in SEQ ID NO: 17 (Met-PRT399), SEQ ID NO: 7 (Met-PRT410), SEQ ID NO: 8 (Met-PRT525), or SEQ ID NO: 9 (Met-PRT799), or (3-ii) an amino acid sequence having 90% or more sequence identity with the amino acid sequence set forth in SEQ ID NO: 17, SEQ ID NO: 7, SEQ ID NO: 8, or SEQ ID NO: 9.

The modified fibroin (3-i) will be described. The amino acid sequence set forth in SEQ ID NO: 17 is an amino acid sequence in which every third $(A)_n$ motif from the N-terminal side to the C-terminal side is deleted from the amino acid sequence set forth in SEQ ID NO: 10 (Met-PRT313) corresponding to a naturally occurring fibroin, and further, one $[(A)_n$ motif-REP] is inserted before the C-terminal sequence. The amino acid sequence set forth in SEQ ID NO: 7, SEQ ID NO: 8, or SEQ ID NO: 9 is as described for the second modified fibroin.

The value of x/y at a Giza ratio of 1:1.8 to 11.3 in the amino acid sequence set forth in SEQ ID NO: 10 (corresponding to a naturally occurring fibroin) is 15.0%. Both the values of x/y in the amino acid sequence set forth in SEQ ID NO: 17 and the amino acid sequence set forth in SEQ ID NO: 7 are 93.4%. The value of x/y in the amino acid sequence set forth in SEQ ID NO: 8 is 92.7%. The value of x/y in the amino acid sequence set forth in SEQ ID NO: 9 is 89.8%. The values of z/w in the amino acid sequences set forth in SEQ ID NO: 10, SEQ ID NO: 17, SEQ ID NO: 7, SEQ ID NO: 8, and SEQ ID NO: 9 are 46.8%, 56.2%, 70.1%, 66.1%, and 70.0%, respectively.

The modified fibroin (3-i) may be a modified fibroin consisting of the amino acid sequence set forth in SEQ ID NO: 17, SEQ ID NO: 7, SEQ ID NO: 8, or SEQ ID NO: 9.

The modified fibroin (3-ii) is a modified fibroin containing an amino acid sequence having 90% or more sequence identity with the amino acid sequence set forth in SEQ ID NO: 17, SEQ ID NO: 7, SEQ ID NO: 8, or SEQ ID NO: 9. The modified fibroin (3-ii) also is a protein containing a domain sequence represented by

protein containing a domain sequence represented by Formula 1: $[(A)_n$ motif-REP$]_m$. The sequence identity is preferably 95% or more.

The sequence identity is preferably 95% or more.

It is preferable that the modified fibroin (3-ii) has 90% or more sequence identity with the amino acid sequence set forth in SEQ ID NO: 17, SEQ ID NO: 7, SEQ ID NO: 8, or SEQ ID NO: 9, and that, when the numbers of amino acid residues of REP of two adjacent $[(A)_n$ motif-REP] units are sequentially compared from the N-terminal side to the C-terminal side, x/y is 64.2% or more, where x is a maximum value of a total value obtained by adding the numbers of amino acid residues of two adjacent $[(A)_n$ motif-REP] units wherein when the number of amino acid residues of one REP having less amino acid residues is taken as 1, the ratio of the number of amino acids residues of the other REP satisfies 1.8 to 11.3 (the Giza ratio is 1:1.8 to 11.3); and where y is a total number of amino acid residues of the domain sequence.

The third modified fibroin may contain a tag sequence described above at either or both of the N-terminal and C-terminal.

A more specific example of the modified fibroin containing a tag sequence may be a modified fibroin containing (3-iii) the amino acid sequence set forth in SEQ ID NO: 18 (PRT399), SEQ ID NO: 13 (PRT410), SEQ ID NO: 14 (PRT525), or SEQ ID NO: 15 (PRT799), or (3-iv) an amino acid sequence having 90% or more sequence identity with the amino acid sequence set forth in SEQ ID NO: 18, SEQ ID NO: 13, SEQ ID NO: 14, or SEQ ID NO: 15.

The amino acid sequences set forth in SEQ ID NO: 18, SEQ ID NO: 13, SEQ ID NO: 14, and SEQ ID NO: 15 are amino acid sequences in which the amino acid sequence (containing a His tag sequence and a hinge sequence) set forth in SEQ ID NO: 11 is added to the N-terminal of the amino acid sequences set forth in SEQ ID NO: 17, SEQ ID NO: 7, SEQ ID NO: 8, and SEQ ID NO: 9, respectively.

The modified fibroin (3-iii) may be a modified fibroin consisting of the amino acid sequence set forth in SEQ ID NO: 18, SEQ ID NO: 13, SEQ ID NO: 14, or SEQ ID NO: 15.

The modified fibroin (3-iv) is a modified fibroin containing an amino acid sequence having 90% or more sequence identity with the amino acid sequence set forth in SEQ ID NO: 18, SEQ ID NO: 13, SEQ ID NO: 14, or SEQ ID NO: 15. The modified fibroin (3-iv) also is a protein containing a domain sequence represented by

protein containing a domain sequence represented by Formula 1: $[(A)_n$ motif-REP$]_m$. The sequence identity is preferably 95% or more.

The sequence identity is preferably 95% or more.

It is preferable that the modified fibroin (3-iv) has 90% or more sequence identity with the amino acid sequence set forth in SEQ ID NO: 18, SEQ ID NO: 13, SEQ ID NO: 14 or SEQ ID NO: 15, and that, when the numbers of amino acid residues of REP of two adjacent $[(A)_n$ motif-REP] units are sequentially compared from the N-terminal side to the C-terminal side, x/y is 64.2% or more, where x is a maximum value of a total value obtained by adding the numbers of amino acid residues of two adjacent $[(A)_n$ motif-REP] units wherein when the number of amino acid residues of one REP having less amino acid residues is taken as 1, the ratio of the number of amino acids residues of the other REP satisfies 1.8 to 11.3; and where y is a total number of amino acid residues of the domain sequence.

The third modified fibroin may contain a secretory signal for releasing the protein produced in a recombinant protein production system to the outside of the host. The sequence of the secretory signal may be set as appropriate, depending on the type of the host.

The fourth modified fibroin has a domain sequence that has an amino acid sequence with a reduced content of glycine residues, in addition to the reduced content of $(A)_n$ motifs, as compared with a naturally occurring fibroin. It can be said that the domain sequence of the fourth modified fibroin is a domain sequence further having an amino acid sequence that corresponds to an amino acid sequence in which at least one or a plurality of glycine residues in REP are substituted with other amino acid residues, in addition to the amino acid sequence in which at least one or a plurality of the $(A)_n$ motifs are deleted, as compared with a naturally occurring fibroin. That is, the fourth modified fibroin is a modified fibroin having both the characteristics of the second modified fibroin and the characteristics of the third modified fibroin described above. Specific embodiments and the like are as described for the second modified fibroin and the third modified fibroin.

More specific examples of the fourth modified fibroin include a modified fibroin containing (4-i) the amino acid sequence set forth in SEQ ID NO: 7 (Met-PRT410), SEQ ID NO: 8 (Met-PRT525), SEQ ID NO: 9 (Met-PRT799), SEQ ID NO: 13 (PRT410), SEQ ID NO: 14 (PRT525), or SEQ ID NO: 15 (PRT799), or (4-ii) an amino acid sequence having 90% or more sequence identity with the amino acid sequence set forth in SEQ ID NO: 7, SEQ ID NO: 8, SEQ ID NO: 9, SEQ ID NO: 13, SEQ ID NO: 14, or SEQ ID NO: 15. Specific embodiments of the modified fibroin containing the amino acid sequence set forth in SEQ ID NO: 7, SEQ ID NO: 8, SEQ ID NO: 9, SEQ ID NO: 13, SEQ ID NO: 14, or SEQ ID NO: 15 are as described above.

The fifth modified fibroin may have a domain sequence that has an amino acid sequence containing a region having a locally high hydropathy index, which corresponds to an amino acid sequence in which one or a plurality of amino acid residues in REP are substituted with an amino acid residue having a high hydropathy index and/or one or a plurality of amino acid residues having a high hydropathy index is inserted into REP, as compared with a naturally occurring fibroin.

It is preferable that the region having a locally high hydropathy index is composed of 2 to 4 consecutive amino acid residues.

It is more preferable that the amino acid residues having a high hydropathy index are selected from isoleucine (I), valine (V), leucine (L), phenylalanine (F), cysteine (C), methionine (M), and alanine (A).

The fifth modified fibroin may further have a modification of an amino acid sequence that corresponds to substitution, deletion, insertion, and/or addition of one or a plurality of amino acid residues, as compared with a naturally occurring fibroin, in addition to the modification that corresponds to substitution of one or a plurality of amino acid residues in REP with amino acid residues having a high hydropathy index and/or insertion of one or a plurality of amino acid residues having a high hydropathy index into REP, as compared with a naturally occurring fibroin.

The fifth modified fibroin may be obtained, for example, from a cloned gene sequence of a naturally occurring fibroin, by substituting one or a plurality of hydrophilic amino acid residues in REP (for example, amino acid residues having a negative hydropathy index) with a hydrophobic amino acid residue (for example, amino acid residues having a positive hydropathy index), and/or inserting one or a plurality of hydrophobic amino acid residues into REP. In addition, the fifth modified fibroin may also be obtained, for example, by designing an amino acid sequence that corresponds to an amino acid sequence in which one or a plurality of hydrophilic amino acid residues in REP are substituted with hydrophobic amino acid residues and/or one or a plurality of hydrophobic amino acid residues are inserted into REP, from an amino acid sequence of a naturally occurring fibroin, and chemically synthesizing a nucleic acid encoding the designed amino acid sequence. In any case, in addition to the modification that corresponds to substitution of one or a plurality of hydrophilic amino acid residues in REP with hydrophobic amino acid residues and/or insertion of one or a plurality of hydrophobic amino acid residues into REP in an amino acid sequence of a naturally occurring fibroin, a modification of an amino acid sequence that corresponds to substitution, deletion, insertion, and/or addition of one or a plurality of amino acid residues may further be performed.

The fifth modified fibroin may be a modified fibroin that contains a domain sequence represented by

contains a domain sequence represented by Formula 1: $[(A)_n$ motif-REP$]_m$, and that has an amino acid sequence in which p/q is 6.2%

and that has an amino acid sequence in which p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence.

With regard to the hydropathy index of an amino acid residue, known index from (Hydropathy index: Kyte J, & Doolittle R (1982) "A simple method for displaying the hydropathic character of a protein", J. Mol. Biol., 157, pp. 105-132) may be used. Specifically, the hydropathy index (hereinafter also referred to as "HI") of each amino acid is as shown in Table 1 below.

[Table 1]

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

A method for calculating p/q will be described in more detail. For calculation, a sequence (hereinafter also referred to as a "Sequence A") obtained by removing, from domain sequence represented by

Formula 1: $[(A)_n$ motif-REP$]_m$, the sequence between the $(A)_n$ motif

the sequence between the $(A)_n$ motif positioned closest to the C-terminal side and the C-terminal of the domain sequence is used. First, in all REPs contained in Sequence A, average values of hydropathy indices of the four consecutive amino acid residues are calculated. The average value of the hydropathy indices is obtained by dividing the sum of HI of each of the amino acid residues contained in the four consecutive amino acid residues by 4 (the number of amino acid residues). The average value of the hydropathy indices is obtained for all four consecutive amino acid residues (each of the amino acid residues is used for calculating the average value 1 to 4 times). Next, a region where the average value of the hydropathy indices of the four consecutive amino acid residues is 2.6 or more is identified. Even if a certain amino acid residues correspond to a plurality of the "four consecutive amino acid residues having an average value of the hydropathy indices of 2.6 or more", the amino acid residue is counted as one amino acid residue in the region. The total number of amino acid residues contained in the region is p. Further, the total number of amino acid residues contained in Sequence A is q.

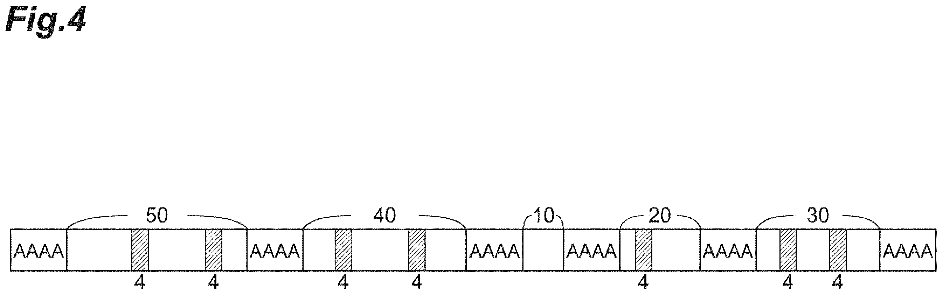

For example, in a case where 20 "four consecutive amino acid residues having an average value of the hydropathy indices of 2.6 or more " are extracted (without an overlap), there are 20 of the four consecutive amino acid residues (without an overlap) in the region in which the average value of the hydropathy indices of the four consecutive amino acid residues is 2.6 or more, and thus p is $20 \times 4 = 80$. In addition, for example, in a case where two "four consecutive amino acid residues having an average value of the hydropathy indices of 2.6 or more" overlap by only one amino acid residue, seven amino acid residues are contained in the region in which the average value of the hydropathy indices of the four consecutive amino acid residues is 2.6 or more ($p = 2 \times 4 - 1 = 7$; "-1" is deduction for overlap). For example, in a case of the domain sequence shown in Fig. 4, since there are seven "four consecutive amino acid residues having an average value of the hydropathy indices of 2.6 or more" without an overlap, p is $7 \times 4 = 28$. Further, for example, in a case of the domain sequence illustrated in Fig. 4, q is $4 + 50 + 4 + 40 + 4 + 10 + 4 + 20 + 4 + 30 = 170$ (the $(A)_n$ motif present at the end of the C-terminal side is not included). Next, p/q (%) can be calculated by dividing p by q. In the case of Fig. 4, p/q (%) is 28/170 = 16.47%.

In the fifth modified fibroin, p/q is preferably 6.2% or more, more preferably 7% or more, still more preferably 10% or more, even still more preferably 20% or more, and still further preferably 30% or more. The upper limit of p/q is not particularly limited, but may be, for example, 45% or less.

The fifth modified fibroin may be obtained, for example, by modifying an amino acid sequence of a cloned naturally occurring fibroin to an amino acid sequence containing a region having a locally high hydropathy index, by substituting one or a plurality of hydrophilic amino acid residues in REP (for example, amino acid residues having a negative hydropathy index) with hydrophobic amino acid residues (for example, amino acid residues having a positive hydropathy index), and/or inserting one or a plurality of hydrophobic amino acid residues into REP, so as to satisfy the above condition of p/q. In addition, the modified fibroin may also be obtained, for example, by designing an amino acid sequence satisfying the above-described condition of p/q based on the amino acid sequence of a naturally occurring fibroin, and chemically synthesizing a nucleic acid encoding the designed amino acid sequence. In any case, in addition to the modification that corresponds substitution of one or a plurality of amino acid residues in REP with amino acid residues having a high hydropathy index and/or insertion of one or a plurality of amino acid residues having a high hydropathy index, as compared with a naturally occurring fibroin, a modification that corresponds to substitution, deletion, insertion, and/or addition of one or a plurality of amino acid residues may further be performed.

The amino acid residue having a high hydropathy index is not particularly limited, but is preferably isoleucine (I), valine (V), leucine (L), phenylalanine (F), cysteine (C), methionine (M), and alanine (A), and more preferably valine (V), leucine (L), and isoleucine (I).

More specific examples of the fifth modified fibroin contains a modified fibroin containing (5-i) the amino acid sequence set forth in SEQ ID NO: 19 (Met-PRT720), SEQ ID NO: 20 (Met-PRT665), or SEQ ID NO: 21 (Met-PRT666), or (5-ii) an amino acid sequence having 90% or more sequence identity with the amino acid sequence set forth in SEQ ID NO: 19, SEQ ID NO: 20, or SEQ ID NO: 21.

The modified fibroin (5-i) will be described. The amino acid sequence set forth in SEQ ID NO: 19 is an amino acid sequence in which two amino acid sequences, each consisting of three amino acid residues (VLI), are inserted for every other REP excluding the terminal domain sequence on the C-terminal side; further, some of the glutamine (Q) residues are substituted with serine (S) residues; and some of the amino acids on the C-terminal side are deleted, with respect to the amino acid sequence set forth in SEQ ID NO: 7 (Met-PRT410). The amino acid sequence set forth in SEQ ID NO: 20 is an amino acid sequence in which one amino acid sequence consisting of three amino acid residues (VLI) is inserted for every other REP with respect to the amino acid sequence set forth in SEQ ID NO: 8 (Met-PRT525). The amino acid sequence set forth in SEQ ID NO: 21 is an amino acid sequence in which two amino acid sequences each consisting of three amino acid residues (VLI) are inserted for every other REP with respect to the amino acid sequence set forth in SEQ ID NO: 8.

The modified fibroin (5-i) may be a modified fibroin consisting of the amino acid sequence set forth in SEQ ID NO: 19, SEQ ID NO: 20, or SEQ ID NO: 21.

The modified fibroin (5-ii) is a modified fibroin containing an amino acid sequence having 90% or more sequence identity with the amino acid sequence set forth in SEQ ID NO: 19, SEQ ID NO: 20, or SEQ ID NO: 21. The modified fibroin (5-ii) also is a protein containing a domain sequence represented by

containing a domain sequence represented by Formula 1: $[(A)_n$ motif-REP$]_m$. The sequence identity is preferably 95% or more.

The sequence identity is preferably 95% or more.

It is preferable that the modified fibroin (5-ii) has 90% or more sequence identity with the amino acid sequence set forth in SEQ ID NO: 19, SEQ ID NO: 20, or SEQ ID NO: 21, and that p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence.

The fifth modified fibroin may contain a tag sequence at either or both of the N-terminal and C-terminal.

More specific examples of the modified fibroin containing a tag sequence include a modified fibroin containing (5-iii) the amino acid sequence set forth in SEQ ID NO: 22 (PRT720), SEQ ID NO: 23 (PRT665), or SEQ ID NO: 24 (PRT666), or (5-iv) an amino acid sequence having 90% or more sequence identity with the amino acid sequence set forth in SEQ ID NO: 22, SEQ ID NO: 23, or SEQ ID NO: 24.

The amino acid sequences set forth in SEQ ID NO: 22, SEQ ID NO: 23, and SEQ ID NO: 24 are amino acid sequences in which the amino acid sequence (containing a His tag sequence and a hinge sequence) set forth in SEQ ID NO: 11 is added to the N-terminal of the amino acid sequences set forth in SEQ ID NO: 19, SEQ ID NO: 20, and SEQ ID NO: 21, respectively.

The modified fibroin (5-iii) may be a modified fibroin consisting of the amino acid sequence set forth in SEQ ID NO: 22, SEQ ID NO: 23, or SEQ ID NO: 24.

The modified fibroin (5-iv) is a modified fibroin containing an amino acid sequence having 90% or more sequence identity with the amino acid sequence set forth in SEQ ID NO: 22, SEQ ID NO: 23, or SEQ ID NO: 24. The modified fibroin (5-iv) also is a protein containing a domain sequence represented by Forumula 1:

containing a domain sequence represented by Formula 1: $[(A)_n$ motif-REP]$_m$. The sequence identity is preferably 95% or more.

The sequence identity is preferably 95% or more.

It is preferable that the modified fibroin (5-iv) has 90% or more sequence identity with the amino acid sequence set forth in SEQ ID NO: 22, SEQ ID NO: 23, or SEQ ID NO: 24, and that p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence.

The fifth modified fibroin may contain a secretory signal for releasing the protein produced in a recombinant protein production system to the outside of the host. The sequence of the secretory signal may be set as appropriate, depending on the type of the host.

The sixth modified fibroin has an amino acid sequence with a reduced content of glutamine residues, as compared with a naturally occurring fibroin.

It is preferable that the sixth modified fibroin contains at least one motif selected from a GGX motif and a GPGXX motif in the amino acid sequence of REP.

In a case where the sixth modified fibroin contains a GPGXX motif in REP, a GPGXX motif content rate is usually 1% or more, may be 5% or more, and is preferably 10% or more. The upper limit of the GPGXX motif content rate is not particularly limited, and it may be 50% or less, and may be 30% or less.

In the present specification, the "GPGXX motif content rate" is a value calculated by the following method.

In a fibroin (a modified fibroin or a naturally occurring fibroin) containing a domain sequence represented by Formula 1: $[(A)_n$motif-REP]$_m$ or Formula 2: $[(A)_n$motif-REP]$_m$-$(A)_n$ motif, the GPGXX motif content rate is calculated as s/t, where s is the number obtained by tripling the total number of the GPGXX motifs (namely, corresponds to the total number of G and P in the GPGXX motifs) contained in all REPs contained in the domain sequence excluding the sequence between the $(A)_n$ motif positioned closest to the C-terminal side and the C-terminal of the domain sequence; and where t is the total number of amino acid residues in all REPs obtained by excluding the sequence between the $(A)_n$ motif positioned closest to the C-terminal side and the C-terminal of the domain sequence from the domain sequence and further excluding the $(A)_n$ motifs.

For the calculation of the GPGXX motif content rate, a "domain sequence excluding the sequence between the $(A)_n$ motif positioned closest to the C-terminal side and the C-terminal of the domain sequence" is used, because "the sequence between the $(A)_n$ motif positioned closest to the C-terminal side and the C-terminal of the domain sequence (sequence corresponding to REP) may contain a sequence having a low correlation with the sequence characteristic of a fibroin, which influences the calculation result of the GPGXX motif content rate when m is small (that is, when the domain sequence is short), and thus, this effect is to be eliminated. In a case where a "GPGXX motif" is located at the C-terminal of REP, it is treated as "GPGXX motif" even if "XX" is, for example, "AA".

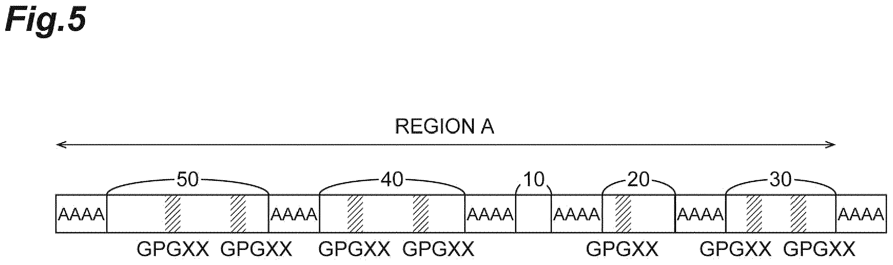

Fig. 5 is a schematic diagram illustrating a domain sequence of a modified fibroin. A method for calculating the GPGXX motif content rate will be specifically described with reference to Fig. 5. First, in a domain sequence of the modified fibroin (which is an [(A)$_n$ motif-REP]$_m$-(A)$_n$ motif] type) illustrated in Fig. 5, the number of GPGXX motifs for calculation of s is 7 and s is $7 \times 3 = 21$, because all REPs are contained in the "domain sequence excluding the sequence between the (A)$_n$ motif positioned closest to the C-terminal side and the C-terminal of the domain sequence" (indicated as "REGION A" in Fig. 5). Similarly, since all REPs are contained in the "domain sequence excluding the sequence between the (A)$_n$ motif positioned closest to the C-terminal side and the C-terminal of the domain sequence" (indicated as "REGION A" in Fig. 5), the total number, t, of amino acid residues in all REPs obtained by further excluding the (A)$_n$ motifs from the "domain sequence excluding the sequence between the (A)$_n$ motif positioned closest to the C-terminal side and the C-terminal of the domain sequence" is $50 + 40 + 10 + 20 + 30 = 150$. Next, s/t (%) can be calculated by dividing s by t, and thus, in a case of the modified fibroin of Fig. 5, s/t (%) is 21/150 = 14.0%.

In the sixth modified fibroin, the glutamine residue content rate is preferably 9% or less, more preferably 7% or less, still more preferably 4% or less, and particularly preferably 0%.

In the present specification, the "glutamine residue content rate" is a value calculated by the following method.

In a fibroin (a modified fibroin or a naturally occurring fibroin) containing a domain sequence represented by Formula 1: [(A)$_n$motif-REP]$_m$ or Formula 2:[(A)$_n$motif-REP]$_m$-(A)$_n$ motif, the glutamine residue content rate is calculated as u/t, where u is the total number of glutamine residues contained in all REPs contained in the domain excluding the sequence between the (A)$_n$ motif positioned closest to the C-terminal side and the C-terminal of the domain sequence (sequence corresponding to the "REGION A" in Fig. 5); and where t is the total number of amino acid residues in all REPs obtained by excluding the sequence between the (A)$_n$ motif positioned closest to the C-terminal side and the C-terminal of the domain sequence from the domain sequence and further excluding the (A)$_n$ motifs. The reason as to why the "domain sequence excluding the sequence between the (A)$_n$ motif positioned closest to the C-terminal side and the C-terminal of the domain sequence" is used for the calculation of the glutamine residue content rate is the same as the reason described above.

The domain sequence of the sixth modified fibroin may be a domain sequence having an amino acid sequence that corresponds to an amino acid sequence in which one or a plurality of glutamine residues in REP are deleted or substituted with other amino acid residues, as compared with a naturally occurring fibroin.

The "other amino acid residues" may be amino acid residues other than the glutamine residue, but are preferably amino acid residues having a higher hydropathy index than that of the glutamine residue. The hydropathy indices of the amino acid residues are as shown in Table 1.

As shown in Table 1, examples of the amino acid residues having a higher hydropathy index than a glutamine residue include an amino acid residue selected from isoleucine (I), valine (V), leucine (L), phenylalanine (F), cysteine (C), methionine (M), alanine (A), glycine (G), threonine (T), serine (S), tryptophan (W), tyrosine (Y), proline (P), and histidine (H). Among these, an amino acid residue selected from isoleucine (I), valine (V), leucine (L), phenylalanine (F), cysteine (C), methionine (M), and alanine (A) is more preferable, and an amino acid residue selected from isoleucine (I), valine (V), leucine (L), and phenylalanine (F) is still more preferable.

In the sixth modified fibroin, the degree of hydrophobicity of REP is preferably -0.8 or more, more preferably -0.7 or more, still more preferably 0 or more, even still more preferably 0.3 or more, and particularly preferably 0.4 or more. The upper limit of the degree of hydrophobicity of REP is not particularly limited, and it may be 1.0 or less, and may be 0.7 or less.

In the present specification, the "degree of hydrophobicity of REP" is a value calculated by the following method.

In a fibroin (a modified fibroin or a naturally occurring fibroin) containing a domain sequence represented by Formula 1: [(A)$_n$ motif-REP]$_m$ or Formula 2:[(A)$_n$motif-REP]$_m$-(A)$_n$motif, the degree of hydrophobicity of REP is calculated as v/t, where v is the total sum of the hydropathy index of each amino acid residue contained in all REPs contained in the domain a sequence excluding the sequence between the (A)$_n$ motif positioned closest to the C-terminal side and the C-terminal of the domain sequence (sequence corresponding to a "REGION A" in Fig. 5); and where t is the total number of amino acid residues in all REPs obtained by excluding the sequence between the (A)$_n$ motif positioned closest to the C-terminal side and the C-terminal of the domain sequence from the domain sequence and further excluding the (A)$_n$ motifs. The reason as to why the "domain sequence excluding the sequence between the (A)$_n$ motif positioned closest to the C-terminal side and the C-terminal of the domain sequence" is used for the calculation of the degree of hydrophobicity of REP is the same as the reason described above.

The domain sequence of the sixth modified fibroin may further have a modification of an amino acid sequence that corresponds to substitution, deletion, insertion, and/or addition of one or a plurality of amino acid residues, in addition to the modification that corresponds to deletion of one or a plurality of glutamine residues in REP and/or substitution of one or a plurality of glutamine residues in REP with other amino acid residues, as compared with a naturally occurring fibroin.

The sixth modified fibroin may be obtained, for example, from a cloned gene sequence of a naturally occurring fibroin, by deleting one or a plurality of glutamine residues in REP and/or substituting one or a plurality of glutamine residues in REP with other amino acid residues. In addition, the sixth modified fibroin may also be obtained by designing an amino acid sequence that corresponds to an amino acid sequence in which one or a plurality of glutamine residues in REP are deleted, and/or one or a plurality of glutamine residues in REP are substituted with other amino acid residues, based on the amino acid sequence of a naturally occurring fibroin, and chemically synthesizing a nucleic acid encoding the designed amino acid sequence.

More specific examples of the sixth modified fibroin include (6-i) a modified fibroin containing the amino acid sequence set forth in SEQ ID NO: 25 (Met-PRT888), SEQ ID NO: 26 (Met-PRT965), SEQ ID NO: 27 (Met-PRT889), SEQ ID NO: 28 (Met-PRT916), SEQ ID NO: 29 (Met-PRT918), SEQ ID NO: 30 (Met-PRT699), SEQ ID NO: 31 (Met-PRT698), or SEQ ID NO: 32 (Met-PRT966), or (6-ii) a modified fibroin containing an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, or SEQ ID NO: 32.

The modified fibroin (6-i) will be described. The amino acid sequence set forth in SEQ ID NO: 25 is obtained by substituting all QQs in an amino acid sequence (Met-PRT410) set forth in SEQ ID NO: 7 with VLs. The amino acid sequence set forth in SEQ ID NO: 26 is obtained by substituting all QQs in the amino acid sequence set forth in SEQ ID NO: 7 with TSs, and substituting the remaining Qs with As. The amino acid sequence set forth in SEQ ID NO: 27 is obtained by substituting all QQs in the amino acid sequence set forth in SEQ ID NO: 7 with VLs, and substituting the remaining Qs with Is. The amino acid sequence set forth in SEQ ID NO: 28 is obtained by substituting all QQs in the amino acid sequence set forth in SEQ ID NO: 7 with VIs, and substituting the remaining Qs with Ls. The amino acid sequence set forth in SEQ ID NO: 29 is obtained by substituting all QQs in the amino acid sequence set forth in SEQ ID NO: 7 with VFs, and substituting the remaining Qs with Is.

The amino acid sequence set forth in SEQ ID NO: 30 is obtained by substituting all QQs in the amino acid sequence (Met-PRT525) set forth in SEQ ID NO: 8 with VLs. The amino acid sequence set forth in SEQ ID NO: 31 is obtained by substituting all QQs in the amino acid sequence set forth in SEQ ID NO: 8 with VLs, and substituting the remaining Qs with Is.

The amino acid sequence set forth in SEQ ID NO: 32 is a sequence obtained by substituting all QQs in a sequence obtained by repeating twice a region of 20 domain sequences present in the amino acid sequence (Met-PRT410) set forth in SEQ ID NO: 7 with VFs, and substituting the remaining Qs with Is.

The glutamine residue content rate of any of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, and SEQ ID NO: 32 is 9% or less (Table 2).

[Table 2]

| Modified fibroin | Glutamine residue content rate | GPGXX motif content rate | Degree of hydrophobicity of REP |
|---|---|---|---|
| Met-PRT410 (SEQ ID NO: 7) | 17.7% | 27.9% | -1.52 |
| Met-PRT888 (SEQ ID NO: 25) | 6.3% | 27.9% | -0.07 |
| Met-PRT965 (SEQ ID NO: 26) | 0.0% | 27.9% | -0.65 |
| Met-PRT889 (SEQ ID NO: 27) | 0.0% | 27.9% | 0.35 |
| Met-PRT916 (SEQ ID NO: 28) | 0.0% | 27.9% | 0.47 |
| Met-PRT918 (SEQ ID NO: 29) | 0.0% | 27.9% | 0.45 |
| Met-PRT699 (SEQ ID NO: 30) | 3.6% | 26.4% | -0.78 |
| Met-PRT698 (SEQ ID NO: 31) | 0.0% | 26.4% | -0.03 |
| Met-PRT966 (SEQ ID NO: 32) | 0.0% | 28.0% | 0.35 |

The modified fibroin (6-i) may be a modified fibroin consisting of the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, or SEQ ID NO: 32.

The modified fibroin (6-ii) may be a modified fibroin containing an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, or SEQ ID NO: 32. The modified fibroin (6-ii) also is a protein containing a domain sequence represented by motif-REP]$_m$ or Formula 2:[(A)$_n$motif-REP]$_m$-(A)$_n$ motif. The sequence identity is preferably 95% or more.

The modified fibroin (6-ii) preferably has a glutamine residue content rate of 9% or less. In addition, the modified fibroin (6-ii) preferably has a GPGXX motif content rate of 10% or more.

The sixth modified fibroin may contain a tag sequence at either or both of the N-terminal and C-terminal. This makes it possible to isolate, immobilize, detect, and visualize the modified fibroin.

More specific examples of the modified fibroin containing a tag sequence include a modified fibroin containing (6-iii) the amino acid sequence set forth in SEQ ID NO: 33 (PRT888), SEQ ID NO: 34 (PRT965), SEQ ID NO: 35 (PRT889), SEQ ID NO: 36 (PRT916), SEQ ID NO: 37. (PRT918), SEQ ID NO: 38 (PRT699), SEQ ID NO: 39 (PRT698), or SEQ ID NO: 40 (PRT966), or a modified fibroin containing (6-iv) an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, or SEQ ID NO: 40.

The amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, and SEQ ID NO: 40 are amino acid sequences in which the amino acid sequence (containing a His tag sequence and a hinge sequence) set forth in SEQ ID NO: 11 is added to the N-terminal of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, and SEQ ID NO: 32, respectively. Since only the tag sequence is added to the N-terminal, the glutamine residue content rate is not changed, and any of the amino acid sequences set forth in SEQ ID NO:33, SEQ ID NO:34, SEQ ID NO:35, SEQ ID NO:36, SEQ ID NO:37, SEQ ID NO:38, SEQ ID NO:39, and SEQ ID NO: 40 has a glutamine residue content rate of 9% or less (Table 3).

[Table 3]

| Modified fibroin | Glutamine residue content rate | GPGXX motif content rate | Degree of hydrophobicity of REP |
|---|---|---|---|
| PRT888 (SEQ ID NO: 33) | 6.3% | 27.9% | -0.07 |
| PRT965 (SEQ ID NO: 34) | 0.0% | 27.9% | -0.65 |
| PRT889 (SEQ ID NO: 35) | 0.0% | 27.9% | 0.35 |
| PRT916 (SEQ ID NO: 36) | 0.0% | 27.9% | 0.47 |
| PRT918 (SEQ ID NO: 37) | 0.0% | 27.9% | 0.45 |
| PRT699 (SEQ ID NO: 38) | 3.6% | 26.4% | -0.78 |
| PRT698 (SEQ ID NO: 39) | 0.0% | 26.4% | -0.03 |
| PRT966 (SEQ ID NO: 40) | 0.0% | 28.0% | 0.35 |

The modified fibroin (6-iii) may be a modified fibroin consisting of the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, or SEQ ID NO: 40.

The modified fibroin (6-iv) is a modified fibroin containing an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, or SEQ ID NO: 40. The modified fibroin (6-iv) also is a protein containing a domain sequence represented by motif-REP]$_m$ or Formula 2:[(A)$_n$motif-REP]$_m$-(A)$_n$ motif. The sequence identity is preferably 95% or more.

The modified fibroin (6-iv) preferably has a glutamine residue content rate of 9% or less. In addition, the modified fibroin (6-iv) preferably has a GPGXX motif content rate of 10% or more.

The sixth modified fibroin may contain a secretory signal for releasing the protein produced in a recombinant protein production system to the outside of the host. The sequence of the secretory signal may be set as appropriate, depending on the type of the host.

The modified fibroin may be a modified fibroin that has at least two or more characteristics in combination among the characteristics of the first modified fibroin, the second modified fibroin, the third modified fibroin, the fourth modified fibroin, the fifth modified fibroin, and the sixth modified fibroin.

The limiting oxygen index (LOI) value of the modified fibroin according to one embodiment may be 18 or more, 20 or more, 22 or more, 24 or more, 26 or more, 28 or more, 29 or more, or 30 or more. In the present specification, the LOI value is a value measured in accordance with "Test Method for Granular or Low-Melting-Point Synthetic Resin" described in "Notice No. 50 of the Office of Hazardous Materials Regulation (as of May 31, 1995)".

The modified fibroin according to one embodiment may have a heat retention index determined according to the following Formula B of 0.20 or more.

$$\text{Formula B: Heat retention index} = \text{Heat retention rate (\%)} / \text{Basis weight of sample (g/m}^2)$$

Here, in the present specification, the heat retention rate means a heat retention rate measured by a dry contact method using a Thermo Labo II type tester (under a wind at 30 cm/sec), and is a value measured by the method described in Test Examples which will be described later.

The heat retention index of the modified fibroin according to one embodiment may be 0.22 or more, may be 0.24 or more, may be 0.26 or more, may be 0.28 or more, may be 0.30 or more, and may be 0.32 or more. The upper limit of the heat retention index is not particularly limited, but may be, for example, 0.60 or less, or 0.40 or less.

The maximum hygroscopic heat generation of the modified fibroin according to one embodiment, which is determined according to Formula A, may be more than 0.025°C/g.

$$\text{Formula A: Maximum hygroscopic heat generation} = \{(\text{Highest temperature of a sample when the sample has been transferred to a high humidity environment after being placed in a low humidity environment until a temperature of the sample reaches equilibrium}) - (\text{Temperature of the sample when the sample is being transferred to the high humidity environment after being placed in the low humidity environment until the temperature of the sample reaches equilibrium})\} \ (°C) / \text{Sample weight (g)}$$

Furthermore, in Formula A, the low humidity environment means an environment of a temperature of 20°C and a relative humidity of 40%, and the high humidity environment means an environment of a temperature of 20°C and a relative humidity of 90%.

The maximum hygroscopic heat generation of the modified fibroin according one embodiment is 0.026°C/g or more, 0.027°C/g or more, 0.028°C/g or more, 0.029°C/g or more, 0.030°C/g or more, 0.035°C/g or more, or 0.040°C/g or more. The upper limit of the maximum hygroscopic heat generation is not particularly limited, but is usually 0.060°C/g or less.

<Method for producing modified fibroin>

The modified fibroin according to any of the embodiments above may also be produced, for example, by expressing a nucleic acid by a host transformed with an expression vector having the nucleic acid sequence encoding the modified fibroin and one or a plurality of regulatory sequences operably linked to the nucleic acid sequence.

A method for producing a nucleic acid encoding a modified fibroin is not particularly limited. For example, the nucleic acid may be produced by a method for performing cloning using a gene encoding a natural fibroin by the amplification with polymerase chain reaction (PCR) or the like and modifying the gene by a genetic engineering method, or a method for chemically synthesizing the nucleic acid. The method for chemically synthesizing a nucleic acid is not particularly limited, and for example, a gene may be chemically synthesized by a method in which oligonucleotides are automatically synthesized by AKTA oligopilot plus 10/100 (GE Healthcare Japan Corporation) or the like, and are linked by PCR or the like, based on the amino acid sequence information of the fibroin obtained from the NCBI web database or the like. At this time, in order to facilitate purification and/or confirmation of the modified fibroin, a nucleic acid encoding a modified fibroin consisting of an amino acid sequence obtained by adding an amino acid sequence consisting of a start codon and a His10 tag to the N-terminal of the amino acid sequence may be synthesized.

The regulatory sequence is a sequence (for example, a promoter, an enhancer, a ribosome binding sequence, or a transcription termination sequence) that controls the expression of a modified fibroin in a host, and may be selected as appropriate, depending on the type of the host. As a promoter, an inducible promoter that functions in a host cell and is capable of inducing the expression of a modified fibroin may be used. An inducible promoter is a promoter that can control transcription by the presence of an inducer (an expression inducer), the absence of a repressor molecule, or physical factors such as an increase or decrease in temperature, osmotic pressure, or pH value.

The type of the expression vector such as a plasmid vector, a viral vector, a cosmid vector, a fosmid vector, or an artificial chromosome vector may be selected as appropriate depending on the type of the host. As the expression vector, an expression vector that can autonomously replicate in a host cell or can be incorporated into a chromosome of a host and which contains a promoter at a position capable of transcribing the nucleic acid that encodes a modified fibroin is suitably used.

Both prokaryotes and eukaryotes such as yeast, filamentous fungi, insect cells, animal cells, and plant cells may be suitably used as a host.

Preferred examples of the prokaryotic host cells include bacteria belonging to the genus Escherichia, the genus Brevibacillus, the genus Serratia, the genus Bacillus, the genus Microbacterium, the genus Brevibacterium, the genus Corynebacterium, and the genus Pseudomonas. Examples of microorganisms belonging to the genus Escherichia may include Escherichia coli. Examples of microorganisms belonging to the genus Brevibacillus may include Brevibacillus agri. Examples of microorganisms belonging to the genus Serratia may include Serratia liquefaciens. Examples of microorganisms belonging to the genus Bacillus may include Bacillus subtilis. Examples of microorganisms belonging to the genus Microbacterium may include Microbacterium ammoniaphilum. Examples of microorganisms belonging to the genus Brevibacterium may include Brevibacterium divaricatum. Examples of microorganisms belonging to the genus Corynebacterium may include Corynebacterium ammoniagenes. Examples of microorganisms belonging to the genus Pseudomonas may include Pseudomonas putida.

In a case where a prokaryote is used as a host, examples of a vector into which a nucleic acid encoding a modified fibroin is introduced include pBTrp2 (manufactured by Boehringer Mannheim), pGEX (manufactured by Pharmacia), pUC18, pBluescriptII, pSupex, pET22b, pCold, pUB110, and pNCO2 (Japanese Unexamined Patent Publication No. 2002-238569).

Examples of the eukaryotic hosts include yeast and filamentous fungi (mold and the like). Examples of the yeasts include yeasts belonging to the genus Saccharomyces, the genus Pichia, and the genus Schizosaccharomyces. Examples of the filamentous fungi include filamentous fungi belonging to the genus Aspergillus, the genus Penicillium, and the genus Trichoderma.

In a case where an eukaryote is used as a host, examples of the vector into which a nucleic acid encoding a modified fibroin is introduced include YEp13 (ATCC37115) and YEp24 (ATCC37051). As a method for introducing an expression vector into the above host cell, any method may be used as long as the method introduces DNA into the host cell. Examples thereof include a method using calcium ions [Proc. Natl. Acad. Sci. USA, 69, 2110 (1972)], electroporation method, spheroplast method, protoplast method, lithium acetate method, and competent method.

As for the method for expressing a nucleic acid using a host transformed with an expression vector, secretory production, fusion protein expression, or the like, in addition to the direct expression, may be carried out in accordance with the methods described in Molecular Cloning, 2nd edition and the like.

The modified fibroin may be produced, for example, by culturing a host transformed with the expression vector in a culture medium, producing and accumulating the modified fibroin in the culture medium, and then collecting the modified fibroin from the culture medium. The method for culturing a host in a culture medium may be carried out according to a method commonly used for culturing a host.

When the host is a prokaryote such as Escherichia coli or a eukaryote such as yeast, any of a natural medium and a synthetic medium may be used as a culture medium, as long as the medium contains a carbon source, a nitrogen source, inorganic salts, and the like which can be utilized by the host and the medium allows for an efficient culturing of the host.

As the carbon source, any carbon source that can be utilized by the transformed microorganism may be used, and for example, glucose, fructose, sucrose, and molasses containing these; carbohydrates such as starch and a starch hydrolyzate; organic acids such as acetic acid and propionic acid; and alcohols such as ethanol and propanol may be used. As the nitrogen source, for example, ammonium salts of inorganic or organic acids such as ammonia, ammonium chloride, ammonium sulfate, ammonium acetate, and ammonium phosphate, other nitrogen-containing compounds, peptone, meat extract, yeast extract, corn steep liquor, casein hydrolysate, soybean cake and soybean cake hydrolysate, and various fermented microbial cells and digested products thereof may be used. As the inorganic salt, potassium dihydrogen phosphate, dipotassium phosphate, magnesium phosphate, magnesium sulfate, sodium chloride, ferrous sulfate, manganese sulfate, copper sulfate, and calcium carbonate may be used.

Culture of a prokaryote such as Escherichia coli or a eukaryote such as yeast may be carried out under aerobic conditions such as shaking culture or deep aeration stirring culture. The culture temperature is, for example, 15°C to 40°C. The culture time is usually 16 hours to 7 days. It is preferable to maintain the pH of the culture medium during the culture at 3.0 to 9.0. The pH of the culture medium may be adjusted using an inorganic acid, an organic acid, an alkali solution, urea, calcium carbonate, ammonia, or the like.

In addition, antibiotics such as ampicillin and tetracycline may be added to the culture medium as necessary during the culture. In a case of culturing a microorganism transformed with an expression vector in which an inducible promoter is used as the promoter, an inducer may be added to the medium as necessary. For example, in a case of culturing a microorganism transformed with an expression vector in which a lac promoter is used, isopropyl-$\beta$-D-thiogalactopyranoside or the like may be added to the medium, and in a case of culturing a microorganism transformed with an expression vector in which a trp promoter is used, indole acrylic acid or the like may be added to the medium.

The isolation and purification of the expressed modified fibroin may be performed by a commonly used method. For example, in a case where the modified fibroin is expressed in a dissolved state in cells, the host cells are recovered by centrifugation after the completion of the culture, suspended in an aqueous buffer solution, and then disrupted using an ultrasonicator, a French press, a Manton-Gaulin homogenizer, a Dyno-Mill, or the like to obtain a cell-free extract. From the supernatant obtained by centrifuging the cell-free extract, a purified preparation may be obtained by a method commonly used for protein isolation and purification, that is, a solvent extraction method, a salting-out method using ammonium sulfate or the like, a desalting method, a precipitation method using an organic solvent, an anion exchange chromatography method using a resin such as diethylaminoethyl (DEAE)-Sepharose or DIAION HPA-75 (manufactured by Mitsubishi Kasei Kogyo Kabushiki Kaisha), an cation exchange chromatography method using a resin such as S-Sepharose FF (manufactured by Pharmacia Corporation), a hydrophobic chromatography method using a resin such as butyl sepharose or phenyl sepharose, a gel filtration method using a molecular sieve, an affinity chromatography method, a chromatofocusing method, or an electrophoresis method such as isoelectric focusing or the like, using the above- methods singly or in combination thereof.

In addition, in a case where the modified fibroin is expressed to form an insoluble body in the cell, the host cells are recovered, disrupted, and centrifuged in the same manner to recover the insoluble body of the modified fibroin as a precipitated fraction. The recovered insoluble body of the modified fibroin may be solubilized with a protein denaturing agent. After this operation, a purified preparation of modified fibroin may be obtained by the same isolation and purification method as described above. In a case where the modified fibroin is secreted extracellularly, the modified fibroin may be recovered from the culture supernatant. That is, a culture supernatant is obtained by treating the culture by a method such as centrifugation, and a purified preparation may be obtained from the culture supernatant by using the same isolation and purification method as described above.

<Modified fibroin fiber and method for producing same>

The modified fibroin fiber according to one embodiment is a synthetic fiber obtained by spinning the above-described modified fibroin and contains the above-described modified fibroin as a main component.

The modified fibroin fiber according to one embodiment may have of an LOI value of 18 or more, 20 or more, 22 or more, 24 or more, 26 or more, 28 or more, 29 or more, and 30 or more.

The modified fibroin fiber according to one embodiment may have a maximum hygroscopic heat generation, as determined according to the following Formula A, of more than 0.025°C/g.

$$\text{Formula A: Maximum hygroscopic heat generation} = \{(\text{Highest}$$

temperature of a sample when the sample has been transferred to a high humidity environment after being placed in a low humidity environment until a temperature of the sample reaches equilibrium) - (Temperature of

the sample when the sample is being transferred to the high humidity environment after being placed in the low humidity environment until the temperature of the sample reaches equilibrium)} (°C) / Sample

weight (g)

In Formula A, the low-humidity environment means an environment of a temperature of 20°C and a relative humidity of 40%, and the high-humidity environment means an environment of a temperature of 20°C and a relative humidity of 90%.

The modified fibroin fiber according to one embodiment may have a maximum hygroscopic heat generation of 0.026°C/g or more, 0.027°C/g or more, 0.028°C/g or more, 0.029°C/g or more, 0.030°C/g or more, 0.035°C/g or more, and 0.040°C/g or more. The upper limit of the maximum hygroscopic heat generation is not particularly limited, but it is usually 0.060°C/g or less.

The modified fibroin fiber according to one embodiment may have a heat retention index determined according to the following Formula B of 0.20 or more.

$$\text{Formula B: Heat retention index} = \text{Heat retention rate (\%)} /$$

$$\text{Basis weight of sample (g/m}^2)$$

The heat retention property index of the modified fibroin fiber according to one embodiment may be 0.22 or more, may be 0.24 or more, may be 0.26 or more, may be 0.28 or more, may be 0.30 or more, and may be 0.32 or more. The upper limit of the heat retention index is not particularly limited, but may be, for example, 0.60 or less, or 0.40 or less.

The modified fibroin fiber may be produced by a known spinning method. That is, for example, in a case of producing a modified fibroin fiber containing a modified fibroin as the main component, first, the modified fibroin produced according to the above-described method is added to a solvent such as dimethyl sulfoxide (DMSO), N,N-dimethylformamide (DMF), formic acid, or hexafluoroisopropanol (HFIP), as necessary, together with an inorganic salt as a dissolution accelerator and dissolved to prepare a doping liquid. Then, using this doping liquid, spinning may be performed by a known spinning method such as wet type spinning, dry type spinning, dry-wet type spinning, or melt spinning to obtain the modified fibroin fiber. A preferred spinning method is wet type spinning and dry-wet type spinning.

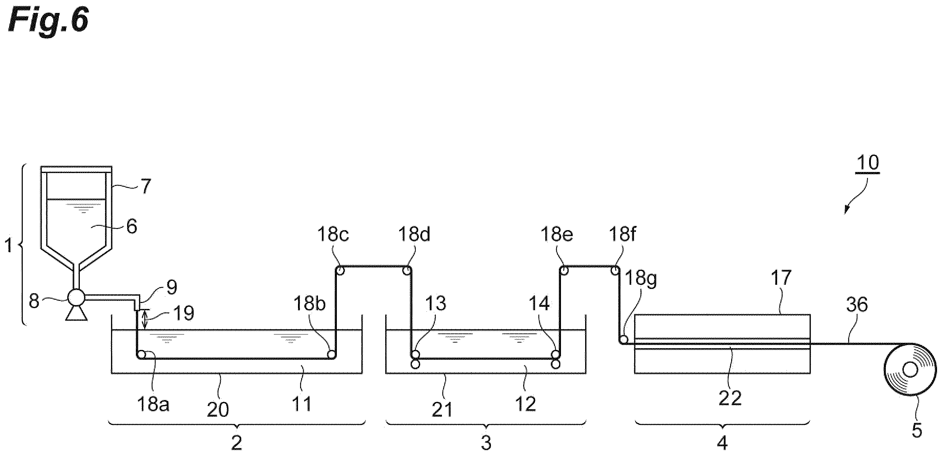

Fig. 6 is an illustrative view schematically illustrating one example of a spinning device for producing a modified fibroin fiber. A spinning device 10 illustrated in Fig. 6 is one example of a spinning device for dry-wet type spinning and includes an extrusion device 1, an undrawn yarn producing device 2, a wet heat drawing device 3, and a drying device 4.

A spinning method using the spinning device 10 will be described. First, a doping liquid 6 stored in a storage tank 7 is extruded from a spinneret 9 by a gear pump 8. In the laboratory scale, the doping liquid may be filled in a cylinder and extruded from a nozzle using a syringe pump. Next, the extruded doping liquid 6 is supplied into a coagulation liquid 11 in a coagulation liquid bath 20 via an air gap 19, the solvent is removed, the modified fibroin is coagulated, and a fibrous coagulate is formed. Then, the fibrous coagulate is supplied into a warm water 12 in a drawing bath 21 and is drawn. A drawing rate is determined according to a speed ratio of a supply nip roller 13 to a withdrawing nip roller 14. Thereafter, the drawn fibrous coagulate is supplied to a drying device 4 and dried in a yarn path 22, and a modified fibroin fiber 36 is obtained as a wound yarn body 5. Reference signs 18a to 18g indicate yarn guides.

The coagulation liquid 11 may be any solvent that can be desolvated, and examples thereof include lower alcohols having 1 to 5 carbon atoms such as methanol, ethanol, and 2-propanol, and acetone. The coagulation liquid 11 may appropriately include water. The temperature of the coagulation liquid 11 is preferably 0°C to 30°C. In a case where a syringe pump having a nozzle with a diameter of 0.1 to 0.6 mm is used as the spinneret 9, the extrusion speed is preferably 0.2 to 6.0 ml/hour, and more preferably 1.4 to 4.0 ml/hour, per hole. A distance that the coagulated modified fibroin passes through the coagulation liquid 11 (substantially a distance from the yarn guide 18a to the yarn guide 18b) may be a length that allows efficient desolvation, and is, for example, 200 to 500 mm. The withdrawing speed of the undrawn yarn may be, for example, 1 to 20 m/min, and is preferably 1 to 3 m/min. The residence time in the coagulation liquid 11 may be, for example, 0.01 to 3 minutes, and is preferably 0.05 to 0.15 minutes. In addition, drawing (pre-drawing) may be performed in the coagulation liquid 11. The coagulation liquid bath 20 may be provided in multiple stages, and the drawing may be performed in each stage or in a specific stage as necessary.

As the drawing performed in a case of obtaining the modified fibroin fiber, for example, a pre-drawing performed in the coagulation liquid bath 20 and a wet heat drawing performed in the drawing bath 21 are employed, and a dry heat drawing is also employed.

The wet heat drawing may be performed in warm water, in a solution obtained by adding an organic solvent or the like to warm water, or in heated steam. The temperature may be, for example, 50°C to 90°C, and is preferably 75°C to 85°C. In the wet heat drawing, the undrawn yarn (or pre-drawn yarn) may be drawn, for example, by 1 to 10 times and preferably by 2 to 8 times.

The dry heat drawing may be performed using an electric tubular furnace, a dry heat plate, or the like. The temperature may be, for example, 140°C to 270°C, and is preferably 160°C to 230°C. In the dry heat drawing, the undrawn yarn (or pre-drawn yarn) may be drawn, for example, by 0.5 to 8 times and preferably by 1 to 4 times.

The wet heat drawing and the dry heat drawing may be performed independently or in combination or may be performed in multiple stages. That is, the wet heat drawing and the dry heat drawing may be performed in combination as appropriate, such as performing the first drawing by wet heat drawing and the second drawing by dry heat drawing; or performing the first drawing by wet heat drawing, the second drawing by wet heat drawing, and the third drawing by dry heat drawing.

The lower limit value of the final drawing rate with respect to the undrawn yarn (or pre-drawn yarn) is preferably any of more than 1 time, 2 times or more, 3 times or more, 4 times or more, 5 times or more, 6 times or more, 7 times or more, 8 times or more, or 9 times or more, and the upper limit value is preferably 40 times or less, 30 times or less, 20 times or less, 15 times or less, 14 times or less, 13 times or less 12 times or less, 11 times or less, or 10 times or less.

<Method for producing artificial fibroin twisted yarn>

The artificial fibroin twisted yarn may obtained, for example, by a method including twisting a yarn containing the modified fibroin fiber described above (twisting step), and heating the yarn while maintaining the twist of the yarn to obtain an artificial fibroin twisted yarn (heating step).

(Twisting step)

In the twisting step, the yarn containing the modified fibroin fiber is twisted. The yarn containing the modified fibroin fiber may be either a single yarn consisting only of the modified fibroin fiber or a composite yarn composed of a combination of the modified fibroin fiber and another fiber. The above another fiber means a fiber containing no modified fibroin, and examples thereof include synthetic fibers such as nylon and polyester, regenerated fibers such as cupra and rayon, and natural fibers such as cotton and hemp. The proportion of the modified fibroin fiber in the yarn containing the modified fibroin fiber may be, for example, 5% by mass or more, 20% by mass or more, or 50% by mass or more. By setting the proportion of the modified fibroin fiber in the above range, the shrinkage rate in the shrinking processing of the artificial fibroin twisted yarn can be adjusted. The yarn containing the modified fibroin fiber may be any of a staple (a short fiber), a filament (long fiber), or a mixture thereof, but a filament is suitable.

As a means for applying twisting, a known means may be used, and for example, a twisting machine may be used. Examples of the twisting machine include twisting machines corresponding to various known twisting types (for example, an up type, a down type, a special crosspiece type, an air bar type, and a rewinder), such as an Italian type twisting machine, a double twisting machine, a ring twisting machine, a composite twisting machine, an interlace, and a panwinder. In a case where the desired artificial fibroin twisted yarn is an artificial fibroin false twisted yarn, false twisting machine such as a pin false twisting machine, a friction false twisting machine, and a belt false twisting machine may also be used as a means for applying twisting. The twisting direction may be S twist (right twist) or Z twist (left twist).

The number of twists to be applied may be, for example, 200 T/m or more, 600 T/m or more, or 800 T/m or more. By setting the number of twists applied to the yarn in the twisting step to 200 T/m or more, twisting can be more efficiently applied to the yarn. Moreover, the number of twists to be applied may be, for example, 1,200 T/m or less or 1,000 T/m or less. Here, the number of twists means how many times the yarn is rotated (that is, how many times the yarn is twisted) per 1 m of yarn. By adjusting the number of twists, so-called soft twist yarn, medium twist yarn, hard twist yarn, or very hard twist yarn can be produced. The number of twists can usually be adjusted by changing the setting of the twisting machine. In a case where the desired artificial fibroin twisted yarn is an artificial fibroin false twisted yarn, the number of twists to be applied may be, for example, 800 T/m or more, 1,000 T/m or more, or 1,200 T/m or more. By setting the number of twists in the twisting step to 800 T/m or more, twisting can be more efficiently applied to the yarn. In addition, in a case where the desired artificial fibroin twisted yarn is an artificial fibroin false twisted yarn, the number of twists may be, for example, 1,800 T/m or less or 1,600 T/m or more.

(Heating step)

The heating step is optionally conducted and is a step of heating the yarn to which twisting has been applied in the twisting step, while maintaining the twist. The twisting step and the heating step may be steps independent of each other, or steps that are conducted simultaneously, but are preferably performed simultaneously.

In the heating step, the method for maintaining the twist of the yarn is not particularly limited, and for example, the entire yarn, wound around a paper tube or the like, may be restrained with a net or the like without sagging. Alternatively, the heating step may be conducted while being twisted by a twisting machine (in-line).

The means for heating is not particularly limited, and a heater, an oven, a thermostat, or the like may be used.

The heating temperature is preferably more than 50°C, more preferably 100°C or more, still more preferably 115°C or more, even still more preferably 130°C or more. When the heating temperature is 50°C or more, fixation of the twist applied to the yarn can be made more sufficient. The heating temperature is preferably 240°C or less, more preferably 180°C or less, and still more preferably 150°C or less, from the viewpoint of suppressing the decomposition of the modified fibroin and the like. For example, the heating step may be a step of heating the yarn in an atmosphere of more than 50°C, or the step of heating the yarn in an atmosphere of 240°C or less. The heating temperature may be constant or may be adjusted to gradually increase. The heating temperature is preferably adjusted to be constant. In the present specification, the heating temperature means the ambient temperature to which the yarn is exposed, and usually means the set temperature of the heating means (for example, a heater) of the subject yarn.

The heating time is altered as appropriate depending on the state of the yarn, the heating temperature, or the like. For example, in a state where twisting is applied by a twisting machine (in-line), the heating time may be 0.1 seconds or more, 1 second or more, or 3 seconds or more. Moreover, in the state where the yarn is wound around a paper tube or the like, the heating time may be 0.5 hours or more, 1 hour or more, 3 hours or more, 6 hours or more, or 12 hours or more. When the heating time is 0.5 hours or more, twisting can be more applied to the yarn with more certainty. The heating time may also be, for example, 24 hours or less or 18 hours or less, from the viewpoint of sufficiently reducing the degradation of the modified fibroin fiber.

(Untwisting step)

In a case where the desired artificial fibroin twisted yarn is an artificial fibroin false twisted yarn, an artificial fibroin false twisted yarn may be obtained optionally by twisting (untwisting step) the artificial fibroin twisted yarn obtained in the heating step in the direction opposite to the direction in the twisting step.

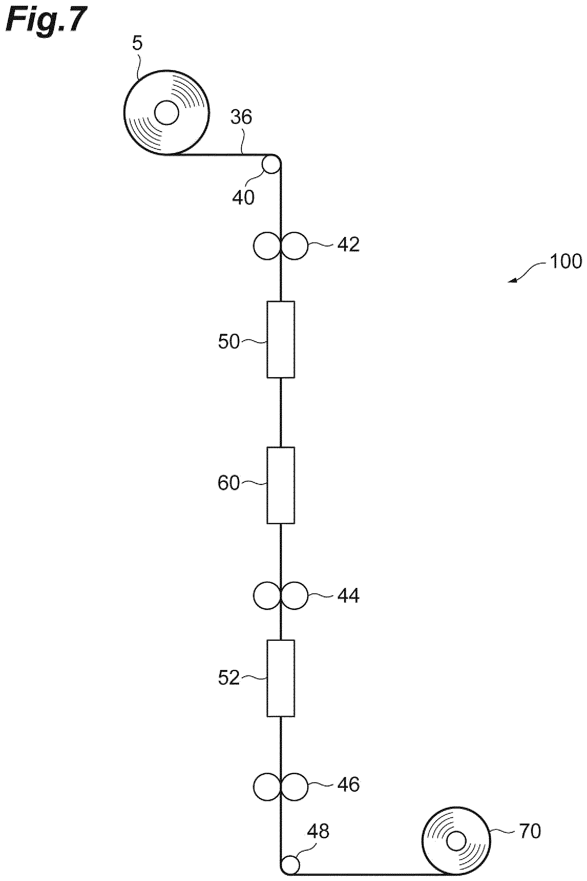

As the untwisting means, for example, a false twisting machine 100 as illustrated in Fig. 7 may be used. The false twisting machine 100 includes a delivery roller 40, a first feed roller 42, a first heater 50, a twisting unit 60, a second feed roller 44, a second heater 52, a third feed roller 46, a winding roller 48 in order from the upstream side. The modified fibroin fiber 36 (which may be a yarn containing a modified fibroin fiber) is delivered from the wound yarn body 5 to the false twisting machine through the delivery roller 40. The delivered modified fibroin fibers 36 are heated in the first heater 50 and twisting is applied thereto at the top of the twisting unit 60 while maintaining the temperature. By applying twisting to the yarn in the heated state, the twisting applied to the yarn is fixed. Next, in the lower part of the twisting unit 60, twisting is applied to the yarn in the direction opposite to the twisting direction in which the first twisting has been applied. As a result, the yarn is untwisted. The untwisted yarn is delivered to the second heater 52 by the second feed roller 44, and further, is wound around a paper tube through the third feed roller 46 and the winding roller, and finally, the desired artificial fibroin false twisted yarn is obtained as a wound product 70. During this time, it is possible to change the staying time of the yarn in the heater and the like by adjusting the driving speeds of the plurality of feed rollers.

<Artificial fibroin twisted yarn>

The artificial fibroin twisted yarn according to one embodiment includes the above-described modified fibroin and is highly shrinkable simply by being brought into contact with water. The expression "highly shrinkable" means that the shrinkage rate defined by the following Formula II is 2% or more.

$$\text{Shrinkage (\%)} = \{1- \text{(length of artificial fibroin twisted yarn subjected to shrinking processing including being brought into contact with water having a temperature lower than a boiling point / length of the artificial fibroin twisted yarn before being subjected to the shrinking processing)}\} \times 100 \cdots \text{(Formula II)}.$$

The artificial fibroin twisted yarn according to one embodiment may have a shrinkage rate of 3% or more, 4% or more, 5% or more, 7% or more, 8% or more, 10% or more, 20% or more, 30% or more, 35% or more, 37% or more, 38% or more, or 39% or more. The upper limit of the shrinkage rate is not particularly limited, but may be 80% or less, 70% or less, 60% or less, 50% or less, or 40% or less.

Further, the artificial fibroin twisted yarn according to one embodiment, including the raw material modified fibroin fiber (which means the fiber after being spun and before being twisted), is more highly shrinkable when it has no contact history with water. That is, when there no contact history with water, the artificial fibroin twisted yarn may have a shrinkage rate of 10% or more, 20% or more, 30% or more, 37% or more, 38% or more, 39% or more, 40% or more, 48% or more, 56% or more, 64% or more, or 72% or more. The upper limit of the shrinkage rate is usually 80% or less.

The artificial fibroin twisted yarn according to one embodiment may have of an LOI value of 18 or more, 20 or more, 22 or more, 24 or more, 26 or more, 28 or more, 29 or more, and 30 or more.

The artificial fibroin twisted yarn according to one embodiment may have a maximum hygroscopic heat generation, as determined according to the following Formula A, of more than 0.025°C/g.

$$\text{Formula A: Maximum hygroscopic heat generation} = \{(\text{Highest temperature of a sample when the sample has been transferred to a high humidity environment after being placed in a low humidity environment until a temperature of the sample reaches equilibrium}) - (\text{Temperature of the sample when the sample is being transferred to the high humidity environment after being placed in the low humidity environment until the temperature of the sample reaches equilibrium})\} \; (°C) \; / \; \text{Sample weight (g)}$$

In Formula A, the low-humidity environment means an environment of a temperature of 20°C and a relative humidity of 40%, and the high-humidity environment means an environment of a temperature of 20°C and a relative humidity of 90%.

The artificial fibroin twisted yarn according to one embodiment may have a maximum hygroscopic heat generation of 0.026°C/g or more, 0.027°C/g or more, 0.028°C/g or more, 0.029°C/g or more, 0.030°C/g or more, 0.035°C/g or more, and 0.040°C/g or more. The upper limit of the maximum hygroscopic heat generation is not particularly limited, but it is usually 0.060°C/g or less.

The artificial fibroin twisted yarn according to one embodiment may have a heat retention index determined according to the following Formula B of 0.20 or more.

$$\text{Formula B: Heat retention index} = \text{Heat retention rate (\%)} \; / \; \text{Basis weight of sample (g/m}^2)$$

The heat retention property index of the artificial fibroin twisted yarn according to one embodiment may be 0.22 or more, may be 0.24 or more, may be 0.26 or more, may be 0.28 or more, may be 0.30 or more, and may be 0.32 or more. The upper limit of the heat retention index is not particularly limited, but may be, for example, 0.60 or less, or 0.40 or less.

The composition of the artificial fibroin twisted yarn is the same as the composition of the yarn containing the modified fibroin fiber described above. In addition, the artificial fibroin twisted yarn may be any one of a staple, a filament, or a mixture thereof, but a filament is suitable.

The number of twists of the artificial fibroin twisted yarn may be, for example, 200 T/m or more, 600 T/m or more, or 800 T/m or more, and may be 1,200 T/m or less or 1,000 T/m or less. The artificial fibroin twisted yarn may be classified into so-called soft twist yarn, medium twist yarn, hard twist yarn, or very hard twist yarn, depending on the number of twists thereof.

In one embodiment, the artificial fibroin twisted yarn may be an artificial fibroin false twisted yarn. In this case, the number of twists of the artificial fibroin false twisted yarn may be, for example, 1,800 T/m or less or 1,600 T/m or less, and the artificial fibroin false twisted yarn may also have no twist (that is, the number of twists may be 0 T/m).

(Shrinking Processing)

The shrinking processing includes bringing the artificial fibroin twisted yarn into contact with water having a temperature lower than the boiling point (hereinafter, also referred to as "contacting step"). By bringing an artificial fibroin twisted yarn into contact with water having a temperature lower than the boiling point, the artificial fibroin twisted yarn can be shrunk without depending on an external force. In contacting step, it is considered that the shrinkage of the artificial fibroin twisted yarn occurs due to reasons as follows. That is, one reason is considered to be due to the secondary structure or tertiary structure of the modified fibroin included in the artificial fibroin twisted yarn. Another reason is considered to be due to the relaxation of residual stress by infiltration of water between the fibers or into the fibers of the modified fibroin fiber having the residual stress caused by drawing in the production step or the like. Accordingly, it is considered that the shrinkage rate of the artificial fibroin twisted yarn in the shrinking processing can be controlled by, for example, adjusting the drawing rate in the production process of the modified fibroin fiber.

The temperature of the water to be brought into contact with the artificial fibroin twisted yarn in the contacting step is lower than the boiling point. At such a temperature, the handling and workability of shrinking processing, and the like are improved. In addition, from the viewpoint of sufficiently shortening the shrinkage time, the lower limit value of the water temperature is preferably 10°C or higher, more preferably 40°C or higher, and further preferably 70°C or higher. The upper limit value of the water temperature is preferably 90°C or less.

In the contacting step, the method for bringing an artificial fibroin twisted yarn into contact with water is not particularly limited. Examples of the method for bringing an artificial fibroin twisted yarn into contact with water include a method that involves immersing an artificial fibroin twisted yarn in water, a method that involves spraying water in a steam state or the like that is at ordinary temperature or heated to an artificial fibroin twisted yarn, and a method that involves exposing an artificial fibroin twisted yarn to a high-humidity environment filled with water vapor. Among these methods, a method that involves immersing an artificial fibroin twisted yarn in water is preferable in the contacting step since the shrinkage time can be effectively shortened and the processing facilities can be simplified.

In addition to the shrinking processing, the contacting step may further include drying the artificial fibroin twisted yarn after being brought into contact with water (hereinafter, also referred to as "drying step").

The drying may be, for example, air drying or forced drying using drying equipment. As the drying equipment, any known contact type or non-contact type drying facilities may be used. The drying temperature is not particularly limited as long as it is lower than the temperature at which the protein contained in the artificial fibroin twisted yarn is decomposed or the modified fibroin is thermally damaged. The drying temperature is generally in the range of 20°C to 150°C and preferably in the range of 50°C to 100°C. When the temperature is in this range, the artificial fibroin twisted yarn is dried more rapidly and efficiently without causing the thermal damage of the modified fibroin or the decomposition of the protein contained in the artificial fibroin twisted yarn. The drying time is set as appropriate depending on the drying temperature and the like, and for example, a time in which the influence of overdrying on the quality and physical properties of the high-shrinkage artificial fibroin twisted yarn can be excluded as much as possible may be adopted.

As described above, the shrinkage rate of the artificial fibroin twisted yarn according to one embodiment may be controlled by adjusting the drawing rate in the production process of the modified fibroin fiber and the amount of the modified fibroin fiber contained in the artificial fibroin twisted yarn. In addition, the shrinkage rate of the artificial fibroin twisted yarn may also be controlled by adjusting the number of twists of the artificial fibroin twisted yarn, the temperature of the water to be brought into contact with the artificial fibroin twisted yarn, the time of contacting with water, and the degree of tensile force which may be optionally applied to the artificial fibroin twisted yarn in the shrinking processing. In a case where the shrinking processing is performed in batch system, the tensile force may be applied, for example, by pulling the artificial fibroin twisted yarn in the direction of the yarn axis or by fixing both ends of the artificial fibroin twisted yarn to the predetermined frame body upon bringing the artificial fibroin twisted yarn into contact with water or drying the artificial fibroin twisted yarn. In a case where the contacting step and the drying step in the shrinking processing are successively performed, more specifically, in a case where the artificial fibroin twisted yarn is brought into contact with water and successively dried while being run between the delivery roller and the winding roller, the tensile force applied to the artificial fibroin twisted yarn may be controlled by adjusting the delivery speed and the winding speed. By adjusting the winding speed to a speed slower than the delivery speed, a higher shrinkage rate can be achieved.

<High-shrinkage artificial fibroin twisted yarn and method for producing same>

Since the artificial fibroin twisted yarn according to one embodiment is highly shrinkable, a high-shrinkage artificial fibroin twisted yarn may be obtained, for example, by undergoing the above-described shrinking processing (the contacting step and the drying step as necessary). Accordingly, in one embodiment, the high-shrinkage artificial fibroin twisted yarn may be produced by a method including a step of shrinking an artificial fibroin twisted yarn containing a modified fibroin by bringing the artificial fibroin twisted yarn into contact with water. Shrinking step may be performed in the same manner as the shrinking processing described above.

The high-shrinkage artificial fibroin twisted yarn according to one embodiment has a shrinkage rate defined by the following Formula I of 2% or more.

$$\text{Shrinkage rate (\%)} = \{1- (\text{length of a shrunk artificial fibroin twisted yarn / length of the artificial fibroin twisted yarn after being twisted and before being brought into contact with water})\} \times 100 \cdots (\text{Formula I}).$$

The high-shrinkage artificial fibroin twisted yarn according to one embodiment may have a shrinkage rate of 3% or more, 4% or more, 5% or more, 7% or more, 8% or more, 10% or more, 20% or more, 30% or more, 35% or more, 37% or more, 38% or more, or 39% or more. The upper limit of the shrinkage rate is not particularly limited, but may be 80% or less, 70% or less, 60% or less, 50% or less, or 40% or less.

Further, the twisted yarn according to one embodiment, including the raw material modified fibroin fiber (which means the fiber after being spun and before being twisted), is more highly shrinkable when it has no contact history with water. That is, when there no contact history with water, the artificial fibroin twisted yarn may have a shrinkage rate of 10% or more, 20% or more, 30% or more, 37% or more, 38% or more, 39% or more, 40% or more, 48% or more, 56% or more, 64% or more, or 72% or more. The upper limit of the shrinkage rate is usually 80% or less.

The high-shrinkage artificial fibroin twisted yarn according to one embodiment may have of an LOI value of 18 or more, 20 or more, 22 or more, 24 or more, 26 or more, 28 or more, 29 or more, and 30 or more.

The high-shrinkage artificial fibroin twisted yarn according to one embodiment may have a maximum hygroscopic heat generation, as determined according to the following Formula A, of more than 0.025°C/g.

$$\text{Formula A: Maximum hygroscopic heat generation} = \{(\text{Highest temperature of a sample when the sample has been transferred to a high humidity environment after being placed in a low humidity environment until a temperature of the sample reaches equilibrium}) - (\text{Temperature of the sample when the sample is being transferred to the high humidity environment after being placed in the low humidity environment until the temperature of the sample reaches equilibrium})\} (°C) / \text{Sample weight (g)}$$

In Formula A, the low-humidity environment means an environment of a temperature of 20°C and a relative humidity of 40%, and the high-humidity environment means an environment of a temperature of 20°C and a relative humidity of 90%.

The high-shrinkage artificial fibroin twisted yarn according to one embodiment may have a maximum hygroscopic heat generation of 0.026°C/g or more, 0.027°C/g or more, 0.028°C/g or more, 0.029°C/g or more, 0.030°C/g or more, 0.035°C/g or more, and 0.040°C/g or more. The upper limit of the maximum hygroscopic heat generation is not particularly limited, but it is usually 0.060°C/g or less.

The high-shrinkage artificial fibroin twisted yarn according to one embodiment may have a heat retention index determined according to the following Formula B of 0.20 or more.

$$\text{Formula B: Heat retention index} = \text{Heat retention rate (\%)} / \text{Basis weight of sample (g/m}^2)$$

The heat retention property index of the high-shrinkage artificial fibroin twisted yarn according to one embodiment may be 0.22 or more, may be 0.24 or more, may be 0.26 or more, may be 0.28 or more, may be 0.30 or more, and may be 0.32 or more. The upper limit of the heat retention index is not particularly limited, but may be, for example, 0.60 or less, or 0.40 or less.

[Method for shrinking artificial fibroin twisted yarn]

The method for shrinking an artificial fibroin twisted yarn according to one embodiment is a method including a step of shrinking an artificial fibroin twisted yarn containing a modified fibroin by bringing the artificial fibroin twisted yarn into contact with water. The shrinking step may further include drying the artificial fibroin twisted yarn after being brought into contact with the water. That is, the method for shrinking an artificial fibroin twisted yarn may be carried out in the same manner as the shrinking processing described above.

**Examples**

Hereinafter, the present invention will be described more specifically based on Examples and the like. However, the present invention is not limited to Examples below.

[Test Example 1: Production of modified fibroin (modified spider silk fibroin)]

(1) Preparation of expression vector for modified fibroin

A modified fibroin having the amino acid sequence set forth in SEQ ID NO: 18 (PRT399), a modified fibroin having the amino acid sequence set forth in SEQ ID NO: 12 (PRT380), a modified fibroin having the amino acid sequence set forth in SEQ ID NO: 13 (PRT410), and a modified fibroin (PRT799) having the amino acid sequence set forth in SEQ ID NO: 15 were designed.

Nucleic acid each encoding a protein having the designed amino acid sequence was synthesized. To the nucleic acid, an NdeI site was added to the 5' terminal and an EcoRI site was added downstream of the stop codon. These five nucleic acids were individually cloned into a cloning vector (pUC118). Thereafter, each of the nucleic acids was enzymatically cleaved by treatment with NdeI and EcoRI, and then recombinated into a protein expression vector pET-22b (+) to obtain an expression vector.

(2) Formula of modified fibroin

Escherichia coli BLR (DE3) was transformed with each of the obtained recombinated expression vectors. The transformed Escherichia coli was cultured in 2 mL of an LB medium containing ampicillin for 15 hours. The culture solution was added to 100 mL of a seed culture medium (Table 4) containing ampicillin so that the $OD_{600}$ was 0.005. While maintaining the temperature of the culture solution at 30°C, flask culturing was performed (for about 15 hours) until the $OD_{600}$ reached 5, thereby obtaining a seed culture solution.

[Table 4]

| Seed culture medium | |
| --- | --- |
| Reagent | Concentration (g/L) |
| Glucose | 5.0 |
| $KH_2PO_4$ | 4.0 |
| $K_2HPO_4$ | 9.3 |
| Yeast Extract | 6.0 |
| Ampicillin | 0.1 |

To a jar fermenter to which 500 mL of a production medium (Table 5) had been added, the seed culture solution was added so that the $OD_{600}$ was 0.05. The culture was carried out while keeping the culture solution temperature at 37°C and controlling the pH constant at 6.9. Further, the concentration of dissolved oxygen in the culture solution was maintained at 20% of the dissolved oxygen saturation concentration.

[Table 5]

| Production medium | |
| --- | --- |
| Reagent | Concentration (g/L) |
| Glucose | 12.0 |
| $KH_2PO_4$ | 9.0 |
| $MgSO_4 \cdot 7H_2O$ | 2.4 |
| Yeast Extract | 15 |
| $FeSO_4 \cdot 7H_2O$ | 0.04 |
| $MnSO_4 \cdot 5H_2O$ | 0.04 |
| $CaCl_2 \cdot 2H_2O$ | 0.04 |
| ADEKA NOL (ADEKA, LG-295S) | 0.1 (mL/L) |

Immediately after glucose in the production medium was completely consumed, a feed solution (455 g/1 L of glucose and 120 g/1 L of Yeast Extract) was added at a rate of 1 mL/min. The culture was carried out while keeping the culture solution temperature at 37°C and controlling the pH constant at 6.9. The culture was performed for 20 hours, while maintaining the concentration of dissolved oxygen in the culture solution at 20% of the dissolved oxygen saturation concentration. Thereafter, 1 M isopropyl-β-thiogalactopyranoside (IPTG) was added to the culture solution at a final concentration of 1 mM to induce the expression of the modified fibroin of interest. 20 hours after the addition of IPTG, the culture solution was centrifuged to recover the bacterial cell pellet. SDS-PAGE was performed using bacterial cell pellets prepared from the culture solution before the addition of IPTG and after the addition of IPTG, and the expression of the modified fibroin of interest was confirmed by the IPTG addition-dependent appearance of a band corresponding to the size of the modified fibroin of interest.

(3) Purification of modified fibroin

The bacterial cell pellet recovered after 2 hours from the addition of IPTG was washed with a 20 mM Tris-HCl buffer solution (pH 7.4). The bacterial cell pellet after washing was suspended in a 20 mM Tris-HCl buffer solution (pH 7.4) containing about 1 mM PMSF, and the cell suspension was disrupted with a high-pressure homogenizer (manufactured by GEA Niro Soavi SpA). The disrupted cells were centrifuged to obtain a precipitate. The obtained precipitate was washed with a 20 mM Tris-HCl buffer solution (pH 7.4) until the obtained precipitate became highly pure. The precipitate after washing was suspended in an 8 M guanidine buffer solution (8 M guanidine hydrochloride, 10 mM sodium dihydrogen phosphate, 20 mM NaCl, 1 mM Tris-HCl, pH 7.0) so that the concentration was 100 mg/mL, and dissolved by stirring with a stirrer at 60°C for 30 minutes. After dissolution, dialysis was carried out in water using a dialysis tube (cellulose tube 36/32 manufactured by Sanko Junyaku Co., Ltd.). The white aggregated protein obtained after dialysis was recovered by centrifugation. The aqueous moisture was removed from the recovered aggregated protein with a freeze dryer to obtain each freeze-dried powder of the modified fibroins (PRT399, PRT380, PRT410, and PRT799) of interest.

[Test Example 2: Production of modified fibroin fiber and evaluation of shrinkability]

Freeze-dried powders of the modified fibroins (PRT399, PRT380, PRT410, and PRT799) was obtained in the same manner as in Test Example 1. Dimethyl sulfoxide (DMSO) in which LiCl was dissolved to be a concentration of 4.0% by mass was prepared as a solvent, and each of the freeze-dried powder of the modified fibroin (PRT399, PRT380, PRT410, and PRT799) was added thereto be a concentration of 18% by mass or 24% by mass (see Table 6) and dissolved for 3 hours using a shaker. Thereafter, insoluble bodies and bubbles were removed, whereby a modified fibroin solution was obtained.

With the obtained modified fibroin solution used as a doping liquid (spinning stock solution), a modified fibroin fiber that is spun and drawn was manufactured by dry-wet type spinning using a spinning device based on the spinning device 10 illustrated in Fig.6. The spinning device used is a spinning device further having an additional second undrawn yarn manufacturing device (second bath) between the undrawn yarn manufacturing device 2 (first bath) and a wet heat drawing device 3 (third bath) in the spinning device 10 illustrated in Fig. 6. The dry-wet type spinning conditions are as follows.

Extrusion nozzle diameter: 0.2 mm
Liquid and temperature in the first to third bath: see Table 6
Total drawing rate: see Table 6
Drying temperature: 60°C

[Table 6]

| | Doping liquid | | First bath | | Second bath | | Third bath | | Total drawing rate (times) |
|---|---|---|---|---|---|---|---|---|---|
| | Modified fibroin | Concentration (% by mass) | Liquid | Temperature (°C) | Liquid | Temperature (°C) | Liquid | Temperature (°C) | |
| Production Example 1 | PRT799 | 24 | | -5 | | 16 | | | 1 |
| Production Example 2 | | | | | | | | | 2 |
| Production Example 3 | | | | | | | | | 3 |
| Example | | | | | | | | | 4 |
| Production Example 5 | | 18 | | | | | | | 1 |
| Production Example 6 | | | | | | | | | 2 |
| Production Example 7 | | | | | | | | | 3 |
| Production Example 8 | | | | | | | | | 4 |

| | Doping liquid | | First bath | | Second bath | | Third bath | | Total drawing rate (times) |
|---|---|---|---|---|---|---|---|---|---|
| | Modified fibroin | Concentration (% by mass) | Liquid | Temperature (°C) | Liquid | Temperature (°C) | Liquid | Temperature (°C) | |
| Production Example 9 | PRT410 | 24 | 100 % Methanol | -11 | 100% Methanol | 14 | Water | 17 | 1 |
| Production Example 10 | | | | | | | | | 2 |
| Production Example 11 | | | | | | | | | 3 |
| Production Example 12 | | | | | | | | | 4 |
| Production Example 13 | PRT399 | | | | | | | | 1 |
| Production Example 14 | | | | | | | | | 2 |
| Production Example 15 | | | | | | | | | 3 |
| Production Example 16 | PRT380 | | | | | 11 | | | 1 |
| Production Example 17 | | | | | | | | | 2 |
| Production Example 18 | | | | | | | | | 3 |
| Production Example 19 | | | | | | | | | 4 |

EP 3 789 525 A1

(Evaluation of shrinkability)

The shrinkage rate of each modified fibroin fiber obtained in Production Examples 1 to 19 was evaluated. That is, with respect to each of the modified fibroin fibers, a shrinkage rate (hereinafter, may be referred to as "primary shrinkage rate") when subjected to a Shrinking Processing 1 composed of bringing into contact with water having a temperature lower than the boiling point, and a shrinkage rate (hereinafter, may be referred to as "secondary shrinkage rate") when subjected to a Shrinking Processing 2 composed of drying in room temperature after bringing into contact with water having the temperature lower than a boiling point were evaluated.

<Primary shrinkage rate>

A plurality of modified fibroin fibers for test, each having a length of 30 cm, were cut out from each of the wound products of the modified fibroin fibers obtained in Production Examples 1 to 19. The plurality of modified fibroin fibers were bundled to obtain a modified fibroin fiber bundle having a fineness of 150 denier. With 0.8 g of lead weight attached to each of the modified fibroin fiber bundles, each of the modified fibroin fiber bundles was immersed in water having a temperature shown in Tables 7 to 10 for 10 minutes (Shrinking Processing 1). Thereafter, the length of each of the modified fibroin fiber bundles was measured in water. The measurement of the length of the modified fibroin fiber bundle in water was carried out with the lead weight of 0.8 g attached to the modified fibroin fiber bundle, in order to eliminate the crimping of the modified fibroin fiber bundle. Subsequently, the primary shrinkage rate (%) of each of the modified fibroin fibers was calculated according to the following Formula III. In Formula III, L0 represents the length (here, 30 cm) of the modified fibroin fiber bundle before being subjected to the shrinking processing and Lw represents the length of the modified fibroin fiber bundle that underwent Shrinking Processing 1.

$$\text{Shrinkage rate (primary shrinkage rate) (\%)} = \{1 - (Lw/L0)\} \times 100 \cdots \text{(Formula III)}$$

<Secondary shrinkage rate>

After immersion in water for the evaluation of the primary shrinkage rate, the modified fibroin fiber bundle was taken out of the water. The modified fibroin fiber bundle taken out was dried at room temperature for 2 hours with 0.8 g lead weight attached (Shrinking Processing 2). After drying, the length of each of the modified fibroin fiber bundles was measured. Subsequently, the secondary shrinkage rate (%) of each of the modified fibroin fibers was calculated according to the following Formula IV. In Formula IV, L0 represents the length (here, 30 cm) of the modified fibroin fiber bundle before being subjected to the shrinking processing and Lwd represents the length of the modified fibroin fiber bundle that underwent Shrinking Processing 2.

$$\text{Shrinkage rate (secondary shrinkage rate) (\%)} = \{1 - (Lwd/L0)\} \times 100 \cdots \text{(Formula IV)}$$

The results are shown in Tables 7 to 10.

[Table 7]

| Modified fibroin fiber | | Temperature of water having temperature lower than boiling point (°C) | Primary shrinkage rate (%) | Secondary shrinkage rate (%) |
|---|---|---|---|---|
| Production Example 1 | 24wt% PRT799 ×1 | 20 | 0.0 | 7.8 |
| Production Example 2 | 24wt% PRT799 ×2 | | -1.2 | 10.3 |
| Production Example 3 | 24wt% PRT799 ×3 | | 7.2 | 21.2 |
| Production Example 4 | 24wt% PRT799 ×4 | | 13.5 | 26.3 |
| Production Example 6 | 18wt% PRT799 ×2 | | -2.3 | 9.5 |
| Production Example 7 | 18wt% PRT799 ×3 | | 6.0 | 19.7 |
| Production Example 8 | 18wt% PRT799 ×4 | | 14.3 | 27.5 |
| Production Example 2 | 24wt% PRT799 ×2 | 40 | -5.3 | 7.2 |
| Production Example 3 | 24wt% PRT799 ×3 | | 8.7 | 21.3 |
| Production Example 4 | 24wt% PRT799 ×4 | | 14.5 | 26.0 |
| Production Example 6 | 18wt% PRT799 ×2 | | -4.3 | 7.3 |
| Production Example 7 | 18wt% PRT799 ×3 | | 6.2 | 18.3 |
| Production Example 8 | 18wt% PRT799 ×4 | | 16.0 | 28.7 |
| Production Example 3 | 24wt% PRT799 ×3 | 60 | 6.8 | 21.0 |
| Production Example 4 | 24wt% PRT799 ×4 | | 15.0 | 27.5 |
| Production Example 6 | 18wt% PRT799 ×2 | | -1.5 | 10.7 |
| Production Example 7 | 18wt% PRT799 ×3 | | 3.3 | 18.2 |
| Production Example 8 | 18wt% PRT799 ×4 | | 16.2 | 29.0 |

[Table 8]

| Modified fibroin fiber | | Temperature of water having temperature lower than boiling point (°C) | Primary shrinkage rate (%) | Secondary shrinkage rate (%) |
|---|---|---|---|---|
| Production Example 10 | 24wt% PRT410 ×2 | 20 | -2.3 | 8.7 |
| Production Example 11 | 24wt% PRT410 ×3 | | 4.7 | 16.7 |
| Production Example 12 | 24wt% PRT410 ×4 | | 10.3 | 22.3 |
| Production Example 11 | 24wt% PRT410 ×3 | 40 | 4.7 | 17.5 |
| Production Example 12 | 24wt% PRT410 ×4 | | 11.5 | 24.0 |
| Production Example 11 | 24wt% PRT410 ×3 | 60 | 2.0 | 16.5 |
| Production Example 12 | 24wt% PRT410 ×4 | | 10.8 | 25.0 |

[Table 9]

| Modified fibroin fiber | | Temperature of water having temperature lower than boiling point (°C) | Primary shrinkage rate (%) | Secondary shrinkage rate (%) |
|---|---|---|---|---|
| Production Example 13 | 24wt% PRT399 ×1 | 20 | -3.5 | 7.6 |
| Producti on Example 14 | 24wt% PRT399 ×2 | | 3.7 | 12.5 |
| Production Example 15 | 24wt% PRT399 ×3 | | 7.0 | 16.8 |
| Production Example 14 | 24wt% PRT399 ×2 | 40 | 3.0 | 12.7 |
| Production Example 15 | 24wt% PRT399 ×3 | | 7.3 | 16.7 |
| Production Example 14 | 24wt% PRT399 ×2 | 60 | 3.3 | 9.3 |
| Production Example 15 | 24wt% PRT399 ×3 | | 6.8 | 14.2 |

EP 3 789 525 A1

[Table 10]

| Modified fibroin fiber | | Temperature of water having temperature lower than boiling point (°C) | Primary shrinkage rate (%) | Secondary shrinkage rate (%) |
|---|---|---|---|---|
| Production Example 16 | 24wt% PRT380 ×1 | 20 | -1.1 | 9.4 |
| Production Example 17 | 24wt% PRT380 ×2 | | 2.7 | 13.3 |
| Production Example 18 | 24wt% PRT380 ×3 | | 7.0 | 17.7 |
| Production Example 19 | 24wt% PRT380 ×4 | | 10.0 | 20.2 |
| Production Example 17 | 24wt% PRT380 ×2 | 40 | 3.3 | 14.2 |
| Production Example 18 | 24wt% PRT380 ×3 | | 7.7 | 19.0 |
| Production Example 19 | 24wt% PRT380 ×4 | | 12.0 | 22.0 |
| Production Example 17 | 24wt% PRT380 ×2 | 60 | 2.7 | 14.3 |
| Production Example 18 | 24wt% PRT380 ×3 | | 8.2 | 20.3 |
| Production Example 19 | 24wt% PRT380 ×4 | | 12.0 | 23.2 |

From the above results, it can be seen that the modified fibroin fiber according to the present invention has a sufficiently high shrinkage rate. Accordingly, it is recognized that the artificial fibroin twisted yarn obtained from the yarn containing the modified fibroin fiber according to the present invention also has a sufficiently high shrinkage rate. In addition, the shrinkage rate of the modified fibroin fiber and the artificial fibroin twisted yarn according to the present invention can be controlled, for example, by controlling the drawing rate in the production process of the modified fibroin fiber, the temperature of the water to be brought into contact, and the time of contacting with water, and the degree of tensile force which may be optionally applied to the artificial fibroin twisted yarn in the shrinking processing.

[Test Example 3: Production and evaluation of twisted yarn]

(1) Example: High-shrinkage twisted yarn

A freeze-dried powder of the modified fibroin (PRT799) was obtained in the same manner as in Test Example 1. DMSO in which LiCl was dissolved to be a concentration of 4.0% by mass was prepared a solvent, and the freeze-dried powder of the modified fibroin (PRT799) was added thereto be a concentration of 24% by mass and dissolved for 3 hours using a shaker. Thereafter, insoluble bodies and bubbles were removed, whereby a modified fibroin solution was obtained. Dry-wet type spinning was carried out using a spinning device based on the spinning device 10, thereby obtaining a modified spider silk fibroin fiber (a multifilament of 24 fibers). The dry-wet type spinning conditions are as follows.

Temperature of coagulation liquid (methanol): 5°C to 10°C
Drawing rate: 5 times
Drying temperature: 80°C

Twisted yarns 1 to 4 were obtained from the modified spider silk fibroin fibers using a known Italian type twisting machine. The twisted yarn 4 (Example 4) was obtained by heating the modified fibroin fiber at 240°C for 2 seconds after spinning, and then twisting. Table 11 shows the number of twists and the fineness of each twisted yarn.

The twisted yarns 1 to 4 cut out to a length of 60 cm were each immersed in water at 40°C for 1 minute and then air-dried. Thereafter, the lengths of the twisted yarns 1 to 4 were measured, and the shrinkage rate was determined according to the following Formula V.

$$\text{Shrinkage rate (\%)} = \{1 - (\text{length of twisted yarn after air drying}$$
$$/ \text{ length of the twisted yarn before immersion in water})\} \times 100 \cdots$$
$$\text{(Formula V)}$$

The results are shown in Table 11. The high-shrinkage artificial fibroin twisted yarns of Examples had excellent touch texture and flexibility.

[Table 11]

| | Twisted yarn | Number of twists (T/m) | Fineness (denier) | Length before shrinkage (cm) | Length after shrinkage (cm) | Shrinkage rate |
|---|---|---|---|---|---|---|
| Example 1 | Twisted yarn 1 | 606 | 160 | 60 | 54 | 10.0% |
| Example 2 | Twisted yarn 2 | 300 | 160 | 60 | 37.6 | 37.3% |
| Example 3 | Twisted yarn 3 | 606 | 160 | 60 | 36.7 | 38.8% |
| Example 4 | Twisted yarn 4 | 914 | 160 | 60 | 36.2 | 39.7% |
| Reference Example | - | 0 | 157 | 60 | 37.5 | 37.5% |
| Comparative Example 1 | Twisted yarn 5 | 500 | 32 | 15 | 15 | 0.0% |

(2) Reference Example: Non-twisted yarn

Instead of the twisted yarn, the shrinkage rate of the untwisted yarn composed of a bundle of 24 fibers of the modified spider silk fibroin fiber obtained in the same manner as in Example was determined in the same manner as in Examples. The results are shown in Table 11.

(3) Comparative Example: polyester twisted yarn

The shrinkage rate of a commercially available twisted yarn formed of a polyester fiber instead of the twisted yarn of the modified spider silk fibroin fiber was determined in the same manner as in Examples. The results are shown in Table 11.

[Test Example 4: Evaluation of flame retardancy of modified fibroin]

A freeze-dried powder of the modified fibroin (PRT799) was obtained in the same manner as in Test Example 1. PRT799 is a hydrophilic modified fibroin having an average HI of 0 or less.

The freeze-dried powder of the modified fibroin (PRT799) was added to a DMSO solution containing LiCl (concentration: 4.0% by mass) such that the concentration of the modified fibroin was 24% by mass, and mixed for 3 hours to be dissolved using a shaker. Then, insoluble bodies and bubbles were removed, whereby a modified fibroin solution (spinning stock solution) was obtained.

The spinning stock solution was filtered at 90°C with a metal filter having a mesh size of 5 $\mu$m, and then allowed to be left in a 30 mL stainless steel syringe, subjected to defoaming, and then discharged from a solid nozzle having a needle diameter of 0.2 mm into 100% by mass of methanol in the coagulation bath. The discharge temperature was 90°C. After coagulation, the obtained raw yarn was wound and subjected to air drying to obtain a modified spider silk fibroin fiber (raw material fiber).

A knitted fabric (thickness: 180 deniers, gauge number: 18) was manufactured by circular knitting using a circular knitting machine, using a twisted yarn in which raw material fibers were twisted with each other. 20 g of the obtained knitted fabric was cut out and used as a test piece.

A flammability test was performed in accordance with "Test Method for Granular or Low-Melting-Point Synthetic Resin" described in "Notice No. 50 of the Office of Hazardous Materials Regulation (as of May 31, 1995)". The test was performed under the conditions of a temperature of 22°C, a relative humidity of 45%, and an atmospheric pressure of 1021 hPa. The measurement results (Oxygen concentration (%), Combustion rate (%), and Converted combustion rate (%)) are shown in Table 12.

[Table 12]

| Oxygen concentration (%) | Combustion rate (%) | Converted combustion rate (%) |
| --- | --- | --- |
| 20.0 | 39.1 | 40.1 |
| 27.0 | 48.1 | 49.3 |
| 28.0 | 51.9 | 53.2 |
| 30.0 | 53.6 | 54.9 |
| 50.0 | 61.2 | 62.7 |
| 70.0 | 91.1 | 93.3 |
| 100.0 | 97.6 | 100.0 |

As a result of the flammability test, the knitted fabric knitted with the modified spider silk fibroin (PRT799) fiber had a limiting oxygen index (LOI) value of 27.2. Generally, LOI value of 26 or more is considered as having flame retardancy. It can be seen that the modified spider silk fibroin has excellent flame retardancy.

[Test Example 5: Evaluation of hygroscopic exothermicity of modified fibroin]

Freeze-dried powders of the modified fibroins (PRT918 and PRT799) were obtained in the same manner as in Test Example 1. PRT918 is a hydrophobic modified fibroin having the amino acid sequence set forth in SEQ ID NO: 37 and having an average HI of more than 0.

The freeze-dried powders of the modified fibroins (PRT918 and PRT799) were added to a DMSO solution containing LiCl (concentration: 4.0% by mass) such that the concentration of the modified fibroin was 24% by mass, and mixed for 3 hours to be dissolved using a shaker. Then, insoluble bodies and bubbles were removed, whereby a modified fibroin solution (spinning stock solution) was obtained.

The spinning stock solution was filtered at 60°C with a metal filter having a mesh size of 5 $\mu$m, and then allowed to be left in a 30 mL stainless steel syringe, subjected to defoaming, and then discharged from a solid nozzle having a needle diameter of 0.2 mm into 100% by mass of methanol in the coagulation bath. The discharge temperature was 60°C. After coagulation, the obtained raw yarn was wound and subjected to air drying to obtain a modified spider silk fibroin fiber (raw material fiber).

For comparison, commercially available wool fibers, cotton fibers, Tencel fibers, rayon fibers, and polyester fibers were prepared as raw material fibers.

Each raw material fiber was used to produce a knitted fabric by flat knitting using a flat knitting machine. Table 13 shows the thickness and the gauge number of the knitted fabric obtained by using the PRT918 fiber or the PRT799 fiber. The thickness and the gauge number of the knitted fabric using other raw material fibers were adjusted so that the cover factor was almost the same as the cover factor of the knitted fabric of the modified fibroin fiber. Specifically, the numerical values are as shown in Table 13.

[Table 13]

| Raw material fiber | Thickness [N] | Gauge number [GG] |
|---|---|---|
| PRT918 | 1/30 (metric count single yarn) | 18 |
| PRT799 | 1/30 (metric count single yarn) | 16 |
| Wool | 2/30 (two folded yarn) | 14 |
| Cotton | 2/34 (two folded yarn) | 14 |
| Tencel | 2/30 (two folded yarn) | 15 |
| Rayon | 1/38 (single yarn) | 14 |
| Polyester | 1/60 (single yarn) | 14 |

Two pieces of knitted fabric cut into 10 cm × 10 cm were put together and the four sides were sewn together to obtain a test piece (sample). After exposing the test piece to a low-humidity environment (temperature of 20°C ± 2°C, relative humidity of 40% ± 5%) for 4 hours or more, the test piece was transferred to a high-humidity environment (temperature of 20°C ± 2°C, relative humidity of 90% ± 5%), and the temperature was measured at an interval of 1 minute for 30 minutes with a temperature sensor attached in the center of the inside of the test piece.

From the measurement results, the maximum hygroscopic heat generation was determined according to the following Formula A.

Formula A: Maximum hygroscopic heat generation = {(Highest temperature of a sample when the sample has been transferred to a high humidity environment after being placed in a low humidity environment until a temperature of the sample reaches equilibrium) - (Temperature of the sample when the sample is being transferred to the high humidity environment after being placed in the low humidity environment until the temperature of the sample reaches equilibrium)} (°C) / Sample weight (g)

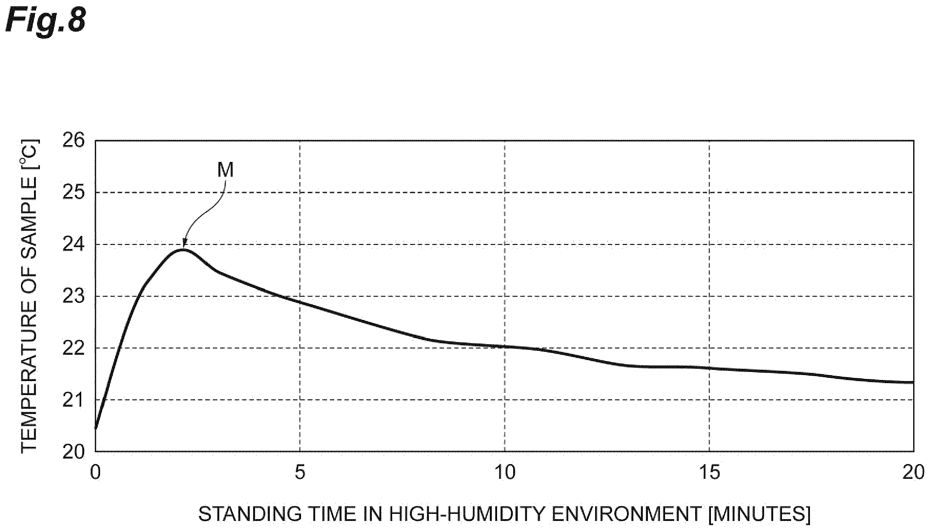

Fig. 8 is a graph showing one example of results of a hygroscopic exothermicity test. The horizontal axis of the graph indicates the time (minutes) left in the high-humidity environment, where 0 is the time when the sample was transferred from the low-humidity environment to the high-humidity environment. The vertical axis of the graph indicates the temperature (sample temperature) measured by the temperature sensor. In the graph shown in Fig. 8, a point indicated as M corresponds to a highest value of the sample temperature.

The results of calculation for the maximum hygroscopic heat generation are shown in Table 14.

[Table 14]

| Raw material fiber | Maximum hygroscopic heat generation (° C/g) |
|---|---|
| PRT918 | 0.040 |
| PRT799 | 0.031 |
| Wool | 0.020 |

(continued)

| Raw material fiber | Maximum hygroscopic heat generation (° C/g) |
|---|---|
| Cotton | 0.021 |
| Tencel | 0.018 |
| Rayon | 0.025 |
| Polyester | 0.010 |

As shown in Table 14, it can be seen that the modified spider silk fibroin (PRT918 and PRT799) fibers have a high value of the maximum hygroscopic heat generation and excellent in hygroscopic exothermicity, as compared with the existing materials.

[Test Example 6: Evaluation of heat retention property of modified fibroin]

Freeze-dried powders of the modified fibroins (PRT966 and PRT799) were obtained in the same manner as in Test Example 1. PRT966 is a hydrophobic modified fibroin having the amino acid sequence set forth in SEQ ID NO: 40 and having an average HI of more than 0.

The freeze-dried powders of the modified fibroins (PRT966 and PRT799) were added to a DMSO solution containing LiCl (concentration: 4.0% by mass) such that the concentration of the modified fibroin was 24% by mass, and mixed for 3 hours to be dissolved using a shaker. Then, insoluble bodies and bubbles were removed, whereby a modified fibroin solution (spinning stock solution) was obtained.

The spinning stock solution was filtered at 60°C with a metal filter having a mesh size of 5 $\mu$m, and then allowed to be left in a 30 mL stainless steel syringe, subjected to defoaming, and then discharged from a solid nozzle having a needle diameter of 0.2 mm into 100% by mass of methanol in the coagulation bath. The discharge temperature was 60°C. After coagulation, the obtained raw yarn was wound and subjected to air drying to obtain a modified spider silk fibroin fiber (raw material fiber).

For comparison, commercially available wool fibers, silk fibers, cotton fibers, rayon fibers, and polyester fibers were prepared as raw material fibers.

Each raw material fiber was used to produce a knitted fabric by flat knitting using a flat knitting machine. The count, the number of pieces of twists, the gauge number, and the unit weight of the knitted fabric using the PRT966 fiber and the PRT799 fiber are shown in Table 15. The thickness and the gauge number of the knitted fabric using other raw material fibers were adjusted so that the cover factor was almost the same as the cover factor of the knitted fabric of the modified fibroin fiber. Specifically, the numerical values are as shown in Table 15.

[Table 15]

| Raw material fiber | Count [Nm] | Number of twists | Gauge number [GG] | Basis weight [g/m$^2$] |
|---|---|---|---|---|
| PRT966 | 30 | 1 | 18 | 90.1 |
| PRT799 | 30 | 1 | 16 | 111.0 |
| Wool | 30 | 2 | 14 | 242.6 |
| Silk | 60 | 2 | 14 | 225.2 |
| Cotton | 34 | 2 | 14 | 194.1 |
| Rayon | 38 | 1 | 14 | 181.8 |
| Polyester | 60 | 1 | 14 | 184.7 |

The heat retention was evaluated by a dry contact method, using a KES-F7 Thermo Labo II tester manufactured by Kato Tech Co., Ltd. The dry contact method is a method assuming a direct contact between the skin and clothes in a dry state. One sheet of the knitted fabric cut into a rectangle of 20 cm $\times$ 20 cm was used as a test piece (sample). The test piece was set on a hot plate set to a constant temperature (30°C), and the quantity of heat (a) dissipated through the test piece was determined under the conditions of a wind velocity in a wind tunnel of 30 cm/sec. The quantity of heat (b) dissipated under the same conditions as described above was determined in a state where the test piece was not set, and the heat retention rate (%) was calculated according to the following formula.

$$\text{Heat retention rate } (\%) = (1 - a/b) \times 100$$

From the measurement results, the heat retention index was calculated according to the following Formula B.

$$\text{Formula B: Heat retention index} = \text{Heat retention rate } (\%) / \text{Basis weight of sample } (g/m^2)$$

The results of calculation of the heat retention property indices are shown in Table 16. It can be evaluated that the higher the heat retention property index is, the more excellent the heat retention property the material has.

[Table 16]

| Raw material fiber | Heat retention index |
|---|---|
| PRT966 | 0.33 |
| PRT799 | 0.22 |
| Wool | 0.16 |
| Silk | 0.11 |
| Cotton | 0.13 |
| Rayon | 0.02 |
| Polyester | 0.18 |

As shown in Table 16, it can be seen that the modified spider silk fibroin (PRT966 and PRT799) fibers have higher heat retention property indices and excellent in heat retention property, as compared with the existing materials.

**Reference Signs List**

1···extrusion device, 2···undrawn yarn producing device, 3···wet heat drawing device, 4···drying device, 6···doping liquid, 10···spinning device, 20···coagulation liquid bath, 21···drawing bath, 36···modified fibroin fiber, 100···false twisting machine.

## Claims

1. A high-shrinkage artificial fibroin twisted yarn, which is a shrunk artificial fibroin twisted yarn comprising a modified fibroin and having a shrinkage rate defined by the following Formula I of 2% or more:

$$\text{Shrinkage rate (\%)} = \{1- (\text{length of a shrunk artificial fibroin twisted yarn} / \text{length of the artificial fibroin twisted yarn after being twisted and before being brought into contact with water})\} \times 100 \cdots \text{(Formula I)}.$$

2. The high-shrinkage artificial fibroin twisted yarn according to claim 1, wherein the modified fibroin is a modified spider silk fibroin.

3. The high-shrinkage artificial fibroin twisted yarn according to claim 1 or 2, wherein the high-shrinkage artificial fibroin twisted yarn is an artificial fibroin twisted yarn shrunk by being brought into contact with water having a temperature lower than a boiling point.

4. The high-shrinkage artificial fibroin twisted yarn according to claim 3, wherein the temperature of the water is 10°C to 90°C.

5. The high-shrinkage artificial fibroin twisted yarn according to claim 3 or 4, wherein the high-shrinkage artificial fibroin twisted yarn is an artificial fibroin twisted yarn further shrunk by drying after being brought into contact with the water.

6. A method for producing a high-shrinkage artificial fibroin twisted yarn, comprising a step of shrinking an artificial fibroin twisted yarn comprising a modified fibroin by bringing the artificial fibroin twisted yarn into contact with water having a temperature lower than a boiling point,
wherein a shrinkage rate defined by the following Formula I is 2% or more,

$$\text{Shrinkage rate (\%)} = \{1- (\text{length of a shrunk artificial fibroin twisted yarn / length of the artificial fibroin twisted yarn after being twisted and before being brought into contact with water})\} \times 100 \cdots \text{(Formula I)}.$$

7. The method for producing a high-shrinkage artificial fibroin twisted yarn according to claim 6, wherein the modified fibroin is a modified spider silk fibroin.

8. The method for producing a high-shrinkage artificial fibroin twisted yarn according to claim 6 or 7, wherein the temperature of the water is 10°C to 90°C.

9. The method for producing a high-shrinkage artificial fibroin twisted yarn according to any one of claims 6 to 8, wherein the shrinking step further comprises drying the artificial fibroin twisted yarn after being brought into contact with the water.

10. A method for shrinking an artificial fibroin twisted yarn, comprising a step of shrinking an artificial fibroin twisted yarn comprising a modified fibroin by bringing the artificial fibroin twisted yarn into contact with water having a temperature lower than a boiling point,
wherein a shrinkage rate defined by the following Formula I is 2% or more,

$$\text{Shrinkage rate (\%)} = \{1- (\text{length of a shrunk artificial fibroin twisted yarn / length of the artificial fibroin twisted yarn after being twisted and before being brought into contact with water})\} \times 100 \cdots \text{(Formula I)}.$$

11. The method for shrinking an artificial fibroin twisted yarn according to claim 10, wherein the modified fibroin is a modified spider silk fibroin.

12. The method for shrinking an artificial fibroin twisted yarn according to claim 10 or 11, wherein the temperature of the water is 10°C to 90°C.

13. The method for shrinking an artificial fibroin twisted yarn according to any one of claims 10 to 12, wherein the shrinking step further comprises drying the artificial fibroin twisted yarn after being brought into contact with the water.

14. An artificial fibroin twisted yarn comprising a modified fibroin and having a shrinkage rate defined by the following Formula II of 2% or more:

Shrinkage (%) = {1- (length of artificial fibroin twisted yarn subjected to shrinking processing including being brought into contact with water having a temperature lower than a boiling point / length of the artificial fibroin twisted yarn before being subjected to the shrinking processing)} $\times$ 100 $\cdots$ (Formula II).

**15.** The high-shrinkage artificial fibroin twisted yarn according to any one of claims 1 to 5, wherein the modified fibroin comprises a domain sequence represented by Formula 1: $[(A)_n motif-REP]_m$, and

the domain sequence has an amino acid sequence with a reduced content of $(A)_n$ motifs, which corresponds to an amino acid sequence in which at least one or a plurality of $(A)_n$ motifs are deleted, as compared with a naturally occurring fibroin,

wherein in Formula 1, the $(A)_n$ motif represents an amino acid sequence composed of 2 to 27 amino acid residues and the number of alanine residues with respect to a total number of amino acid residues in the $(A)_n$ motif is 83% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

**16.** The high-shrinkage artificial fibroin twisted yarn according to any one of claims 1 to 5, wherein the modified fibroin comprises a domain sequence represented by

Formula 1: $[(A)_n motif-REP]_m$,

and

the domain sequence has an amino acid sequence with a reduced content of glycine residues, which corresponds to an amino acid sequence in which at least one or a plurality of glycine residues in REP are substituted with other amino acid residues, as compared with a naturally occurring fibroin,

wherein in Formula 1, the $(A)_n$ motif represents an amino acid sequence composed of 2 to 27 amino acid residues and the number of alanine residues with respect to a total number of amino acid residues in the $(A)_n$ motif is 83% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

**17.** The high-shrinkage artificial fibroin twisted yarn according to any one of claims 1 to 5, wherein the modified fibroin comprises a domain sequence represented by

Formula 1: $[(A)_n motif-REP]_m$,

and

the domain sequence has an amino acid sequence comprising a region having a locally high hydropathy index, which corresponds to an amino acid sequence in which one or a plurality of amino acid residues in REP are substituted with an amino acid residue having a high hydropathy index and/or one or a plurality of amino acid residues having a high hydropathy index is inserted into REP, as compared with a naturally occurring fibroin,

wherein in Formula 1, the $(A)_n$ motif represents an amino acid sequence composed of 2 to 27 amino acid residues and the number of alanine residues with respect to a total number of amino acid residues in the $(A)_n$ motif is 83% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

18. The high-shrinkage artificial fibroin twisted yarn according to any one of claims 1 to 5, wherein the modified fibroin comprises a domain sequence represented by Formula 1: $[(A)_n\text{motif-REP}]_m$ or Formula 2 $[(A)_n\text{motif-REP}]_m\text{-}(A)_n\text{motif}$, and

the domain sequence has an amino acid sequence with a reduced content of glutamine residues, which corresponds to an amino acid sequence in which one or a plurality of glutamine residues in REP are deleted or substituted with other amino acid residues, as compared with a naturally occurring fibroin,

wherein in Formula 1 and Formula 2, the $(A)_n$ motif represents an amino acid sequence composed of 2 to 27 amino acid residues and the number of alanine residues with respect to a total number of amino acid residues in the $(A)_n$ motif is 80% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

19. The high-shrinkage artificial fibroin twisted yarn according to any one of claims 1 to 5, wherein the modified fibroin has a limiting oxygen index (LOI) value of 26.0 or more.

20. The high-shrinkage artificial fibroin twisted yarn according to any one of claims 1 to 5, wherein a maximum hygroscopic heat generation, as determined according to the following Formula A, of the modified fibroin is more than 0.025°C/g:

$$\text{Formula A: Maximum hygroscopic heat generation} = \{(\text{Highest}$$
$$\text{temperature of a sample when the sample has been transferred to a high}$$
$$\text{humidity environment after being placed in a low humidity environment}$$
$$\text{until a temperature of the sample reaches equilibrium)} - (\text{Temperature of}$$
$$\text{the sample when the sample is being transferred to the high humidity}$$
$$\text{environment after being placed in the low humidity environment until}$$
$$\text{the temperature of the sample reaches equilibrium)}\} \ (°C) \ / \ \text{Sample}$$
$$\text{weight (g)},$$

wherein in Formula A, the low humidity environment means an environment of a temperature of 20°C and a relative humidity of 40%, and the high humidity environment means an environment of a temperature of 20°C and a relative humidity of 90%.

**Fig.1**

Fig.2

EP 3 789 525 A1

EP 3 789 525 A1

## Fig.3

Fig.4

# Fig.5

**Fig.6**

**Fig.7**

5

36

40

42

50

60

44

52

46

48

70

100

*Fig.8*

EP 3 789 525 A1

## INTERNATIONAL SEARCH REPORT

| International application No. |
| --- |
| PCT/JP2019/014898 |

**A. CLASSIFICATION OF SUBJECT MATTER**
Int.Cl. D02G3/22(2006.01)i, C07K14/435(2006.01)i, D01F4/02(2006.01)i

According to International Patent Classification (IPC) or to both national classification and IPC

**B. FIELDS SEARCHED**

Minimum documentation searched (classification system followed by classification symbols)
Int.Cl. D02G1/00-3/48, D02J1/00-13/00, D01F1/00-6/96, D01F9/00-9/04, D06M11/00, C07K14/435

Documentation searched other than minimum documentation to the extent that such documents are included in the fields searched
Published examined utility model applications of Japan      1922-1996
Published unexamined utility model applications of Japan      1971-2019
Registered utility model specifications of Japan      1996-2019
Published registered utility model applications of Japan      1994-2019

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 111700 C2 (KENGYOU SHIKENSHO) 24 July 1935, claims, property of invention and point of | 1, 3-6, 8-10, 12-14, 19-20 |
| A | purpose, page 2, lines 8-10, page 2, line 17 to page 3, lines 1-3 (Family: none) | 2, 7, 11, 15-18 |
| A | JP 2013-506058 A (TUFTS UNIVERSITY/TRUSTEES OF TUFTS COLLEGE) 21 February 2013, claims & US 9074302 B2, claims & WO 2011/038401 A2 & EP 2483460 A2 | 1-20 |

☒ Further documents are listed in the continuation of Box C.      ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 14.05.2019 | 28.05.2019 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**171**

**INTERNATIONAL SEARCH REPORT**

| International application No. |
| --- |
| PCT/JP2019/014898 |

C (Continuation).    DOCUMENTS CONSIDERED TO BE RELEVANT

| Category* | Citation of document, with indication, where appropriate, of the relevant passages | Relevant to claim No. |
| --- | --- | --- |
| P, X | WO 2019/022163 A1 (SPIBER INC.) 31 January 2019, claims <br> (Family: none) | 1-20 |
| P, X | WO 2018/165595 A1 (BOLT THREADS, INC.) 13 September 2018, claims <br> & US 2018/0282937 A1 | 1-20 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009121003 A

- JP 2002238569 A

**Non-patent literature cited in the description**

- *Nucleic Acid Res.,* 1982, vol. 10, 6487
- *Methods in Enzymology,* 1983, vol. 100, 448
- **KYTE J ; DOOLITTLE R.** A simple method for displaying the hydropathic character of a protein. *J. Mol. Biol.,* vol. 157, 105-132

- *Notice No. 50 of the Office of Hazardous Materials Regulation,* 31 May 1995
- *Proc. Natl. Acad. Sci. USA,* 1972, vol. 69, 2110
- Molecular Cloning

SEQUENCE LISTING

<110> Spiber Inc.
      KOJIMA INDUSTRIES CORPORATION

<120> A highly-constricted twisted yarn of modified fibroin fiber and a
      method for producing the same

<130> FP19-0339-00

<150> JP2018-071894
<151> 2018-04-03

<160> 40

<170> PatentIn version 3.5

<210> 1
<211> 50
<212> PRT
<213> Araneus diadematus

<400> 1

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn Tyr Gly
            20                  25                  30

Ala Ser Ala Gln Tyr Thr Gln Met Val Gly Gln Ser Val Ala Gln Ala
            35                  40                  45

Leu Ala
    50

<210> 2
<211> 30
<212> PRT
<213> Araneus diadematus

<400> 2

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn
            20                  25                  30

<210> 3
<211> 21
<212> PRT
<213> Araneus diadematus

<400> 3

Ser Gly Cys Asp Val Leu Val Gln Ala Leu Leu Glu Val Val Ser Ala

```
          1                  5                        10                      15


          Leu Val Ser Ile Leu
                        20


          <210>   4
          <211>   1154
          <212>   PRT
          <213>   Artificial Sequence

          <220>
          <223>   recombinant spider silk protein ADF3KaiLargeNRSH1

          <400>   4

          Met His His His His His His His His His His Ser Ser Gly Ser Ser
          1                  5                        10                      15


          Leu Glu Val Leu Phe Gln Gly Pro Ala Arg Ala Gly Ser Gly Gln Gln
                        20                      25                      30


          Gly Pro Gly Gln Gln Gly Pro Gly Gln Gln Gly Pro Gly Gln Gln Gly
                        35                      40                      45


          Pro Tyr Gly Pro Gly Ala Ser Ala Ala Ala Ala Ala Ala Gly Gly Tyr
                   50                      55                      60


          Gly Pro Gly Ser Gly Gln Gln Gly Pro Ser Gln Gln Gly Pro Gly Gln
          65                      70                      75                      80


          Gln Gly Pro Gly Gly Gln Gly Pro Tyr Gly Pro Gly Ala Ser Ala Ala
                             85                      90                      95


          Ala Ala Ala Ala Gly Gly Tyr Gly Pro Gly Ser Gly Gln Gln Gly Pro
                        100                     105                     110


          Gly Gly Gln Gly Pro Tyr Gly Pro Gly Ser Ser Ala Ala Ala Ala Ala
                   115                     120                     125


          Ala Gly Gly Asn Gly Pro Gly Ser Gly Gln Gln Gly Ala Gly Gln Gln
                   130                     135                     140


          Gly Pro Gly Gln Gln Gly Pro Gly Ala Ser Ala Ala Ala Ala Ala Ala
          145                     150                     155                     160


          Gly Gly Tyr Gly Pro Gly Ser Gly Gln Gln Gly Pro Gly Gln Gln Gly
                             165                     170                     175


          Pro Gly Gly Gln Gly Pro Tyr Gly Pro Gly Ala Ser Ala Ala Ala Ala
                        180                     185                     190
```

```
Ala Ala Gly Gly Tyr Gly Pro Gly Ser Gly Gln Gly Pro Gly Gln Gln
        195             200             205

Gly Pro Gly Gly Gln Gly Pro Tyr Gly Pro Gly Ala Ser Ala Ala Ala
        210             215             220

Ala Ala Ala Gly Gly Tyr Gly Pro Gly Ser Gly Gln Gln Gly Pro Gly
225             230             235             240

Gln Gln Gly Pro Gly Gln Gln Gly Pro Gly Gly Gln Gly Pro Tyr Gly
            245             250             255

Pro Gly Ala Ser Ala Ala Ala Ala Ala Ala Gly Gly Tyr Gly Pro Gly
            260             265             270

Tyr Gly Gln Gln Gly Pro Gly Gln Gln Gly Pro Gly Gly Gln Gly Pro
        275             280             285

Tyr Gly Pro Gly Ala Ser Ala Ala Ser Ala Ala Ser Gly Gly Tyr Gly
    290             295             300

Pro Gly Ser Gly Gln Gln Gly Pro Gly Gln Gln Gly Pro Gly Gly Gln
305             310             315             320

Gly Pro Tyr Gly Pro Gly Ala Ser Ala Ala Ala Ala Ala Ala Gly Gly
            325             330             335

Tyr Gly Pro Gly Ser Gly Gln Gln Gly Pro Gly Gln Gln Gly Pro Gly
        340             345             350

Gln Gln Gly Pro Gly Gln Gln Gly Pro Gly Gly Gln Gly Pro Tyr Gly
        355             360             365

Pro Gly Ala Ser Ala Ala Ala Ala Ala Ala Gly Gly Tyr Gly Pro Gly
    370             375             380

Ser Gly Gln Gln Gly Pro Gly Gln Gln Gly Pro Gly Gln Gln Gly Pro
385             390             395             400

Gly Gln Gln Gly Pro Gly Gln Gln Gly Pro Gly Gln Gln Gly Pro Gly
            405             410             415

Gln Gln Gly Pro Gly Gln Gln Gly Pro Gly Gln Gln Gly Pro Gly Gly
        420             425             430

Gln Gly Ala Tyr Gly Pro Gly Ala Ser Ala Ala Ala Gly Ala Ala Gly
```

                435                    440                    445

Gly Tyr Gly Pro Gly Ser Gly Gln Gln Gly Pro Gly Gln Gln Gly Pro
    450                 455                 460

Gly Gln Gln Gly Pro Gly Gln Gln Gly Pro Gly Gln Gln Gly Pro Gly
465                 470                 475                 480

Gln Gln Gly Pro Gly Gln Gln Gly Pro Gly Gln Gln Gly Pro Tyr Gly
                485                 490                 495

Pro Gly Ala Ser Ala Ala Ala Ala Ala Ala Gly Gly Tyr Gly Pro Gly
            500                 505                 510

Ser Gly Gln Gln Gly Pro Gly Gln Gln Gly Pro Gly Gln Gln Gly Pro
        515                 520                 525

Gly Gly Gln Gly Pro Tyr Gly Pro Gly Ala Ala Ser Ala Ala Val Ser
    530                 535                 540

Val Ser Arg Ala Arg Ala Gly Ser Gly Gln Gln Gly Pro Gly Gln Gln
545                 550                 555                 560

Gly Pro Gly Gln Gln Gly Pro Gly Gln Gln Gly Pro Tyr Gly Pro Gly
                565                 570                 575

Ala Ser Ala Ala Ala Ala Ala Ala Gly Gly Tyr Gly Pro Gly Ser Gly
        580                 585                 590

Gln Gln Gly Pro Ser Gln Gln Gly Pro Gly Gln Gln Gly Pro Gly Gly
        595                 600                 605

Gln Gly Pro Tyr Gly Pro Gly Ala Ser Ala Ala Ala Ala Ala Ala Gly
    610                 615                 620

Gly Tyr Gly Pro Gly Ser Gly Gln Gln Gly Pro Gly Gly Gln Gly Pro
625                 630                 635                 640

Tyr Gly Pro Gly Ser Ser Ala Ala Ala Ala Ala Ala Gly Gly Asn Gly
            645                 650                 655

Pro Gly Ser Gly Gln Gln Gly Ala Gly Gln Gln Gly Pro Gly Gln Gln
        660                 665                 670

Gly Pro Gly Ala Ser Ala Ala Ala Ala Ala Ala Gly Gly Tyr Gly Pro
    675                 680                 685

```
Gly Ser Gly Gln Gln Gly Pro Gly Gln Gln Gly Pro Gly Gly Gln Gly
    690               695               700

Pro Tyr Gly Pro Gly Ala Ser Ala Ala Ala Ala Ala Ala Gly Gly Tyr
705               710               715               720

Gly Pro Gly Ser Gly Gln Gly Pro Gly Gln Gln Gly Pro Gly Gly Gln
                725               730               735

Gly Pro Tyr Gly Pro Gly Ala Ser Ala Ala Ala Ala Ala Ala Gly Gly
            740               745               750

Tyr Gly Pro Gly Ser Gly Gln Gln Gly Pro Gly Gln Gln Gly Pro Gly
            755               760               765

Gln Gln Gly Pro Gly Gly Gln Gly Pro Tyr Gly Pro Gly Ala Ser Ala
    770               775               780

Ala Ala Ala Ala Ala Gly Gly Tyr Gly Pro Gly Tyr Gly Gln Gln Gly
785               790               795               800

Pro Gly Gln Gln Gly Pro Gly Gly Gln Gly Pro Tyr Gly Pro Gly Ala
            805               810               815

Ser Ala Ala Ser Ala Ala Ser Gly Gly Tyr Gly Pro Gly Ser Gly Gln
            820               825               830

Gln Gly Pro Gly Gln Gln Gly Pro Gly Gly Gln Gly Pro Tyr Gly Pro
        835               840               845

Gly Ala Ser Ala Ala Ala Ala Ala Ala Gly Gly Tyr Gly Pro Gly Ser
    850               855               860

Gly Gln Gln Gly Pro Gly Gln Gln Gly Pro Gly Gln Gln Gly Pro Gly
865               870               875               880

Gln Gln Gly Pro Gly Gly Gln Gly Pro Tyr Gly Pro Gly Ala Ser Ala
            885               890               895

Ala Ala Ala Ala Gly Gly Tyr Gly Pro Gly Ser Gly Gln Gln Gly
            900               905               910

Pro Gly Gln Gln Gly Pro Gly Gln Gln Gly Pro Gly Gln Gln Gly Pro
        915               920               925

Gly Gln Gln Gly Pro Gly Gln Gln Gly Pro Gly Gln Gln Gly Pro Gly
    930               935               940
```

51

```
Gln Gln Gly Pro Gly Gln Gln Gly Pro Gly Gly Gln Gly Ala Tyr Gly
945                 950             955                 960

Pro Gly Ala Ser Ala Ala Ala Gly Ala Ala Gly Gly Tyr Gly Pro Gly
                965             970             975

Ser Gly Gln Gln Gly Pro Gly Gln Gln Gly Pro Gly Gln Gln Gly Pro
            980             985             990

Gly Gln Gln Gly Pro Gly Gln Gln  Gly Pro Gly Gln Gln  Gly Pro Gly
        995             1000                1005

Gln Gln  Gly Pro Gly Gln Gln  Gly Pro Tyr Gly Pro  Gly Ala Ser
    1010             1015             1020

Ala Ala  Ala Ala Ala Ala Gly  Gly Tyr Gly Pro Gly  Ser Gly Gln
    1025             1030             1035

Gln Gly  Pro Gly Gln Gln Gly  Pro Gly Gln Gln Gly  Pro Gly Gly
    1040             1045             1050

Gln Gly  Pro Tyr Gly Pro Gly  Ala Ala Ser Ala Ala  Val Ser Val
    1055             1060             1065

Gly Gly  Tyr Gly Pro Gln Ser  Ser Ser Val Pro Val  Ala Ser Ala
    1070             1075             1080

Val Ala  Ser Arg Leu Ser Ser  Pro Ala Ala Ser Ser  Arg Val Ser
    1085             1090             1095

Ser Ala  Val Ser Ser Leu Val  Ser Ser Gly Pro Thr  Lys His Ala
    1100             1105             1110

Ala Leu  Ser Asn Thr Ile Ser  Ser Val Val Ser Gln  Val Ser Ala
    1115             1120             1125

Ser Asn  Pro Gly Leu Ser Gly  Cys Asp Val Leu Val  Gln Ala Leu
    1130             1135             1140

Leu Glu  Val Val Ser Ala Leu  Val Ser Ile Leu
    1145             1150
```

```
<210>  5
<211>  24
<212>  PRT
<213>  Artificial Sequence

<220>
```

<223> His tag and start codon

<400> 5

Met His His His His His His His His His Ser Ser Gly Ser Ser
1                   5                   10                  15

Leu Glu Val Leu Phe Gln Gly Pro
                20

<210> 6
<211> 597
<212> PRT
<213> Artificial Sequence

<220>
<223> Met-PRT380

<400> 6

Met Gly Pro Gly Gln Gln Gly Pro Tyr Gly Pro Gly Ala Ser Ala Ala
1                   5                   10                  15

Ala Ala Ala Gly Gln Asn Gly Pro Gly Ser Gly Gln Gln Gly Pro Gly
                20                  25                  30

Gln Ser Ala Ala Ala Ala Ala Gly Gln Tyr Gly Pro Gly Gln Gln Gly
                35                  40                  45

Pro Gly Gln Gln Gly Pro Gly Ser Ser Ala Ala Ala Ala Ala Gly Pro
        50                  55                  60

Gly Gln Tyr Gly Pro Gly Gln Gln Gly Pro Ser Ala Ser Ala Ala Ala
65                  70                  75                  80

Ala Ala Gly Pro Gly Ser Gly Gln Gln Gly Pro Gly Ala Ser Ala Ala
                85                  90                  95

Ala Ala Ala Gly Gln Tyr Gly Pro Gly Gln Gln Gly Pro Gly Gln Gln
                100                 105                 110

Gly Pro Gly Ser Ser Ala Ala Ala Ala Ala Gly Gln Tyr Gly Ser Gly
        115                 120                 125

Pro Gly Gln Gln Gly Pro Tyr Gly Ser Ala Ala Ala Ala Ala Gly Pro
        130                 135                 140

Gly Ser Gly Gln Tyr Gly Gln Gly Pro Tyr Gly Pro Gly Ala Ser Ala
145                 150                 155                 160

Ala Ala Ala Gly Pro Gly Gln Tyr Gly Pro Gly Gln Gln Gly Pro

|     |     |     | 165 |     |     |     |     |     | 170 |     |     |     | 175 |     |     |
|-----|-----|-----|-----|-----|-----|-----|-----|-----|-----|-----|-----|-----|-----|-----|-----|

Ser Ala Ser Ala Ala Ala Ala Ala Gly Ser Gly Gln Gln Gly Pro Gly
          180            185           190

Gln Tyr Gly Pro Tyr Ala Ser Ala Ala Ala Ala Gly Gln Tyr Gly
        195          200          205

Ser Gly Pro Gly Gln Gln Gly Pro Tyr Gly Pro Gly Gln Ser Ala Ala
        210          215          220

Ala Ala Ala Gly Ser Gly Gln Gln Gly Pro Gly Gln Gln Gly Pro Tyr
225           230         235         240

Ala Ser Ala Ala Ala Ala Ala Gly Pro Gly Gln Gln Gly Pro Tyr Gly
          245        250         255

Pro Gly Ser Ser Ala Ala Ala Ala Ala Gly Gln Tyr Gly Tyr Gly Pro
        260          265         270

Gly Gln Gln Gly Pro Tyr Gly Pro Gly Ala Ser Ala Ala Ala Ala Ala
        275          280         285

Gly Gln Asn Gly Pro Gly Ser Gly Gln Tyr Gly Pro Gly Gln Gln Gly
     290          295        300

Pro Gly Gln Ser Ala Ala Ala Ala Ala Gly Pro Gly Gln Gln Gly Pro
305           310         315         320

Tyr Gly Pro Gly Ala Ser Ala Ala Ala Ala Ala Gly Gln Tyr Gly Pro
        325          330         335

Gly Gln Gln Gly Pro Gly Gln Tyr Gly Pro Gly Ser Ser Ala Ala Ala
        340          345        350

Ala Ala Gly Pro Gly Gln Gln Gly Pro Tyr Gly Pro Gly Ser Ser Ala
        355          360        365

Ala Ala Ala Ala Gly Gln Tyr Gly Pro Gly Gln Gln Gly Pro Tyr Gly
        370          375        380

Pro Gly Gln Ser Ala Ala Ala Ala Ala Gly Gln Tyr Gln Gln Gly Pro
385           390         395         400

Gly Gln Gln Gly Pro Tyr Gly Pro Gly Ala Ser Ala Ala Ala Ala Ala
        405          410        415

Gly Pro Gly Gln Gln Gly Pro Tyr Gly Pro Gly Ala Ser Ala Ala Ala
420 425 430

Ala Ala Gly Pro Gly Gln Tyr Gly Pro Gly Gln Gln Gly Pro Ser Ala
435 440 445

Ser Ala Ala Ala Ala Ala Gly Gln Tyr Gly Ser Gly Pro Gly Gln Tyr
450 455 460

Gly Pro Tyr Gly Pro Gly Gln Ser Ala Ala Ala Ala Ala Gly Pro Gly
465 470 475 480

Ser Gly Gln Gln Gly Gln Gly Pro Tyr Gly Pro Gly Ala Ser Ala Ala
485 490 495

Ala Ala Ala Gly Gln Tyr Gly Pro Gly Gln Gln Gly Pro Tyr Gly Pro
500 505 510

Gly Gln Ser Ala Ala Ala Ala Ala Gly Pro Gly Ser Gly Gln Tyr Gly
515 520 525

Pro Gly Ala Ser Ala Ala Ala Ala Ala Gly Gln Asn Gly Pro Gly Ser
530 535 540

Gly Gln Tyr Gly Pro Gly Gln Gln Gly Pro Gly Gln Ser Ala Ala Ala
545 550 555 560

Ala Ala Gly Gln Tyr Gln Gln Gly Pro Gly Gln Gln Gly Pro Tyr Gly
565 570 575

Pro Gly Ala Ser Ala Ala Ala Ala Ala Gly Pro Gly Ser Gly Gln Gln
580 585 590

Gly Pro Gly Ala Ser
595


<210> 7
<211> 590
<212> PRT
<213> Artificial Sequence

<220>
<223> Met-PRT410

<400> 7

Met Gly Pro Gly Gln Gln Gly Pro Tyr Gly Pro Gly Ala Ser Ala Ala
1 5 10 15

Ala Ala Ala Gly Gln Asn Gly Pro Gly Ser Gly Gln Gln Gly Pro Gly

|    |    | 20 |    |    |    |    | 25 |    |    |    |    | 30 |    |    |
|----|----|----|----|----|----|----|----|----|----|----|----|----|----|----|

Gln Ser Gly Gln Tyr Gly Pro Gly Gln Gln Gly Pro Gly Gln Gln Gly
35                40                45

Pro Gly Ser Ser Ala Ala Ala Ala Ala Gly Pro Gly Gln Tyr Gly Pro
50                55                60

Gly Gln Gln Gly Pro Ser Ala Ser Ala Ala Ala Ala Gly Pro Gly
65                70                75                80

Ser Gly Gln Gln Gly Pro Gly Ala Ser Gly Gln Tyr Gly Pro Gly Gln
85                90                95

Gln Gly Pro Gly Gln Gln Gly Pro Gly Ser Ser Ala Ala Ala Ala Ala
100                105                110

Gly Gln Tyr Gly Ser Gly Pro Gly Gln Gln Gly Pro Tyr Gly Ser Ala
115                120                125

Ala Ala Ala Ala Gly Pro Gly Ser Gly Gln Tyr Gly Gln Gly Pro Tyr
130                135                140

Gly Pro Gly Ala Ser Gly Pro Gly Gln Tyr Gly Pro Gly Gln Gln Gly
145                150                155                160

Pro Ser Ala Ser Ala Ala Ala Ala Ala Gly Ser Gly Gln Gln Gly Pro
165                170                175

Gly Gln Tyr Gly Pro Tyr Ala Ser Ala Ala Ala Ala Gly Gln Tyr
180                185                190

Gly Ser Gly Pro Gly Gln Gln Gly Pro Tyr Gly Pro Gly Gln Ser Gly
195                200                205

Ser Gly Gln Gln Gly Pro Gly Gln Gln Gly Pro Tyr Ala Ser Ala Ala
210                215                220

Ala Ala Ala Gly Pro Gly Gln Gln Gly Pro Tyr Gly Pro Gly Ser Ser
225                230                235                240

Ala Ala Ala Ala Ala Gly Gln Tyr Gly Tyr Gly Pro Gly Gln Gln Gly
245                250                255

Pro Tyr Gly Pro Gly Ala Ser Gly Gln Asn Gly Pro Gly Ser Gly Gln
260                265                270

Tyr Gly Pro Gly Gln Gln Gly Pro Gly Gln Ser Ala Ala Ala Ala
        275                 280             285

Gly Pro Gly Gln Gln Gly Pro Tyr Gly Pro Gly Ala Ser Ala Ala Ala
        290                 295             300

Ala Ala Gly Gln Tyr Gly Pro Gly Gln Gln Gly Pro Gly Gln Tyr Gly
305             310                 315                 320

Pro Gly Ser Ser Gly Pro Gly Gln Gln Gly Pro Tyr Gly Pro Gly Ser
            325                 330             335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Gly Gln Tyr Gln Gln
        355                 360                 365

Gly Pro Gly Gln Gln Gly Pro Tyr Gly Pro Gly Ala Ser Gly Pro Gly
        370                 375                 380

Gln Gln Gly Pro Tyr Gly Pro Gly Ala Ser Ala Ala Ala Ala Gly
385                 390                 395                 400

Pro Gly Gln Tyr Gly Pro Gly Gln Gln Gly Pro Ser Ala Ser Ala Ala
                405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Gly Gln Tyr Gly Pro
        450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
            485                 490                 495

Pro Gly Ser Gly Gln Tyr Gly Pro Gly Gln Gln Gly Pro Gly Gln Ser
        500                 505                 510

Ala Ala Ala Ala Ala Gly Gln Tyr Gln Gln Gly Pro Gly Gln Gln Gly
        515                 520                 525

```
Pro Tyr Gly Pro Gly Ala Ser Ala Ala Ala Ala Gly Gln Tyr Gly
    530                 535                 540

Ser Gly Pro Gly Gln Gln Gly Pro Tyr Gly Pro Gly Gln Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
            580                 585                 590


<210>   8
<211>   565
<212>   PRT
<213>   Artificial Sequence

<220>
<223>   Met-PRT525

<400>   8

Met Gly Pro Gly Gln Gln Gly Pro Tyr Gly Pro Gly Ala Ser Ala Ala
1                   5                   10                  15

Ala Ala Ala Ala Ala Gly Ser Asn Gly Pro Gly Ser Gly Gln Gln Gly
            20                  25                  30

Pro Gly Gln Ser Gly Gln Tyr Gly Pro Gly Gln Gln Gly Pro Gly Gln
            35                  40                  45

Gln Gly Pro Gly Ser Ser Ala Ala Ala Ala Ala Ala Gly Pro Gly
            50                  55                  60

Gln Tyr Gly Pro Gly Gln Gln Gly Pro Ser Ala Ser Ala Ala Ala Ala
65                  70                  75                  80

Ala Ala Ala Gly Pro Gly Ser Gly Gln Gln Gly Pro Gly Ala Ser Gly
            85                  90                  95

Gln Tyr Gly Pro Gly Gln Gln Gly Pro Gly Gln Gln Gly Pro Gly Ser
            100                 105                 110

Ser Ala Ala Ala Ala Ala Ala Ala Gly Ser Tyr Gly Ser Gly Pro Gly
            115                 120                 125

Gln Gln Gly Pro Tyr Gly Ser Ala Ala Ala Ala Ala Ala Gly Pro
            130                 135                 140
```

Gly Ser Gly Gln Tyr Gly Gln Gly Pro Tyr Gly Pro Gly Ala Ser Gly
145                 150                 155                 160

Pro Gly Gln Tyr Gly Pro Gly Gln Gln Gly Pro Ser Ala Ser Ala Ala
                165                 170                 175

Ala Ala Ala Ala Ala Gly Ser Gly Gln Gln Gly Pro Gly Gln Tyr Gly
                180                 185                 190

Pro Tyr Ala Ser Ala Ala Ala Ala Ala Ala Gly Ser Tyr Gly Ser
        195                 200                 205

Gly Pro Gly Gln Gln Gly Pro Tyr Gly Pro Gly Gln Ser Gly Ser Gly
        210                 215                 220

Gln Gln Gly Pro Gly Gln Gln Gly Pro Tyr Ala Ser Ala Ala Ala Ala
225                 230                 235                 240

Ala Ala Ala Gly Pro Gly Gln Gln Gly Pro Tyr Gly Pro Gly Ser Ser
                245                 250                 255

Ala Ala Ala Ala Ala Ala Ala Gly Ser Tyr Gly Tyr Gly Pro Gly Gln
                260                 265                 270

Gln Gly Pro Tyr Gly Pro Gly Ala Ser Gly Gln Asn Gly Pro Gly Ser
        275                 280                 285

Gly Gln Tyr Gly Pro Gly Gln Gln Gly Pro Gly Pro Ser Ala Ala Ala
        290                 295                 300

Ala Ala Ala Ala Gly Pro Gly Gln Gln Gly Pro Tyr Gly Pro Gly Ala
305                 310                 315                 320

Ser Ala Ala Ala Ala Ala Ala Ala Gly Ser Tyr Gly Pro Gly Gln Gln
                325                 330                 335

Gly Pro Gly Gln Tyr Gly Pro Gly Ser Ser Gly Pro Gly Gln Gln Gly
        340                 345                 350

Pro Tyr Gly Pro Gly Ser Ser Ala Ala Ala Ala Ala Ala Ala Gly Ser
        355                 360                 365

Tyr Gly Pro Gly Gln Gln Gly Pro Tyr Gly Pro Gly Pro Ser Ala Ala
        370                 375                 380

Ala Ala Ala Ala Ala Gly Ser Tyr Gln Gln Gly Pro Gly Gln Gln Gly
385                 390                 395                 400

```
Pro Tyr Gly Pro Gly Ala Ser Gly Pro Gly Gln Gln Gly Pro Tyr Gly
             405                 410                 415

Pro Gly Ala Ser Ala Ala Ala Ala Ala Ala Ala Gly Pro Gly Gln Tyr
             420                 425                 430

Gly Pro Gly Gln Gln Gly Pro Ser Ala Ser Ala Ala Ala Ala Ala Ala
             435                 440                 445

Ala Gly Ser Tyr Gly Ser Gly Pro Gly Gln Tyr Gly Pro Tyr Gly Pro
         450                 455                 460

Gly Gln Ser Gly Pro Gly Ser Gly Gln Gln Gly Gln Gly Pro Tyr Gly
465                 470                 475                 480

Pro Gly Ala Ser Ala Ala Ala Ala Ala Ala Ala Gly Ser Tyr Gly Pro
                 485                 490                 495

Gly Gln Gln Gly Pro Tyr Gly Pro Gly Pro Ser Ala Ala Ala Ala Ala
             500                 505                 510

Ala Ala Gly Pro Gly Ser Gly Gln Tyr Gly Pro Gly Ala Ser Gly Gln
         515                 520                 525

Asn Gly Pro Gly Ser Gly Gln Tyr Gly Pro Gly Gln Gln Gly Pro Gly
         530                 535                 540

Pro Ser Ala Ala Ala Ala Ala Ala Ala Gly Pro Gly Ser Gly Gln Gln
545                 550                 555                 560

Gly Pro Gly Ala Ser
                 565
```

<210> 9
<211> 2364
<212> PRT
<213> Artificial Sequence

<220>
<223> Met-PRT799

<400> 9

```
Met Gly Pro Gly Gln Gln Gly Pro Tyr Gly Pro Gly Ala Ser Ala Ala
1               5                 10                  15

Ala Ala Ala Gly Gln Asn Gly Pro Gly Ser Gly Gln Gln Gly Pro Gly
             20                  25                  30
```

Gln Ser Gly Gln Tyr Gly Pro Gly Gln Gln Gly Pro Gly Gln Gln Gly
        35              40              45

Pro Gly Ser Ser Ala Ala Ala Ala Ala Gly Pro Gly Gln Tyr Gly Pro
        50              55              60

Gly Gln Gln Gly Pro Ser Ala Ser Ala Ala Ala Ala Gly Pro Gly
65              70              75              80

Ser Gly Gln Gln Gly Pro Gly Ala Ser Gly Gln Tyr Gly Pro Gly Gln
                85              90              95

Gln Gly Pro Gly Gln Gln Gly Pro Gly Ser Ser Ala Ala Ala Ala Ala
            100             105             110

Gly Gln Tyr Gly Ser Gly Pro Gly Gln Gln Gly Pro Tyr Gly Ser Ala
        115             120             125

Ala Ala Ala Ala Gly Pro Gly Ser Gly Gln Tyr Gly Gln Gly Pro Tyr
    130             135             140

Gly Pro Gly Ala Ser Gly Pro Gly Gln Tyr Gly Pro Gly Gln Gln Gly
145             150             155             160

Pro Ser Ala Ser Ala Ala Ala Ala Ala Gly Ser Gly Gln Gln Gly Pro
            165             170             175

Gly Gln Tyr Gly Pro Tyr Ala Ser Ala Ala Ala Ala Gly Gln Tyr
        180             185             190

Gly Ser Gly Pro Gly Gln Gln Gly Pro Tyr Gly Pro Gly Gln Ser Gly
    195             200             205

Ser Gly Gln Gln Gly Pro Gly Gln Gln Gly Pro Tyr Ala Ser Ala Ala
    210             215             220

Ala Ala Ala Gly Pro Gly Gln Gln Gly Pro Tyr Gly Pro Gly Ser Ser
225             230             235             240

Ala Ala Ala Ala Ala Gly Gln Tyr Gly Tyr Gly Pro Gly Gln Gln Gly
            245             250             255

Pro Tyr Gly Pro Gly Ala Ser Gly Gln Asn Gly Pro Gly Ser Gly Gln
        260             265             270

Tyr Gly Pro Gly Gln Gln Gly Pro Gly Gln Ser Ala Ala Ala Ala Ala
        275             280             285

Gly Pro Gly Gln Gln Gly Pro Tyr Gly Pro Gly Ala Ser Ala Ala Ala
290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
            325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
            355                 360                 365

Gly Pro Gly Gln Gln Gly Pro Tyr Gly Pro Gly Ala Ser Gly Pro Gly
            370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
            405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
            435                 440                 445

Tyr Gly Pro Gly Ala Ser Ala Ala Ala Ala Gly Gln Tyr Gly Pro
            450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
            485                 490                 495

Pro Gly Ser Gly Gln Tyr Gly Pro Gly Gln Gln Gly Pro Gly Gln Ser
            500                 505                 510

Ala Ala Ala Ala Ala Gly Gln Tyr Gln Gln Gly Pro Gly Gln Gln Gly
            515                 520                 525

Pro Tyr Gly Pro Gly Ala Ser Ala Ala Ala Ala Ala Gly Gln Tyr Gly
            530                 535                 540

```
Ser Gly Pro Gly Gln Gln Gly Pro Tyr Gly Pro Gly Gln Ser Gly Ser
545                 550             555             560


Gly Gln Gln Gly Pro Gly Gln Gln Gly Pro Tyr Ala Ser Ala Ala Ala
                565             570             575


Ala Ala Gly Pro Gly Ser Gly Gln Gln Gly Pro Gly Ala Ser Gly Gln
            580             585             590


Gln Gly Pro Tyr Gly Pro Gly Ala Ser Ala Ala Ala Ala Ala Gly Gln
            595             600             605


Asn Gly Pro Gly Ser Gly Gln Gln Gly Pro Gly Gln Ser Gly Gln Tyr
    610             615             620


Gly Pro Gly Gln Gln Gly Pro Gly Gln Gln Gly Pro Gly Ser Ser Ala
625             630             635             640


Ala Ala Ala Ala Gly Pro Gly Gln Tyr Gly Pro Gly Gln Gln Gly Pro
            645             650             655


Ser Ala Ser Ala Ala Ala Ala Ala Gly Pro Gly Ser Gly Gln Gln Gly
            660             665             670


Pro Gly Ala Ser Gly Gln Tyr Gly Pro Gly Gln Gln Gly Pro Gly Gln
            675             680             685


Gln Gly Pro Gly Ser Ser Ala Ala Ala Ala Gly Gln Tyr Gly Ser
    690             695             700


Gly Pro Gly Gln Gln Gly Pro Tyr Gly Ser Ala Ala Ala Ala Ala Gly
705             710             715             720


Pro Gly Ser Gly Gln Tyr Gly Gln Gly Pro Tyr Gly Pro Gly Ala Ser
            725             730             735


Gly Pro Gly Gln Tyr Gly Pro Gly Gln Gln Gly Pro Ser Ala Ser Ala
            740             745             750


Ala Ala Ala Ala Gly Ser Gly Gln Gln Gly Pro Gly Gln Tyr Gly Pro
            755             760             765


Tyr Ala Ser Ala Ala Ala Ala Ala Gly Gln Tyr Gly Ser Gly Pro Gly
    770             775             780


Gln Gln Gly Pro Tyr Gly Pro Gly Gln Ser Gly Ser Gly Gln Gln Gly
```

785                    790                    795                    800

Pro Gly Gln Gln Gly Pro Tyr Ala Ser Ala Ala Ala Ala Gly Pro
               805                   810              815

Gly Gln Gln Gly Pro Tyr Gly Pro Gly Ser Ser Ala Ala Ala Ala
          820                825               830

Gly Gln Tyr Gly Tyr Gly Pro Gly Gln Gln Gly Pro Tyr Gly Pro Gly
      835               840              845

Ala Ser Gly Gln Asn Gly Pro Gly Ser Gly Gln Tyr Gly Pro Gly Gln
      850             855              860

Gln Gly Pro Gly Gln Ser Ala Ala Ala Ala Gly Pro Gly Gln Gln
865               870              875              880

Gly Pro Tyr Gly Pro Gly Ala Ser Ala Ala Ala Ala Gly Gln Tyr
          885               890              895

Gly Pro Gly Gln Gln Gly Pro Gly Gln Tyr Gly Pro Gly Ser Ser Gly
          900               905              910

Pro Gly Gln Gln Gly Pro Tyr Gly Pro Gly Ser Ser Ala Ala Ala Ala
      915               920              925

Ala Gly Gln Tyr Gly Pro Gly Gln Gln Gly Pro Tyr Gly Pro Gly Gln
      930               935              940

Ser Ala Ala Ala Ala Ala Gly Gln Tyr Gln Gln Gly Pro Gly Gln Gln
945               950              955              960

Gly Pro Tyr Gly Pro Gly Ala Ser Gly Pro Gly Gln Gln Gly Pro Tyr
          965               970              975

Gly Pro Gly Ala Ser Ala Ala Ala Ala Gly Pro Gly Gln Tyr Gly
          980               985              990

Pro Gly Gln Gln Gly Pro Ser Ala Ser Ala Ala Ala Ala Ala Gly Gln
      995             1000            1005

Tyr Gly Ser Gly Pro Gly Gln Tyr Gly Pro Tyr Gly Pro Gly Gln
     1010              1015             1020

Ser Gly Pro Gly Ser Gly Gln Gln Gly Gln Gly Pro Tyr Gly Pro
     1025              1030             1035

Gly Ala Ser Ala Ala Ala Ala Ala Gly Gln Tyr Gly Pro Gly Gln
1040 1045 1050

Gln Gly Pro Tyr Gly Pro Gly Gln Ser Ala Ala Ala Ala Ala Gly
1055 1060 1065

Pro Gly Ser Gly Gln Tyr Gly Pro Gly Ala Ser Gly Gln Asn Gly
1070 1075 1080

Pro Gly Ser Gly Gln Tyr Gly Pro Gly Gln Gln Gly Pro Gly Gln
1085 1090 1095

Ser Ala Ala Ala Ala Ala Gly Gln Tyr Gln Gln Gly Pro Gly Gln
1100 1105 1110

Gln Gly Pro Tyr Gly Pro Gly Ala Ser Ala Ala Ala Ala Ala Gly
1115 1120 1125

Gln Tyr Gly Ser Gly Pro Gly Gln Gln Gly Pro Tyr Gly Pro Gly
1130 1135 1140

Gln Ser Gly Ser Gly Gln Gln Gly Pro Gly Gln Gln Gly Pro Tyr
1145 1150 1155

Ala Ser Ala Ala Ala Ala Ala Gly Pro Gly Ser Gly Gln Gln Gly
1160 1165 1170

Pro Gly Ala Ser Gly Gln Gln Gly Pro Tyr Gly Pro Gly Ala Ser
1175 1180 1185

Ala Ala Ala Ala Ala Gly Gln Asn Gly Pro Gly Ser Gly Gln Gln
1190 1195 1200

Gly Pro Gly Gln Ser Gly Gln Tyr Gly Pro Gly Gln Gln Gly Pro
1205 1210 1215

Gly Gln Gln Gly Pro Gly Ser Ser Ala Ala Ala Ala Gly Pro
1220 1225 1230

Gly Gln Tyr Gly Pro Gly Gln Gln Gly Pro Ser Ala Ser Ala Ala
1235 1240 1245

Ala Ala Ala Gly Pro Gly Ser Gly Gln Gln Gly Pro Gly Ala Ser
1250 1255 1260

Gly Gln Tyr Gly Pro Gly Gln Gln Gly Pro Gly Gln Gln Gly Pro
1265 1270 1275

```
        Gly  Ser   Ser  Ala  Ala  Ala  Ala   Ala  Gly  Gln  Tyr  Gly   Ser  Gly  Pro
        1280                      1285                     1290

        Gly  Gln   Gln  Gly  Pro  Tyr  Gly   Ser  Ala  Ala  Ala  Ala   Ala  Gly  Pro
        1295                      1300                     1305

        Gly  Ser   Gly  Gln  Tyr  Gly  Gln   Gly  Pro  Tyr  Gly  Pro   Gly  Ala  Ser
        1310                      1315                     1320

        Gly  Pro   Gly  Gln  Tyr  Gly  Pro   Gly  Gln  Gln  Gly  Pro   Ser  Ala  Ser
        1325                      1330                     1335

        Ala  Ala   Ala  Ala  Ala  Gly  Ser   Gly  Gln  Gln  Gly  Pro   Gly  Gln  Tyr
        1340                      1345                     1350

        Gly  Pro   Tyr  Ala  Ser  Ala  Ala   Ala  Ala  Ala  Gly  Gln   Tyr  Gly  Ser
        1355                      1360                     1365

        Gly  Pro   Gly  Gln  Gln  Gly  Pro   Tyr  Gly  Pro  Gly  Gln   Ser  Gly  Ser
        1370                      1375                     1380

        Gly  Gln   Gln  Gly  Pro  Gly  Gln   Gln  Gly  Pro  Tyr  Ala   Ser  Ala  Ala
        1385                      1390                     1395

        Ala  Ala   Ala  Gly  Pro  Gly  Gln   Gln  Gly  Pro  Tyr  Gly   Pro  Gly  Ser
        1400                      1405                     1410

        Ser  Ala   Ala  Ala  Ala  Ala  Gly   Gln  Tyr  Gly  Tyr  Gly   Pro  Gly  Gln
        1415                      1420                     1425

        Gln  Gly   Pro  Tyr  Gly  Pro  Gly   Ala  Ser  Gly  Gln  Asn   Gly  Pro  Gly
        1430                      1435                     1440

        Ser  Gly   Gln  Tyr  Gly  Pro  Gly   Gln  Gln  Gly  Pro  Gly   Gln  Ser  Ala
        1445                      1450                     1455

        Ala  Ala   Ala  Ala  Gly  Pro  Gly   Gln  Gln  Gly  Pro  Tyr   Gly  Pro  Gly
        1460                      1465                     1470

        Ala  Ser   Ala  Ala  Ala  Ala  Ala   Gly  Gln  Tyr  Gly  Pro   Gly  Gln  Gln
        1475                      1480                     1485

        Gly  Pro   Gly  Gln  Tyr  Gly  Pro   Gly  Ser  Ser  Gly  Pro   Gly  Gln  Gln
        1490                      1495                     1500

        Gly  Pro   Tyr  Gly  Pro  Gly  Ser   Ser  Ala  Ala  Ala  Ala   Ala  Gly  Gln
        1505                      1510                     1515
```

66

Tyr Gly Pro Gly Gln Gln Gly Pro Tyr Gly Pro Gly Gln Ser Ala
    1520             1525             1530

Ala Ala Ala Ala Gly Gln Tyr Gln Gln Gly Pro Gly Gln Gln Gly
    1535             1540             1545

Pro Tyr Gly Pro Gly Ala Ser Gly Pro Gly Gln Gln Gly Pro Tyr
    1550             1555             1560

Gly Pro Gly Ala Ser Ala Ala Ala Ala Ala Gly Pro Gly Gln Tyr
    1565             1570             1575

Gly Pro Gly Gln Gln Gly Pro Ser Ala Ser Ala Ala Ala Ala Ala
    1580             1585             1590

Gly Gln Tyr Gly Ser Gly Pro Gly Gln Tyr Gly Pro Tyr Gly Pro
    1595             1600             1605

Gly Gln Ser Gly Pro Gly Ser Gly Gln Gln Gly Gln Gly Pro Tyr
    1610             1615             1620

Gly Pro Gly Ala Ser Ala Ala Ala Ala Ala Gly Gln Tyr Gly Pro
    1625             1630             1635

Gly Gln Gln Gly Pro Tyr Gly Pro Gly Gln Ser Ala Ala Ala Ala
    1640             1645             1650

Ala Gly Pro Gly Ser Gly Gln Tyr Gly Pro Gly Ala Ser Gly Gln
    1655             1660             1665

Asn Gly Pro Gly Ser Gly Gln Tyr Gly Pro Gly Gln Gln Gly Pro
    1670             1675             1680

Gly Gln Ser Ala Ala Ala Ala Ala Gly Gln Tyr Gln Gln Gly Pro
    1685             1690             1695

Gly Gln Gln Gly Pro Tyr Gly Pro Gly Ala Ser Ala Ala Ala Ala
    1700             1705             1710

Ala Gly Gln Tyr Gly Ser Gly Pro Gly Gln Gln Gly Pro Tyr Gly
    1715             1720             1725

Pro Gly Gln Ser Gly Ser Gly Gln Gln Gly Pro Gly Gln Gln Gly
    1730             1735             1740

Pro Tyr Ala Ser Ala Ala Ala Ala Ala Gly Pro Gly Ser Gly Gln

|      | 1745 |     |     |     |     | 1750 |     |     |     |     | 1755 |     |     |     |
| ---- | ---- | --- | --- | --- | --- | ---- | --- | --- | --- | --- | ---- | --- | --- | --- |

Gln Gly Pro Gly Ala Ser Gly Gln Gln Gly Pro Tyr Gly Pro Gly
        1760                1765                1770

Ala Ser Ala Ala Ala Ala Ala Gly Gln Asn Gly Pro Gly Ser Gly
        1775                1780                1785

Gln Gln Gly Pro Gly Gln Ser Gly Gln Tyr Gly Pro Gly Gln Gln
        1790                1795                1800

Gly Pro Gly Gln Gln Gly Pro Gly Ser Ser Ala Ala Ala Ala Ala
        1805                1810                1815

Gly Pro Gly Gln Tyr Gly Pro Gly Gln Gln Gly Pro Ser Ala Ser
        1820                1825                1830

Ala Ala Ala Ala Ala Gly Pro Gly Ser Gly Gln Gln Gly Pro Gly
        1835                1840                1845

Ala Ser Gly Gln Tyr Gly Pro Gly Gln Gln Gly Pro Gly Gln Gln
        1850                1855                1860

Gly Pro Gly Ser Ser Ala Ala Ala Ala Ala Gly Gln Tyr Gly Ser
        1865                1870                1875

Gly Pro Gly Gln Gln Gly Pro Tyr Gly Ser Ala Ala Ala Ala Ala
        1880                1885                1890

Gly Pro Gly Ser Gly Gln Tyr Gly Gln Gly Pro Tyr Gly Pro Gly
        1895                1900                1905

Ala Ser Gly Pro Gly Gln Tyr Gly Pro Gly Gln Gln Gly Pro Ser
        1910                1915                1920

Ala Ser Ala Ala Ala Ala Ala Gly Ser Gly Gln Gln Gly Pro Gly
        1925                1930                1935

Gln Tyr Gly Pro Tyr Ala Ser Ala Ala Ala Ala Ala Gly Gln Tyr
        1940                1945                1950

Gly Ser Gly Pro Gly Gln Gln Gly Pro Tyr Gly Pro Gly Gln Ser
        1955                1960                1965

Gly Ser Gly Gln Gln Gly Pro Gly Gln Gln Gly Pro Tyr Ala Ser
        1970                1975                1980

```
Ala Ala  Ala Ala Ala Gly Pro  Gly Gln Gln Gly Pro  Tyr Gly Pro
    1985             1990                 1995

Gly Ser  Ser Ala Ala Ala Ala  Ala Gly Gln Tyr Gly  Tyr Gly Pro
    2000             2005                 2010

Gly Gln  Gln Gly Pro Tyr Gly  Pro Gly Ala Ser Gly  Gln Asn Gly
    2015             2020                 2025

Pro Gly  Ser Gly Gln Tyr Gly  Pro Gly Gln Gln Gly  Pro Gly Gln
    2030             2035                 2040

Ser Ala  Ala Ala Ala Ala Gly  Pro Gly Gln Gln Gly  Pro Tyr Gly
    2045             2050                 2055

Pro Gly  Ala Ser Ala Ala Ala  Ala Ala Gly Gln Tyr  Gly Pro Gly
    2060             2065                 2070

Gln Gln  Gly Pro Gly Gln Tyr  Gly Pro Gly Ser Ser  Gly Pro Gly
    2075             2080                 2085

Gln Gln  Gly Pro Tyr Gly Pro  Gly Ser Ser Ala Ala  Ala Ala Ala
    2090             2095                 2100

Gly Gln  Tyr Gly Pro Gly Gln  Gln Gly Pro Tyr Gly  Pro Gly Gln
    2105             2110                 2115

Ser Ala  Ala Ala Ala Ala Gly  Gln Tyr Gln Gln Gly  Pro Gly Gln
    2120             2125                 2130

Gln Gly  Pro Tyr Gly Pro Gly  Ala Ser Gly Pro Gly  Gln Gln Gly
    2135             2140                 2145

Pro Tyr  Gly Pro Gly Ala Ser  Ala Ala Ala Ala Ala  Gly Pro Gly
    2150             2155                 2160

Gln Tyr  Gly Pro Gly Gln Gln  Gly Pro Ser Ala Ser  Ala Ala Ala
    2165             2170                 2175

Ala Ala  Gly Gln Tyr Gly Ser  Gly Pro Gly Gln Tyr  Gly Pro Tyr
    2180             2185                 2190

Gly Pro  Gly Gln Ser Gly Pro  Gly Ser Gly Gln Gln  Gly Gln Gly
    2195             2200                 2205

Pro Tyr  Gly Pro Gly Ala Ser  Ala Ala Ala Ala Ala  Gly Gln Tyr
    2210             2215                 2220
```

69

```
Gly Pro  Gly Gln Gln Gly Pro  Tyr Gly Pro Gly Gln  Ser Ala Ala
    2225                2230                2235


Ala Ala  Ala Gly Pro Gly Ser  Gly Gln Tyr Gly Pro  Gly Ala Ser
    2240                2245                2250


Gly Gln  Asn Gly Pro Gly Ser  Gly Gln Tyr Gly Pro  Gly Gln Gln
    2255                2260                2265


Gly Pro  Gly Gln Ser Ala Ala  Ala Ala Ala Gly Gln  Tyr Gln Gln
    2270                2275                2280


Gly Pro  Gly Gln Gln Gly Pro  Tyr Gly Pro Gly Ala  Ser Ala Ala
    2285                2290                2295


Ala Ala  Ala Gly Gln Tyr Gly  Ser Gly Pro Gly Gln  Gln Gly Pro
    2300                2305                2310


Tyr Gly  Pro Gly Gln Ser Gly  Ser Gly Gln Gln Gly  Pro Gly Gln
    2315                2320                2325


Gln Gly  Pro Tyr Ala Ser Ala  Ala Ala Ala Ala Gly  Pro Gly Ser
    2330                2335                2340


Gly Gln  Gln Gly Ser Ser Val  Asp Lys Leu Ala Ala  Ala Leu Glu
    2345                2350                2355


His His  His His His His
    2360
```

```
<210>  10
<211>  597
<212>  PRT
<213>  Artificial Sequence

<220>
<223>  Met-PRT313

<400>  10
```

```
Met Gly Pro Gly Gly Gln Gly Pro Tyr Gly Pro Gly Ala Ser Ala Ala
1               5                10               15


Ala Ala Ala Gly Gly Asn Gly Pro Gly Ser Gly Gln Gln Gly Pro Gly
            20               25               30


Gly Ser Ala Ala Ala Ala Ala Gly Gly Tyr Gly Pro Gly Gly Gln Gly
            35               40               45
```

```
Pro Gly Gln Gln Gly Pro Gly Ser Ser Ala Ala Ala Ala Ala Gly Pro
    50                  55              60

Gly Gly Tyr Gly Pro Gly Gly Gln Gly Pro Ser Ala Ser Ala Ala Ala
65                  70              75                  80

Ala Ala Gly Pro Gly Ser Gly Gln Gln Gly Pro Gly Ala Ser Ala Ala
                85              90              95

Ala Ala Ala Gly Gly Tyr Gly Pro Gly Gly Gln Gly Pro Gly Gln Gln
            100             105             110

Gly Pro Gly Ser Ser Ala Ala Ala Ala Gly Gly Tyr Gly Ser Gly
        115             120             125

Pro Gly Gln Gln Gly Pro Tyr Gly Ser Ala Ala Ala Ala Ala Gly Pro
    130             135             140

Gly Ser Gly Gly Tyr Gly Gln Gly Pro Tyr Gly Pro Gly Ala Ser Ala
145             150             155             160

Ala Ala Ala Gly Pro Gly Gly Tyr Gly Pro Gly Gly Gln Gly Pro
            165             170             175

Ser Ala Ser Ala Ala Ala Ala Ala Gly Ser Gly Gln Gln Gly Pro Gly
        180             185             190

Gly Tyr Gly Pro Tyr Ala Ser Ala Ala Ala Ala Gly Gly Tyr Gly
    195             200             205

Ser Gly Pro Gly Gln Gln Gly Pro Tyr Gly Pro Gly Gly Ser Ala Ala
    210             215             220

Ala Ala Ala Gly Ser Gly Gln Gln Gly Pro Gly Gln Gln Gly Pro Tyr
225             230             235             240

Ala Ser Ala Ala Ala Ala Ala Gly Pro Gly Gly Gln Gly Pro Tyr Gly
        245             250             255

Pro Gly Ser Ser Ala Ala Ala Ala Ala Gly Gly Tyr Gly Tyr Gly Pro
        260             265             270

Gly Gly Gln Gly Pro Tyr Gly Pro Gly Ala Ser Ala Ala Ala Ala Ala
    275             280             285

Gly Gly Asn Gly Pro Gly Ser Gly Gly Tyr Gly Pro Gly Gln Gln Gly
    290             295             300
```

Pro Gly Gly Ser Ala Ala Ala Ala Ala Gly Pro Gly Gly Gln Gly Pro
305                 310                 315                 320

Tyr Gly Pro Gly Ala Ser Ala Ala Ala Ala Ala Gly Gly Tyr Gly Pro
                325                 330                 335

Gly Gly Gln Gly Pro Gly Gly Tyr Gly Pro Gly Ser Ser Ala Ala Ala
            340                 345                 350

Ala Ala Gly Pro Gly Gly Gln Gly Pro Tyr Gly Pro Gly Ser Ser Ala
        355                 360                 365

Ala Ala Ala Gly Gly Tyr Gly Pro Gly Gln Gln Gly Pro Tyr Gly
        370                 375                 380

Pro Gly Gly Ser Ala Ala Ala Ala Ala Gly Gly Tyr Gln Gln Gly Pro
385                 390                 395                 400

Gly Gly Gln Gly Pro Tyr Gly Pro Gly Ala Ser Ala Ala Ala Ala Ala
            405                 410                 415

Gly Pro Gly Gly Gln Gly Pro Tyr Gly Pro Gly Ala Ser Ala Ala Ala
        420                 425                 430

Ala Ala Gly Pro Gly Gly Tyr Gly Pro Gly Gly Gln Gly Pro Ser Ala
        435                 440                 445

Ser Ala Ala Ala Ala Ala Gly Gly Tyr Gly Ser Gly Pro Gly Gly Tyr
    450                 455                 460

Gly Pro Tyr Gly Pro Gly Gly Ser Ala Ala Ala Ala Ala Gly Pro Gly
465                 470                 475                 480

Ser Gly Gln Gln Gly Gln Gly Pro Tyr Gly Pro Gly Ala Ser Ala Ala
            485                 490                 495

Ala Ala Ala Gly Gly Tyr Gly Pro Gly Gln Gln Gly Pro Tyr Gly Pro
        500                 505                 510

Gly Gly Ser Ala Ala Ala Ala Ala Gly Pro Gly Ser Gly Gly Tyr Gly
        515                 520                 525

Pro Gly Ala Ser Ala Ala Ala Ala Ala Gly Gly Asn Gly Pro Gly Ser
    530                 535                 540

Gly Gly Tyr Gly Pro Gly Gln Gln Gly Pro Gly Gly Ser Ala Ala Ala
545                 550                 555                 560

```
Ala Ala Gly Gly Tyr Gln Gln Gly Pro Gly Gly Gln Gly Pro Tyr Gly
            565             570             575

Pro Gly Ala Ser Ala Ala Ala Ala Ala Gly Pro Gly Ser Gly Gln Gln
            580             585             590

Gly Pro Gly Ala Ser
            595
```

<210> 11
<211> 12
<212> PRT
<213> Artificial Sequence

<220>
<223> HisTag

<400> 11

```
Met His His His His His His Ser Ser Gly Ser Ser
1               5               10
```

<210> 12
<211> 608
<212> PRT
<213> Artificial Sequence

<220>
<223> PRT380

<400> 12

```
Met His His His His His His Ser Ser Gly Ser Ser Gly Pro Gly Gln
1               5               10              15

Gln Gly Pro Tyr Gly Pro Gly Ala Ser Ala Ala Ala Ala Ala Gly Gln
            20              25              30

Asn Gly Pro Gly Ser Gly Gln Gln Gly Pro Gly Gln Ser Ala Ala Ala
            35              40              45

Ala Ala Gly Gln Tyr Gly Pro Gly Gln Gln Gly Pro Gly Gln Gln Gly
      50              55              60

Pro Gly Ser Ser Ala Ala Ala Ala Ala Gly Pro Gly Gln Tyr Gly Pro
65              70              75              80

Gly Gln Gln Gly Pro Ser Ala Ser Ala Ala Ala Ala Gly Pro Gly
            85              90              95

Ser Gly Gln Gln Gly Pro Gly Ala Ser Ala Ala Ala Ala Ala Gly Gln
```

```
                100                      105                       110


    Tyr Gly Pro Gly Gln Gln Gly Pro Gly Gln Gln Gly Pro Gly Ser Ser
            115                 120                 125


    Ala Ala Ala Ala Ala Gly Gln Tyr Gly Ser Gly Pro Gly Gln Gln Gly
        130                 135                 140


    Pro Tyr Gly Ser Ala Ala Ala Ala Ala Gly Pro Gly Ser Gly Gln Tyr
    145                 150                 155                 160


    Gly Gln Gly Pro Tyr Gly Pro Gly Ala Ser Ala Ala Ala Ala Ala Gly
                    165                 170                 175


    Pro Gly Gln Tyr Gly Pro Gly Gln Gln Gly Pro Ser Ala Ser Ala Ala
                180                 185                 190


    Ala Ala Ala Gly Ser Gly Gln Gln Gly Pro Gly Gln Tyr Gly Pro Tyr
            195                 200                 205


    Ala Ser Ala Ala Ala Ala Ala Gly Gln Tyr Gly Ser Gly Pro Gly Gln
        210                 215                 220


    Gln Gly Pro Tyr Gly Pro Gly Gln Ser Ala Ala Ala Ala Ala Gly Ser
    225                 230                 235                 240


    Gly Gln Gln Gly Pro Gly Gln Gln Gly Pro Tyr Ala Ser Ala Ala Ala
                245                 250                 255


    Ala Ala Gly Pro Gly Gln Gln Gly Pro Tyr Gly Pro Gly Ser Ser Ala
            260                 265                 270


    Ala Ala Ala Gly Gln Tyr Gly Tyr Gly Pro Gly Gln Gln Gly Pro
            275                 280                 285


    Tyr Gly Pro Gly Ala Ser Ala Ala Ala Ala Gly Gln Asn Gly Pro
        290                 295                 300


    Gly Ser Gly Gln Tyr Gly Pro Gly Gln Gln Gly Pro Gly Gln Ser Ala
    305                 310                 315                 320


    Ala Ala Ala Gly Pro Gly Gln Gln Gly Pro Tyr Gly Pro Gly Ala
                325                 330                 335


    Ser Ala Ala Ala Ala Ala Gly Gln Tyr Gly Pro Gly Gln Gln Gly Pro
        340                 345                 350
```

Gly Gln Tyr Gly Pro Gly Ser Ser Ala Ala Ala Ala Ala Gly Pro Gly
        355             360         365

Gln Gln Gly Pro Tyr Gly Pro Gly Ser Ser Ala Ala Ala Ala Ala Gly
        370             375         380

Gln Tyr Gly Pro Gly Gln Gln Gly Pro Tyr Gly Pro Gly Gln Ser Ala
385             390             395             400

Ala Ala Ala Ala Gly Gln Tyr Gln Gln Gly Pro Gly Gln Gln Gly Pro
            405             410             415

Tyr Gly Pro Gly Ala Ser Ala Ala Ala Ala Gly Pro Gly Gln Gln
        420             425             430

Gly Pro Tyr Gly Pro Gly Ala Ser Ala Ala Ala Ala Gly Pro Gly
        435             440             445

Gln Tyr Gly Pro Gly Gln Gln Gly Pro Ser Ala Ser Ala Ala Ala Ala
    450             455             460

Ala Gly Gln Tyr Gly Ser Gly Pro Gly Gln Tyr Gly Pro Tyr Gly Pro
465             470             475             480

Gly Gln Ser Ala Ala Ala Ala Ala Gly Pro Gly Ser Gly Gln Gln Gly
            485             490             495

Gln Gly Pro Tyr Gly Pro Gly Ala Ser Ala Ala Ala Ala Ala Gly Gln
        500             505             510

Tyr Gly Pro Gly Gln Gln Gly Pro Tyr Gly Pro Gly Gln Ser Ala Ala
        515             520             525

Ala Ala Ala Gly Pro Gly Ser Gly Gln Tyr Gly Pro Gly Ala Ser Ala
    530             535             540

Ala Ala Ala Gly Gln Asn Gly Pro Gly Ser Gly Gln Tyr Gly Pro
545             550             555             560

Gly Gln Gln Gly Pro Gly Gln Ser Ala Ala Ala Ala Ala Gly Gln Tyr
            565             570             575

Gln Gln Gly Pro Gly Gln Gln Gly Pro Tyr Gly Pro Gly Ala Ser Ala
        580             585             590

Ala Ala Ala Gly Pro Gly Ser Gly Gln Gln Gly Pro Gly Ala Ser
    595             600             605

```
<210>  13
<211>  601
<212>  PRT
<213>  Artificial Sequence

<220>
<223>  PRT410

<400>  13

Met His His His His His His Ser Ser Gly Ser Ser Gly Pro Gly Gln
1                   5                   10                  15


Gln Gly Pro Tyr Gly Pro Gly Ala Ser Ala Ala Ala Ala Ala Gly Gln
            20                  25                  30


Asn Gly Pro Gly Ser Gly Gln Gln Gly Pro Gly Gln Ser Gly Gln Tyr
            35                  40                  45


Gly Pro Gly Gln Gln Gly Pro Gly Gln Gln Gly Pro Gly Ser Ser Ala
    50                  55                  60


Ala Ala Ala Ala Gly Pro Gly Gln Tyr Gly Pro Gly Gln Gln Gly Pro
65                  70                  75                  80


Ser Ala Ser Ala Ala Ala Ala Ala Gly Pro Gly Ser Gly Gln Gln Gly
                85                  90                  95


Pro Gly Ala Ser Gly Gln Tyr Gly Pro Gly Gln Gln Gly Pro Gly Gln
            100                 105                 110


Gln Gly Pro Gly Ser Ser Ala Ala Ala Ala Ala Gly Gln Tyr Gly Ser
            115                 120                 125


Gly Pro Gly Gln Gln Gly Pro Tyr Gly Ser Ala Ala Ala Ala Ala Gly
    130                 135                 140


Pro Gly Ser Gly Gln Tyr Gly Gln Gly Pro Tyr Gly Pro Gly Ala Ser
145                 150                 155                 160


Gly Pro Gly Gln Tyr Gly Pro Gly Gln Gln Gly Pro Ser Ala Ser Ala
            165                 170                 175


Ala Ala Ala Ala Gly Ser Gly Gln Gln Gly Pro Gly Gln Tyr Gly Pro
            180                 185                 190


Tyr Ala Ser Ala Ala Ala Ala Ala Gly Gln Tyr Gly Ser Gly Pro Gly
            195                 200                 205
```

Gln Gln Gly Pro Tyr Gly Pro Gly Gln Ser Gly Ser Gly Gln Gln Gly
210                     215                 220

Pro Gly Gln Gln Gly Pro Tyr Ala Ser Ala Ala Ala Ala Ala Gly Pro
225                     230                 235                 240

Gly Gln Gln Gly Pro Tyr Gly Pro Gly Ser Ser Ala Ala Ala Ala Ala
245                 250                 255

Gly Gln Tyr Gly Tyr Gly Pro Gly Gln Gln Gly Pro Tyr Gly Pro Gly
260                 265                 270

Ala Ser Gly Gln Asn Gly Pro Gly Ser Gly Gln Tyr Gly Pro Gly Gln
275                 280                 285

Gln Gly Pro Gly Gln Ser Ala Ala Ala Ala Gly Pro Gly Gln Gln
290                 295                 300

Gly Pro Tyr Gly Pro Gly Ala Ser Ala Ala Ala Ala Ala Gly Gln Tyr
305                 310                 315                 320

Gly Pro Gly Gln Gln Gly Pro Gly Gln Tyr Gly Pro Gly Ser Ser Gly
325                 330                 335

Pro Gly Gln Gln Gly Pro Tyr Gly Pro Gly Ser Ser Ala Ala Ala Ala
340                 345                 350

Ala Gly Gln Tyr Gly Pro Gly Gln Gln Gly Pro Tyr Gly Pro Gly Gln
355                 360                 365

Ser Ala Ala Ala Ala Ala Gly Gln Tyr Gln Gln Gly Pro Gly Gln Gln
370                 375                 380

Gly Pro Tyr Gly Pro Gly Ala Ser Gly Pro Gly Gln Gln Gly Pro Tyr
385                 390                 395                 400

Gly Pro Gly Ala Ser Ala Ala Ala Ala Gly Pro Gly Gln Tyr Gly
405                 410                 415

Pro Gly Gln Gln Gly Pro Ser Ala Ser Ala Ala Ala Ala Gly Gln
420                 425                 430

Tyr Gly Ser Gly Pro Gly Gln Tyr Gly Pro Tyr Gly Pro Gly Gln Ser
435                 440                 445

Gly Pro Gly Ser Gly Gln Gln Gly Gln Gly Pro Tyr Gly Pro Gly Ala
450                 455                 460

```
Ser Ala Ala Ala Ala Ala Gly Gln Tyr Gly Pro Gly Gln Gln Gly Pro
465             470             475             480


Tyr Gly Pro Gly Gln Ser Ala Ala Ala Ala Ala Gly Pro Gly Ser Gly
            485             490             495


Gln Tyr Gly Pro Gly Ala Ser Gly Gln Asn Gly Pro Gly Ser Gly Gln
        500             505             510


Tyr Gly Pro Gly Gln Gln Gly Pro Gly Gln Ser Ala Ala Ala Ala Ala
    515             520             525


Gly Gln Tyr Gln Gln Gly Pro Gly Gln Gln Gly Pro Tyr Gly Pro Gly
    530             535             540


Ala Ser Ala Ala Ala Ala Ala Gly Gln Tyr Gly Ser Gly Pro Gly Gln
545             550             555             560


Gln Gly Pro Tyr Gly Pro Gly Gln Ser Gly Ser Gly Gln Gln Gly Pro
            565             570             575


Gly Gln Gln Gly Pro Tyr Ala Ser Ala Ala Ala Ala Ala Gly Pro Gly
        580             585             590


Ser Gly Gln Gln Gly Pro Gly Ala Ser
    595             600
```

```
<210>  14
<211>  576
<212>  PRT
<213>  Artificial Sequence

<220>
<223>  PRT525

<400>  14
```

```
Met His His His His His His Ser Ser Gly Ser Ser Gly Pro Gly Gln
1               5               10              15


Gln Gly Pro Tyr Gly Pro Gly Ala Ser Ala Ala Ala Ala Ala Ala Ala
            20              25              30


Gly Ser Asn Gly Pro Gly Ser Gly Gln Gln Gly Pro Gly Gln Ser Gly
        35              40              45


Gln Tyr Gly Pro Gly Gln Gln Gly Pro Gly Gln Gln Gly Pro Gly Ser
    50              55              60
```

Ser Ala Ala Ala Ala Ala Ala Ala Gly Pro Gly Gln Tyr Gly Pro Gly
65                  70              75                  80

Gln Gln Gly Pro Ser Ala Ser Ala Ala Ala Ala Ala Ala Ala Gly Pro
                85                  90                  95

Gly Ser Gly Gln Gln Gly Pro Gly Ala Ser Gly Gln Tyr Gly Pro Gly
                100                 105                 110

Gln Gln Gly Pro Gly Gln Gln Gly Pro Gly Ser Ser Ala Ala Ala Ala
            115                 120                 125

Ala Ala Ala Gly Ser Tyr Gly Ser Gly Pro Gly Gln Gln Gly Pro Tyr
            130                 135                 140

Gly Ser Ala Ala Ala Ala Ala Ala Ala Gly Pro Gly Ser Gly Gln Tyr
145                 150                 155                 160

Gly Gln Gly Pro Tyr Gly Pro Gly Ala Ser Gly Pro Gly Gln Tyr Gly
                165                 170                 175

Pro Gly Gln Gln Gly Pro Ser Ala Ser Ala Ala Ala Ala Ala Ala Ala
            180                 185                 190

Gly Ser Gly Gln Gln Gly Pro Gly Gln Tyr Gly Pro Tyr Ala Ser Ala
            195                 200                 205

Ala Ala Ala Ala Ala Gly Ser Tyr Gly Ser Gly Pro Gly Gln Gln
            210                 215                 220

Gly Pro Tyr Gly Pro Gly Gln Ser Gly Ser Gly Gln Gln Gly Pro Gly
225                 230                 235                 240

Gln Gln Gly Pro Tyr Ala Ser Ala Ala Ala Ala Ala Ala Ala Gly Pro
                245                 250                 255

Gly Gln Gln Gly Pro Tyr Gly Pro Gly Ser Ser Ala Ala Ala Ala Ala
            260                 265                 270

Ala Ala Gly Ser Tyr Gly Tyr Gly Pro Gly Gln Gln Gly Pro Tyr Gly
            275                 280                 285

Pro Gly Ala Ser Gly Gln Asn Gly Pro Gly Ser Gly Gln Tyr Gly Pro
            290                 295                 300

Gly Gln Gln Gly Pro Gly Pro Ser Ala Ala Ala Ala Ala Ala Ala Gly
305                 310                 315                 320

Pro Gly Gln Gln Gly Pro Tyr Gly Pro Gly Ala Ser Ala Ala Ala Ala
            325             330             335

Ala Ala Ala Gly Ser Tyr Gly Pro Gly Gln Gln Gly Pro Gly Gln Tyr
            340             345             350

Gly Pro Gly Ser Ser Gly Pro Gly Gln Gln Gly Pro Tyr Gly Pro Gly
            355             360             365

Ser Ser Ala Ala Ala Ala Ala Ala Ala Gly Ser Tyr Gly Pro Gly Gln
            370             375             380

Gln Gly Pro Tyr Gly Pro Gly Pro Ser Ala Ala Ala Ala Ala Ala Ala
385             390             395             400

Gly Ser Tyr Gln Gln Gly Pro Gly Gln Gln Gly Pro Tyr Gly Pro Gly
            405             410             415

Ala Ser Gly Pro Gly Gln Gln Gly Pro Tyr Gly Pro Gly Ala Ser Ala
            420             425             430

Ala Ala Ala Ala Ala Ala Gly Pro Gly Gln Tyr Gly Pro Gly Gln Gln
            435             440             445

Gly Pro Ser Ala Ser Ala Ala Ala Ala Ala Ala Gly Ser Tyr Gly
            450             455             460

Ser Gly Pro Gly Gln Tyr Gly Pro Tyr Gly Pro Gly Gln Ser Gly Pro
465             470             475             480

Gly Ser Gly Gln Gln Gly Gln Gly Pro Tyr Gly Pro Gly Ala Ser Ala
            485             490             495

Ala Ala Ala Ala Ala Gly Ser Tyr Gly Pro Gly Gln Gln Gly Pro
            500             505             510

Tyr Gly Pro Gly Pro Ser Ala Ala Ala Ala Ala Ala Gly Pro Gly
            515             520             525

Ser Gly Gln Tyr Gly Pro Gly Ala Ser Gly Gln Asn Gly Pro Gly Ser
            530             535             540

Gly Gln Tyr Gly Pro Gly Gln Gln Gly Pro Gly Pro Ser Ala Ala Ala
545             550             555             560

Ala Ala Ala Ala Gly Pro Gly Ser Gly Gln Gln Gly Pro Gly Ala Ser
            565             570             575

<210> 15
<211> 2375
<212> PRT
<213> Artificial Sequence

<220>
<223> PRT799

<400> 15

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
                20                  25                  30

Asn Gly Pro Gly Ser Gly Gln Gln Gly Pro Gly Gln Ser Gly Gln Tyr
            35                  40                  45

Gly Pro Gly Gln Gln Gly Pro Gly Gln Gln Gly Pro Gly Ser Ser Ala
        50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
            115                 120                 125

Gly Pro Gly Gln Gln Gly Pro Tyr Gly Ser Ala Ala Ala Ala Ala Gly
        130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
            165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
210            215              220

Pro Gly Gln Gln Gly Pro Tyr Ala Ser Ala Ala Ala Ala Ala Gly Pro
225            230              235                240

Gly Gln Gln Gly Pro Tyr Gly Pro Gly Ser Ser Ala Ala Ala Ala Ala
245              250                255

Gly Gln Tyr Gly Tyr Gly Pro Gly Gln Gln Gly Pro Tyr Gly Pro Gly
260              265              270

Ala Ser Gly Gln Asn Gly Pro Gly Ser Gly Gln Tyr Gly Pro Gly Gln
275              280              285

Gln Gly Pro Gly Gln Ser Ala Ala Ala Ala Gly Pro Gly Gln Gln
290              295              300

Gly Pro Tyr Gly Pro Gly Ala Ser Ala Ala Ala Ala Ala Gly Gln Tyr
305              310              315              320

Gly Pro Gly Gln Gln Gly Pro Gly Gln Tyr Gly Pro Gly Ser Ser Gly
325              330              335

Pro Gly Gln Gln Gly Pro Tyr Gly Pro Gly Ser Ser Ala Ala Ala Ala
340              345              350

Ala Gly Gln Tyr Gly Pro Gly Gln Gln Gly Pro Tyr Gly Pro Gly Gln
355              360              365

Ser Ala Ala Ala Ala Ala Gly Gln Tyr Gln Gln Gly Pro Gly Gln Gln
370              375              380

Gly Pro Tyr Gly Pro Gly Ala Ser Gly Pro Gly Gln Gln Gly Pro Tyr
385              390              395              400

Gly Pro Gly Ala Ser Ala Ala Ala Ala Ala Gly Pro Gly Gln Tyr Gly
405              410              415

Pro Gly Gln Gln Gly Pro Ser Ala Ser Ala Ala Ala Ala Ala Gly Gln
420              425              430

Tyr Gly Ser Gly Pro Gly Gln Tyr Gly Pro Tyr Gly Pro Gly Gln Ser
435              440              445

Gly Pro Gly Ser Gly Gln Gln Gly Gln Gly Pro Tyr Gly Pro Gly Ala
450              455              460

Ser Ala Ala Ala Ala Ala Gly Gln Tyr Gly Pro Gly Gln Gln Gly Pro
465                 470             475                 480

Tyr Gly Pro Gly Gln Ser Ala Ala Ala Ala Ala Gly Pro Gly Ser Gly
                485                 490             495

Gln Tyr Gly Pro Gly Ala Ser Gly Gln Asn Gly Pro Gly Ser Gly Gln
                500             505             510

Tyr Gly Pro Gly Gln Gln Gly Pro Gly Gln Ser Ala Ala Ala Ala Ala
        515             520             525

Gly Gln Tyr Gln Gln Gly Pro Gly Gln Gln Gly Pro Tyr Gly Pro Gly
        530             535             540

Ala Ser Ala Ala Ala Ala Ala Gly Gln Tyr Gly Ser Gly Pro Gly Gln
545             550             555                 560

Gln Gly Pro Tyr Gly Pro Gly Gln Ser Gly Ser Gly Gln Gln Gly Pro
            565             570             575

Gly Gln Gln Gly Pro Tyr Ala Ser Ala Ala Ala Ala Gly Pro Gly
            580             585             590

Ser Gly Gln Gln Gly Pro Gly Ala Ser Gly Gln Gln Gly Pro Tyr Gly
            595             600             605

Pro Gly Ala Ser Ala Ala Ala Ala Gly Gln Asn Gly Pro Gly Ser
        610             615             620

Gly Gln Gln Gly Pro Gly Gln Ser Gly Gln Tyr Gly Pro Gly Gln Gln
625             630             635                 640

Gly Pro Gly Gln Gln Gly Pro Gly Ser Ser Ala Ala Ala Ala Gly
            645             650             655

Pro Gly Gln Tyr Gly Pro Gly Gln Gln Gly Pro Ser Ala Ser Ala Ala
            660             665             670

Ala Ala Ala Gly Pro Gly Ser Gly Gln Gln Gly Pro Gly Ala Ser Gly
        675             680             685

Gln Tyr Gly Pro Gly Gln Gln Gly Pro Gly Gln Gln Gly Pro Gly Ser
        690             695             700

Ser Ala Ala Ala Ala Ala Gly Gln Tyr Gly Ser Gly Pro Gly Gln Gln

|     |     |     |     |     |     |     |     |     |     |     |     |     |     |     |     |
|-----|-----|-----|-----|-----|-----|-----|-----|-----|-----|-----|-----|-----|-----|-----|-----|
| 705 |     |     |     | 710 |     |     |     | 715 |     |     |     | 720 |     |     |     |

Gly Pro Tyr Gly Ser Ala Ala Ala Ala Ala Gly Pro Gly Ser Gly Gln
            725             730             735

Tyr Gly Gln Gly Pro Tyr Gly Pro Gly Ala Ser Gly Pro Gly Gln Tyr
            740             745             750

Gly Pro Gly Gln Gln Gly Pro Ser Ala Ser Ala Ala Ala Ala Ala Gly
            755             760             765

Ser Gly Gln Gln Gly Pro Gly Gln Tyr Gly Pro Tyr Ala Ser Ala Ala
            770             775             780

Ala Ala Ala Gly Gln Tyr Gly Ser Gly Pro Gly Gln Gln Gly Pro Tyr
785             790             795             800

Gly Pro Gly Gln Ser Gly Ser Gly Gln Gln Gly Pro Gly Gln Gln Gly
            805             810             815

Pro Tyr Ala Ser Ala Ala Ala Ala Ala Gly Pro Gly Gln Gln Gly Pro
            820             825             830

Tyr Gly Pro Gly Ser Ser Ala Ala Ala Ala Gly Gln Tyr Gly Tyr
            835             840             845

Gly Pro Gly Gln Gln Gly Pro Tyr Gly Pro Gly Ala Ser Gly Gln Asn
            850             855             860

Gly Pro Gly Ser Gly Gln Tyr Gly Pro Gly Gln Gln Gly Pro Gly Gln
865             870             875             880

Ser Ala Ala Ala Ala Ala Gly Pro Gly Gln Gln Gly Pro Tyr Gly Pro
            885             890             895

Gly Ala Ser Ala Ala Ala Ala Ala Gly Gln Tyr Gly Pro Gly Gln Gln
            900             905             910

Gly Pro Gly Gln Tyr Gly Pro Gly Ser Ser Gly Pro Gly Gln Gln Gly
            915             920             925

Pro Tyr Gly Pro Gly Ser Ser Ala Ala Ala Ala Gly Gln Tyr Gly
            930             935             940

Pro Gly Gln Gln Gly Pro Tyr Gly Pro Gly Gln Ser Ala Ala Ala Ala
945             950             955             960

84

Ala Gly Gln Tyr Gln Gln Gly Pro Gly Gln Gln Gly Pro Tyr Gly Pro
                965                      970                      975

Gly Ala Ser Gly Pro Gly Gln Gln Gly Pro Tyr Gly Pro Gly Ala Ser
                980                      985                      990

Ala Ala Ala Ala Ala Gly Pro Gly  Gln Tyr Gly Pro Gly  Gln Gln Gly
         995              1000                     1005

Pro Ser  Ala Ser Ala Ala Ala  Ala Ala Gly Gln Tyr  Gly Ser Gly
    1010              1015              1020

Pro Gly  Gln Tyr Gly Pro Tyr  Gly Pro Gly Gln Ser  Gly Pro Gly
    1025              1030              1035

Ser Gly  Gln Gln Gly Gln Gly  Pro Tyr Gly Pro Gly  Ala Ser Ala
    1040              1045              1050

Ala Ala  Ala Ala Gly Gln Tyr  Gly Pro Gly Gln Gln  Gly Pro Tyr
    1055              1060              1065

Gly Pro  Gly Gln Ser Ala Ala  Ala Ala Ala Gly Pro  Gly Ser Gly
    1070              1075              1080

Gln Tyr  Gly Pro Gly Ala Ser  Gly Gln Asn Gly Pro  Gly Ser Gly
    1085              1090              1095

Gln Tyr  Gly Pro Gly Gln Gln  Gly Pro Gly Gln Ser  Ala Ala Ala
    1100              1105              1110

Ala Ala  Gly Gln Tyr Gln Gln  Gly Pro Gly Gln Gln  Gly Pro Tyr
    1115              1120              1125

Gly Pro  Gly Ala Ser Ala Ala  Ala Ala Ala Gly Gln  Tyr Gly Ser
    1130              1135              1140

Gly Pro  Gly Gln Gln Gly Pro  Tyr Gly Pro Gly Gln  Ser Gly Ser
    1145              1150              1155

Gly Gln  Gln Gly Pro Gly Gln  Gln Gly Pro Tyr Ala  Ser Ala Ala
    1160              1165              1170

Ala Ala  Ala Gly Pro Gly Ser  Gly Gln Gln Gly Pro  Gly Ala Ser
    1175              1180              1185

Gly Gln  Gln Gly Pro Tyr Gly  Pro Gly Ala Ser Ala  Ala Ala Ala
    1190              1195              1200

```
Ala Gly  Gln Asn Gly Pro Gly  Ser Gly Gln Gln Gly  Pro Gly Gln
    1205              1210                1215

Ser Gly  Gln Tyr Gly Pro Gly  Gln Gln Gly Pro Gly  Gln Gln Gly
    1220              1225                1230

Pro Gly  Ser Ser Ala Ala Ala  Ala Ala Gly Pro Gly  Gln Tyr Gly
    1235              1240                1245

Pro Gly  Gln Gln Gly Pro Ser  Ala Ser Ala Ala Ala  Ala Ala Gly
    1250              1255                1260

Pro Gly  Ser Gly Gln Gln Gly  Pro Gly Ala Ser Gly  Gln Tyr Gly
    1265              1270                1275

Pro Gly  Gln Gln Gly Pro Gly  Gln Gln Gly Pro Gly  Ser Ser Ala
    1280              1285                1290

Ala Ala  Ala Ala Gly Gln Tyr  Gly Ser Gly Pro Gly  Gln Gln Gly
    1295              1300                1305

Pro Tyr  Gly Ser Ala Ala Ala  Ala Ala Gly Pro Gly  Ser Gly Gln
    1310              1315                1320

Tyr Gly  Gln Gly Pro Tyr Gly  Pro Gly Ala Ser Gly  Pro Gly Gln
    1325              1330                1335

Tyr Gly  Pro Gly Gln Gln Gly  Pro Ser Ala Ser Ala  Ala Ala Ala
    1340              1345                1350

Ala Gly  Ser Gly Gln Gln Gly  Pro Gly Gln Tyr Gly  Pro Tyr Ala
    1355              1360                1365

Ser Ala  Ala Ala Ala Ala Gly  Gln Tyr Gly Ser Gly  Pro Gly Gln
    1370              1375                1380

Gln Gly  Pro Tyr Gly Pro Gly  Gln Ser Gly Ser Gly  Gln Gln Gly
    1385              1390                1395

Pro Gly  Gln Gln Gly Pro Tyr  Ala Ser Ala Ala Ala  Ala Ala Gly
    1400              1405                1410

Pro Gly  Gln Gln Gly Pro Tyr  Gly Pro Gly Ser Ser  Ala Ala Ala
    1415              1420                1425

Ala Ala  Gly Gln Tyr Gly Tyr  Gly Pro Gly Gln Gln  Gly Pro Tyr
    1430              1435                1440
```

86

```
Gly Pro  Gly Ala Ser Gly Gln  Asn Gly Pro Gly Ser  Gly Gln Tyr
    1445             1450             1455

Gly Pro  Gly Gln Gln Gly Pro  Gly Gln Ser Ala Ala  Ala Ala Ala
    1460             1465             1470

Gly Pro  Gly Gln Gln Gly Pro  Tyr Gly Pro Gly Ala  Ser Ala Ala
    1475             1480             1485

Ala Ala  Ala Gly Gln Tyr Gly  Pro Gly Gln Gln Gly  Pro Gly Gln
    1490             1495             1500

Tyr Gly  Pro Gly Ser Ser Gly  Pro Gly Gln Gln Gly  Pro Tyr Gly
    1505             1510             1515

Pro Gly  Ser Ser Ala Ala Ala  Ala Ala Gly Gln Tyr  Gly Pro Gly
    1520             1525             1530

Gln Gln  Gly Pro Tyr Gly Pro  Gly Gln Ser Ala Ala  Ala Ala Ala
    1535             1540             1545

Gly Gln  Tyr Gln Gln Gly Pro  Gly Gln Gln Gly Pro  Tyr Gly Pro
    1550             1555             1560

Gly Ala  Ser Gly Pro Gly Gln  Gln Gly Pro Tyr Gly  Pro Gly Ala
    1565             1570             1575

Ser Ala  Ala Ala Ala Ala Gly  Pro Gly Gln Tyr Gly  Pro Gly Gln
    1580             1585             1590

Gln Gly  Pro Ser Ala Ser Ala  Ala Ala Ala Gly Gln  Tyr Gly
    1595             1600             1605

Ser Gly  Pro Gly Gln Tyr Gly  Pro Tyr Gly Pro Gly  Gln Ser Gly
    1610             1615             1620

Pro Gly  Ser Gly Gln Gln Gly  Gln Gly Pro Tyr Gly  Pro Gly Ala
    1625             1630             1635

Ser Ala  Ala Ala Ala Ala Gly  Gln Tyr Gly Pro Gly  Gln Gln Gly
    1640             1645             1650

Pro Tyr  Gly Pro Gly Gln Ser  Ala Ala Ala Ala Ala  Gly Pro Gly
    1655             1660             1665

Ser Gly  Gln Tyr Gly Pro Gly  Ala Ser Gly Gln Asn  Gly Pro Gly
```

<pre>
        1670                          1675                          1680


    Ser Gly Gln Tyr Gly Pro Gly Gln Gln Gly Pro Gly Gln Ser Ala
        1685              1690              1695


    Ala Ala Ala Ala Gly Gln Tyr Gln Gln Gly Pro Gly Gln Gln Gly
        1700              1705              1710


    Pro Tyr Gly Pro Gly Ala Ser Ala Ala Ala Ala Ala Gly Gln Tyr
        1715              1720              1725


    Gly Ser Gly Pro Gly Gln Gln Gly Pro Tyr Gly Pro Gly Gln Ser
        1730              1735              1740


    Gly Ser Gly Gln Gln Gly Pro Gly Gln Gln Gly Pro Tyr Ala Ser
        1745              1750              1755


    Ala Ala Ala Ala Ala Gly Pro Gly Ser Gly Gln Gln Gly Pro Gly
        1760              1765              1770


    Ala Ser Gly Gln Gln Gly Pro Tyr Gly Pro Gly Ala Ser Ala Ala
        1775              1780              1785


    Ala Ala Ala Gly Gln Asn Gly Pro Gly Ser Gly Gln Gln Gly Pro
        1790              1795              1800


    Gly Gln Ser Gly Gln Tyr Gly Pro Gly Gln Gln Gly Pro Gly Gln
        1805              1810              1815


    Gln Gly Pro Gly Ser Ser Ala Ala Ala Ala Ala Gly Pro Gly Gln
        1820              1825              1830


    Tyr Gly Pro Gly Gln Gln Gly Pro Ser Ala Ser Ala Ala Ala Ala
        1835              1840              1845


    Ala Gly Pro Gly Ser Gly Gln Gln Gly Pro Gly Ala Ser Gly Gln
        1850              1855              1860


    Tyr Gly Pro Gly Gln Gln Gly Pro Gly Gln Gln Gly Pro Gly Ser
        1865              1870              1875


    Ser Ala Ala Ala Ala Ala Gly Gln Tyr Gly Ser Gly Pro Gly Gln
        1880              1885              1890


    Gln Gly Pro Tyr Gly Ser Ala Ala Ala Ala Ala Gly Pro Gly Ser
        1895              1900              1905
</pre>

```
Gly Gln  Tyr Gly Gln Gly Pro  Tyr Gly Pro Gly Ala  Ser Gly Pro
    1910                 1915                 1920

Gly Gln  Tyr Gly Pro Gly Gln  Gln Gly Pro Ser Ala  Ser Ala Ala
    1925                 1930                 1935

Ala Ala  Ala Gly Ser Gly Gln  Gln Gly Pro Gly Gln  Tyr Gly Pro
    1940                 1945                 1950

Tyr Ala  Ser Ala Ala Ala Ala  Ala Gly Gln Tyr Gly  Ser Gly Pro
    1955                 1960                 1965

Gly Gln  Gln Gly Pro Tyr Gly  Pro Gly Gln Ser Gly  Ser Gly Gln
    1970                 1975                 1980

Gln Gly  Pro Gly Gln Gln Gly  Pro Tyr Ala Ser Ala  Ala Ala Ala
    1985                 1990                 1995

Ala Gly  Pro Gly Gln Gln Gly  Pro Tyr Gly Pro Gly  Ser Ser Ala
    2000                 2005                 2010

Ala Ala  Ala Ala Gly Gln Tyr  Gly Tyr Gly Pro Gly  Gln Gln Gly
    2015                 2020                 2025

Pro Tyr  Gly Pro Gly Ala Ser  Gly Gln Asn Gly Pro  Gly Ser Gly
    2030                 2035                 2040

Gln Tyr  Gly Pro Gly Gln Gln  Gly Pro Gly Gln Ser  Ala Ala Ala
    2045                 2050                 2055

Ala Ala  Gly Pro Gly Gln Gln  Gly Pro Tyr Gly Pro  Gly Ala Ser
    2060                 2065                 2070

Ala Ala  Ala Ala Ala Gly Gln  Tyr Gly Pro Gly Gln  Gln Gly Pro
    2075                 2080                 2085

Gly Gln  Tyr Gly Pro Gly Ser  Ser Gly Pro Gly Gln  Gln Gly Pro
    2090                 2095                 2100

Tyr Gly  Pro Gly Ser Ser Ala  Ala Ala Ala Ala Gly  Gln Tyr Gly
    2105                 2110                 2115

Pro Gly  Gln Gln Gly Pro Tyr  Gly Pro Gly Gln Ser  Ala Ala Ala
    2120                 2125                 2130

Ala Ala  Gly Gln Tyr Gln Gln  Gly Pro Gly Gln Gln  Gly Pro Tyr
    2135                 2140                 2145
```

Gly Pro Gly Ala Ser Gly Pro Gly Gln Gln Gly Pro Tyr Gly Pro
2150          2155              2160

Gly Ala Ser Ala Ala Ala Ala Ala Gly Pro Gly Gln Tyr Gly Pro
2165          2170              2175

Gly Gln Gln Gly Pro Ser Ala Ser Ala Ala Ala Ala Gly Gln
2180          2185              2190

Tyr Gly Ser Gly Pro Gly Gln Tyr Gly Pro Tyr Gly Pro Gly Gln
2195          2200              2205

Ser Gly Pro Gly Ser Gly Gln Gln Gly Gln Gly Pro Tyr Gly Pro
2210          2215              2220

Gly Ala Ser Ala Ala Ala Ala Ala Gly Gln Tyr Gly Pro Gly Gln
2225          2230              2235

Gln Gly Pro Tyr Gly Pro Gly Gln Ser Ala Ala Ala Ala Ala Gly
2240          2245              2250

Pro Gly Ser Gly Gln Tyr Gly Pro Gly Ala Ser Gly Gln Asn Gly
2255          2260              2265

Pro Gly Ser Gly Gln Tyr Gly Pro Gly Gln Gln Gly Pro Gly Gln
2270          2275              2280

Ser Ala Ala Ala Ala Ala Gly Gln Tyr Gln Gln Gly Pro Gly Gln
2285          2290              2295

Gln Gly Pro Tyr Gly Pro Gly Ala Ser Ala Ala Ala Ala Ala Gly
2300          2305              2310

Gln Tyr Gly Ser Gly Pro Gly Gln Gln Gly Pro Tyr Gly Pro Gly
2315          2320              2325

Gln Ser Gly Ser Gly Gln Gln Gly Pro Gly Gln Gln Gly Pro Tyr
2330          2335              2340

Ala Ser Ala Ala Ala Ala Ala Gly Pro Gly Ser Gly Gln Gln Gly
2345          2350              2355

Ser Ser Val Asp Lys Leu Ala Ala Ala Leu Glu His His His His
2360          2365              2370

His His
2375

<210> 16
<211> 608
<212> PRT
<213> Artificial Sequence

<220>
<223> PRT313

<400> 16

Met His His His His His His Ser Ser Gly Ser Ser Gly Pro Gly Gly
1               5                   10                  15

Gln Gly Pro Tyr Gly Pro Gly Ala Ser Ala Ala Ala Ala Ala Gly Gly
            20                  25                  30

Asn Gly Pro Gly Ser Gly Gln Gln Gly Pro Gly Gly Ser Ala Ala Ala
            35                  40                  45

Ala Ala Gly Gly Tyr Gly Pro Gly Gly Gln Gly Pro Gly Gln Gln Gly
        50                  55                  60

Pro Gly Ser Ser Ala Ala Ala Ala Ala Gly Pro Gly Gly Tyr Gly Pro
65                  70                  75                  80

Gly Gly Gln Gly Pro Ser Ala Ser Ala Ala Ala Ala Ala Gly Pro Gly
                85                  90                  95

Ser Gly Gln Gln Gly Pro Gly Ala Ser Ala Ala Ala Ala Ala Gly Gly
            100                 105                 110

Tyr Gly Pro Gly Gly Gln Gly Pro Gly Gln Gln Gly Pro Gly Ser Ser
            115                 120                 125

Ala Ala Ala Ala Ala Gly Gly Tyr Gly Ser Gly Pro Gly Gln Gln Gly
        130                 135                 140

Pro Tyr Gly Ser Ala Ala Ala Ala Ala Gly Pro Gly Ser Gly Gly Tyr
145                 150                 155                 160

Gly Gln Gly Pro Tyr Gly Pro Gly Ala Ser Ala Ala Ala Ala Ala Gly
            165                 170                 175

Pro Gly Gly Tyr Gly Pro Gly Gly Gln Gly Pro Ser Ala Ser Ala Ala
            180                 185                 190

Ala Ala Ala Gly Ser Gly Gln Gln Gly Pro Gly Gly Tyr Gly Pro Tyr
        195                 200                 205

Ala Ser Ala Ala Ala Ala Ala Gly Gly Tyr Gly Ser Gly Pro Gly Gln
210               215               220

Gln Gly Pro Tyr Gly Pro Gly Gly Ser Ala Ala Ala Ala Ala Gly Ser
225               230               235               240

Gly Gln Gln Gly Pro Gly Gln Gln Gly Pro Tyr Ala Ser Ala Ala Ala
245               250               255

Ala Ala Gly Pro Gly Gly Gln Gly Pro Tyr Gly Pro Gly Ser Ser Ala
260               265               270

Ala Ala Ala Ala Gly Gly Tyr Gly Tyr Gly Pro Gly Gly Gln Gly Pro
275               280               285

Tyr Gly Pro Gly Ala Ser Ala Ala Ala Ala Gly Gly Asn Gly Pro
290               295               300

Gly Ser Gly Gly Tyr Gly Pro Gly Gln Gln Gly Pro Gly Gly Ser Ala
305               310               315               320

Ala Ala Ala Gly Pro Gly Gly Gln Gly Pro Tyr Gly Pro Gly Ala
325               330               335

Ser Ala Ala Ala Ala Ala Gly Gly Tyr Gly Pro Gly Gly Gln Gly Pro
340               345               350

Gly Gly Tyr Gly Pro Gly Ser Ser Ala Ala Ala Ala Ala Gly Pro Gly
355               360               365

Gly Gln Gly Pro Tyr Gly Pro Gly Ser Ser Ala Ala Ala Ala Ala Gly
370               375               380

Gly Tyr Gly Pro Gly Gln Gln Gly Pro Tyr Gly Pro Gly Gly Ser Ala
385               390               395               400

Ala Ala Ala Gly Gly Tyr Gln Gln Gly Pro Gly Gly Gln Gly Pro
405               410               415

Tyr Gly Pro Gly Ala Ser Ala Ala Ala Ala Gly Pro Gly Gly Gln
420               425               430

Gly Pro Tyr Gly Pro Gly Ala Ser Ala Ala Ala Ala Gly Pro Gly
435               440               445

Gly Tyr Gly Pro Gly Gly Gln Gly Pro Ser Ala Ser Ala Ala Ala Ala
450               455               460

92

Ala Gly Gly Tyr Gly Ser Gly Pro Gly Gly Tyr Gly Pro Tyr Gly Pro
465             470             475             480

Gly Gly Ser Ala Ala Ala Ala Ala Gly Pro Gly Ser Gly Gln Gln Gly
            485             490             495

Gln Gly Pro Tyr Gly Pro Gly Ala Ser Ala Ala Ala Ala Ala Gly Gly
            500             505             510

Tyr Gly Pro Gly Gln Gln Gly Pro Tyr Gly Pro Gly Gly Ser Ala Ala
            515             520             525

Ala Ala Ala Gly Pro Gly Ser Gly Gly Tyr Gly Pro Gly Ala Ser Ala
    530             535             540

Ala Ala Ala Gly Gly Asn Gly Pro Gly Ser Gly Gly Tyr Gly Pro
545             550             555             560

Gly Gln Gln Gly Pro Gly Gly Ser Ala Ala Ala Ala Ala Gly Gly Tyr
            565             570             575

Gln Gln Gly Pro Gly Gly Gln Gly Pro Tyr Gly Pro Gly Ala Ser Ala
            580             585             590

Ala Ala Ala Gly Pro Gly Ser Gly Gln Gln Gly Pro Gly Ala Ser
    595             600             605

<210>   17
<211>   590
<212>   PRT
<213>   Artificial Sequence

<220>
<223>   Met-PRT399

<400>   17

Met Gly Pro Gly Gly Gln Gly Pro Tyr Gly Pro Gly Ala Ser Ala Ala
1               5               10              15

Ala Ala Ala Gly Gly Asn Gly Pro Gly Ser Gly Gln Gln Gly Pro Gly
            20              25              30

Gly Ser Gly Gly Tyr Gly Pro Gly Gly Gln Gly Pro Gly Gln Gln Gly
            35              40              45

Pro Gly Ser Ser Ala Ala Ala Ala Ala Gly Pro Gly Gly Tyr Gly Pro
    50              55              60

93

Gly Gly Gln Gly Pro Ser Ala Ser Ala Ala Ala Ala Ala Gly Pro Gly
65                70              75                    80

Ser Gly Gln Gln Gly Pro Gly Ala Ser Gly Gly Tyr Gly Pro Gly Gly
                85              90              95

Gln Gly Pro Gly Gln Gln Gly Pro Gly Ser Ser Ala Ala Ala Ala Ala
            100             105             110

Gly Gly Tyr Gly Ser Gly Pro Gly Gln Gln Gly Pro Tyr Gly Ser Ala
        115             120             125

Ala Ala Ala Ala Gly Pro Gly Ser Gly Gly Tyr Gly Gln Gly Pro Tyr
    130             135             140

Gly Pro Gly Ala Ser Gly Pro Gly Gly Tyr Gly Pro Gly Gly Gln Gly
145             150             155             160

Pro Ser Ala Ser Ala Ala Ala Ala Ala Gly Ser Gly Gln Gln Gly Pro
            165             170             175

Gly Gly Tyr Gly Pro Tyr Ala Ser Ala Ala Ala Ala Gly Gly Tyr
        180             185             190

Gly Ser Gly Pro Gly Gln Gln Gly Pro Tyr Gly Pro Gly Gly Ser Gly
        195             200             205

Ser Gly Gln Gln Gly Pro Gly Gln Gln Gly Pro Tyr Ala Ser Ala Ala
    210             215             220

Ala Ala Ala Gly Pro Gly Gly Gln Gly Pro Tyr Gly Pro Gly Ser Ser
225             230             235             240

Ala Ala Ala Ala Ala Gly Gly Tyr Gly Tyr Gly Pro Gly Gly Gln Gly
            245             250             255

Pro Tyr Gly Pro Gly Ala Ser Gly Gly Asn Gly Pro Gly Ser Gly Gly
        260             265             270

Tyr Gly Pro Gly Gln Gln Gly Pro Gly Gly Ser Ala Ala Ala Ala Ala
        275             280             285

Gly Pro Gly Gly Gln Gly Pro Tyr Gly Pro Gly Ala Ser Ala Ala Ala
    290             295             300

Ala Ala Gly Gly Tyr Gly Pro Gly Gly Gln Gly Pro Gly Gly Tyr Gly
305             310             315             320

94

```
Pro Gly Ser Ser Gly Pro Gly Gly Gln Gly Pro Tyr Gly Pro Gly Ser
            325                     330                 335

Ser Ala Ala Ala Ala Ala Gly Gly Tyr Gly Pro Gly Gln Gln Gly Pro
            340                     345                 350

Tyr Gly Pro Gly Gly Ser Ala Ala Ala Ala Ala Gly Gly Tyr Gln Gln
            355                     360                 365

Gly Pro Gly Gly Gln Gly Pro Tyr Gly Pro Gly Ala Ser Gly Pro Gly
            370                     375                 380

Gly Gln Gly Pro Tyr Gly Pro Gly Ala Ser Ala Ala Ala Ala Ala Gly
    385                     390                     395                 400

Pro Gly Gly Tyr Gly Pro Gly Gly Gln Gly Pro Ser Ala Ser Ala Ala
            405                     410                 415

Ala Ala Ala Gly Gly Tyr Gly Ser Gly Pro Gly Gly Tyr Gly Pro Tyr
            420                     425                 430

Gly Pro Gly Gly Ser Gly Pro Gly Ser Gly Gln Gln Gly Gln Gly Pro
            435                     440                 445

Tyr Gly Pro Gly Ala Ser Ala Ala Ala Ala Gly Gly Tyr Gly Pro
    450                     455                     460

Gly Gln Gln Gly Pro Tyr Gly Pro Gly Gly Ser Ala Ala Ala Ala Ala
    465                     470                     475                 480

Gly Pro Gly Ser Gly Gly Tyr Gly Pro Gly Ala Ser Gly Gly Asn Gly
            485                     490                 495

Pro Gly Ser Gly Gly Tyr Gly Pro Gly Gln Gln Gly Pro Gly Gly Ser
            500                     505                 510

Ala Ala Ala Ala Ala Gly Gly Tyr Gln Gln Gly Pro Gly Gly Gln Gly
            515                     520                 525

Pro Tyr Gly Pro Gly Ala Ser Ala Ala Ala Ala Gly Gly Tyr Gly
    530                     535                     540

Ser Gly Pro Gly Gln Gln Gly Pro Tyr Gly Pro Gly Gly Ser Gly Ser
    545                     550                     555                 560

Gly Gln Gln Gly Pro Gly Gln Gln Gly Pro Tyr Ala Ser Ala Ala Ala
```

95

```
                  565                     570                        575


         Ala Ala Gly Pro Gly Ser Gly Gln Gln Gly Pro Gly Ala Ser
                     580                 585                  590


         <210>  18
         <211>  601
         <212>  PRT
         <213>  Artificial Sequence

         <220>
         <223>  PRT399

         <400>  18

         Met His His His His His His Ser Ser Gly Ser Ser Gly Pro Gly Gly
         1               5                   10                  15


         Gln Gly Pro Tyr Gly Pro Gly Ala Ser Ala Ala Ala Ala Ala Gly Gly
                     20                  25                  30


         Asn Gly Pro Gly Ser Gly Gln Gln Gly Pro Gly Gly Ser Gly Gly Tyr
                     35                  40                  45


         Gly Pro Gly Gly Gln Gly Pro Gly Gln Gln Gly Pro Gly Ser Ser Ala
                     50                  55                  60


         Ala Ala Ala Ala Gly Pro Gly Gly Tyr Gly Pro Gly Gly Gln Gly Pro
         65                  70                  75                  80


         Ser Ala Ser Ala Ala Ala Ala Ala Gly Pro Gly Ser Gly Gln Gln Gly
                     85                  90                  95


         Pro Gly Ala Ser Gly Gly Tyr Gly Pro Gly Gly Gln Gly Pro Gly Gln
                     100                 105                 110


         Gln Gly Pro Gly Ser Ser Ala Ala Ala Ala Ala Gly Gly Tyr Gly Ser
                     115                 120                 125


         Gly Pro Gly Gln Gln Gly Pro Tyr Gly Ser Ala Ala Ala Ala Ala Gly
                     130                 135                 140


         Pro Gly Ser Gly Gly Tyr Gly Gln Gly Pro Tyr Gly Pro Gly Ala Ser
         145                 150                 155                 160


         Gly Pro Gly Gly Tyr Gly Pro Gly Gly Gln Gly Pro Ser Ala Ser Ala
                     165                 170                 175


         Ala Ala Ala Ala Gly Ser Gly Gln Gln Gly Pro Gly Gly Tyr Gly Pro
                     180                 185                 190
```

96

Tyr Ala Ser Ala Ala Ala Ala Ala Gly Gly Tyr Gly Ser Gly Pro Gly
    195                  200              205

Gln Gln Gly Pro Tyr Gly Pro Gly Gly Ser Gly Ser Gly Gln Gln Gly
    210                  215              220

Pro Gly Gln Gln Gly Pro Tyr Ala Ser Ala Ala Ala Ala Ala Gly Pro
225                230          235              240

Gly Gly Gln Gly Pro Tyr Gly Pro Gly Ser Ser Ala Ala Ala Ala Ala
            245            250            255

Gly Gly Tyr Gly Tyr Gly Pro Gly Gly Gln Gly Pro Tyr Gly Pro Gly
        260              265          270

Ala Ser Gly Gly Asn Gly Pro Gly Ser Gly Gly Tyr Gly Pro Gly Gln
    275                280          285

Gln Gly Pro Gly Gly Ser Ala Ala Ala Ala Gly Pro Gly Gly Gln
    290              295          300

Gly Pro Tyr Gly Pro Gly Ala Ser Ala Ala Ala Ala Gly Gly Tyr
305              310          315          320

Gly Pro Gly Gly Gln Gly Pro Gly Gly Tyr Gly Pro Gly Ser Ser Gly
        325              330          335

Pro Gly Gly Gln Gly Pro Tyr Gly Pro Gly Ser Ser Ala Ala Ala Ala
        340            345          350

Ala Gly Gly Tyr Gly Pro Gly Gln Gln Gly Pro Tyr Gly Pro Gly Gly
        355            360          365

Ser Ala Ala Ala Ala Ala Gly Gly Tyr Gln Gln Gly Pro Gly Gly Gln
    370                375          380

Gly Pro Tyr Gly Pro Gly Ala Ser Gly Pro Gly Gly Gln Gly Pro Tyr
385              390          395          400

Gly Pro Gly Ala Ser Ala Ala Ala Ala Ala Gly Pro Gly Gly Tyr Gly
            405            410          415

Pro Gly Gly Gln Gly Pro Ser Ala Ser Ala Ala Ala Ala Ala Gly Gly
        420            425          430

Tyr Gly Ser Gly Pro Gly Gly Tyr Gly Pro Tyr Gly Pro Gly Gly Ser

```
                 435                        440                         445


        Gly Pro Gly Ser Gly Gln Gln Gly Gln Gly Pro Tyr Gly Pro Gly Ala
            450                 455                 460


        Ser Ala Ala Ala Ala Ala Gly Gly Tyr Gly Pro Gly Gln Gln Gly Pro
        465                 470                 475                 480


        Tyr Gly Pro Gly Gly Ser Ala Ala Ala Ala Gly Pro Gly Ser Gly
                        485                 490                 495


        Gly Tyr Gly Pro Gly Ala Ser Gly Gly Asn Gly Pro Gly Ser Gly Gly
                    500                 505                 510


        Tyr Gly Pro Gly Gln Gln Gly Pro Gly Gly Ser Ala Ala Ala Ala Ala
                    515                 520                 525


        Gly Gly Tyr Gln Gln Gly Pro Gly Gly Gln Gly Pro Tyr Gly Pro Gly
            530                 535                 540


        Ala Ser Ala Ala Ala Ala Ala Gly Gly Tyr Gly Ser Gly Pro Gly Gln
        545                 550                 555                 560


        Gln Gly Pro Tyr Gly Pro Gly Gly Ser Gly Ser Gly Gln Gln Gly Pro
                        565                 570                 575


        Gly Gln Gln Gly Pro Tyr Ala Ser Ala Ala Ala Ala Gly Pro Gly
                    580                 585                 590


        Ser Gly Gln Gln Gly Pro Gly Ala Ser
            595                 600


        <210>   19
        <211>   612
        <212>   PRT
        <213>   Artificial Sequence

        <220>
        <223>   Met-PRT720

        <400>   19

        Met Gly Pro Gly Gln Gln Gly Pro Tyr Gly Pro Gly Ala Ser Ala Ala
        1                   5                   10                  15


        Ala Ala Ala Gly Gln Asn Gly Pro Gly Ser Gly Gln Gln Gly Pro Gly
                    20                  25                  30


        Gln Ser Gly Gln Tyr Gly Pro Gly Gln Gln Gly Pro Gly Gln Gln Gly
                35                  40                  45
```

98

```
Pro Gly Ser Ser Ala Ala Ala Ala Ala Gly Pro Gly Gln Tyr Val Leu
    50                  55                  60

Ile Gly Pro Gly Gln Gln Val Leu Ile Gly Pro Ser Ala Ser Ala Ala
65                  70                  75                  80

Ala Ala Ala Gly Pro Gly Ser Gly Gln Gln Gly Pro Gly Ala Ser Gly
                85                  90                  95

Gln Tyr Gly Pro Gly Gln Gln Gly Pro Gly Gln Gln Gly Pro Gly Ser
            100                 105                 110

Ser Ala Ala Ala Ala Ala Gly Ser Tyr Gly Ser Val Leu Ile Gly Pro
            115                 120                 125

Gly Gln Gln Val Leu Ile Gly Pro Tyr Gly Ser Ala Ala Ala Ala Ala
    130                 135                 140

Gly Pro Gly Ser Gly Gln Tyr Gly Gln Gly Pro Tyr Gly Pro Gly Ala
145                 150                 155                 160

Ser Gly Pro Gly Gln Tyr Gly Pro Gly Gln Gln Gly Pro Ser Ala Ser
            165                 170                 175

Ala Ala Ala Ala Ala Gly Ser Gly Gln Gln Val Leu Ile Gly Pro Gly
        180                 185                 190

Gln Tyr Val Leu Ile Gly Pro Tyr Ala Ser Ala Ala Ala Ala Ala Gly
        195                 200                 205

Gln Tyr Gly Ser Gly Pro Gly Gln Gln Gly Pro Tyr Gly Pro Gly Gln
    210                 215                 220

Ser Gly Ser Gly Gln Gln Gly Pro Gly Gln Gln Gly Pro Tyr Ala Ser
225                 230                 235                 240

Ala Ala Ala Ala Ala Gly Pro Gly Gln Gln Val Leu Ile Gly Pro Tyr
            245                 250                 255

Val Leu Ile Gly Pro Gly Ser Ser Ala Ala Ala Ala Gly Gln Tyr
            260                 265                 270

Gly Tyr Gly Pro Gly Gln Gln Gly Pro Tyr Gly Pro Gly Ala Ser Gly
    275                 280                 285

Gln Asn Gly Pro Gly Ser Gly Gln Tyr Gly Pro Gly Gln Gln Gly Pro
```

                290                          295                          300

Gly Gln Ser Ala Ala Ala Ala Ala Gly Pro Gly Gln Gln Val Leu Ile
305                310                315                320

Gly Pro Tyr Val Leu Ile Gly Pro Gly Ala Ser Ala Ala Ala Ala Ala
                325                330                335

Gly Gln Tyr Gly Pro Gly Gln Gln Gly Pro Gly Gln Tyr Gly Pro Gly
                340                345                350

Ser Ser Gly Pro Gly Gln Gln Gly Pro Tyr Gly Pro Gly Ser Ser Ala
                355                360                365

Ala Ala Ala Ala Gly Ser Tyr Gly Pro Gly Gln Gln Val Leu Ile Gly
                370                375                380

Pro Tyr Val Leu Ile Gly Pro Gly Pro Ser Ala Ala Ala Ala Ala Gly
385                390                395                400

Gln Tyr Gln Gln Gly Pro Gly Gln Gln Gly Pro Tyr Gly Pro Gly Ala
                405                410                415

Ser Gly Pro Gly Gln Gln Gly Pro Tyr Gly Pro Gly Ala Ser Ala Ala
                420                425                430

Ala Ala Ala Gly Pro Gly Gln Tyr Val Leu Ile Gly Pro Gly Gln Gln
                435                440                445

Val Leu Ile Gly Pro Ser Ala Ser Ala Ala Ala Ala Gly Gln Tyr
                450                455                460

Gly Ser Gly Pro Gly Gln Tyr Gly Pro Tyr Gly Pro Gly Gln Ser Gly
465                470                475                480

Pro Gly Ser Gly Gln Gln Gly Gln Gly Pro Tyr Gly Pro Gly Ala Ser
                485                490                495

Ala Ala Ala Ala Ala Gly Ser Tyr Gly Pro Gly Gln Gln Val Leu Ile
                500                505                510

Gly Pro Tyr Val Leu Ile Gly Pro Gly Pro Ser Ala Ala Ala Ala Ala
                515                520                525

Gly Pro Gly Ser Gly Gln Tyr Gly Pro Gly Ala Ser Gly Gln Asn Gly
                530                535                540

```
Pro Gly Ser Gly Gln Tyr Gly Pro Gly Gln Gln Gly Pro Gly Gln Ser
545                 550                 555                 560


Ala Ala Ala Ala Ala Gly Gln Tyr Gln Gln Val Leu Ile Gly Pro Gly
                565                 570                 575


Gln Gln Gly Pro Tyr Val Leu Ile Gly Pro Gly Ala Ser Ala Ala Ala
            580                 585                 590


Ala Ala Gly Pro Gly Ser Gly Gln Gln Val Leu Ile Gly Pro Gly Ala
            595                 600                 605


Ser Val Leu Ile
        610



<210>   20
<211>   592
<212>   PRT
<213>   Artificial Sequence

<220>
<223>   Met-PRT665

<400>   20

Met Gly Pro Gly Gln Gln Gly Pro Tyr Gly Pro Gly Ala Ser Ala Ala
1               5                   10                  15


Ala Ala Ala Ala Ala Gly Ser Asn Gly Pro Gly Ser Gly Gln Gln Gly
                20                  25                  30


Pro Gly Gln Ser Gly Gln Tyr Gly Pro Gly Gln Gln Gly Pro Gly Gln
            35                  40                  45


Gln Gly Pro Gly Ser Ser Ala Ala Ala Ala Ala Ala Gly Pro Gly
        50                  55                  60


Gln Tyr Val Leu Ile Gly Pro Gly Gln Gln Gly Pro Ser Ala Ser Ala
65                  70                  75                  80


Ala Ala Ala Ala Ala Gly Pro Gly Ser Gly Gln Gln Gly Pro Gly
                85                  90                  95


Ala Ser Gly Gln Tyr Gly Pro Gly Gln Gln Gly Pro Gly Gln Gln Gly
            100                 105                 110


Pro Gly Ser Ser Ala Ala Ala Ala Ala Ala Ala Gly Ser Tyr Gly Ser
            115                 120                 125


Val Leu Ile Gly Pro Gly Gln Gln Gly Pro Tyr Gly Ser Ala Ala Ala
```

```
                130                        135                         140


        Ala Ala Ala Ala Gly Pro Gly Ser Gly Gln Tyr Gly Gln Gly Pro Tyr
        145                 150                 155                 160


        Gly Pro Gly Ala Ser Gly Pro Gly Gln Tyr Gly Pro Gly Gln Gln Gly
                        165                 170                 175


        Pro Ser Ala Ser Ala Ala Ala Ala Ala Ala Ala Gly Ser Gly Gln Gln
                    180                 185                 190


        Val Leu Ile Gly Pro Gly Gln Tyr Gly Pro Tyr Ala Ser Ala Ala Ala
                    195                 200                 205


        Ala Ala Ala Ala Gly Ser Tyr Gly Ser Gly Pro Gly Gln Gln Gly Pro
                    210                 215                 220


        Tyr Gly Pro Gly Gln Ser Gly Ser Gly Gln Gln Gly Pro Gly Gln Gln
        225                 230                 235                 240


        Gly Pro Tyr Ala Ser Ala Ala Ala Ala Ala Ala Gly Pro Gly Gln
                        245                 250                 255


        Gln Val Leu Ile Gly Pro Tyr Gly Pro Gly Ser Ser Ala Ala Ala Ala
                    260                 265                 270


        Ala Ala Ala Gly Ser Tyr Gly Tyr Gly Pro Gly Gln Gln Gly Pro Tyr
                275                 280                 285


        Gly Pro Gly Ala Ser Gly Gln Asn Gly Pro Gly Ser Gly Gln Tyr Gly
                290                 295                 300


        Pro Gly Gln Gln Gly Pro Gly Pro Ser Ala Ala Ala Ala Ala Ala Ala
        305                 310                 315                 320


        Gly Pro Gly Gln Gln Val Leu Ile Gly Pro Tyr Gly Pro Gly Ala Ser
                        325                 330                 335


        Ala Ala Ala Ala Ala Ala Ala Gly Ser Tyr Gly Pro Gly Gln Gln Gly
                    340                 345                 350


        Pro Gly Gln Tyr Gly Pro Gly Ser Ser Gly Pro Gly Gln Gln Gly Pro
                    355                 360                 365


        Tyr Gly Pro Gly Ser Ser Ala Ala Ala Ala Ala Ala Ala Gly Ser Tyr
        370                 375                 380
```

```
Gly Pro Gly Gln Gln Val Leu Ile Gly Pro Tyr Gly Pro Gly Pro Ser
385                 390                 395                 400

Ala Ala Ala Ala Ala Ala Ala Gly Ser Tyr Gln Gln Gly Pro Gly Gln
                405                 410                 415

Gln Gly Pro Tyr Gly Pro Gly Ala Ser Gly Pro Gly Gln Gln Gly Pro
            420                 425                 430

Tyr Gly Pro Gly Ala Ser Ala Ala Ala Ala Ala Ala Ala Gly Pro Gly
            435                 440                 445

Gln Tyr Val Leu Ile Gly Pro Gly Gln Gln Gly Pro Ser Ala Ser Ala
    450                 455                 460

Ala Ala Ala Ala Ala Ala Gly Ser Tyr Gly Ser Gly Pro Gly Gln Tyr
465                 470                 475                 480

Gly Pro Tyr Gly Pro Gly Gln Ser Gly Pro Gly Ser Gly Gln Gln Gly
            485                 490                 495

Gln Gly Pro Tyr Gly Pro Gly Ala Ser Ala Ala Ala Ala Ala Ala Ala
            500                 505                 510

Gly Ser Tyr Gly Pro Gly Gln Gln Val Leu Ile Gly Pro Tyr Gly Pro
    515                 520                 525

Gly Pro Ser Ala Ala Ala Ala Ala Ala Ala Gly Pro Gly Ser Gly Gln
    530                 535                 540

Tyr Gly Pro Gly Ala Ser Gly Gln Asn Gly Pro Gly Ser Gly Gln Tyr
545                 550                 555                 560

Gly Pro Gly Gln Gln Gly Pro Gly Pro Ser Ala Ala Ala Ala Ala Ala
            565                 570                 575

Ala Gly Pro Gly Ser Gly Gln Gln Gly Pro Gly Ala Ser Val Leu Ile
            580                 585                 590
```

```
<210>  21
<211>  619
<212>  PRT
<213>  Artificial Sequence

<220>
<223>  Met-PRT666

<400>  21

Met Gly Pro Gly Gln Gln Gly Pro Tyr Gly Pro Gly Ala Ser Ala Ala
```

```
            1                    5                        10                              15

            Ala Ala Ala Ala Ala Gly Ser Asn Gly Pro Gly Ser Gly Gln Gln Gly
                    20                  25                  30

            Pro Gly Gln Ser Gly Gln Tyr Gly Pro Gly Gln Gln Gly Pro Gly Gln
                    35                  40                  45

            Gln Gly Pro Gly Ser Ser Ala Ala Ala Ala Ala Ala Gly Pro Gly
                    50                  55                  60

            Gln Tyr Val Leu Ile Gly Pro Gly Gln Gln Val Leu Ile Gly Pro Ser
            65                  70                  75                  80

            Ala Ser Ala Ala Ala Ala Ala Ala Gly Pro Gly Ser Gly Gln Gln
                            85                  90                  95

            Gly Pro Gly Ala Ser Gly Gln Tyr Gly Pro Gly Gln Gln Gly Pro Gly
                    100                 105                 110

            Gln Gln Gly Pro Gly Ser Ser Ala Ala Ala Ala Ala Ala Ala Gly Ser
                    115                 120                 125

            Tyr Gly Ser Val Leu Ile Gly Pro Gly Gln Gln Val Leu Ile Gly Pro
                    130                 135                 140

            Tyr Gly Ser Ala Ala Ala Ala Ala Ala Ala Gly Pro Gly Ser Gly Gln
            145                 150                 155                 160

            Tyr Gly Gln Gly Pro Tyr Gly Pro Gly Ala Ser Gly Pro Gly Gln Tyr
                        165                 170                 175

            Gly Pro Gly Gln Gln Gly Pro Ser Ala Ser Ala Ala Ala Ala Ala Ala
                    180                 185                 190

            Ala Gly Ser Gly Gln Gln Val Leu Ile Gly Pro Gly Gln Tyr Val Leu
                    195                 200                 205

            Ile Gly Pro Tyr Ala Ser Ala Ala Ala Ala Ala Ala Gly Ser Tyr
                    210                 215                 220

            Gly Ser Gly Pro Gly Gln Gln Gly Pro Tyr Gly Pro Gly Gln Ser Gly
            225                 230                 235                 240

            Ser Gly Gln Gln Gly Pro Gly Gln Gln Gly Pro Tyr Ala Ser Ala Ala
                        245                 250                 255
```

```
Ala Ala Ala Ala Ala Gly Pro Gly Gln Gln Val Leu Ile Gly Pro Tyr
            260             265             270

Val Leu Ile Gly Pro Gly Ser Ser Ala Ala Ala Ala Ala Ala Ala Gly
            275             280             285

Ser Tyr Gly Tyr Gly Pro Gly Gln Gln Gly Pro Tyr Gly Pro Gly Ala
            290             295             300

Ser Gly Gln Asn Gly Pro Gly Ser Gly Gln Tyr Gly Pro Gly Gln Gln
305             310             315             320

Gly Pro Gly Pro Ser Ala Ala Ala Ala Ala Ala Ala Gly Pro Gly Gln
            325             330             335

Gln Val Leu Ile Gly Pro Tyr Val Leu Ile Gly Pro Gly Ala Ser Ala
            340             345             350

Ala Ala Ala Ala Ala Ala Gly Ser Tyr Gly Pro Gly Gln Gln Gly Pro
            355             360             365

Gly Gln Tyr Gly Pro Gly Ser Ser Gly Pro Gly Gln Gln Gly Pro Tyr
            370             375             380

Gly Pro Gly Ser Ser Ala Ala Ala Ala Ala Ala Gly Ser Tyr Gly
385             390             395             400

Pro Gly Gln Gln Val Leu Ile Gly Pro Tyr Val Leu Ile Gly Pro Gly
            405             410             415

Pro Ser Ala Ala Ala Ala Ala Ala Gly Ser Tyr Gln Gln Gly Pro
            420             425             430

Gly Gln Gln Gly Pro Tyr Gly Pro Gly Ala Ser Gly Pro Gly Gln Gln
            435             440             445

Gly Pro Tyr Gly Pro Gly Ala Ser Ala Ala Ala Ala Ala Ala Gly
            450             455             460

Pro Gly Gln Tyr Val Leu Ile Gly Pro Gly Gln Gln Val Leu Ile Gly
465             470             475             480

Pro Ser Ala Ser Ala Ala Ala Ala Ala Ala Gly Ser Tyr Gly Ser
            485             490             495

Gly Pro Gly Gln Tyr Gly Pro Tyr Gly Pro Gly Gln Ser Gly Pro Gly
            500             505             510
```

```
Ser Gly Gln Gln Gly Gln Gly Pro Tyr Gly Pro Gly Ala Ser Ala Ala
        515                 520             525

Ala Ala Ala Ala Ala Gly Ser Tyr Gly Pro Gly Gln Gln Val Leu Ile
        530                 535             540

Gly Pro Tyr Val Leu Ile Gly Pro Gly Pro Ser Ala Ala Ala Ala Ala
545                 550             555                 560

Ala Ala Gly Pro Gly Ser Gly Gln Tyr Gly Pro Gly Ala Ser Gly Gln
                565                 570             575

Asn Gly Pro Gly Ser Gly Gln Tyr Gly Pro Gly Gln Gln Gly Pro Gly
            580                 585             590

Pro Ser Ala Ala Ala Ala Ala Ala Ala Gly Pro Gly Ser Gly Gln Gln
        595                 600             605

Val Leu Ile Gly Pro Gly Ala Ser Val Leu Ile
    610                 615
```

```
<210>   22
<211>   623
<212>   PRT
<213>   Artificial Sequence

<220>
<223>   PRT720

<400>   22
```

```
Met His His His His His His Ser Ser Gly Ser Ser Gly Pro Gly Gln
1               5               10              15

Gln Gly Pro Tyr Gly Pro Gly Ala Ser Ala Ala Ala Ala Gly Gln
            20              25              30

Asn Gly Pro Gly Ser Gly Gln Gln Gly Pro Gly Gln Ser Gly Gln Tyr
            35              40              45

Gly Pro Gly Gln Gln Gly Pro Gly Gln Gln Gly Pro Gly Ser Ser Ala
    50                  55                  60

Ala Ala Ala Gly Pro Gly Gln Tyr Val Leu Ile Gly Pro Gly Gln
65                  70              75                  80

Gln Val Leu Ile Gly Pro Ser Ala Ser Ala Ala Ala Ala Gly Pro
                85                  90                  95
```

```
Gly Ser Gly Gln Gln Gly Pro Gly Ala Ser Gly Gln Tyr Gly Pro Gly
        100             105             110

Gln Gln Gly Pro Gly Gln Gln Gly Pro Gly Ser Ser Ala Ala Ala Ala
        115             120             125

Ala Gly Ser Tyr Gly Ser Val Leu Ile Gly Pro Gly Gln Gln Val Leu
        130             135             140

Ile Gly Pro Tyr Gly Ser Ala Ala Ala Ala Gly Pro Gly Ser Gly
145             150             155             160

Gln Tyr Gly Gln Gly Pro Tyr Gly Pro Gly Ala Ser Gly Pro Gly Gln
        165             170             175

Tyr Gly Pro Gly Gln Gln Gly Pro Ser Ala Ser Ala Ala Ala Ala Ala
        180             185             190

Gly Ser Gly Gln Gln Val Leu Ile Gly Pro Gly Gln Tyr Val Leu Ile
        195             200             205

Gly Pro Tyr Ala Ser Ala Ala Ala Ala Gly Gln Tyr Gly Ser Gly
        210             215             220

Pro Gly Gln Gln Gly Pro Tyr Gly Pro Gly Gln Ser Gly Ser Gly Gln
225             230             235             240

Gln Gly Pro Gly Gln Gln Gly Pro Tyr Ala Ser Ala Ala Ala Ala Ala
        245             250             255

Gly Pro Gly Gln Gln Val Leu Ile Gly Pro Tyr Val Leu Ile Gly Pro
        260             265             270

Gly Ser Ser Ala Ala Ala Ala Ala Gly Gln Tyr Gly Tyr Gly Pro Gly
        275             280             285

Gln Gln Gly Pro Tyr Gly Pro Gly Ala Ser Gly Gln Asn Gly Pro Gly
        290             295             300

Ser Gly Gln Tyr Gly Pro Gly Gln Gln Gly Pro Gly Gln Ser Ala Ala
305             310             315             320

Ala Ala Ala Gly Pro Gly Gln Gln Val Leu Ile Gly Pro Tyr Val Leu
        325             330             335

Ile Gly Pro Gly Ala Ser Ala Ala Ala Ala Ala Gly Gln Tyr Gly Pro
        340             345             350
```

Gly Gln Gln Gly Pro Gly Gln Tyr Gly Pro Gly Ser Ser Gly Pro Gly
        355                 360                 365

Gln Gln Gly Pro Tyr Gly Pro Gly Ser Ser Ala Ala Ala Ala Ala Gly
        370                 375                 380

Ser Tyr Gly Pro Gly Gln Gln Val Leu Ile Gly Pro Tyr Val Leu Ile
385                 390                 395                 400

Gly Pro Gly Pro Ser Ala Ala Ala Ala Ala Gly Gln Tyr Gln Gln Gly
                405                 410                 415

Pro Gly Gln Gln Gly Pro Tyr Gly Pro Gly Ala Ser Gly Pro Gly Gln
                420                 425                 430

Gln Gly Pro Tyr Gly Pro Gly Ala Ser Ala Ala Ala Ala Ala Gly Pro
        435                 440                 445

Gly Gln Tyr Val Leu Ile Gly Pro Gly Gln Gln Val Leu Ile Gly Pro
        450                 455                 460

Ser Ala Ser Ala Ala Ala Ala Ala Gly Gln Tyr Gly Ser Gly Pro Gly
465                 470                 475                 480

Gln Tyr Gly Pro Tyr Gly Pro Gly Gln Ser Gly Pro Gly Ser Gly Gln
                485                 490                 495

Gln Gly Gln Gly Pro Tyr Gly Pro Gly Ala Ser Ala Ala Ala Ala Ala
                500                 505                 510

Gly Ser Tyr Gly Pro Gly Gln Gln Val Leu Ile Gly Pro Tyr Val Leu
        515                 520                 525

Ile Gly Pro Gly Pro Ser Ala Ala Ala Ala Gly Pro Gly Ser Gly
        530                 535                 540

Gln Tyr Gly Pro Gly Ala Ser Gly Gln Asn Gly Pro Gly Ser Gly Gln
545                 550                 555                 560

Tyr Gly Pro Gly Gln Gln Gly Pro Gly Gln Ser Ala Ala Ala Ala Ala
                565                 570                 575

Gly Gln Tyr Gln Gln Val Leu Ile Gly Pro Gly Gln Gln Gly Pro Tyr
        580                 585                 590

Val Leu Ile Gly Pro Gly Ala Ser Ala Ala Ala Ala Ala Gly Pro Gly
        595                 600                 605

```
Ser Gly Gln Gln Val Leu Ile Gly Pro Gly Ala Ser Val Leu Ile
    610             615             620
```

<210> 23
<211> 603
<212> PRT
<213> Artificial Sequence

<220>
<223> PRT665

<400> 23

```
Met His His His His His His Ser Ser Gly Ser Ser Gly Pro Gly Gln
1               5               10              15
```

```
Gln Gly Pro Tyr Gly Pro Gly Ala Ser Ala Ala Ala Ala Ala Ala Ala
            20              25              30
```

```
Gly Ser Asn Gly Pro Gly Ser Gly Gln Gln Gly Pro Gly Gln Ser Gly
        35              40              45
```

```
Gln Tyr Gly Pro Gly Gln Gln Gly Pro Gly Gln Gln Gly Pro Gly Ser
    50              55              60
```

```
Ser Ala Ala Ala Ala Ala Ala Ala Gly Pro Gly Gln Tyr Val Leu Ile
65              70              75              80
```

```
Gly Pro Gly Gln Gln Gly Pro Ser Ala Ser Ala Ala Ala Ala Ala Ala
            85              90              95
```

```
Ala Gly Pro Gly Ser Gly Gln Gln Gly Pro Gly Ala Ser Gly Gln Tyr
        100             105             110
```

```
Gly Pro Gly Gln Gln Gly Pro Gly Gln Gln Gly Pro Gly Ser Ser Ala
        115             120             125
```

```
Ala Ala Ala Ala Ala Gly Ser Tyr Gly Ser Val Leu Ile Gly Pro
    130             135             140
```

```
Gly Gln Gln Gly Pro Tyr Gly Ser Ala Ala Ala Ala Ala Ala Gly
145             150             155             160
```

```
Pro Gly Ser Gly Gln Tyr Gly Gln Gly Pro Tyr Gly Pro Gly Ala Ser
            165             170             175
```

```
Gly Pro Gly Gln Tyr Gly Pro Gly Gln Gln Gly Pro Ser Ala Ser Ala
        180             185             190
```

Ala Ala Ala Ala Ala Ala Gly Ser Gly Gln Gln Val Leu Ile Gly Pro
    195               200            205

Gly Gln Tyr Gly Pro Tyr Ala Ser Ala Ala Ala Ala Ala Ala Ala Gly
    210               215            220

Ser Tyr Gly Ser Gly Pro Gly Gln Gln Gly Pro Tyr Gly Pro Gly Gln
225              230           235            240

Ser Gly Ser Gly Gln Gln Gly Pro Gly Gln Gln Gly Pro Tyr Ala Ser
           245          250          255

Ala Ala Ala Ala Ala Ala Ala Gly Pro Gly Gln Gln Val Leu Ile Gly
    260              265           270

Pro Tyr Gly Pro Gly Ser Ser Ala Ala Ala Ala Ala Ala Gly Ser
    275              280          285

Tyr Gly Tyr Gly Pro Gly Gln Gln Gly Pro Tyr Gly Pro Gly Ala Ser
    290             295          300

Gly Gln Asn Gly Pro Gly Ser Gly Gln Tyr Gly Pro Gly Gln Gln Gly
305              310           315            320

Pro Gly Pro Ser Ala Ala Ala Ala Ala Ala Gly Pro Gly Gln Gln
           325          330          335

Val Leu Ile Gly Pro Tyr Gly Pro Gly Ala Ser Ala Ala Ala Ala Ala
    340              345          350

Ala Ala Gly Ser Tyr Gly Pro Gly Gln Gln Gly Pro Gly Gln Tyr Gly
    355              360          365

Pro Gly Ser Ser Gly Pro Gly Gln Gln Gly Pro Tyr Gly Pro Gly Ser
    370              375          380

Ser Ala Ala Ala Ala Ala Ala Gly Ser Tyr Gly Pro Gly Gln Gln
385              390           395            400

Val Leu Ile Gly Pro Tyr Gly Pro Gly Pro Ser Ala Ala Ala Ala Ala
    405              410          415

Ala Ala Gly Ser Tyr Gln Gln Gly Pro Gly Gln Gln Gly Pro Tyr Gly
    420              425          430

Pro Gly Ala Ser Gly Pro Gly Gln Gln Gly Pro Tyr Gly Pro Gly Ala
    435              440          445

```
Ser Ala Ala Ala Ala Ala Ala Ala Gly Pro Gly Gln Tyr Val Leu Ile
    450                 455             460

Gly Pro Gly Gln Gln Gly Pro Ser Ala Ser Ala Ala Ala Ala Ala Ala
465                 470             475                 480

Ala Gly Ser Tyr Gly Ser Gly Pro Gly Gln Tyr Gly Pro Tyr Gly Pro
                485             490                 495

Gly Gln Ser Gly Pro Gly Ser Gly Gln Gln Gly Gln Gly Pro Tyr Gly
            500             505             510

Pro Gly Ala Ser Ala Ala Ala Ala Ala Ala Ala Gly Ser Tyr Gly Pro
            515             520             525

Gly Gln Gln Val Leu Ile Gly Pro Tyr Gly Pro Gly Pro Ser Ala Ala
    530             535             540

Ala Ala Ala Ala Ala Gly Pro Gly Ser Gly Gln Tyr Gly Pro Gly Ala
545             550             555             560

Ser Gly Gln Asn Gly Pro Gly Ser Gly Gln Tyr Gly Pro Gly Gln Gln
            565             570             575

Gly Pro Gly Pro Ser Ala Ala Ala Ala Ala Ala Ala Gly Pro Gly Ser
            580             585             590

Gly Gln Gln Gly Pro Gly Ala Ser Val Leu Ile
            595             600
```

```
<210>   24
<211>   630
<212>   PRT
<213>   Artificial Sequence

<220>
<223>   PRT666

<400>   24
```

```
Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Ala Ala
            20                  25                  30

Gly Ser Asn Gly Pro Gly Ser Gly Gln Gln Gly Pro Gly Gln Ser Gly
            35                  40                  45
```

Gln Tyr Gly Pro Gly Gln Gln Gly Pro Gly Gln Gln Gly Pro Gly Ser
    50                  55                  60

Ser Ala Ala Ala Ala Ala Ala Ala Gly Pro Gly Gln Tyr Val Leu Ile
65                  70                  75                  80

Gly Pro Gly Gln Gln Val Leu Ile Gly Pro Ser Ala Ser Ala Ala Ala
                85                  90                  95

Ala Ala Ala Ala Gly Pro Gly Ser Gly Gln Gln Gly Pro Gly Ala Ser
            100                 105                 110

Gly Gln Tyr Gly Pro Gly Gln Gln Gly Pro Gly Gln Gln Gly Pro Gly
        115                 120                 125

Ser Ser Ala Ala Ala Ala Ala Ala Ala Gly Ser Tyr Gly Ser Val Leu
    130                 135                 140

Ile Gly Pro Gly Gln Gln Val Leu Ile Gly Pro Tyr Gly Ser Ala Ala
145                 150                 155                 160

Ala Ala Ala Ala Ala Gly Pro Gly Ser Gly Gln Tyr Gly Gln Gly Pro
            165                 170                 175

Tyr Gly Pro Gly Ala Ser Gly Pro Gly Gln Tyr Gly Pro Gly Gln Gln
        180                 185                 190

Gly Pro Ser Ala Ser Ala Ala Ala Ala Ala Ala Gly Ser Gly Gln
    195                 200                 205

Gln Val Leu Ile Gly Pro Gly Gln Tyr Val Leu Ile Gly Pro Tyr Ala
    210                 215                 220

Ser Ala Ala Ala Ala Ala Ala Gly Ser Tyr Gly Ser Gly Pro Gly
225                 230                 235                 240

Gln Gln Gly Pro Tyr Gly Pro Gly Gln Ser Gly Ser Gly Gln Gln Gly
        245                 250                 255

Pro Gly Gln Gln Gly Pro Tyr Ala Ser Ala Ala Ala Ala Ala Ala Ala
        260                 265                 270

Gly Pro Gly Gln Gln Val Leu Ile Gly Pro Tyr Val Leu Ile Gly Pro
    275                 280                 285

Gly Ser Ser Ala Ala Ala Ala Ala Ala Ala Gly Ser Tyr Gly Tyr Gly
    290                 295                 300

Pro Gly Gln Gln Gly Pro Tyr Gly Pro Gly Ala Ser Gly Gln Asn Gly
305                 310             315                 320

Pro Gly Ser Gly Gln Tyr Gly Pro Gly Gln Gln Gly Pro Gly Pro Ser
            325             330             335

Ala Ala Ala Ala Ala Ala Ala Gly Pro Gly Gln Gln Val Leu Ile Gly
            340             345             350

Pro Tyr Val Leu Ile Gly Pro Gly Ala Ser Ala Ala Ala Ala Ala Ala
        355             360             365

Ala Gly Ser Tyr Gly Pro Gly Gln Gln Gly Pro Gly Gln Tyr Gly Pro
    370             375             380

Gly Ser Ser Gly Pro Gly Gln Gln Gly Pro Tyr Gly Pro Gly Ser Ser
385             390             395                 400

Ala Ala Ala Ala Ala Ala Ala Gly Ser Tyr Gly Pro Gly Gln Gln Val
            405             410             415

Leu Ile Gly Pro Tyr Val Leu Ile Gly Pro Gly Pro Ser Ala Ala Ala
        420             425             430

Ala Ala Ala Ala Gly Ser Tyr Gln Gln Gly Pro Gly Gln Gln Gly Pro
    435             440             445

Tyr Gly Pro Gly Ala Ser Gly Pro Gly Gln Gln Gly Pro Tyr Gly Pro
    450             455             460

Gly Ala Ser Ala Ala Ala Ala Ala Ala Gly Pro Gly Gln Tyr Val
465             470             475                 480

Leu Ile Gly Pro Gly Gln Gln Val Leu Ile Gly Pro Ser Ala Ser Ala
            485             490             495

Ala Ala Ala Ala Ala Gly Ser Tyr Gly Ser Gly Pro Gly Gln Tyr
    500             505             510

Gly Pro Tyr Gly Pro Gly Gln Ser Gly Pro Gly Ser Gly Gln Gln Gly
    515             520             525

Gln Gly Pro Tyr Gly Pro Gly Ala Ser Ala Ala Ala Ala Ala Ala
    530             535             540

Gly Ser Tyr Gly Pro Gly Gln Gln Val Leu Ile Gly Pro Tyr Val Leu

545                        550                        555                        560

Ile Gly Pro Gly Pro Ser Ala Ala Ala Ala Ala Ala Gly Pro Gly
                565                570                575

Ser Gly Gln Tyr Gly Pro Gly Ala Ser Gly Gln Asn Gly Pro Gly Ser
            580                585                590

Gly Gln Tyr Gly Pro Gly Gln Gln Gly Pro Gly Pro Ser Ala Ala Ala
        595                600                605

Ala Ala Ala Ala Gly Pro Gly Ser Gly Gln Gln Val Leu Ile Gly Pro
    610                615                620

Gly Ala Ser Val Leu Ile
625                630

<210> 25
<211> 593
<212> PRT
<213> Artificial Sequence

<220>
<223> Met-PRT888

<400> 25

Met Gly Ser Ser Gly Pro Gly Val Leu Gly Pro Tyr Gly Pro Gly Ala
1            5                10                15

Ser Ala Ala Ala Ala Ala Gly Gln Asn Gly Pro Gly Ser Gly Val Leu
            20                25                30

Gly Pro Gly Gln Ser Gly Gln Tyr Gly Pro Gly Val Leu Gly Pro Gly
        35                40                45

Val Leu Gly Pro Gly Ser Ser Ala Ala Ala Ala Ala Gly Pro Gly Gln
    50                55                60

Tyr Gly Pro Gly Val Leu Gly Pro Ser Ala Ser Ala Ala Ala Ala Ala
65                70                75                80

Gly Pro Gly Ser Gly Val Leu Gly Pro Gly Ala Ser Gly Gln Tyr Gly
            85                90                95

Pro Gly Val Leu Gly Pro Gly Val Leu Gly Pro Gly Ser Ser Ala Ala
        100                105                110

Ala Ala Ala Gly Gln Tyr Gly Ser Gly Pro Gly Val Leu Gly Pro Tyr
    115                120                125

114

```
Gly Ser Ala Ala Ala Ala Ala Gly Pro Gly Ser Gly Gln Tyr Gly Gln
    130             135                 140

Gly Pro Tyr Gly Pro Gly Ala Ser Gly Pro Gly Gln Tyr Gly Pro Gly
    145             150                 155                 160

Val Leu Gly Pro Ser Ala Ser Ala Ala Ala Ala Ala Gly Ser Gly Val
                165             170                 175

Leu Gly Pro Gly Gln Tyr Gly Pro Tyr Ala Ser Ala Ala Ala Ala Ala
            180             185                 190

Gly Gln Tyr Gly Ser Gly Pro Gly Val Leu Gly Pro Tyr Gly Pro Gly
        195                 200                 205

Gln Ser Gly Ser Gly Val Leu Gly Pro Gly Val Leu Gly Pro Tyr Ala
    210             215                 220

Ser Ala Ala Ala Ala Ala Gly Pro Gly Val Leu Gly Pro Tyr Gly Pro
225             230                 235                 240

Gly Ser Ser Ala Ala Ala Ala Ala Gly Gln Tyr Gly Tyr Gly Pro Gly
            245                 250                 255

Val Leu Gly Pro Tyr Gly Pro Gly Ala Ser Gly Gln Asn Gly Pro Gly
        260                 265                 270

Ser Gly Gln Tyr Gly Pro Gly Val Leu Gly Pro Gly Gln Ser Ala Ala
        275                 280                 285

Ala Ala Ala Gly Pro Gly Val Leu Gly Pro Tyr Gly Pro Gly Ala Ser
    290                 295                 300

Ala Ala Ala Ala Ala Gly Gln Tyr Gly Pro Gly Val Leu Gly Pro Gly
305                 310                 315                 320

Gln Tyr Gly Pro Gly Ser Ser Gly Pro Gly Val Leu Gly Pro Tyr Gly
            325                 330                 335

Pro Gly Ser Ser Ala Ala Ala Ala Ala Gly Gln Tyr Gly Pro Gly Val
            340                 345                 350

Leu Gly Pro Tyr Gly Pro Gly Gln Ser Ala Ala Ala Ala Ala Gly Gln
        355                 360                 365

Tyr Val Leu Gly Pro Gly Val Leu Gly Pro Tyr Gly Pro Gly Ala Ser
```

```
            370                    375                      380

    Gly Pro Gly Val Leu Gly Pro Tyr Gly Pro Gly Ala Ser Ala Ala Ala
    385                 390                 395                 400

    Ala Ala Gly Pro Gly Gln Tyr Gly Pro Gly Val Leu Gly Pro Ser Ala
                405                 410                 415

    Ser Ala Ala Ala Ala Ala Gly Gln Tyr Gly Ser Gly Pro Gly Gln Tyr
            420                 425                 430

    Gly Pro Tyr Gly Pro Gly Gln Ser Gly Pro Gly Ser Gly Val Leu Gly
            435                 440                 445

    Gln Gly Pro Tyr Gly Pro Gly Ala Ser Ala Ala Ala Ala Gly Gln
        450                 455                 460

    Tyr Gly Pro Gly Val Leu Gly Pro Tyr Gly Pro Gly Gln Ser Ala Ala
    465                 470                 475                 480

    Ala Ala Ala Gly Pro Gly Ser Gly Gln Tyr Gly Pro Gly Ala Ser Gly
                485                 490                 495

    Gln Asn Gly Pro Gly Ser Gly Gln Tyr Gly Pro Gly Val Leu Gly Pro
            500                 505                 510

    Gly Gln Ser Ala Ala Ala Ala Ala Gly Gln Tyr Val Leu Gly Pro Gly
            515                 520                 525

    Val Leu Gly Pro Tyr Gly Pro Gly Ala Ser Ala Ala Ala Ala Ala Gly
        530                 535                 540

    Gln Tyr Gly Ser Gly Pro Gly Val Leu Gly Pro Tyr Gly Pro Gly Gln
    545                 550                 555                 560

    Ser Gly Ser Gly Val Leu Gly Pro Gly Val Leu Gly Pro Tyr Ala Ser
                565                 570                 575

    Ala Ala Ala Ala Ala Gly Pro Gly Ser Gly Val Leu Gly Pro Gly Ala
                580                 585                 590

    Ser


    <210>   26
    <211>   590
    <212>   PRT
    <213>   Artificial Sequence
```

<220>
<223>  Met-PRT965

<400>   26

Met Gly Pro Gly Thr Ser Gly Pro Tyr Gly Pro Gly Ala Ser Ala Ala
1               5                   10                  15

Ala Ala Ala Gly Ala Asn Gly Pro Gly Ser Gly Thr Ser Gly Pro Gly
            20                  25                  30

Ala Ser Gly Ala Tyr Gly Pro Gly Thr Ser Gly Pro Gly Thr Ser Gly
        35                  40                  45

Pro Gly Ser Ser Ala Ala Ala Ala Ala Gly Pro Gly Ala Tyr Gly Pro
    50                  55                  60

Gly Thr Ser Gly Pro Ser Ala Ser Ala Ala Ala Ala Gly Pro Gly
65                  70                  75                  80

Ser Gly Thr Ser Gly Pro Gly Ala Ser Gly Ala Tyr Gly Pro Gly Thr
            85                  90                  95

Ser Gly Pro Gly Thr Ser Gly Pro Gly Ser Ser Ala Ala Ala Ala Ala
            100                 105                 110

Gly Ala Tyr Gly Ser Gly Pro Gly Thr Ser Gly Pro Tyr Gly Ser Ala
        115                 120                 125

Ala Ala Ala Ala Gly Pro Gly Ser Gly Ala Tyr Gly Ala Gly Pro Tyr
    130                 135                 140

Gly Pro Gly Ala Ser Gly Pro Gly Ala Tyr Gly Pro Gly Thr Ser Gly
145                 150                 155                 160

Pro Ser Ala Ser Ala Ala Ala Ala Ala Gly Ser Gly Thr Ser Gly Pro
            165                 170                 175

Gly Ala Tyr Gly Pro Tyr Ala Ser Ala Ala Ala Ala Gly Ala Tyr
        180                 185                 190

Gly Ser Gly Pro Gly Thr Ser Gly Pro Tyr Gly Pro Gly Ala Ser Gly
        195                 200                 205

Ser Gly Thr Ser Gly Pro Gly Thr Ser Gly Pro Tyr Ala Ser Ala Ala
    210                 215                 220

Ala Ala Ala Gly Pro Gly Thr Ser Gly Pro Tyr Gly Pro Gly Ser Ser

|     | 225 |     |     |     | 230 |     |     |     | 235 |     |     |     | 240 |
|-----|-----|-----|-----|-----|-----|-----|-----|-----|-----|-----|-----|-----|-----|

Ala Ala Ala Ala Ala Gly Ala Tyr Gly Tyr Gly Pro Gly Thr Ser Gly
245                     250                     255

Pro Tyr Gly Pro Gly Ala Ser Gly Ala Asn Gly Pro Gly Ser Gly Ala
260                     265                     270

Tyr Gly Pro Gly Thr Ser Gly Pro Gly Ala Ser Ala Ala Ala Ala Ala
275                     280                     285

Gly Pro Gly Thr Ser Gly Pro Tyr Gly Pro Gly Ala Ser Ala Ala Ala
290                     295                     300

Ala Ala Gly Ala Tyr Gly Pro Gly Thr Ser Gly Pro Gly Ala Tyr Gly
305                     310                     315                     320

Pro Gly Ser Ser Gly Pro Gly Thr Ser Gly Pro Tyr Gly Pro Gly Ser
325                     330                     335

Ser Ala Ala Ala Ala Ala Gly Ala Tyr Gly Pro Gly Thr Ser Gly Pro
340                     345                     350

Tyr Gly Pro Gly Ala Ser Ala Ala Ala Ala Gly Ala Tyr Thr Ser
355                     360                     365

Gly Pro Gly Thr Ser Gly Pro Tyr Gly Pro Gly Ala Ser Gly Pro Gly
370                     375                     380

Thr Ser Gly Pro Tyr Gly Pro Gly Ala Ser Ala Ala Ala Ala Ala Gly
385                     390                     395                     400

Pro Gly Ala Tyr Gly Pro Gly Thr Ser Gly Pro Ser Ala Ser Ala Ala
405                     410                     415

Ala Ala Ala Gly Ala Tyr Gly Ser Gly Pro Gly Ala Tyr Gly Pro Tyr
420                     425                     430

Gly Pro Gly Ala Ser Gly Pro Gly Ser Gly Thr Ser Gly Ala Gly Pro
435                     440                     445

Tyr Gly Pro Gly Ala Ser Ala Ala Ala Ala Gly Ala Tyr Gly Pro
450                     455                     460

Gly Thr Ser Gly Pro Tyr Gly Pro Gly Ala Ser Ala Ala Ala Ala Ala
465                     470                     475                     480

Gly Pro Gly Ser Gly Ala Tyr Gly Pro Gly Ala Ser Gly Ala Asn Gly
485               490               495

Pro Gly Ser Gly Ala Tyr Gly Pro Gly Thr Ser Gly Pro Gly Ala Ser
500               505               510

Ala Ala Ala Ala Ala Gly Ala Tyr Thr Ser Gly Pro Gly Thr Ser Gly
515               520               525

Pro Tyr Gly Pro Gly Ala Ser Ala Ala Ala Ala Ala Gly Ala Tyr Gly
530               535               540

Ser Gly Pro Gly Thr Ser Gly Pro Tyr Gly Pro Gly Ala Ser Gly Ser
545               550               555               560

Gly Thr Ser Gly Pro Gly Thr Ser Gly Pro Tyr Ala Ser Ala Ala Ala
565               570               575

Ala Ala Gly Pro Gly Ser Gly Thr Ser Gly Pro Gly Ala Ser
580               585               590

<210>   27
<211>   593
<212>   PRT
<213>   Artificial Sequence

<220>
<223>   Met-PRT889

<400>   27

Met Gly Ser Ser Gly Pro Gly Val Leu Gly Pro Tyr Gly Pro Gly Ala
1               5               10              15

Ser Ala Ala Ala Ala Ala Gly Ile Asn Gly Pro Gly Ser Gly Val Leu
20              25              30

Gly Pro Gly Ile Ser Gly Ile Tyr Gly Pro Gly Val Leu Gly Pro Gly
35              40              45

Val Leu Gly Pro Gly Ser Ser Ala Ala Ala Ala Gly Pro Gly Ile
50              55              60

Tyr Gly Pro Gly Val Leu Gly Pro Ser Ala Ser Ala Ala Ala Ala Ala
65              70              75              80

Gly Pro Gly Ser Gly Val Leu Gly Pro Gly Ala Ser Gly Ile Tyr Gly
85              90              95

Pro Gly Val Leu Gly Pro Gly Val Leu Gly Pro Gly Ser Ser Ala Ala

                        100                          105                          110


        Ala Ala Ala Gly Ile Tyr Gly Ser Gly Pro Gly Val Leu Gly Pro Tyr
                115                          120                          125


        Gly Ser Ala Ala Ala Ala Ala Gly Pro Gly Ser Gly Ile Tyr Gly Ile
                130                          135                          140


        Gly Pro Tyr Gly Pro Gly Ala Ser Gly Pro Gly Ile Tyr Gly Pro Gly
        145                          150                          155                          160


        Val Leu Gly Pro Ser Ala Ser Ala Ala Ala Ala Gly Ser Gly Val
                                165                          170                          175


        Leu Gly Pro Gly Ile Tyr Gly Pro Tyr Ala Ser Ala Ala Ala Ala Ala
                        180                          185                          190


        Gly Ile Tyr Gly Ser Gly Pro Gly Val Leu Gly Pro Tyr Gly Pro Gly
                        195                          200                          205


        Ile Ser Gly Ser Gly Val Leu Gly Pro Gly Val Leu Gly Pro Tyr Ala
                210                          215                          220


        Ser Ala Ala Ala Ala Ala Gly Pro Gly Val Leu Gly Pro Tyr Gly Pro
        225                          230                          235                          240


        Gly Ser Ser Ala Ala Ala Ala Gly Ile Tyr Gly Tyr Gly Pro Gly
                        245                          250                          255


        Val Leu Gly Pro Tyr Gly Pro Gly Ala Ser Gly Ile Asn Gly Pro Gly
                        260                          265                          270


        Ser Gly Ile Tyr Gly Pro Gly Val Leu Gly Pro Gly Ile Ser Ala Ala
                        275                          280                          285


        Ala Ala Ala Gly Pro Gly Val Leu Gly Pro Tyr Gly Pro Gly Ala Ser
                290                          295                          300


        Ala Ala Ala Ala Ala Gly Ile Tyr Gly Pro Gly Val Leu Gly Pro Gly
        305                          310                          315                          320


        Ile Tyr Gly Pro Gly Ser Ser Gly Pro Gly Val Leu Gly Pro Tyr Gly
                        325                          330                          335


        Pro Gly Ser Ser Ala Ala Ala Ala Ala Gly Ile Tyr Gly Pro Gly Val
                340                          345                          350

Leu Gly Pro Tyr Gly Pro Gly Ile Ser Ala Ala Ala Ala Gly Ile
355 360 365

Tyr Val Leu Gly Pro Gly Val Leu Gly Pro Tyr Gly Pro Gly Ala Ser
370 375 380

Gly Pro Gly Val Leu Gly Pro Tyr Gly Pro Gly Ala Ser Ala Ala Ala
385 390 395 400

Ala Ala Gly Pro Gly Ile Tyr Gly Pro Gly Val Leu Gly Pro Ser Ala
405 410 415

Ser Ala Ala Ala Ala Ala Gly Ile Tyr Gly Ser Gly Pro Gly Ile Tyr
420 425 430

Gly Pro Tyr Gly Pro Gly Ile Ser Gly Pro Gly Ser Gly Val Leu Gly
435 440 445

Ile Gly Pro Tyr Gly Pro Gly Ala Ser Ala Ala Ala Ala Ala Gly Ile
450 455 460

Tyr Gly Pro Gly Val Leu Gly Pro Tyr Gly Pro Gly Ile Ser Ala Ala
465 470 475 480

Ala Ala Ala Gly Pro Gly Ser Gly Ile Tyr Gly Pro Gly Ala Ser Gly
485 490 495

Ile Asn Gly Pro Gly Ser Gly Ile Tyr Gly Pro Gly Val Leu Gly Pro
500 505 510

Gly Ile Ser Ala Ala Ala Ala Ala Gly Ile Tyr Val Leu Gly Pro Gly
515 520 525

Val Leu Gly Pro Tyr Gly Pro Gly Ala Ser Ala Ala Ala Ala Ala Gly
530 535 540

Ile Tyr Gly Ser Gly Pro Gly Val Leu Gly Pro Tyr Gly Pro Gly Ile
545 550 555 560

Ser Gly Ser Gly Val Leu Gly Pro Gly Val Leu Gly Pro Tyr Ala Ser
565 570 575

Ala Ala Ala Ala Ala Gly Pro Gly Ser Gly Val Leu Gly Pro Gly Ala
580 585 590

Ser

<210> 28
<211> 590
<212> PRT
<213> Artificial Sequence

<220>
<223> Met-PRT916

<400> 28

```
Met Gly Pro Gly Val Ile Gly Pro Tyr Gly Pro Gly Ala Ser Ala Ala
1               5                   10                  15

Ala Ala Ala Gly Leu Asn Gly Pro Gly Ser Gly Val Ile Gly Pro Gly
            20                  25                  30

Leu Ser Gly Leu Tyr Gly Pro Gly Val Ile Gly Pro Gly Val Ile Gly
            35                  40                  45

Pro Gly Ser Ser Ala Ala Ala Ala Ala Gly Pro Gly Leu Tyr Gly Pro
        50                  55                  60

Gly Val Ile Gly Pro Ser Ala Ser Ala Ala Ala Ala Gly Pro Gly
65                  70                  75                  80

Ser Gly Val Ile Gly Pro Gly Ala Ser Gly Leu Tyr Gly Pro Gly Val
                85                  90                  95

Ile Gly Pro Gly Val Ile Gly Pro Gly Ser Ser Ala Ala Ala Ala Ala
            100                 105                 110

Gly Leu Tyr Gly Ser Gly Pro Gly Val Ile Gly Pro Tyr Gly Ser Ala
        115                 120                 125

Ala Ala Ala Gly Pro Gly Ser Gly Leu Tyr Gly Leu Gly Pro Tyr
        130                 135                 140

Gly Pro Gly Ala Ser Gly Pro Gly Leu Tyr Gly Pro Gly Val Ile Gly
145                 150                 155                 160

Pro Ser Ala Ser Ala Ala Ala Ala Ala Gly Ser Gly Val Ile Gly Pro
                165                 170                 175

Gly Leu Tyr Gly Pro Tyr Ala Ser Ala Ala Ala Ala Gly Leu Tyr
        180                 185                 190

Gly Ser Gly Pro Gly Val Ile Gly Pro Tyr Gly Pro Gly Leu Ser Gly
        195                 200                 205
```

Ser Gly Val Ile Gly Pro Gly Val Ile Gly Pro Tyr Ala Ser Ala Ala
210                 215                 220

Ala Ala Ala Gly Pro Gly Val Ile Gly Pro Tyr Gly Pro Gly Ser Ser
225                 230                 235                 240

Ala Ala Ala Ala Ala Gly Leu Tyr Gly Tyr Gly Pro Gly Val Ile Gly
                245                 250                 255

Pro Tyr Gly Pro Gly Ala Ser Gly Leu Asn Gly Pro Gly Ser Gly Leu
            260                 265                 270

Tyr Gly Pro Gly Val Ile Gly Pro Gly Leu Ser Ala Ala Ala Ala Ala
            275                 280                 285

Gly Pro Gly Val Ile Gly Pro Tyr Gly Pro Gly Ala Ser Ala Ala Ala
    290                 295                 300

Ala Ala Gly Leu Tyr Gly Pro Gly Val Ile Gly Pro Gly Leu Tyr Gly
305                 310                 315                 320

Pro Gly Ser Ser Gly Pro Gly Val Ile Gly Pro Tyr Gly Pro Gly Ser
            325                 330                 335

Ser Ala Ala Ala Ala Ala Gly Leu Tyr Gly Pro Gly Val Ile Gly Pro
            340                 345                 350

Tyr Gly Pro Gly Leu Ser Ala Ala Ala Ala Ala Gly Leu Tyr Val Ile
    355                 360                 365

Gly Pro Gly Val Ile Gly Pro Tyr Gly Pro Gly Ala Ser Gly Pro Gly
    370                 375                 380

Val Ile Gly Pro Tyr Gly Pro Gly Ala Ser Ala Ala Ala Ala Ala Gly
385                 390                 395                 400

Pro Gly Leu Tyr Gly Pro Gly Val Ile Gly Pro Ser Ala Ser Ala Ala
            405                 410                 415

Ala Ala Ala Gly Leu Tyr Gly Ser Gly Pro Gly Leu Tyr Gly Pro Tyr
            420                 425                 430

Gly Pro Gly Leu Ser Gly Pro Gly Ser Gly Val Ile Gly Leu Gly Pro
    435                 440                 445

Tyr Gly Pro Gly Ala Ser Ala Ala Ala Ala Ala Gly Leu Tyr Gly Pro
450                 455                 460

123

```
Gly Val Ile Gly Pro Tyr Gly Pro Gly Leu Ser Ala Ala Ala Ala Ala
465             470             475                     480


Gly Pro Gly Ser Gly Leu Tyr Gly Pro Gly Ala Ser Gly Leu Asn Gly
                485             490                     495


Pro Gly Ser Gly Leu Tyr Gly Pro Gly Val Ile Gly Pro Gly Leu Ser
                500             505                     510


Ala Ala Ala Ala Ala Gly Leu Tyr Val Ile Gly Pro Gly Val Ile Gly
            515             520                     525


Pro Tyr Gly Pro Gly Ala Ser Ala Ala Ala Ala Gly Leu Tyr Gly
    530             535                     540


Ser Gly Pro Gly Val Ile Gly Pro Tyr Gly Pro Gly Leu Ser Gly Ser
545             550             555                     560


Gly Val Ile Gly Pro Gly Val Ile Gly Pro Tyr Ala Ser Ala Ala Ala
                565             570                     575


Ala Ala Gly Pro Gly Ser Gly Val Ile Gly Pro Gly Ala Ser
            580             585                     590


<210>  29
<211>  590
<212>  PRT
<213>  Artificial Sequence

<220>
<223>  Met-PRT918

<400>  29

Met Gly Pro Gly Val Phe Gly Pro Tyr Gly Pro Gly Ala Ser Ala Ala
1               5               10                      15


Ala Ala Ala Gly Ile Asn Gly Pro Gly Ser Gly Val Phe Gly Pro Gly
            20              25                      30


Ile Ser Gly Ile Tyr Gly Pro Gly Val Phe Gly Pro Gly Val Phe Gly
            35              40                      45


Pro Gly Ser Ser Ala Ala Ala Ala Ala Gly Pro Gly Ile Tyr Gly Pro
    50              55                      60


Gly Val Phe Gly Pro Ser Ala Ser Ala Ala Ala Ala Ala Gly Pro Gly
65              70              75                      80
```

```
Ser Gly Val Phe Gly Pro Gly Ala Ser Gly Ile Tyr Gly Pro Gly Val
                85                      90                  95

Phe Gly Pro Gly Val Phe Gly Pro Gly Ser Ser Ala Ala Ala Ala Ala
            100                 105                 110

Gly Ile Tyr Gly Ser Gly Pro Gly Val Phe Gly Pro Tyr Gly Ser Ala
            115                 120                 125

Ala Ala Ala Ala Gly Pro Gly Ser Gly Ile Tyr Gly Ile Gly Pro Tyr
    130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
                165                 170                 175

Gly Ile Tyr Gly Pro Tyr Ala Ser Ala Ala Ala Ala Gly Ile Tyr
            180                 185                 190

Gly Ser Gly Pro Gly Val Phe Gly Pro Tyr Gly Pro Gly Ile Ser Gly
            195                 200                 205

Ser Gly Val Phe Gly Pro Gly Val Phe Gly Pro Tyr Ala Ser Ala Ala
    210                 215                 220

Ala Ala Ala Gly Pro Gly Val Phe Gly Pro Tyr Gly Pro Gly Ser Ser
225                 230                 235                 240

Ala Ala Ala Ala Ala Gly Ile Tyr Gly Tyr Gly Pro Gly Val Phe Gly
                245                 250                 255

Pro Tyr Gly Pro Gly Ala Ser Gly Ile Asn Gly Pro Gly Ser Gly Ile
            260                 265                 270

Tyr Gly Pro Gly Val Phe Gly Pro Gly Ile Ser Ala Ala Ala Ala Ala
            275                 280                 285

Gly Pro Gly Val Phe Gly Pro Tyr Gly Pro Gly Ala Ser Ala Ala Ala
    290                 295                 300

Ala Ala Gly Ile Tyr Gly Pro Gly Val Phe Gly Pro Gly Ile Tyr Gly
305                 310                 315                 320

Pro Gly Ser Ser Gly Pro Gly Val Phe Gly Pro Tyr Gly Pro Gly Ser
            325                 330                 335
```

```
Ser Ala Ala Ala Ala Ala Gly Ile Tyr Gly Pro Gly Val Phe Gly Pro
        340                 345             350

Tyr Gly Pro Gly Ile Ser Ala Ala Ala Ala Ala Gly Ile Tyr Val Phe
        355                 360             365

Gly Pro Gly Val Phe Gly Pro Tyr Gly Pro Gly Ala Ser Gly Pro Gly
    370                 375             380

Val Phe Gly Pro Tyr Gly Pro Gly Ala Ser Ala Ala Ala Ala Ala Gly
385                 390             395                 400

Pro Gly Ile Tyr Gly Pro Gly Val Phe Gly Pro Ser Ala Ser Ala Ala
                405             410                 415

Ala Ala Ala Gly Ile Tyr Gly Ser Gly Pro Gly Ile Tyr Gly Pro Tyr
        420                 425             430

Gly Pro Gly Ile Ser Gly Pro Gly Ser Gly Val Phe Gly Ile Gly Pro
    435                 440             445

Tyr Gly Pro Gly Ala Ser Ala Ala Ala Ala Gly Ile Tyr Gly Pro
    450                 455             460

Gly Val Phe Gly Pro Tyr Gly Pro Gly Ile Ser Ala Ala Ala Ala Ala
465                 470             475                 480

Gly Pro Gly Ser Gly Ile Tyr Gly Pro Gly Ala Ser Gly Ile Asn Gly
                485             490             495

Pro Gly Ser Gly Ile Tyr Gly Pro Gly Val Phe Gly Pro Gly Ile Ser
        500             505             510

Ala Ala Ala Ala Ala Gly Ile Tyr Val Phe Gly Pro Gly Val Phe Gly
        515             520             525

Pro Tyr Gly Pro Gly Ala Ser Ala Ala Ala Ala Gly Ile Tyr Gly
    530             535             540

Ser Gly Pro Gly Val Phe Gly Pro Tyr Gly Pro Gly Ile Ser Gly Ser
545             550             555             560

Gly Val Phe Gly Pro Gly Val Phe Gly Pro Tyr Ala Ser Ala Ala Ala
            565             570             575

Ala Ala Gly Pro Gly Ser Gly Val Phe Gly Pro Gly Ala Ser
        580             585             590
```

```
<210>   30
<211>   565
<212>   PRT
<213>   Artificial Sequence

<220>
<223>   Met-PRT699

<400>   30
```

Met Gly Pro Gly Val Leu Gly Pro Tyr Gly Pro Gly Ala Ser Ala Ala
1               5                   10                  15

Ala Ala Ala Ala Ala Gly Ser Asn Gly Pro Gly Ser Gly Val Leu Gly
            20                  25                  30

Pro Gly Gln Ser Gly Gln Tyr Gly Pro Gly Val Leu Gly Pro Gly Val
            35                  40                  45

Leu Gly Pro Gly Ser Ser Ala Ala Ala Ala Ala Ala Gly Pro Gly
        50                  55                  60

Gln Tyr Gly Pro Gly Val Leu Gly Pro Ser Ala Ser Ala Ala Ala Ala
65                  70                  75                  80

Ala Ala Ala Gly Pro Gly Ser Gly Val Leu Gly Pro Gly Ala Ser Gly
                85                  90                  95

Gln Tyr Gly Pro Gly Val Leu Gly Pro Gly Val Leu Gly Pro Gly Ser
            100                 105                 110

Ser Ala Ala Ala Ala Ala Ala Ala Gly Ser Tyr Gly Ser Gly Pro Gly
            115                 120                 125

Val Leu Gly Pro Tyr Gly Ser Ala Ala Ala Ala Ala Ala Gly Pro
        130                 135                 140

Gly Ser Gly Gln Tyr Gly Gln Gly Pro Tyr Gly Pro Gly Ala Ser Gly
145                 150                 155                 160

Pro Gly Gln Tyr Gly Pro Gly Val Leu Gly Pro Ser Ala Ser Ala Ala
            165                 170                 175

Ala Ala Ala Ala Ala Gly Ser Gly Val Leu Gly Pro Gly Gln Tyr Gly
            180                 185                 190

Pro Tyr Ala Ser Ala Ala Ala Ala Ala Ala Ala Gly Ser Tyr Gly Ser
        195                 200                 205

Gly Pro Gly Val Leu Gly Pro Tyr Gly Pro Gly Gln Ser Gly Ser Gly
210            215            220

Val Leu Gly Pro Gly Val Leu Gly Pro Tyr Ala Ser Ala Ala Ala Ala
225            230            235            240

Ala Ala Ala Gly Pro Gly Val Leu Gly Pro Tyr Gly Pro Gly Ser Ser
245            250            255

Ala Ala Ala Ala Ala Ala Ala Gly Ser Tyr Gly Tyr Gly Pro Gly Val
260            265            270

Leu Gly Pro Tyr Gly Pro Gly Ala Ser Gly Gln Asn Gly Pro Gly Ser
275            280            285

Gly Gln Tyr Gly Pro Gly Val Leu Gly Pro Gly Pro Ser Ala Ala Ala
290            295            300

Ala Ala Ala Ala Gly Pro Gly Val Leu Gly Pro Tyr Gly Pro Gly Ala
305            310            315            320

Ser Ala Ala Ala Ala Ala Ala Ala Gly Ser Tyr Gly Pro Gly Val Leu
325            330            335

Gly Pro Gly Gln Tyr Gly Pro Gly Ser Ser Gly Pro Gly Val Leu Gly
340            345            350

Pro Tyr Gly Pro Gly Ser Ser Ala Ala Ala Ala Ala Ala Gly Ser
355            360            365

Tyr Gly Pro Gly Val Leu Gly Pro Tyr Gly Pro Gly Pro Ser Ala Ala
370            375            380

Ala Ala Ala Ala Ala Gly Ser Tyr Val Leu Gly Pro Gly Val Leu Gly
385            390            395            400

Pro Tyr Gly Pro Gly Ala Ser Gly Pro Gly Val Leu Gly Pro Tyr Gly
405            410            415

Pro Gly Ala Ser Ala Ala Ala Ala Ala Ala Gly Pro Gly Gln Tyr
420            425            430

Gly Pro Gly Val Leu Gly Pro Ser Ala Ser Ala Ala Ala Ala Ala Ala
435            440            445

Ala Gly Ser Tyr Gly Ser Gly Pro Gly Gln Tyr Gly Pro Tyr Gly Pro
450            455            460

128

```
Gly Gln Ser Gly Pro Gly Ser Gly Val Leu Gly Gln Gly Pro Tyr Gly
465             470             475             480

Pro Gly Ala Ser Ala Ala Ala Ala Ala Ala Ala Gly Ser Tyr Gly Pro
            485             490             495

Gly Val Leu Gly Pro Tyr Gly Pro Gly Pro Ser Ala Ala Ala Ala Ala
            500             505             510

Ala Ala Gly Pro Gly Ser Gly Gln Tyr Gly Pro Gly Ala Ser Gly Gln
        515             520             525

Asn Gly Pro Gly Ser Gly Gln Tyr Gly Pro Gly Val Leu Gly Pro Gly
    530             535             540

Pro Ser Ala Ala Ala Ala Ala Ala Ala Gly Pro Gly Ser Gly Val Leu
545             550             555             560

Gly Pro Gly Ala Ser
                565
```

```
<210>   31
<211>   565
<212>   PRT
<213>   Artificial Sequence

<220>
<223>   Met-PRT698

<400>   31
```

```
Met Gly Pro Gly Val Leu Gly Pro Tyr Gly Pro Gly Ala Ser Ala Ala
1               5               10              15

Ala Ala Ala Ala Ala Gly Ser Asn Gly Pro Gly Ser Gly Val Leu Gly
            20              25              30

Pro Gly Ile Ser Gly Ile Tyr Gly Pro Gly Val Leu Gly Pro Gly Val
        35              40              45

Leu Gly Pro Gly Ser Ser Ala Ala Ala Ala Ala Ala Gly Pro Gly
    50              55              60

Ile Tyr Gly Pro Gly Val Leu Gly Pro Ser Ala Ser Ala Ala Ala Ala
65              70              75              80

Ala Ala Ala Gly Pro Gly Ser Gly Val Leu Gly Pro Gly Ala Ser Gly
            85              90              95
```

Ile Tyr Gly Pro Gly Val Leu Gly Pro Gly Val Leu Gly Pro Gly Ser
                100                 105                 110

Ser Ala Ala Ala Ala Ala Ala Ala Gly Ser Tyr Gly Ser Gly Pro Gly
        115                 120                 125

Val Leu Gly Pro Tyr Gly Ser Ala Ala Ala Ala Ala Ala Ala Gly Pro
        130                 135                 140

Gly Ser Gly Ile Tyr Gly Ile Gly Pro Tyr Gly Pro Gly Ala Ser Gly
145                 150                 155                 160

Pro Gly Ile Tyr Gly Pro Gly Val Leu Gly Pro Ser Ala Ser Ala Ala
                165                 170                 175

Ala Ala Ala Ala Ala Gly Ser Gly Val Leu Gly Pro Gly Ile Tyr Gly
        180                 185                 190

Pro Tyr Ala Ser Ala Ala Ala Ala Ala Ala Ala Gly Ser Tyr Gly Ser
        195                 200                 205

Gly Pro Gly Val Leu Gly Pro Tyr Gly Pro Gly Ile Ser Gly Ser Gly
    210                 215                 220

Val Leu Gly Pro Gly Val Leu Gly Pro Tyr Ala Ser Ala Ala Ala Ala
225                 230                 235                 240

Ala Ala Ala Gly Pro Gly Val Leu Gly Pro Tyr Gly Pro Gly Ser Ser
            245                 250                 255

Ala Ala Ala Ala Ala Ala Ala Gly Ser Tyr Gly Tyr Gly Pro Gly Val
        260                 265                 270

Leu Gly Pro Tyr Gly Pro Gly Ala Ser Gly Ile Asn Gly Pro Gly Ser
        275                 280                 285

Gly Ile Tyr Gly Pro Gly Val Leu Gly Pro Gly Pro Ser Ala Ala Ala
        290                 295                 300

Ala Ala Ala Ala Gly Pro Gly Val Leu Gly Pro Tyr Gly Pro Gly Ala
305                 310                 315                 320

Ser Ala Ala Ala Ala Ala Ala Ala Gly Ser Tyr Gly Pro Gly Val Leu
        325                 330                 335

Gly Pro Gly Ile Tyr Gly Pro Gly Ser Ser Gly Pro Gly Val Leu Gly
        340                 345                 350

```
Pro Tyr Gly Pro Gly Ser Ser Ala Ala Ala Ala Ala Ala Ala Gly Ser
        355                 360                 365

Tyr Gly Pro Gly Val Leu Gly Pro Tyr Gly Pro Gly Pro Ser Ala Ala
        370                 375                 380

Ala Ala Ala Ala Ala Gly Ser Tyr Val Leu Gly Pro Gly Val Leu Gly
385                 390                 395                 400

Pro Tyr Gly Pro Gly Ala Ser Gly Pro Gly Val Leu Gly Pro Tyr Gly
                405                 410                 415

Pro Gly Ala Ser Ala Ala Ala Ala Ala Ala Gly Pro Gly Ile Tyr
            420                 425                 430

Gly Pro Gly Val Leu Gly Pro Ser Ala Ser Ala Ala Ala Ala Ala Ala
        435                 440                 445

Ala Gly Ser Tyr Gly Ser Gly Pro Gly Ile Tyr Gly Pro Tyr Gly Pro
        450                 455                 460

Gly Ile Ser Gly Pro Gly Ser Gly Val Leu Gly Ile Gly Pro Tyr Gly
465                 470                 475                 480

Pro Gly Ala Ser Ala Ala Ala Ala Ala Ala Gly Ser Tyr Gly Pro
            485                 490                 495

Gly Val Leu Gly Pro Tyr Gly Pro Gly Pro Ser Ala Ala Ala Ala Ala
            500                 505                 510

Ala Ala Gly Pro Gly Ser Gly Ile Tyr Gly Pro Gly Ala Ser Gly Ile
        515                 520                 525

Asn Gly Pro Gly Ser Gly Ile Tyr Gly Pro Gly Val Leu Gly Pro Gly
        530                 535                 540

Pro Ser Ala Ala Ala Ala Ala Ala Ala Gly Pro Gly Ser Gly Val Leu
545                 550                 555                 560

Gly Pro Gly Ala Ser
                565
```

```
<210>    32
<211>    1179
<212>    PRT
<213>    Artificial Sequence
```

<220>
<223>   Met-PRT966

<400>   32

Met Gly Pro Gly Val Phe Gly Pro Tyr Gly Pro Gly Ala Ser Ala Ala
1               5                   10                  15

Ala Ala Ala Gly Ile Asn Gly Pro Gly Ser Gly Val Phe Gly Pro Gly
            20                  25                  30

Ile Ser Gly Ile Tyr Gly Pro Gly Val Phe Gly Pro Gly Val Phe Gly
            35                  40                  45

Pro Gly Ser Ser Ala Ala Ala Ala Ala Gly Pro Gly Ile Tyr Gly Pro
        50                  55                  60

Gly Val Phe Gly Pro Ser Ala Ser Ala Ala Ala Ala Gly Pro Gly
65                  70                  75                  80

Ser Gly Val Phe Gly Pro Gly Ala Ser Gly Ile Tyr Gly Pro Gly Val
            85                  90                  95

Phe Gly Pro Gly Val Phe Gly Pro Gly Ser Ser Ala Ala Ala Ala Ala
            100                 105                 110

Gly Ile Tyr Gly Ser Gly Pro Gly Val Phe Gly Pro Tyr Gly Ser Ala
            115                 120                 125

Ala Ala Ala Ala Gly Pro Gly Ser Gly Ile Tyr Gly Ile Gly Pro Tyr
            130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
                165                 170                 175

Gly Ile Tyr Gly Pro Tyr Ala Ser Ala Ala Ala Ala Gly Ile Tyr
            180                 185                 190

Gly Ser Gly Pro Gly Val Phe Gly Pro Tyr Gly Pro Gly Ile Ser Gly
            195                 200                 205

Ser Gly Val Phe Gly Pro Gly Val Phe Gly Pro Tyr Ala Ser Ala Ala
            210                 215                 220

Ala Ala Ala Gly Pro Gly Val Phe Gly Pro Tyr Gly Pro Gly Ser Ser
225                 230                 235                 240

Ala Ala Ala Ala Ala Gly Ile Tyr Gly Tyr Gly Pro Gly Val Phe Gly
                245                 250                 255

Pro Tyr Gly Pro Gly Ala Ser Gly Ile Asn Gly Pro Gly Ser Gly Ile
        260                 265                 270

Tyr Gly Pro Gly Val Phe Gly Pro Gly Ile Ser Ala Ala Ala Ala Ala
        275                 280                 285

Gly Pro Gly Val Phe Gly Pro Tyr Gly Pro Gly Ala Ser Ala Ala Ala
    290                 295                 300

Ala Ala Gly Ile Tyr Gly Pro Gly Val Phe Gly Pro Gly Ile Tyr Gly
305                 310                 315                 320

Pro Gly Ser Ser Gly Pro Gly Val Phe Gly Pro Tyr Gly Pro Gly Ser
            325                 330                 335

Ser Ala Ala Ala Ala Ala Gly Ile Tyr Gly Pro Gly Val Phe Gly Pro
        340                 345                 350

Tyr Gly Pro Gly Ile Ser Ala Ala Ala Ala Ala Gly Ile Tyr Val Phe
        355                 360                 365

Gly Pro Gly Val Phe Gly Pro Tyr Gly Pro Gly Ala Ser Gly Pro Gly
    370                 375                 380

Val Phe Gly Pro Tyr Gly Pro Gly Ala Ser Ala Ala Ala Ala Ala Gly
385                 390                 395                 400

Pro Gly Ile Tyr Gly Pro Gly Val Phe Gly Pro Ser Ala Ser Ala Ala
            405                 410                 415

Ala Ala Ala Gly Ile Tyr Gly Ser Gly Pro Gly Ile Tyr Gly Pro Tyr
        420                 425                 430

Gly Pro Gly Ile Ser Gly Pro Gly Ser Gly Val Phe Gly Ile Gly Pro
        435                 440                 445

Tyr Gly Pro Gly Ala Ser Ala Ala Ala Ala Gly Ile Tyr Gly Pro
    450                 455                 460

Gly Val Phe Gly Pro Tyr Gly Pro Gly Ile Ser Ala Ala Ala Ala Ala
465                 470                 475                 480

Gly Pro Gly Ser Gly Ile Tyr Gly Pro Gly Ala Ser Gly Ile Asn Gly

485 490 495

Pro Gly Ser Gly Ile Tyr Gly Pro Gly Val Phe Gly Pro Gly Ile Ser
500 505 510

Ala Ala Ala Ala Ala Gly Ile Tyr Val Phe Gly Pro Gly Val Phe Gly
515 520 525

Pro Tyr Gly Pro Gly Ala Ser Ala Ala Ala Ala Gly Ile Tyr Gly
530 535 540

Ser Gly Pro Gly Val Phe Gly Pro Tyr Gly Pro Gly Ile Ser Gly Ser
545 550 555 560

Gly Val Phe Gly Pro Gly Val Phe Gly Pro Tyr Ala Ser Ala Ala Ala
565 570 575

Ala Ala Gly Pro Gly Ser Gly Val Phe Gly Pro Gly Ala Ser Gly Pro
580 585 590

Gly Val Phe Gly Pro Tyr Gly Pro Gly Ala Ser Ala Ala Ala Ala Ala
595 600 605

Gly Ile Asn Gly Pro Gly Ser Gly Val Phe Gly Pro Gly Ile Ser Gly
610 615 620

Ile Tyr Gly Pro Gly Val Phe Gly Pro Gly Val Phe Gly Pro Gly Ser
625 630 635 640

Ser Ala Ala Ala Ala Ala Gly Pro Gly Ile Tyr Gly Pro Gly Val Phe
645 650 655

Gly Pro Ser Ala Ser Ala Ala Ala Ala Gly Pro Gly Ser Gly Val
660 665 670

Phe Gly Pro Gly Ala Ser Gly Ile Tyr Gly Pro Gly Val Phe Gly Pro
675 680 685

Gly Val Phe Gly Pro Gly Ser Ser Ala Ala Ala Ala Gly Ile Tyr
690 695 700

Gly Ser Gly Pro Gly Val Phe Gly Pro Tyr Gly Ser Ala Ala Ala Ala
705 710 715 720

Ala Gly Pro Gly Ser Gly Ile Tyr Gly Ile Gly Pro Tyr Gly Pro Gly
725 730 735

```
Ala Ser Gly Pro Gly Ile Tyr Gly Pro Gly Val Phe Gly Pro Ser Ala
        740             745             750

Ser Ala Ala Ala Ala Ala Gly Ser Gly Val Phe Gly Pro Gly Ile Tyr
        755             760             765

Gly Pro Tyr Ala Ser Ala Ala Ala Ala Ala Gly Ile Tyr Gly Ser Gly
        770             775             780

Pro Gly Val Phe Gly Pro Tyr Gly Pro Gly Ile Ser Gly Ser Gly Val
785             790             795             800

Phe Gly Pro Gly Val Phe Gly Pro Tyr Ala Ser Ala Ala Ala Ala Ala
                805             810             815

Gly Pro Gly Val Phe Gly Pro Tyr Gly Pro Gly Ser Ser Ala Ala Ala
            820             825             830

Ala Ala Gly Ile Tyr Gly Tyr Gly Pro Gly Val Phe Gly Pro Tyr Gly
        835             840             845

Pro Gly Ala Ser Gly Ile Asn Gly Pro Gly Ser Gly Ile Tyr Gly Pro
    850             855             860

Gly Val Phe Gly Pro Gly Ile Ser Ala Ala Ala Ala Gly Pro Gly
865             870             875             880

Val Phe Gly Pro Tyr Gly Pro Gly Ala Ser Ala Ala Ala Ala Ala Gly
            885             890             895

Ile Tyr Gly Pro Gly Val Phe Gly Pro Gly Ile Tyr Gly Pro Gly Ser
        900             905             910

Ser Gly Pro Gly Val Phe Gly Pro Tyr Gly Pro Gly Ser Ser Ala Ala
        915             920             925

Ala Ala Ala Gly Ile Tyr Gly Pro Gly Val Phe Gly Pro Tyr Gly Pro
    930             935             940

Gly Ile Ser Ala Ala Ala Ala Gly Ile Tyr Val Phe Gly Pro Gly
945             950             955             960

Val Phe Gly Pro Tyr Gly Pro Gly Ala Ser Gly Pro Gly Val Phe Gly
            965             970             975

Pro Tyr Gly Pro Gly Ala Ser Ala Ala Ala Ala Ala Gly Pro Gly Ile
        980             985             990
```

```
Tyr Gly Pro Gly Val Phe Gly Pro  Ser Ala Ser Ala Ala  Ala Ala Ala
        995              1000              1005


Gly Ile  Tyr Gly Ser Gly Pro  Gly Ile Tyr Gly Pro  Tyr Gly Pro
    1010              1015              1020


Gly Ile  Ser Gly Pro Gly Ser  Gly Val Phe Gly Ile  Gly Pro Tyr
    1025              1030              1035


Gly Pro  Gly Ala Ser Ala Ala  Ala Ala Ala Gly Ile  Tyr Gly Pro
    1040              1045              1050


Gly Val  Phe Gly Pro Tyr Gly  Pro Gly Ile Ser Ala  Ala Ala Ala
    1055              1060              1065


Ala Gly  Pro Gly Ser Gly Ile  Tyr Gly Pro Gly Ala  Ser Gly Ile
    1070              1075              1080


Asn Gly  Pro Gly Ser Gly Ile  Tyr Gly Pro Gly Val  Phe Gly Pro
    1085              1090              1095


Gly Ile  Ser Ala Ala Ala Ala  Ala Gly Ile Tyr Val  Phe Gly Pro
    1100              1105              1110


Gly Val  Phe Gly Pro Tyr Gly  Pro Gly Ala Ser Ala  Ala Ala Ala
    1115              1120              1125


Ala Gly  Ile Tyr Gly Ser Gly  Pro Gly Val Phe Gly  Pro Tyr Gly
    1130              1135              1140


Pro Gly  Ile Ser Gly Ser Gly  Val Phe Gly Pro Gly  Val Phe Gly
    1145              1150              1155


Pro Tyr  Ala Ser Ala Ala Ala  Ala Ala Gly Pro Gly  Ser Gly Val
    1160              1165              1170


Phe Gly  Pro Gly Ala Ser
    1175


<210>  33
<211>  601
<212>  PRT
<213>  Artificial Sequence

<220>
<223>  PRT888

<400>  33
```

```
Met His His His His His His Ser Ser Gly Ser Ser Gly Pro Gly Val
1                   5                   10                  15

Leu Gly Pro Tyr Gly Pro Gly Ala Ser Ala Ala Ala Ala Ala Gly Gln
            20                  25                  30

Asn Gly Pro Gly Ser Gly Val Leu Gly Pro Gly Gln Ser Gly Gln Tyr
        35                  40                  45

Gly Pro Gly Val Leu Gly Pro Gly Val Leu Gly Pro Gly Ser Ser Ala
    50                  55                  60

Ala Ala Ala Ala Gly Pro Gly Gln Tyr Gly Pro Gly Val Leu Gly Pro
65                  70                  75                  80

Ser Ala Ser Ala Ala Ala Ala Ala Gly Pro Gly Ser Gly Val Leu Gly
            85                  90                  95

Pro Gly Ala Ser Gly Gln Tyr Gly Pro Gly Val Leu Gly Pro Gly Val
        100                 105                 110

Leu Gly Pro Gly Ser Ser Ala Ala Ala Ala Ala Gly Gln Tyr Gly Ser
        115                 120                 125

Gly Pro Gly Val Leu Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Val Leu Gly Pro Ser Ala Ser Ala
            165                 170                 175

Ala Ala Ala Ala Gly Ser Gly Val Leu Gly Pro Gly Gln Tyr Gly Pro
        180                 185                 190

Tyr Ala Ser Ala Ala Ala Ala Ala Gly Gln Tyr Gly Ser Gly Pro Gly
        195                 200                 205

Val Leu Gly Pro Tyr Gly Pro Gly Gln Ser Gly Ser Gly Val Leu Gly
        210                 215                 220

Pro Gly Val Leu Gly Pro Tyr Ala Ser Ala Ala Ala Ala Ala Gly Pro
225                 230                 235                 240

Gly Val Leu Gly Pro Tyr Gly Pro Gly Ser Ser Ala Ala Ala Ala Ala
            245                 250                 255
```

```
Gly Gln Tyr Gly Tyr Gly Pro Gly Val Leu Gly Pro Tyr Gly Pro Gly
            260             265             270

Ala Ser Gly Gln Asn Gly Pro Gly Ser Gly Gln Tyr Gly Pro Gly Val
            275             280             285

Leu Gly Pro Gly Gln Ser Ala Ala Ala Ala Ala Gly Pro Gly Val Leu
            290             295             300

Gly Pro Tyr Gly Pro Gly Ala Ser Ala Ala Ala Ala Ala Gly Gln Tyr
305             310             315             320

Gly Pro Gly Val Leu Gly Pro Gly Gln Tyr Gly Pro Gly Ser Ser Gly
            325             330             335

Pro Gly Val Leu Gly Pro Tyr Gly Pro Gly Ser Ser Ala Ala Ala Ala
            340             345             350

Ala Gly Gln Tyr Gly Pro Gly Val Leu Gly Pro Tyr Gly Pro Gly Gln
            355             360             365

Ser Ala Ala Ala Ala Ala Gly Gln Tyr Val Leu Gly Pro Gly Val Leu
370             375             380

Gly Pro Tyr Gly Pro Gly Ala Ser Gly Pro Gly Val Leu Gly Pro Tyr
385             390             395             400

Gly Pro Gly Ala Ser Ala Ala Ala Ala Ala Gly Pro Gly Gln Tyr Gly
            405             410             415

Pro Gly Val Leu Gly Pro Ser Ala Ser Ala Ala Ala Ala Gly Gln
            420             425             430

Tyr Gly Ser Gly Pro Gly Gln Tyr Gly Pro Tyr Gly Pro Gly Gln Ser
            435             440             445

Gly Pro Gly Ser Gly Val Leu Gly Gln Gly Pro Tyr Gly Pro Gly Ala
            450             455             460

Ser Ala Ala Ala Ala Gly Gln Tyr Gly Pro Gly Val Leu Gly Pro
465             470             475             480

Tyr Gly Pro Gly Gln Ser Ala Ala Ala Ala Gly Pro Gly Ser Gly
            485             490             495

Gln Tyr Gly Pro Gly Ala Ser Gly Gln Asn Gly Pro Gly Ser Gly Gln
            500             505             510
```

138

```
Tyr Gly Pro Gly Val Leu Gly Pro Gly Gln Ser Ala Ala Ala Ala Ala
        515             520         525

Gly Gln Tyr Val Leu Gly Pro Gly Val Leu Gly Pro Tyr Gly Pro Gly
    530             535             540

Ala Ser Ala Ala Ala Ala Ala Gly Gln Tyr Gly Ser Gly Pro Gly Val
545             550             555             560

Leu Gly Pro Tyr Gly Pro Gly Gln Ser Gly Ser Gly Val Leu Gly Pro
            565             570             575

Gly Val Leu Gly Pro Tyr Ala Ser Ala Ala Ala Ala Ala Gly Pro Gly
        580             585             590

Ser Gly Val Leu Gly Pro Gly Ala Ser
        595             600


<210>  34
<211>  601
<212>  PRT
<213>  Artificial Sequence

<220>
<223>  PRT965

<400>  34

Met His His His His His His Ser Ser Gly Ser Ser Gly Pro Gly Thr
1               5               10              15

Ser Gly Pro Tyr Gly Pro Gly Ala Ser Ala Ala Ala Ala Ala Gly Ala
            20              25              30

Asn Gly Pro Gly Ser Gly Thr Ser Gly Pro Gly Ala Ser Gly Ala Tyr
        35              40              45

Gly Pro Gly Thr Ser Gly Pro Gly Thr Ser Gly Pro Gly Ser Ser Ala
    50              55              60

Ala Ala Ala Ala Gly Pro Gly Ala Tyr Gly Pro Gly Thr Ser Gly Pro
65              70              75              80

Ser Ala Ser Ala Ala Ala Ala Ala Gly Pro Gly Ser Gly Thr Ser Gly
            85              90              95

Pro Gly Ala Ser Gly Ala Tyr Gly Pro Gly Thr Ser Gly Pro Gly Thr
        100             105             110
```

139

Ser Gly Pro Gly Ser Ser Ala Ala Ala Ala Ala Gly Ala Tyr Gly Ser
        115                 120                 125

Gly Pro Gly Thr Ser Gly Pro Tyr Gly Ser Ala Ala Ala Ala Ala Gly
        130                 135                 140

Pro Gly Ser Gly Ala Tyr Gly Ala Gly Pro Tyr Gly Pro Gly Ala Ser
145                 150                 155                 160

Gly Pro Gly Ala Tyr Gly Pro Gly Thr Ser Gly Pro Ser Ala Ser Ala
                165                 170                 175

Ala Ala Ala Ala Gly Ser Gly Thr Ser Gly Pro Gly Ala Tyr Gly Pro
        180                 185                 190

Tyr Ala Ser Ala Ala Ala Ala Ala Gly Ala Tyr Gly Ser Gly Pro Gly
        195                 200                 205

Thr Ser Gly Pro Tyr Gly Pro Gly Ala Ser Gly Ser Gly Thr Ser Gly
        210                 215                 220

Pro Gly Thr Ser Gly Pro Tyr Ala Ser Ala Ala Ala Ala Ala Gly Pro
225                 230                 235                 240

Gly Thr Ser Gly Pro Tyr Gly Pro Gly Ser Ser Ala Ala Ala Ala Ala
                245                 250                 255

Gly Ala Tyr Gly Tyr Gly Pro Gly Thr Ser Gly Pro Tyr Gly Pro Gly
                260                 265                 270

Ala Ser Gly Ala Asn Gly Pro Gly Ser Gly Ala Tyr Gly Pro Gly Thr
        275                 280                 285

Ser Gly Pro Gly Ala Ser Ala Ala Ala Ala Gly Pro Gly Thr Ser
        290                 295                 300

Gly Pro Tyr Gly Pro Gly Ala Ser Ala Ala Ala Ala Gly Ala Tyr
305                 310                 315                 320

Gly Pro Gly Thr Ser Gly Pro Gly Ala Tyr Gly Pro Gly Ser Ser Gly
                325                 330                 335

Pro Gly Thr Ser Gly Pro Tyr Gly Pro Gly Ser Ser Ala Ala Ala Ala
        340                 345                 350

Ala Gly Ala Tyr Gly Pro Gly Thr Ser Gly Pro Tyr Gly Pro Gly Ala
        355                 360                 365

```
Ser Ala Ala Ala Ala Ala Gly Ala Tyr Thr Ser Gly Pro Gly Thr Ser
    370                 375             380

Gly Pro Tyr Gly Pro Gly Ala Ser Gly Pro Gly Thr Ser Gly Pro Tyr
    385                 390             395                 400

Gly Pro Gly Ala Ser Ala Ala Ala Ala Ala Gly Pro Gly Ala Tyr Gly
                405                 410                 415

Pro Gly Thr Ser Gly Pro Ser Ala Ser Ala Ala Ala Ala Ala Gly Ala
            420                 425                 430

Tyr Gly Ser Gly Pro Gly Ala Tyr Gly Pro Tyr Gly Pro Gly Ala Ser
            435                 440             445

Gly Pro Gly Ser Gly Thr Ser Gly Ala Gly Pro Tyr Gly Pro Gly Ala
    450                 455                 460

Ser Ala Ala Ala Ala Ala Gly Ala Tyr Gly Pro Gly Thr Ser Gly Pro
465                 470                 475                 480

Tyr Gly Pro Gly Ala Ser Ala Ala Ala Ala Ala Gly Pro Gly Ser Gly
                485                 490                 495

Ala Tyr Gly Pro Gly Ala Ser Gly Ala Asn Gly Pro Gly Ser Gly Ala
        500                 505                 510

Tyr Gly Pro Gly Thr Ser Gly Pro Gly Ala Ser Ala Ala Ala Ala Ala
        515                 520                 525

Gly Ala Tyr Thr Ser Gly Pro Gly Thr Ser Gly Pro Tyr Gly Pro Gly
    530                 535                 540

Ala Ser Ala Ala Ala Ala Ala Gly Ala Tyr Gly Ser Gly Pro Gly Thr
545                 550                 555                 560

Ser Gly Pro Tyr Gly Pro Gly Ala Ser Gly Ser Gly Thr Ser Gly Pro
                565                 570                 575

Gly Thr Ser Gly Pro Tyr Ala Ser Ala Ala Ala Ala Ala Gly Pro Gly
            580                 585                 590

Ser Gly Thr Ser Gly Pro Gly Ala Ser
            595                 600
```

<210> 35

141

<211> 601
<212> PRT
<213> Artificial Sequence

<220>
<223> PRT889

<400> 35

Met His His His His His His Ser Ser Gly Ser Ser Gly Pro Gly Val
1               5                   10                  15

Leu Gly Pro Tyr Gly Pro Gly Ala Ser Ala Ala Ala Ala Ala Gly Ile
            20                  25                  30

Asn Gly Pro Gly Ser Gly Val Leu Gly Pro Gly Ile Ser Gly Ile Tyr
            35                  40                  45

Gly Pro Gly Val Leu Gly Pro Gly Val Leu Gly Pro Gly Ser Ser Ala
        50                  55                  60

Ala Ala Ala Ala Gly Pro Gly Ile Tyr Gly Pro Gly Val Leu Gly Pro
65                  70                  75                  80

Ser Ala Ser Ala Ala Ala Ala Ala Gly Pro Gly Ser Gly Val Leu Gly
                85                  90                  95

Pro Gly Ala Ser Gly Ile Tyr Gly Pro Gly Val Leu Gly Pro Gly Val
            100                 105                 110

Leu Gly Pro Gly Ser Ser Ala Ala Ala Ala Ala Gly Ile Tyr Gly Ser
            115                 120                 125

Gly Pro Gly Val Leu Gly Pro Tyr Gly Ser Ala Ala Ala Ala Ala Gly
        130                 135                 140

Pro Gly Ser Gly Ile Tyr Gly Ile Gly Pro Tyr Gly Pro Gly Ala Ser
145                 150                 155                 160

Gly Pro Gly Ile Tyr Gly Pro Gly Val Leu Gly Pro Ser Ala Ser Ala
                165                 170                 175

Ala Ala Ala Ala Gly Ser Gly Val Leu Gly Pro Gly Ile Tyr Gly Pro
                180                 185                 190

Tyr Ala Ser Ala Ala Ala Ala Ala Gly Ile Tyr Gly Ser Gly Pro Gly
        195                 200                 205

Val Leu Gly Pro Tyr Gly Pro Gly Ile Ser Gly Ser Gly Val Leu Gly
    210                 215                 220

Pro Gly Val Leu Gly Pro Tyr Ala Ser Ala Ala Ala Ala Ala Gly Pro
225                 230             235                 240

Gly Val Leu Gly Pro Tyr Gly Pro Gly Ser Ser Ala Ala Ala Ala Ala
                245             250                 255

Gly Ile Tyr Gly Tyr Gly Pro Gly Val Leu Gly Pro Tyr Gly Pro Gly
            260             265                 270

Ala Ser Gly Ile Asn Gly Pro Gly Ser Gly Ile Tyr Gly Pro Gly Val
            275             280                 285

Leu Gly Pro Gly Ile Ser Ala Ala Ala Ala Gly Pro Gly Val Leu
    290             295                 300

Gly Pro Tyr Gly Pro Gly Ala Ser Ala Ala Ala Ala Gly Ile Tyr
305             310                 315                 320

Gly Pro Gly Val Leu Gly Pro Gly Ile Tyr Gly Pro Gly Ser Ser Gly
                325                 330                 335

Pro Gly Val Leu Gly Pro Tyr Gly Pro Gly Ser Ser Ala Ala Ala Ala
            340             345                 350

Ala Gly Ile Tyr Gly Pro Gly Val Leu Gly Pro Tyr Gly Pro Gly Ile
        355             360                 365

Ser Ala Ala Ala Ala Ala Gly Ile Tyr Val Leu Gly Pro Gly Val Leu
    370                 375                 380

Gly Pro Tyr Gly Pro Gly Ala Ser Gly Pro Gly Val Leu Gly Pro Tyr
385             390                 395                 400

Gly Pro Gly Ala Ser Ala Ala Ala Ala Ala Gly Pro Gly Ile Tyr Gly
            405                 410                 415

Pro Gly Val Leu Gly Pro Ser Ala Ser Ala Ala Ala Ala Ala Gly Ile
            420                 425                 430

Tyr Gly Ser Gly Pro Gly Ile Tyr Gly Pro Tyr Gly Pro Gly Ile Ser
        435                 440                 445

Gly Pro Gly Ser Gly Val Leu Gly Ile Gly Pro Tyr Gly Pro Gly Ala
    450                 455                 460

Ser Ala Ala Ala Ala Ala Gly Ile Tyr Gly Pro Gly Val Leu Gly Pro

143

465    470    475    480

```
Tyr Gly Pro Gly Ile Ser Ala Ala Ala Ala Gly Pro Gly Ser Gly
                485             490             495

Ile Tyr Gly Pro Gly Ala Ser Gly Ile Asn Gly Pro Gly Ser Gly Ile
        500             505             510

Tyr Gly Pro Gly Val Leu Gly Pro Gly Ile Ser Ala Ala Ala Ala Ala
        515             520             525

Gly Ile Tyr Val Leu Gly Pro Gly Val Leu Gly Pro Tyr Gly Pro Gly
        530             535             540

Ala Ser Ala Ala Ala Ala Ala Gly Ile Tyr Gly Ser Gly Pro Gly Val
545             550             555             560

Leu Gly Pro Tyr Gly Pro Gly Ile Ser Gly Ser Gly Val Leu Gly Pro
            565             570             575

Gly Val Leu Gly Pro Tyr Ala Ser Ala Ala Ala Ala Ala Gly Pro Gly
            580             585             590

Ser Gly Val Leu Gly Pro Gly Ala Ser
            595             600
```

```
<210>  36
<211>  601
<212>  PRT
<213>  Artificial Sequence

<220>
<223>  PRT916

<400>  36
```

```
Met His His His His His His Ser Ser Gly Ser Ser Gly Pro Gly Val
1               5               10              15

Ile Gly Pro Tyr Gly Pro Gly Ala Ser Ala Ala Ala Ala Ala Gly Leu
            20              25              30

Asn Gly Pro Gly Ser Gly Val Ile Gly Pro Gly Leu Ser Gly Leu Tyr
        35              40              45

Gly Pro Gly Val Ile Gly Pro Gly Val Ile Gly Pro Gly Ser Ser Ala
        50              55              60

Ala Ala Ala Ala Gly Pro Gly Leu Tyr Gly Pro Gly Val Ile Gly Pro
65              70              75              80
```

Ser Ala Ser Ala Ala Ala Ala Ala Gly Pro Gly Ser Gly Val Ile Gly
                85                    90                    95

Pro Gly Ala Ser Gly Leu Tyr Gly Pro Gly Val Ile Gly Pro Gly Val
            100                    105                   110

Ile Gly Pro Gly Ser Ser Ala Ala Ala Ala Gly Leu Tyr Gly Ser
        115                    120                   125

Gly Pro Gly Val Ile Gly Pro Tyr Gly Ser Ala Ala Ala Ala Gly
    130                    135                   140

Pro Gly Ser Gly Leu Tyr Gly Leu Gly Pro Tyr Gly Pro Gly Ala Ser
145                    150                   155                   160

Gly Pro Gly Leu Tyr Gly Pro Gly Val Ile Gly Pro Ser Ala Ser Ala
                165                    170                   175

Ala Ala Ala Ala Gly Ser Gly Val Ile Gly Pro Gly Leu Tyr Gly Pro
            180                    185                   190

Tyr Ala Ser Ala Ala Ala Ala Ala Gly Leu Tyr Gly Ser Gly Pro Gly
        195                    200                   205

Val Ile Gly Pro Tyr Gly Pro Gly Leu Ser Gly Ser Gly Val Ile Gly
    210                    215                   220

Pro Gly Val Ile Gly Pro Tyr Ala Ser Ala Ala Ala Ala Ala Gly Pro
225                    230                   235                   240

Gly Val Ile Gly Pro Tyr Gly Pro Gly Ser Ser Ala Ala Ala Ala Ala
                245                    250                   255

Gly Leu Tyr Gly Tyr Gly Pro Gly Val Ile Gly Pro Tyr Gly Pro Gly
            260                    265                   270

Ala Ser Gly Leu Asn Gly Pro Gly Ser Gly Leu Tyr Gly Pro Gly Val
        275                    280                   285

Ile Gly Pro Gly Leu Ser Ala Ala Ala Ala Gly Pro Gly Val Ile
    290                    295                   300

Gly Pro Tyr Gly Pro Gly Ala Ser Ala Ala Ala Ala Gly Leu Tyr
305                    310                   315                   320

Gly Pro Gly Val Ile Gly Pro Gly Leu Tyr Gly Pro Gly Ser Ser Gly

```
                    325                    330                    335

Pro Gly Val Ile Gly Pro Tyr Gly Pro Gly Ser Ser Ala Ala Ala Ala
            340                    345                    350

Ala Gly Leu Tyr Gly Pro Gly Val Ile Gly Pro Tyr Gly Pro Gly Leu
            355                    360                    365

Ser Ala Ala Ala Ala Ala Gly Leu Tyr Val Ile Gly Pro Gly Val Ile
            370                    375                    380

Gly Pro Tyr Gly Pro Gly Ala Ser Gly Pro Gly Val Ile Gly Pro Tyr
385                    390                    395                    400

Gly Pro Gly Ala Ser Ala Ala Ala Ala Gly Pro Gly Leu Tyr Gly
                405                    410                    415

Pro Gly Val Ile Gly Pro Ser Ala Ser Ala Ala Ala Ala Gly Leu
            420                    425                    430

Tyr Gly Ser Gly Pro Gly Leu Tyr Gly Pro Tyr Gly Pro Gly Leu Ser
            435                    440                    445

Gly Pro Gly Ser Gly Val Ile Gly Leu Gly Pro Tyr Gly Pro Gly Ala
        450                    455                    460

Ser Ala Ala Ala Ala Ala Gly Leu Tyr Gly Pro Gly Val Ile Gly Pro
465                    470                    475                    480

Tyr Gly Pro Gly Leu Ser Ala Ala Ala Ala Gly Pro Gly Ser Gly
            485                    490                    495

Leu Tyr Gly Pro Gly Ala Ser Gly Leu Asn Gly Pro Gly Ser Gly Leu
        500                    505                    510

Tyr Gly Pro Gly Val Ile Gly Pro Gly Leu Ser Ala Ala Ala Ala Ala
        515                    520                    525

Gly Leu Tyr Val Ile Gly Pro Gly Val Ile Gly Pro Tyr Gly Pro Gly
    530                    535                    540

Ala Ser Ala Ala Ala Ala Ala Gly Leu Tyr Gly Ser Gly Pro Gly Val
545                    550                    555                    560

Ile Gly Pro Tyr Gly Pro Gly Leu Ser Gly Ser Gly Val Ile Gly Pro
            565                    570                    575
```

```
Gly Val Ile Gly Pro Tyr Ala Ser Ala Ala Ala Ala Gly Pro Gly
            580                 585             590


Ser Gly Val Ile Gly Pro Gly Ala Ser
            595                 600


<210>  37
<211>  601
<212>  PRT
<213>  Artificial Sequence

<220>
<223>  PRT918

<400>  37

Met His His His His His His Ser Ser Gly Ser Ser Gly Pro Gly Val
1               5                   10                  15


Phe Gly Pro Tyr Gly Pro Gly Ala Ser Ala Ala Ala Ala Ala Gly Ile
            20                  25                  30


Asn Gly Pro Gly Ser Gly Val Phe Gly Pro Gly Ile Ser Gly Ile Tyr
            35                  40                  45


Gly Pro Gly Val Phe Gly Pro Gly Val Phe Gly Pro Gly Ser Ser Ala
    50                  55                  60


Ala Ala Ala Ala Gly Pro Gly Ile Tyr Gly Pro Gly Val Phe Gly Pro
65                  70                  75                  80


Ser Ala Ser Ala Ala Ala Ala Ala Gly Pro Gly Ser Gly Val Phe Gly
                85                  90                  95


Pro Gly Ala Ser Gly Ile Tyr Gly Pro Gly Val Phe Gly Pro Gly Val
            100                 105                 110


Phe Gly Pro Gly Ser Ser Ala Ala Ala Ala Ala Gly Ile Tyr Gly Ser
            115                 120                 125


Gly Pro Gly Val Phe Gly Pro Tyr Gly Ser Ala Ala Ala Ala Ala Gly
    130                 135                 140


Pro Gly Ser Gly Ile Tyr Gly Ile Gly Pro Tyr Gly Pro Gly Ala Ser
145                 150                 155                 160


Gly Pro Gly Ile Tyr Gly Pro Gly Val Phe Gly Pro Ser Ala Ser Ala
                165                 170                 175


Ala Ala Ala Ala Gly Ser Gly Val Phe Gly Pro Gly Ile Tyr Gly Pro
```

180                        185                        190

Tyr Ala Ser Ala Ala Ala Ala Ala Gly Ile Tyr Gly Ser Gly Pro Gly
        195                 200              205

Val Phe Gly Pro Tyr Gly Pro Gly Ile Ser Gly Ser Gly Val Phe Gly
        210              215              220

Pro Gly Val Phe Gly Pro Tyr Ala Ser Ala Ala Ala Ala Gly Pro
225              230              235              240

Gly Val Phe Gly Pro Tyr Gly Pro Gly Ser Ser Ala Ala Ala Ala
            245              250              255

Gly Ile Tyr Gly Tyr Gly Pro Gly Val Phe Gly Pro Tyr Gly Pro Gly
        260              265              270

Ala Ser Gly Ile Asn Gly Pro Gly Ser Gly Ile Tyr Gly Pro Gly Val
        275              280              285

Phe Gly Pro Gly Ile Ser Ala Ala Ala Ala Gly Pro Gly Val Phe
    290              295              300

Gly Pro Tyr Gly Pro Gly Ala Ser Ala Ala Ala Ala Gly Ile Tyr
305              310              315              320

Gly Pro Gly Val Phe Gly Pro Gly Ile Tyr Gly Pro Gly Ser Ser Gly
            325              330              335

Pro Gly Val Phe Gly Pro Tyr Gly Pro Gly Ser Ser Ala Ala Ala Ala
        340              345              350

Ala Gly Ile Tyr Gly Pro Gly Val Phe Gly Pro Tyr Gly Pro Gly Ile
        355              360              365

Ser Ala Ala Ala Ala Ala Gly Ile Tyr Val Phe Gly Pro Gly Val Phe
    370              375              380

Gly Pro Tyr Gly Pro Gly Ala Ser Gly Pro Gly Val Phe Gly Pro Tyr
385              390              395              400

Gly Pro Gly Ala Ser Ala Ala Ala Ala Gly Pro Gly Ile Tyr Gly
            405              410              415

Pro Gly Val Phe Gly Pro Ser Ala Ser Ala Ala Ala Ala Ala Gly Ile
        420              425              430

```
Tyr Gly Ser Gly Pro Gly Ile Tyr Gly Pro Tyr Gly Pro Gly Ile Ser
        435                 440                 445

Gly Pro Gly Ser Gly Val Phe Gly Ile Gly Pro Tyr Gly Pro Gly Ala
        450                 455                 460

Ser Ala Ala Ala Ala Ala Gly Ile Tyr Gly Pro Gly Val Phe Gly Pro
465                 470                 475                 480

Tyr Gly Pro Gly Ile Ser Ala Ala Ala Ala Gly Pro Gly Ser Gly
                485                 490                 495

Ile Tyr Gly Pro Gly Ala Ser Gly Ile Asn Gly Pro Gly Ser Gly Ile
        500                 505                 510

Tyr Gly Pro Gly Val Phe Gly Pro Gly Ile Ser Ala Ala Ala Ala Ala
        515                 520                 525

Gly Ile Tyr Val Phe Gly Pro Gly Val Phe Gly Pro Tyr Gly Pro Gly
        530                 535                 540

Ala Ser Ala Ala Ala Ala Ala Gly Ile Tyr Gly Ser Gly Pro Gly Val
545                 550                 555                 560

Phe Gly Pro Tyr Gly Pro Gly Ile Ser Gly Ser Gly Val Phe Gly Pro
                565                 570                 575

Gly Val Phe Gly Pro Tyr Ala Ser Ala Ala Ala Ala Ala Gly Pro Gly
        580                 585                 590

Ser Gly Val Phe Gly Pro Gly Ala Ser
        595                 600
```

```
<210>   38
<211>   576
<212>   PRT
<213>   Artificial Sequence

<220>
<223>   PRT699

<400>   38
```

```
Met His His His His His His Ser Ser Gly Ser Ser Gly Pro Gly Val
1               5                   10                  15

Leu Gly Pro Tyr Gly Pro Gly Ala Ser Ala Ala Ala Ala Ala Ala Ala
            20                  25                  30

Gly Ser Asn Gly Pro Gly Ser Gly Val Leu Gly Pro Gly Gln Ser Gly
```

```
                    35                      40                      45

        Gln Tyr Gly Pro Gly Val Leu Gly Pro Gly Val Leu Gly Pro Gly Ser
            50                  55                  60

        Ser Ala Ala Ala Ala Ala Ala Ala Gly Pro Gly Gln Tyr Gly Pro Gly
        65                  70                  75                  80

        Val Leu Gly Pro Ser Ala Ser Ala Ala Ala Ala Ala Ala Gly Pro
                        85                  90                  95

        Gly Ser Gly Val Leu Gly Pro Gly Ala Ser Gly Gln Tyr Gly Pro Gly
                    100                 105                 110

        Val Leu Gly Pro Gly Val Leu Gly Pro Gly Ser Ser Ala Ala Ala Ala
                    115                 120                 125

        Ala Ala Ala Gly Ser Tyr Gly Ser Gly Pro Gly Val Leu Gly Pro Tyr
            130                 135                 140

        Gly Ser Ala Ala Ala Ala Ala Ala Ala Gly Pro Gly Ser Gly Gln Tyr
        145                 150                 155                 160

        Gly Gln Gly Pro Tyr Gly Pro Gly Ala Ser Gly Pro Gly Gln Tyr Gly
                        165                 170                 175

        Pro Gly Val Leu Gly Pro Ser Ala Ser Ala Ala Ala Ala Ala Ala Ala
                    180                 185                 190

        Gly Ser Gly Val Leu Gly Pro Gly Gln Tyr Gly Pro Tyr Ala Ser Ala
                    195                 200                 205

        Ala Ala Ala Ala Ala Gly Ser Tyr Gly Ser Gly Pro Gly Val Leu
            210                 215                 220

        Gly Pro Tyr Gly Pro Gly Gln Ser Gly Ser Gly Val Leu Gly Pro Gly
        225                 230                 235                 240

        Val Leu Gly Pro Tyr Ala Ser Ala Ala Ala Ala Ala Ala Gly Pro
                        245                 250                 255

        Gly Val Leu Gly Pro Tyr Gly Pro Gly Ser Ser Ala Ala Ala Ala Ala
                    260                 265                 270

        Ala Ala Gly Ser Tyr Gly Tyr Gly Pro Gly Val Leu Gly Pro Tyr Gly
            275                 280                 285
```

```
Pro Gly Ala Ser Gly Gln Asn Gly Pro Gly Ser Gly Gln Tyr Gly Pro
    290                 295             300

Gly Val Leu Gly Pro Gly Pro Ser Ala Ala Ala Ala Ala Ala Ala Gly
    305             310             315                 320

Pro Gly Val Leu Gly Pro Tyr Gly Pro Gly Ala Ser Ala Ala Ala Ala
                325             330                 335

Ala Ala Ala Gly Ser Tyr Gly Pro Gly Val Leu Gly Pro Gly Gln Tyr
            340             345             350

Gly Pro Gly Ser Ser Gly Pro Gly Val Leu Gly Pro Tyr Gly Pro Gly
        355             360             365

Ser Ser Ala Ala Ala Ala Ala Ala Ala Gly Ser Tyr Gly Pro Gly Val
    370             375             380

Leu Gly Pro Tyr Gly Pro Gly Pro Ser Ala Ala Ala Ala Ala Ala Ala
385             390             395                 400

Gly Ser Tyr Val Leu Gly Pro Gly Val Leu Gly Pro Tyr Gly Pro Gly
            405             410             415

Ala Ser Gly Pro Gly Val Leu Gly Pro Tyr Gly Pro Gly Ala Ser Ala
            420             425             430

Ala Ala Ala Ala Ala Gly Pro Gly Gln Tyr Gly Pro Gly Val Leu
            435             440             445

Gly Pro Ser Ala Ser Ala Ala Ala Ala Ala Ala Gly Ser Tyr Gly
    450             455             460

Ser Gly Pro Gly Gln Tyr Gly Pro Tyr Gly Pro Gly Gln Ser Gly Pro
465             470             475                 480

Gly Ser Gly Val Leu Gly Gln Gly Pro Tyr Gly Pro Gly Ala Ser Ala
            485             490             495

Ala Ala Ala Ala Ala Gly Ser Tyr Gly Pro Gly Val Leu Gly Pro
            500             505             510

Tyr Gly Pro Gly Pro Ser Ala Ala Ala Ala Ala Ala Gly Pro Gly
    515             520             525

Ser Gly Gln Tyr Gly Pro Gly Ala Ser Gly Gln Asn Gly Pro Gly Ser
    530             535             540
```

151

```
Gly Gln Tyr Gly Pro Gly Val Leu Gly Pro Gly Pro Ser Ala Ala Ala
545                 550             555                 560


Ala Ala Ala Ala Gly Pro Gly Ser Gly Val Leu Gly Pro Gly Ala Ser
                565             570                 575


<210>  39
<211>  576
<212>  PRT
<213>  Artificial Sequence

<220>
<223>  PRT698

<400>  39

Met His His His His His His Ser Ser Gly Ser Ser Gly Pro Gly Val
1               5                 10                  15


Leu Gly Pro Tyr Gly Pro Gly Ala Ser Ala Ala Ala Ala Ala Ala Ala
                20              25                  30


Gly Ser Asn Gly Pro Gly Ser Gly Val Leu Gly Pro Gly Ile Ser Gly
        35              40                  45


Ile Tyr Gly Pro Gly Val Leu Gly Pro Gly Val Leu Gly Pro Gly Ser
        50              55                  60


Ser Ala Ala Ala Ala Ala Ala Ala Gly Pro Gly Ile Tyr Gly Pro Gly
65                  70                  75                  80


Val Leu Gly Pro Ser Ala Ser Ala Ala Ala Ala Ala Ala Ala Gly Pro
                85                  90                  95


Gly Ser Gly Val Leu Gly Pro Gly Ala Ser Gly Ile Tyr Gly Pro Gly
            100             105                 110


Val Leu Gly Pro Gly Val Leu Gly Pro Gly Ser Ser Ala Ala Ala Ala
            115             120                 125


Ala Ala Ala Gly Ser Tyr Gly Ser Gly Pro Gly Val Leu Gly Pro Tyr
        130             135                 140


Gly Ser Ala Ala Ala Ala Ala Ala Ala Gly Pro Gly Ser Gly Ile Tyr
145                 150                 155                 160


Gly Ile Gly Pro Tyr Gly Pro Gly Ala Ser Gly Pro Gly Ile Tyr Gly
                165             170                 175
```

```
Pro Gly Val Leu Gly Pro Ser Ala Ser Ala Ala Ala Ala Ala Ala
        180                 185                 190

Gly Ser Gly Val Leu Gly Pro Gly Ile Tyr Gly Pro Tyr Ala Ser Ala
        195                 200                 205

Ala Ala Ala Ala Ala Ala Gly Ser Tyr Gly Ser Gly Pro Gly Val Leu
        210                 215                 220

Gly Pro Tyr Gly Pro Gly Ile Ser Gly Ser Gly Val Leu Gly Pro Gly
225                 230                 235                 240

Val Leu Gly Pro Tyr Ala Ser Ala Ala Ala Ala Ala Ala Gly Pro
                245                 250                 255

Gly Val Leu Gly Pro Tyr Gly Pro Gly Ser Ser Ala Ala Ala Ala
        260                 265                 270

Ala Ala Gly Ser Tyr Gly Tyr Gly Pro Gly Val Leu Gly Pro Tyr Gly
        275                 280                 285

Pro Gly Ala Ser Gly Ile Asn Gly Pro Gly Ser Gly Ile Tyr Gly Pro
        290                 295                 300

Gly Val Leu Gly Pro Gly Pro Ser Ala Ala Ala Ala Ala Ala Gly
305                 310                 315                 320

Pro Gly Val Leu Gly Pro Tyr Gly Pro Gly Ala Ser Ala Ala Ala Ala
                325                 330                 335

Ala Ala Ala Gly Ser Tyr Gly Pro Gly Val Leu Gly Pro Gly Ile Tyr
        340                 345                 350

Gly Pro Gly Ser Ser Gly Pro Gly Val Leu Gly Pro Tyr Gly Pro Gly
        355                 360                 365

Ser Ser Ala Ala Ala Ala Ala Ala Gly Ser Tyr Gly Pro Gly Val
370                 375                 380

Leu Gly Pro Tyr Gly Pro Gly Pro Ser Ala Ala Ala Ala Ala Ala
385                 390                 395                 400

Gly Ser Tyr Val Leu Gly Pro Gly Val Leu Gly Pro Tyr Gly Pro Gly
            405                 410                 415

Ala Ser Gly Pro Gly Val Leu Gly Pro Tyr Gly Pro Gly Ala Ser Ala
        420                 425                 430
```

Ala Ala Ala Ala Ala Ala Gly Pro Gly Ile Tyr Gly Pro Gly Val Leu
435 440 445

Gly Pro Ser Ala Ser Ala Ala Ala Ala Ala Ala Gly Ser Tyr Gly
450 455 460

Ser Gly Pro Gly Ile Tyr Gly Pro Tyr Gly Pro Gly Ile Ser Gly Pro
465 470 475 480

Gly Ser Gly Val Leu Gly Ile Gly Pro Tyr Gly Pro Gly Ala Ser Ala
485 490 495

Ala Ala Ala Ala Ala Ala Gly Ser Tyr Gly Pro Gly Val Leu Gly Pro
500 505 510

Tyr Gly Pro Gly Pro Ser Ala Ala Ala Ala Ala Ala Gly Pro Gly
515 520 525

Ser Gly Ile Tyr Gly Pro Gly Ala Ser Gly Ile Asn Gly Pro Gly Ser
530 535 540

Gly Ile Tyr Gly Pro Gly Val Leu Gly Pro Gly Pro Ser Ala Ala Ala
545 550 555 560

Ala Ala Ala Ala Gly Pro Gly Ser Gly Val Leu Gly Pro Gly Ala Ser
565 570 575

<210> 40
<211> 1190
<212> PRT
<213> Artificial Sequence

<220>
<223> PRT966

<400> 40

Met His His His His His His Ser Ser Gly Ser Ser Gly Pro Gly Val
1 5 10 15

Phe Gly Pro Tyr Gly Pro Gly Ala Ser Ala Ala Ala Ala Gly Ile
20 25 30

Asn Gly Pro Gly Ser Gly Val Phe Gly Pro Gly Ile Ser Gly Ile Tyr
35 40 45

Gly Pro Gly Val Phe Gly Pro Gly Val Phe Gly Pro Gly Ser Ser Ala
50 55 60

154

```
Ala Ala Ala Ala Gly Pro Gly Ile Tyr Gly Pro Gly Val Phe Gly Pro
65              70              75              80

Ser Ala Ser Ala Ala Ala Ala Ala Gly Pro Gly Ser Gly Val Phe Gly
            85              90              95

Pro Gly Ala Ser Gly Ile Tyr Gly Pro Gly Val Phe Gly Pro Gly Val
        100             105             110

Phe Gly Pro Gly Ser Ser Ala Ala Ala Ala Gly Ile Tyr Gly Ser
        115             120             125

Gly Pro Gly Val Phe Gly Pro Tyr Gly Ser Ala Ala Ala Ala Gly
    130             135             140

Pro Gly Ser Gly Ile Tyr Gly Ile Gly Pro Tyr Gly Pro Gly Ala Ser
145             150             155             160

Gly Pro Gly Ile Tyr Gly Pro Gly Val Phe Gly Pro Ser Ala Ser Ala
            165             170             175

Ala Ala Ala Ala Gly Ser Gly Val Phe Gly Pro Gly Ile Tyr Gly Pro
        180             185             190

Tyr Ala Ser Ala Ala Ala Ala Gly Ile Tyr Gly Ser Gly Pro Gly
    195             200             205

Val Phe Gly Pro Tyr Gly Pro Gly Ile Ser Gly Ser Gly Val Phe Gly
    210             215             220

Pro Gly Val Phe Gly Pro Tyr Ala Ser Ala Ala Ala Ala Gly Pro
225             230             235             240

Gly Val Phe Gly Pro Tyr Gly Pro Gly Ser Ser Ala Ala Ala Ala
        245             250             255

Gly Ile Tyr Gly Tyr Gly Pro Gly Val Phe Gly Pro Tyr Gly Pro Gly
        260             265             270

Ala Ser Gly Ile Asn Gly Pro Gly Ser Gly Ile Tyr Gly Pro Gly Val
        275             280             285

Phe Gly Pro Gly Ile Ser Ala Ala Ala Ala Gly Pro Gly Val Phe
    290             295             300

Gly Pro Tyr Gly Pro Gly Ala Ser Ala Ala Ala Ala Gly Ile Tyr
305             310             315             320
```

155

Gly Pro Gly Val Phe Gly Pro Gly Ile Tyr Gly Pro Gly Ser Ser Gly
                    325                 330                 335

Pro Gly Val Phe Gly Pro Tyr Gly Pro Gly Ser Ser Ala Ala Ala Ala
                    340                 345                 350

Ala Gly Ile Tyr Gly Pro Gly Val Phe Gly Pro Tyr Gly Pro Gly Ile
        355                 360                 365

Ser Ala Ala Ala Ala Ala Gly Ile Tyr Val Phe Gly Pro Gly Val Phe
        370                 375                 380

Gly Pro Tyr Gly Pro Gly Ala Ser Gly Pro Gly Val Phe Gly Pro Tyr
385                 390                 395                 400

Gly Pro Gly Ala Ser Ala Ala Ala Ala Ala Gly Pro Gly Ile Tyr Gly
                    405                 410                 415

Pro Gly Val Phe Gly Pro Ser Ala Ser Ala Ala Ala Ala Ala Gly Ile
            420                 425                 430

Tyr Gly Ser Gly Pro Gly Ile Tyr Gly Pro Tyr Gly Pro Gly Ile Ser
        435                 440                 445

Gly Pro Gly Ser Gly Val Phe Gly Ile Gly Pro Tyr Gly Pro Gly Ala
    450                 455                 460

Ser Ala Ala Ala Ala Ala Gly Ile Tyr Gly Pro Gly Val Phe Gly Pro
465                 470                 475                 480

Tyr Gly Pro Gly Ile Ser Ala Ala Ala Ala Gly Pro Gly Ser Gly
                485                 490                 495

Ile Tyr Gly Pro Gly Ala Ser Gly Ile Asn Gly Pro Gly Ser Gly Ile
            500                 505                 510

Tyr Gly Pro Gly Val Phe Gly Pro Gly Ile Ser Ala Ala Ala Ala Ala
        515                 520                 525

Gly Ile Tyr Val Phe Gly Pro Gly Val Phe Gly Pro Tyr Gly Pro Gly
    530                 535                 540

Ala Ser Ala Ala Ala Ala Gly Ile Tyr Gly Ser Gly Pro Gly Val
545                 550                 555                 560

Phe Gly Pro Tyr Gly Pro Gly Ile Ser Gly Ser Gly Val Phe Gly Pro
                565                 570                 575

Gly Val Phe Gly Pro Tyr Ala Ser Ala Ala Ala Ala Ala Gly Pro Gly
580 585 590

Ser Gly Val Phe Gly Pro Gly Ala Ser Gly Pro Gly Val Phe Gly Pro
595 600 605

Tyr Gly Pro Gly Ala Ser Ala Ala Ala Ala Ala Gly Ile Asn Gly Pro
610 615 620

Gly Ser Gly Val Phe Gly Pro Gly Ile Ser Gly Ile Tyr Gly Pro Gly
625 630 635 640

Val Phe Gly Pro Gly Val Phe Gly Pro Gly Ser Ser Ala Ala Ala Ala
645 650 655

Ala Gly Pro Gly Ile Tyr Gly Pro Gly Val Phe Gly Pro Ser Ala Ser
660 665 670

Ala Ala Ala Ala Ala Gly Pro Gly Ser Gly Val Phe Gly Pro Gly Ala
675 680 685

Ser Gly Ile Tyr Gly Pro Gly Val Phe Gly Pro Gly Val Phe Gly Pro
690 695 700

Gly Ser Ser Ala Ala Ala Ala Ala Gly Ile Tyr Gly Ser Gly Pro Gly
705 710 715 720

Val Phe Gly Pro Tyr Gly Ser Ala Ala Ala Ala Ala Gly Pro Gly Ser
725 730 735

Gly Ile Tyr Gly Ile Gly Pro Tyr Gly Pro Gly Ala Ser Gly Pro Gly
740 745 750

Ile Tyr Gly Pro Gly Val Phe Gly Pro Ser Ala Ser Ala Ala Ala Ala
755 760 765

Ala Gly Ser Gly Val Phe Gly Pro Gly Ile Tyr Gly Pro Tyr Ala Ser
770 775 780

Ala Ala Ala Ala Ala Gly Ile Tyr Gly Ser Gly Pro Gly Val Phe Gly
785 790 795 800

Pro Tyr Gly Pro Gly Ile Ser Gly Ser Gly Val Phe Gly Pro Gly Val
805 810 815

Phe Gly Pro Tyr Ala Ser Ala Ala Ala Ala Ala Gly Pro Gly Val Phe

```
                820                      825                      830


        Gly Pro Tyr Gly Pro Gly Ser Ser Ala Ala Ala Ala Ala Gly Ile Tyr
                835                 840                 845


        Gly Tyr Gly Pro Gly Val Phe Gly Pro Tyr Gly Pro Gly Ala Ser Gly
                850                 855                 860


        Ile Asn Gly Pro Gly Ser Gly Ile Tyr Gly Pro Gly Val Phe Gly Pro
        865                 870                 875                 880


        Gly Ile Ser Ala Ala Ala Ala Ala Gly Pro Gly Val Phe Gly Pro Tyr
                    885                 890                 895


        Gly Pro Gly Ala Ser Ala Ala Ala Ala Ala Gly Ile Tyr Gly Pro Gly
                    900                 905                 910


        Val Phe Gly Pro Gly Ile Tyr Gly Pro Gly Ser Ser Gly Pro Gly Val
                915                 920                 925


        Phe Gly Pro Tyr Gly Pro Gly Ser Ser Ala Ala Ala Ala Ala Gly Ile
                930                 935                 940


        Tyr Gly Pro Gly Val Phe Gly Pro Tyr Gly Pro Gly Ile Ser Ala Ala
        945                 950                 955                 960


        Ala Ala Ala Gly Ile Tyr Val Phe Gly Pro Gly Val Phe Gly Pro Tyr
                    965                 970                 975


        Gly Pro Gly Ala Ser Gly Pro Gly Val Phe Gly Pro Tyr Gly Pro Gly
                    980                 985                 990


        Ala Ser Ala Ala Ala Ala Ala Gly  Pro Gly Ile Tyr Gly  Pro Gly Val
                    995                 1000                1005


        Phe Gly  Pro Ser Ala Ser Ala  Ala Ala Ala Ala Gly  Ile Tyr Gly
             1010                 1015                1020


        Ser Gly  Pro Gly Ile Tyr Gly  Pro Tyr Gly Pro Gly  Ile Ser Gly
             1025                 1030                1035


        Pro Gly  Ser Gly Val Phe Gly  Ile Gly Pro Tyr Gly  Pro Gly Ala
             1040                 1045                1050


        Ser Ala  Ala Ala Ala Ala Gly  Ile Tyr Gly Pro Gly  Val Phe Gly
             1055                 1060                1065
```

```
Pro Tyr  Gly Pro Gly Ile Ser  Ala Ala Ala Ala Ala  Gly Pro Gly
    1070              1075                  1080


Ser Gly  Ile Tyr Gly Pro Gly  Ala Ser Gly Ile Asn  Gly Pro Gly
    1085              1090                  1095


Ser Gly  Ile Tyr Gly Pro Gly  Val Phe Gly Pro Gly  Ile Ser Ala
    1100              1105                  1110


Ala Ala  Ala Ala Gly Ile Tyr  Val Phe Gly Pro Gly  Val Phe Gly
    1115              1120                  1125


Pro Tyr  Gly Pro Gly Ala Ser  Ala Ala Ala Ala Ala  Gly Ile Tyr
    1130              1135                  1140


Gly Ser  Gly Pro Gly Val Phe  Gly Pro Tyr Gly Pro  Gly Ile Ser
    1145              1150                  1155


Gly Ser  Gly Val Phe Gly Pro  Gly Val Phe Gly Pro  Tyr Ala Ser
    1160              1165                  1170


Ala Ala  Ala Ala Ala Gly Pro  Gly Ser Gly Val Phe  Gly Pro Gly
    1175              1180                  1185


Ala Ser
    1190
```